# EUROPEAN PATENT APPLICATION

(11) **EP 3 848 367 A1**
(43) Date of publication of application: **14.07.2021**
(21) Application number: 19857378.4
(22) Date of filing: 09.09.2019
(51) Int. Cl.: C07D 413/00, C07D 413/04, C07D 413/14, C07D 498/00, C07D 498/02, A61K 31/42, A61P 35/00

(54) **TRICYCLIC COMPOUNDS ACTING ON CRBN PROTEINS**

(30) Priority: 07.09.2018 CN 201811045941; 14.11.2018 CN 201811354986; 22.03.2019 CN 201910222597
(71) Applicant: Chia Tai Tianqing Pharmaceutical Group Co., Ltd., Lianyungang, Jiangsu 222062 (CN); Medshine Discovery Inc., Nanjing, Jiangsu 210032 (CN)
(72) Inventor: LEI, Maoyi, Shanghai 200131 (CN); LUO, Yunfu, Shanghai 200131 (CN); XU, Yu, Shanghai 200131 (CN); ZHANG, Guoli, Shanghai 200131 (CN); YU, Zhijuan, Shanghai 200131 (CN); LI, Jian, Shanghai 200131 (CN); CHEN, Shuhui, Shanghai 200131 (CN)
(74) Representative: Grünecker Patent- und Rechtsanwälte PartG mbB
(86) International application number: PCT/CN2019/104996
(87) International publication number: WO 2020/048548

(57) **Abstract**

The present invention discloses a series of tricyclic compounds and use thereof in preparing a medicament for treating a disease related to CRBN protein. Specifically, the present invention discloses a derivative compound of formula (1) or a pharmaceutically acceptable salt thereof.

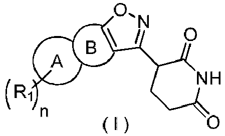

## Description

### CROSS-REFERENCE TO RELATED APPLICATIONS

The present application claims the benefits and priority to the Chinese Patent Application No. 201811045941.1 filed with the National Intellectual Property Administration, PRC on September 07, 2018, the Chinese Patent Application No. 201811354986.7 filed with the National Intellectual Property Administration, PRC on November 14, 2018, and the Chinese Patent Application No. 201910222597.7 filed with the National Intellectual Property Administration, PRC on March 22, 2019, the content of each of which is incorporated herein by reference in its entirety.

### TECHNICAL FIELD

The present invention relates to a series of tricyclic substituted piperidine dione compounds and use thereof in preparing a medicament for treating a disease related to CRBN protein, specifically to a derivative compound of formula (I) or a pharmaceutically acceptable salt thereof.

### BACKGROUND

Thalidomide, sold under the trade name THALOMID®, was first synthesized by Grünenthal Group of Germany. From the latter half of the 1950s to the early 1960s, it was sold as a sedative in over 40 countries and also widely used as an antiemetic for pregnant women. It was withdrawn from the market in the end due to the tragedy it caused, namely tens of thousands of infants were born with phocomelia (morphogenesis disorder).

After the thalidomide event, the mechanism of action of thalidomide teratogenicity has aroused great interest of researchers. Cereblon (CRBN) protein has been proved to be the target protein for thalidomide teratogenicity. Thalidomide forms an E3 ubiquitin ligase complex by combining with CRBN, Damaged DNA Binding Protein 1 (DDB1), CuLlin-4A (CUL4A) and Regulator of CuLlins 1 (ROC1) to ubiquitinate a plurality of substrate proteins and form ubiquitinated chains, so that the substrate proteins are recognized and hydrolyzed by proteasomes. Domide drugs, called Immunomodulatory Drugs (IMiDs), activate the E3 ubiquitin ligase complex formed with CRBN to ubiquitinate transcription factors IKZF1 and IKZF3, which are then recognized and degraded by proteasomes, thereby generating toxic effects on multiple myeloma. Absence of these two transcription factors will terminate the growth of myeloma. Domide drugs such as lenalidomide and pomalidomide are now first-line drugs for treating multiple myeloma.

CRBN is a protein that is conserved from plant to human and has 442 amino acids, and it is located on the short arm p26.3 of human chromosome 3, with a molecular weight of 51 kDa. In humans, CRBN gene has been identified as a candidate gene of Autosomal Recessive Inheritance of Non-Syndromic Mental Retardation (ARNSMR). CRBN is widely expressed in testis, spleen, prostate, liver, pancreas, placenta, kidney, lung, skeletal muscle, ovary, small intestine, peripheral blood leukocytes, colon, brain, and retina, and its expression in brain tissue (including retina) and testis is significantly higher than in other tissues.

Taking CRBN as an important target of anti-tumor and immune regulator drugs has been proved to have definite efficacy on various hematologic malignant tumors such as multiple myeloma and chronic lymphocytic leukemia, skin diseases such as erythema nodosum leprosum, and autoimmune diseases such as systemic lupus erythematosus. The domide drugs all have many side effects, especially peripheral neuropathy. There is an urgent need to develop CRBN modulator drugs with no teratogenic effect, fewer peripheral neuropathies, stronger immunomodulatory effect and higher anti-tumor activity to improve clinical therapeutic effects, reduce clinical side effects, and facilitate long-term use by patients.

### SUMMARY

The present invention provides a compound of formula (I), an isomer thereof or a pharmaceutically acceptable salt thereof, 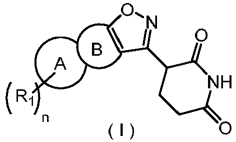 wherein,
n is selected from the group consisting of 0, 1, 2 and 3;
each R₁ is independently selected from the group consisting of H, F, Cl, Br, I, OH, NH₂, CN, C₁₋₆ alkyl, C₃₋₁₀ cycloalkyl, C₁₋₆ alkoxy, C₁₋₆ alkylamino, C₂₋₆ alkenyl, 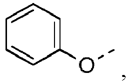 -S(=O)₂NH₂, -NHS(=O)₂-C₁₋₆ alkyl, -N[S(=O)₂-C₁₋₆ alkyl]₂, -N[C(=O)-C₁₋₆ alkyl]₂, -NHC(=O)-C₁₋₆ alkyl and -C(=O)NH₂, wherein the OH, NH₂, C₁₋₆ alkyl, C₃₋₁₀ cycloalkyl, C₁₋₆ alkoxy, C₁₋₆ alkylamino, C₂₋₆ alkenyl, 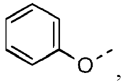 -S(=O)₂NH₂, -NHS(=O)₂-C₁₋₆ alkyl, -N[S(=O)₂-C₁₋₆ alkyl]₂, -N[C(=O)-C₁₋₆ alkyl]₂, -NHC(=O)-C₁₋₆ alkyl and -C(=O)NH₂ are optionally substituted with 1, 2 or 3 Rₐ;
ring A is selected from the group consisting of 5-6 membered heteroaryl, phenyl, C₄₋₆ cycloalkyl, 4-7 membered heterocycloalkyl and 4-7 membered heterocycloalkenyl;
ring B is selected from the group consisting of 5-6 membered heteroaryl and phenyl;
each Rₐ is independently selected from the group consisting of F, Cl, Br, I, OH, NH₂, C₁₋₁₀ alkyl, C₁₋₁₀ alkoxy, C₁₋₁₀ alkylamino, -C(=O)NH-C₁₋₁₀ alkyl, -NHC(=O)-C₁₋₁₀ alkyl, C₃₋₁₀ cycloalkyl, C₃₋₁₀ cycloalkylamino, 4-10 membered heterocycloalkyl, 4-10 membered heterocycloalkylamino and 4-10 membered heterocycloalkyl substituted with one carbonyl, wherein the OH, NH₂, C₁₋₁₀ alkyl, C₁₋₁₀ alkoxy, C₁₋₁₀ alkylamino, -C(=O)NH-C₁₋₁₀ alkyl, -NHC(=O)-C₁₋₁₀ alkyl, -COOC₁₋₁₀ alkyl, C₃₋₁₀ cycloalkyl, C₃₋₁₀ cycloalkylamino, 4-10 membered heterocycloalkyl and 4-10 membered heterocycloalkylamino are optionally substituted with 1, 2 or 3 R;
each R is independently selected from the group consisting of F, Cl, Br, I, OH, NH₂, C₁₋₃ alkyl, C₁₋₃ alkoxy, C₁₋₃ alkylamino, C₃₋₅ cycloalkyl, -C(=O)-C₁₋₃ alkyl, -C(=O)O-C₁₋₆ alkyl, -S(=O)₂-C₁₋₃ alkyl, 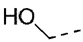 and 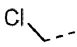
the 5-6 membered heteroaryl, 4-7 membered heterocycloalkyl, 4-10 membered heterocycloalkyl, 4-10 membered heterocycloalkylamino, 4-7 membered heterocycloalkenyl and 4-10 membered heterocycloalkyl substituted with one carbonyl each contain 1, 2, 3 or 4 heteroatoms or heteroatom groups independently selected from the group consisting of -NH-, -O-, -S- and N.

In some embodiments of the present invention, provided is a compound of formula (I), an isomer thereof or a pharmaceutically acceptable salt thereof, 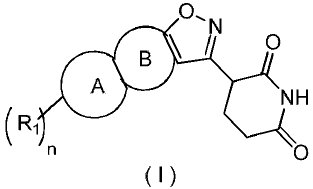 wherein,
n is selected from the group consisting of 0, 1, 2 and 3;
each R₁ is independently selected from the group consisting of H, F, Cl, Br, I, OH, NH₂, CN, C₁₋₆ alkyl, C₁₋₆ alkoxy and -C(=O)NH₂, wherein the C₁₋₆ alkyl, C₁₋₆ alkoxy and -C(=O)NH₂ are optionally substituted with 1, 2 or 3 Ra;
ring A is selected from the group consisting of 5-6 membered heteroaryl, phenyl, C₄₋₆ cycloalkyl and 4-7 membered heterocycloalkyl;
ring B is selected from the group consisting of 5-6 membered heteroaryl and phenyl;
each Rₐ is independently selected from the group consisting of F, Cl, Br, I, OH, NH₂, C₁₋₃ alkyl, C₁₋₃ alkoxy and C₁₋₅ alkylamino, wherein the C₁₋₃ alkyl, C₁₋₃ alkoxy and C₁₋₅ alkylamino are optionally substituted with 1, 2 or 3 R;
each R is independently selected from the group consisting of F, Cl, Br, I, OH, NH₂, C₁₋₃ alkyl, C₁₋₃ alkoxy, C₁₋₃ alkylamino and C₃₋₅ cycloalkyl;
the 5-6 membered heteroaryl and 4-7 membered heterocycloalkyl each contain 1, 2, 3 or 4 heteroatoms or heteroatom groups independently selected from the group consisting of -NH-, -O-, -S- and N.

In some embodiments of the present invention, provided is a compound of formula (I), an isomer thereof or a pharmaceutically acceptable salt thereof, 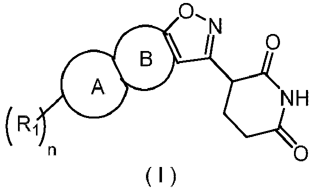 wherein,
n is selected from the group consisting of 0, 1, 2 and 3;
each R₁ is independently selected from the group consisting of halogen, OH, NH₂ and C₁₋₆ alkyl, wherein the C₁₋₆ alkyl is optionally substituted with 1, 2 or 3 Rₐ;
ring A is selected from the group consisting of 5-6 membered heteroaryl, phenyl, C₄₋₆ cycloalkyl and 4-7 membered heterocycloalkyl;
ring B is selected from phenyl;
each Rₐ is independently selected from the group consisting of F, Cl, Br, I, OH and NH₂;
the 5-6 membered heteroaryl and 4-7 membered heterocycloalkyl each contain 1, 2, 3 or 4 heteroatoms or heteroatom groups independently selected from the group consisting of -NH-, -O-, -S- and N.

In some embodiments of the present invention, each Rₐ is independently selected from the group consisting of F, Cl, Br, I, OH, NH₂, C₁₋₁₀ alkyl, C₁₋₁₀ alkoxy, C₁₋₁₀ alkylamino, -C(=O)NH-C₁₋₁₀ alkyl, -NHC(=O)-C₁₋₁₀ alkyl, C₃₋₁₀ cycloalkyl, C₃₋₁₀ cycloalkylamino, 4-10 membered heterocycloalkyl, 4-10 membered heterocycloalkylamino and 4-10 membered heterocycloalkyl substituted with one carbonyl, wherein the C₁₋₁₀ alkyl, C₁₋₁₀ alkoxy, C₁₋₁₀ alkylamino, -C(=O)NH-C₁₋₁₀ alkyl, -NHC(=O)-C₁₋₁₀ alkyl, -COOC₁₋₁₀ alkyl, C₃₋₁₀ cycloalkyl, C₃₋₁₀ cycloalkylamino, 4-10 membered heterocycloalkyl and 4-10 membered heterocycloalkylamino are optionally substituted with 1, 2 or 3 R.

In some embodiments of the present invention, each Rₐ is independently selected from the group consisting of F, Cl, Br, I, OH, NH₂, C₁₋₆ alkyl, C₁₋₆ alkoxy, C₁₋₆ alkylamino, -C(=O)NH-C₁₋₆ alkyl, -NHC(=O)-C₁₋₆ alkyl, C₃₋₈ cycloalkyl, C₃₋₈ cycloalkylamino, 4-6 membered heterocycloalkyl, 4-6 membered heterocycloalkylamino and 4-10 membered heterocycloalkyl substituted with one carbonyl, wherein the C₁₋₆ alkyl, C₁₋₆ alkoxy, C₁₋₆ alkylamino, -C(=O)NH-C₁₋₆ alkyl, -NHC(=O)-C₁₋₆ alkyl, -COOC₁₋₆ alkyl, C₃₋₈ cycloalkyl, C₃₋₈ cycloalkylamino, 4-6 membered heterocycloalkyl and 4-6 membered heterocycloalkylamino are optionally substituted with 1, 2 or 3 R, while the other variables are defined as herein.

In some embodiments of the present invention, each Rₐ is independently selected from the group consisting of F, Cl, Br, I, OH, NH₂, C₁₋₃ alkyl, C₁₋₃ alkoxy, C₁₋₃ alkylamino, -C(=O)NH-C₁₋₃ alkyl, -NHC(=O)-C₁₋₃ alkyl, C₃₋₆ cycloalkyl, cyclohexylamino, azetidinyl, pyrrolidin-2-one, tetrahydropyrrolyl, piperidinyl, piperazinyl, morpholinyl, tetrahydropyranyl, 3-azabicyclo[3,1,0]hexyl, azetidinylamino, tetrahydropyrrolylamino, tetrahydropyrrolyl, piperidinylamino, piperazinylamino, morpholinylamino, tetrahydropyranylamino and 3-azabicyclo[3,1,0]hexylamino, wherein the C₁₋₃ alkyl, C₁₋₃ alkoxy, C₁₋₃ alkylamino, -C(=O)NH-C₁₋₃ alkyl, -NHC(=O)-C₁₋₃ alkyl, -COOC₁₋₄ alkyl, C₃₋₆ cycloalkyl, cyclohexylamino, azetidinyl, tetrahydropyrrolyl, piperidinyl, piperazinyl, morpholinyl, tetrahydropyranyl, 3-azabicyclo[3,1,0]hexyl, azetidinylamino, tetrahydropyrrolylamino, piperidinylamino, piperazinylamino, morpholinylamino, tetrahydropyranylamino and 3-azabicyclo[3,1,0]hexylamino are optionally substituted with 1, 2 or 3 R, while the other variables are defined as herein.

In some embodiments of the present invention, each Rₐ is independently selected from the group consisting of F, Cl, Br, I, OH, NH₂, C₁₋₃ alkyl, C₁₋₃ alkoxy, C₁₋₃ alkylamino, -C(=O)NH-C₁₋₃ alkyl, -NHC(=O)-C₁₋₃ alkyl, C₃₋₆ cycloalkyl, cyclohexylamino, azetidinyl, pyrrolidin-2-one, piperidinyl, piperazinyl, morpholinyl, 3-azabicyclo[3,1,0]hexyl, tetrahydropyrrolyl, piperidinylamino and tetrahydropyranylamino, wherein the C₁₋₃ alkyl, C₁₋₃ alkoxy, C₁₋₃ alkylamino, -C(=O)NH-C₁₋₃ alkyl, -NHC(=O)-C₁₋₃ alkyl, C₃₋₆ cycloalkyl, cyclohexylamino, azetidinyl, pyrrolidin-2-one, piperidinyl, piperazinyl, morpholinyl, 3-azabicyclo[3,1,0]hexyl, tetrahydropyrrolyl, piperidinylamino and tetrahydropyranylamino are optionally substituted with 1, 2 or 3 R, while the other variables are defined as herein.

In some embodiments of the present invention, each Rₐ is independently selected from the group consisting of F, Cl, Br, I, OH, NH₂, -CH₃, -CH₂CH₃, 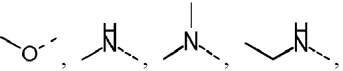 -C(=O)NHCH₃, -NHC(=O)CH₃, 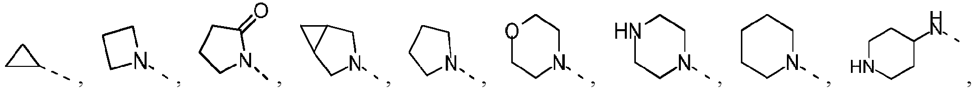 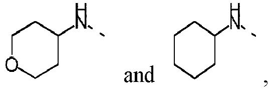 wherein the CH₃, -CH₂CH₃, 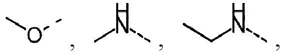 -C(=O)NHCH₃, -NHC(=O)CH₃, -COOt-Bu, 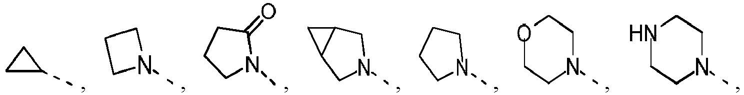 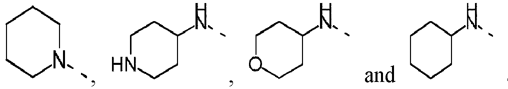 are optionally substituted with 1, 2 or 3 R, while the other variables are defined as herein.

In some embodiments of the present invention, each Rₐ is independently selected from the group consisting of F, Cl, Br, I, OH, NH₂, -CH₃, -CH₂CH₃, 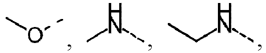 -C(=O)NHCH₃, -NHC(=O)CH₃, 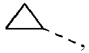 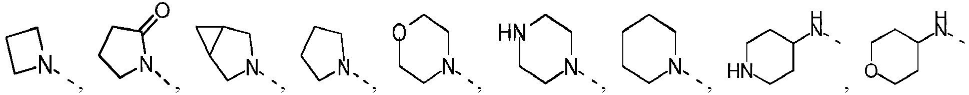 and 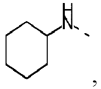 wherein the -CH₃, -CH₂CH₃, 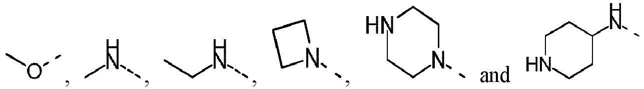 are optionally substituted with 1, 2 or 3 R, while the other variables are defined as herein.

In some embodiments of the present invention, each Rₐ is selected from the group consisting of F, Cl, Br, I, OH, NH₂, -CH₂-, -CH₂CH₂-, 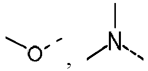 and 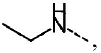 wherein the -CH₂-, -CH₂CH₂-, 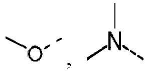 and 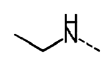 are optionally substituted with 1, 2 or 3 R, while the other variables are defined as herein.

In some embodiments of the present invention, each Rₐ is independently selected from the group consisting of F, Cl, Br, I, Me, OH, NH₂, 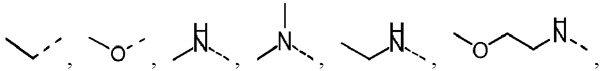 -C(=O)NHCH₃, -NHC(=O)CH₃, -CH₂COOt-Bu, 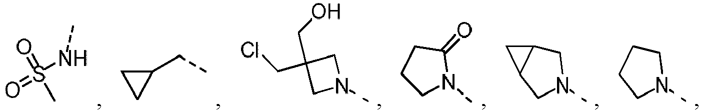 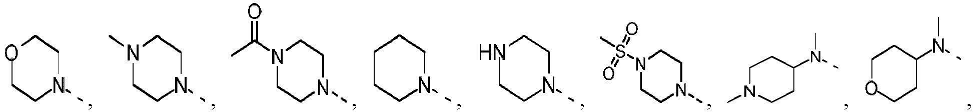 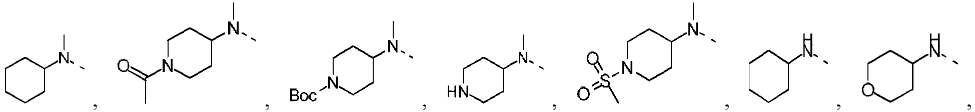 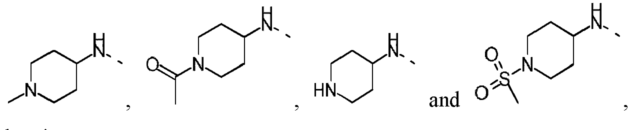 while the other variables are defined as herein.

In some embodiments of the present invention, each R is independently selected from the group consisting of C₁₋₃
alkyl, C₁₋₃ alkoxy, -C(=O)-C₁₋₃ alkyl, C₃₋₅ cycloalkyl, -C (=O)O-C₁₋₄ alkyl, -S(=O)₂-C₁₋₃ alkyl, 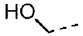 and

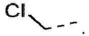

In some embodiments of the present invention, each R is independently selected from the group consisting of -CH₃, 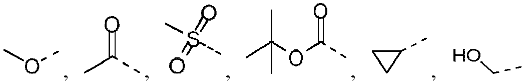 and 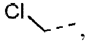 while the other variables are defined as herein.

In some embodiments of the present invention, each R is independently selected from the group consisting of F, Cl, Br, I, OH, NH₂, -CH₂-, -CH₂CH₂-, 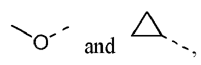 , while the other variables are defined as herein.

In some embodiments of the present invention, n is selected from the group consisting of 0, 1 and 2.

In some embodiments of the present invention, n is selected from the group consisting of 0 and 1.

In some embodiments of the present invention each R₁ is independently selected from the group consisting of H, F, Cl, Br, I, OH, NH₂, CN, C₁₋₆ alkyl, C₃₋₁₀ cycloalkyl, C₁₋₆ alkoxy, C₁₋₆ alkylamino, C₂₋₆ alkenyl, 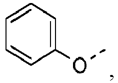 -S(=O)₂NH₂, -NHS(=O)₂-C₁₋₆ alkyl, -N[S(=O)₂-C₁₋₆ alkyl]₂, -N[C(=O)-C₁₋₆ alkyl]₂, -NHC(=O)-C₁₋₆ alkyl and -C(=O)NH₂, wherein the C₁₋₆ alkyl, C₃₋₁₀ cycloalkyl, C₁₋₆ alkoxy, C₁₋₆ alkylamino, C₂₋₆ alkenyl, 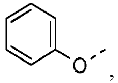 -S(=O)₂NH₂, -NHS(=O)₂-C₁₋₆ alkyl, -N[S(=O)₂-C₁₋₆ alkyl]₂, -N[C(=O)-C₁₋₆ alkyl]₂, -NHC(=O)-C₁₋₆ alkyl and -C(=O)NH₂ are optionally substituted with 1, 2 or 3 Rₐ.

In some embodiments of the present invention, each R₁ is independently selected from the group consisting of H, F, Cl, Br, I, OH, NH₂, CN, C₁₋₃ alkyl, C₃₋₅ cycloalkyl, C₁₋₃ alkoxy, C₁₋₃ alkylamino, C₂₋₄ alkenyl, 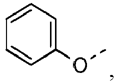 -S(=O)₂NH₂, -S(=O)₂NH-C₁₋₃ alkyl, -NHS(=O)₂-C₁₋₃ alkyl, -N[S(=O)₂-C₁₋₃ alkyl]₂, -N[C(=O)-C₁₋₃ alkyl]₂, -NHC(=O)-C₁₋₃ alkyl, -C(=O)NH₂ and -C(=O)NH-C₁₋₃ alkyl, wherein the C₁₋₃ alkyl, C₃₋₅ cycloalkyl, C₁₋₃ alkoxy, C₁₋₃ alkylamino, C₂₋₄ alkenyl, 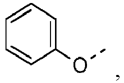 -S(=O)₂NH₂, -S(=O)₂NH-C₁₋₃ alkyl, -NHS(=O)₂-C₁₋₃ alkyl, -N[S(=O)₂-C₁₋₃ alkyl]₂, -N[C(=O)-C₁₋₃ alkyl]₂, -NHC(=O)-C₁₋₃ alkyl, -C(=O)NH₂ and -C(=O)NH-C₁₋₃ alkyl are optionally substituted with 1, 2 or 3 Rₐ, while the other variables are defined as herein.

In some embodiments of the present invention, each R₁ is independently selected from the group consisting of H, F, Cl, Br, I, OH, NH₂, CN, C₁₋₃ alkyl, C₁₋₃ alkoxy and -C(=O)NH₂, wherein the C₁₋₃ alkyl, C₁₋₃ alkoxy and -C(=O)NH₂ are optionally substituted with 1, 2 or 3 Rₐ, while the other variables are defined as herein.

In some embodiments of the present invention, each R₁ is independently selected from the group consisting of H, F, Cl, Br, I, OH, NH₂, CN, Me, 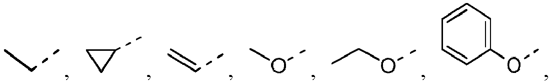 -S(=O)₂NH₂, 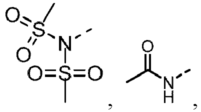 -NHCH₃ and -C(=O)NH₂, wherein the Me, 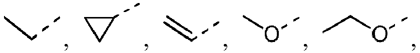 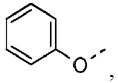 -S(=O)₂NH₂, 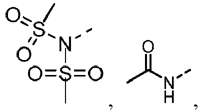 -NHCH₃ and -C(=O)NH₂ are optionally substituted with 1, 2 or 3 Rₐ, while the other variables are defined as herein.

In some embodiments of the present invention, each R₁ is independently selected from the group consisting of H, F, Cl, Br, I, OH, NH₂, Me, 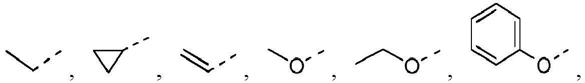 -S(=O)₂NH₂, 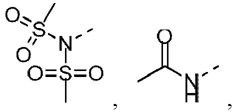 -NHCH₃ and -C(=O)NH₂, wherein the Me, 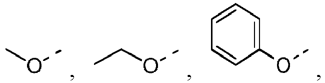 -S(=O)₂NH₂, -NHCH₃ and -C(=O)NH₂ are optionally substituted with 1, 2 or 3 Rₐ, while the other variables are defined as herein.

In some embodiments of the present invention, each R₁ is independently selected from the group consisting of H, F, Cl, Br, I, OH, NH₂, CN, Me, 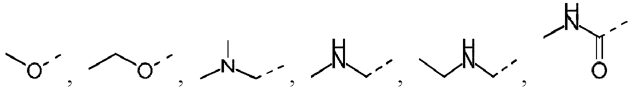 and 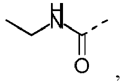 wherein the Me, 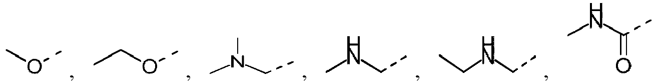 and 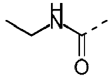 are optionally substituted with 1, 2 or 3 Rₐ, while the other variables are defined as herein.

In some embodiments of the present invention, each R₁ is independently selected from the group consisting of H, Me, OH, 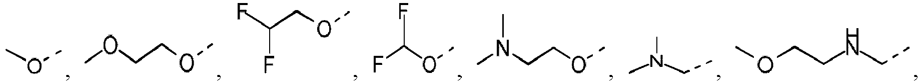 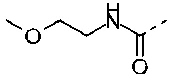 and 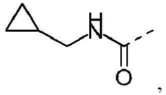 while the other variables are defined as herein.

In some embodiments of the present invention, each R₁ is independently selected from the group consisting of F, Cl, Br, I, OH, NH₂ and C₁₋₃ alkyl, wherein the C₁₋₃ alkyl is optionally substituted with 1, 2 or 3 Rₐ, while the other variables are defined as herein.

In some embodiments of the present invention, each R₁ is independently selected from the group consisting of F, Cl, Br, I, OH, NH₂ and Me, wherein the Me is optionally substituted with 1, 2 or 3 Rₐ, while the other variables are defined as herein.

In some embodiments of the present invention, each R₁ is independently selected from the group consisting of H, F, Cl, Br, I, Me, OH, NH₂, 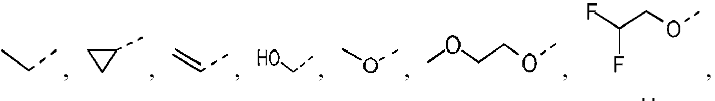 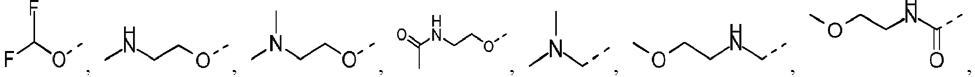 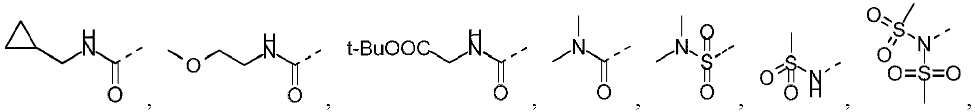 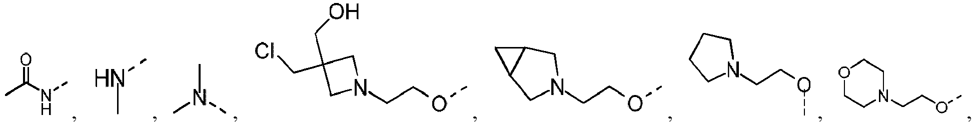 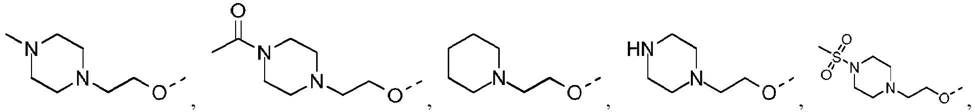 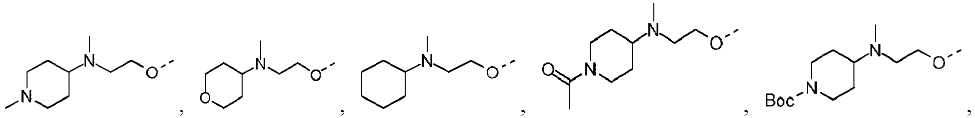 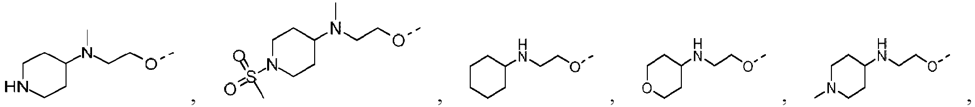 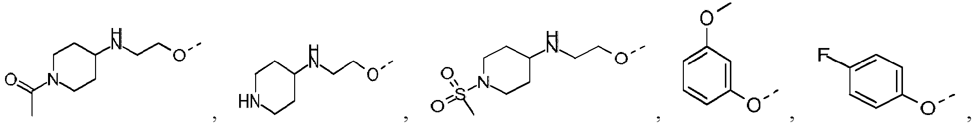 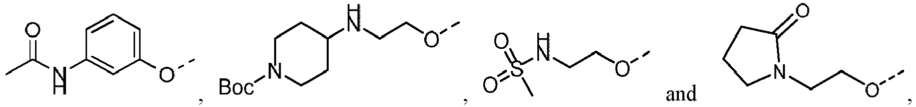 while the other variables are defined as herein.

In some embodiments of the present invention, each R₁ is independently selected from Me, while the other variables are defined as herein.

In some embodiments of the present invention, ring A is selected from the group consisting of 5-6 membered heteroaryl, phenyl, 4-7 membered heterocycloalkyl and 4-7 membered heterocycloalkenyl.

In some embodiments of the present invention, ring A is selected from the group consisting of 5-6 membered heteroaryl, phenyl and 4-7 membered heterocycloalkenyl.

In some embodiments of the present invention, ring A is selected from the group consisting of phenyl, 1,3-cyclohexadienyl, 1,3-dioxolanyl, 1,3-dioxolyl, morpholinyl, oxazolyl, cyclobutyl, oxapanyl, thiazolyl, tetrahydrothiazolyl, furanyl, 2,3-dihydrofuranyl, 1,4-oxazepanyl, pyridinyl, 2,3-dihydropyridinyl, pyrazolyl, 4,5-dihydro-1*H*-pyrazolyl, oxazolyl, 4,5-dihydrooxazolyl, pyrrolyl and 2,3-dihydro-1*H*-pyrrolyl, while the other variables are defined as herein.

In some embodiments of the present invention, ring A is selected from the group consisting of phenyl, 1,3-cyclohexadienyl, 1,3-dioxolanyl, 1,3-dioxolyl, furanyl, 2,3-dihydrofuranyl, pyridinyl, 2,3-dihydropyridinyl, pyrazolyl, 4,5-dihydro-1*H*-pyrazolyl, oxazolyl, 4,5-dihydrooxazolyl, pyrrolyl and 2,3-dihydro-1*H*-pyrrolyl, while the other variables are defined as herein.

In some embodiments of the present invention, ring A is selected from the group consisting of phenyl, 1,3-dioxolanyl, morpholinyl, oxazolyl, cyclobutyl, oxapanyl, thiazolyl, tetrahydrothiazolyl, furanyl, 1,4-oxazepanyl, pyridinyl and pyrrolyl, while the other variables are defined as herein.

In some embodiments of the present invention, ring A is selected from the group consisting of phenyl, 1,3-dioxolanyl, furanyl, pyridinyl and pyrrolyl, while the other variables are defined as herein.

In some embodiments of the present invention, ring A is selected from the group consisting of phenyl, 1,3-dioxolanyl, morpholinyl, oxazolyl, cyclobutyl, oxapanyl, thiazolyl, tetrahydrothiazolyl, furanyl, tetrahydrofuranyl and 1,4-oxazepanyl, while the other variables are defined as herein.

In some embodiments of the present invention, ring A is selected from the group consisting of phenyl, 1,3-dioxolanyl and furanyl, while the other variables are defined as herein.

In some embodiments of the present invention, ring A is selected from the group consisting of phenyl, 1,3-dioxolanyl, morpholinyl, oxazolyl, cyclobutyl, oxapanyl and 1,4-oxazepanyl, while the other variables are defined as herein.

In some embodiments of the present invention, ring A is selected from the group consisting of phenyl, 1,3-dioxolanyl, 1,3-dioxolyl, pyridinyl, furanyl, pyrrolyl, oxazolyl and pyrazolyl, while the other variables are defined as herein.

In some embodiments of the present invention, the structural unit 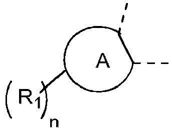 is selected from the group consisting of 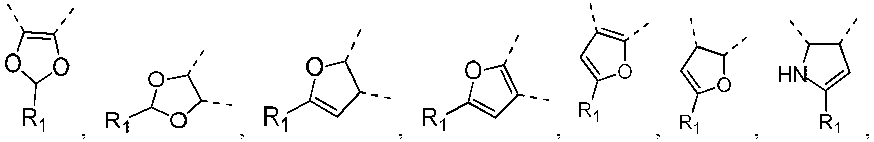 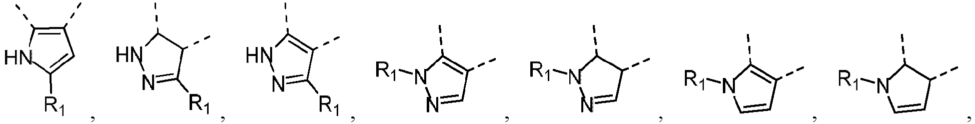 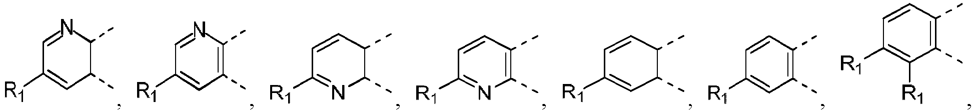 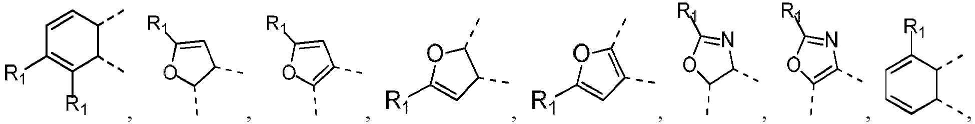 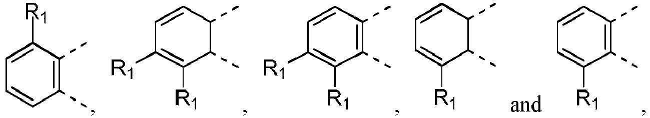 while the other variables are defined as herein.

In some embodiments of the present invention, the structural unit 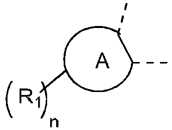 is selected from the group consisting of 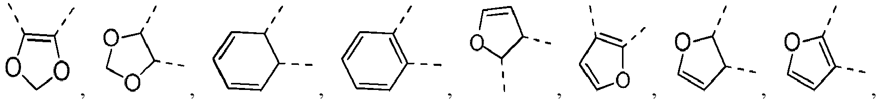 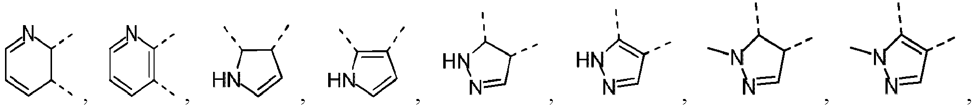 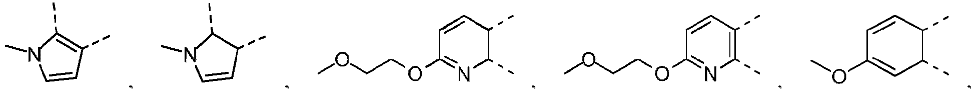 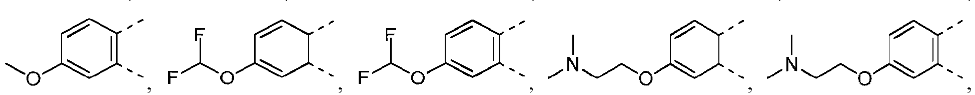 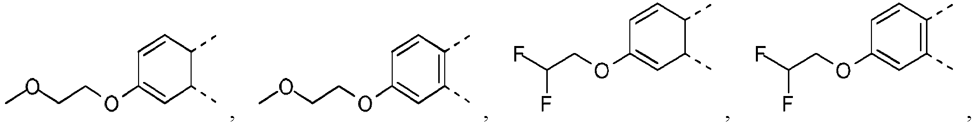 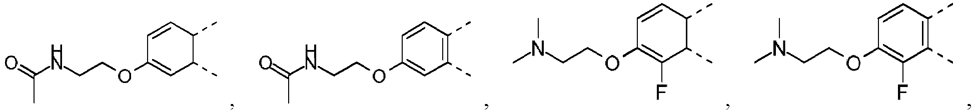 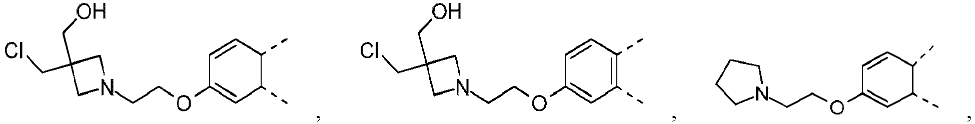 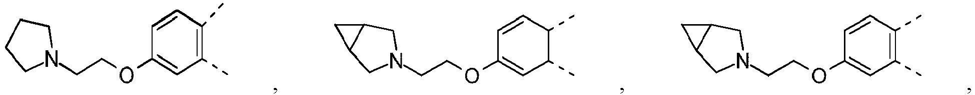 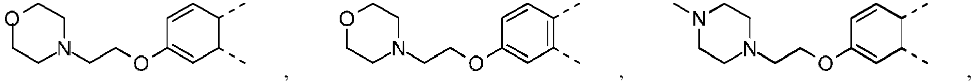 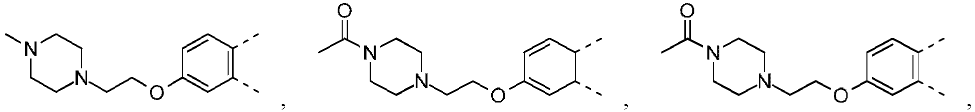 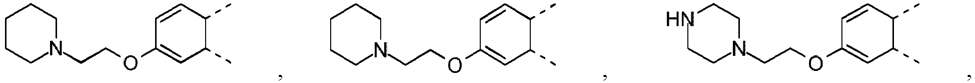 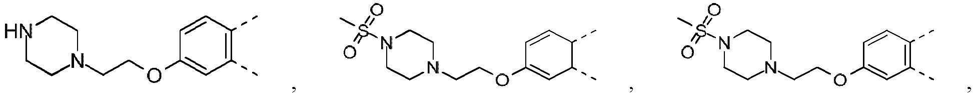 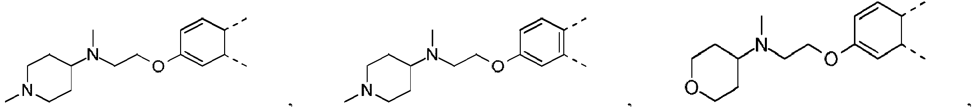 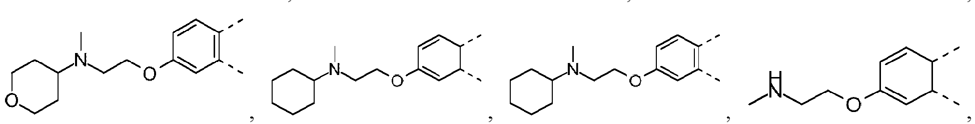 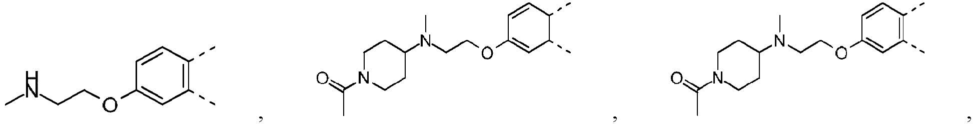 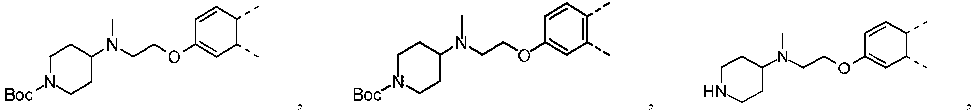 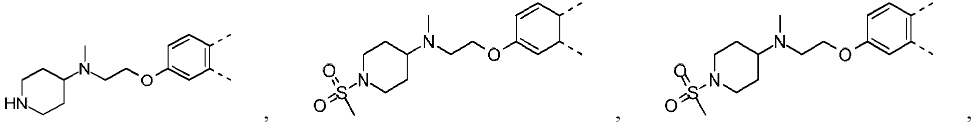 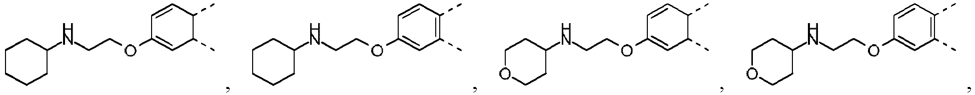 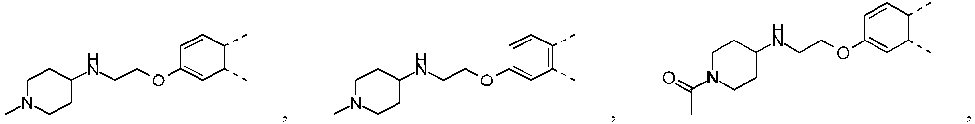 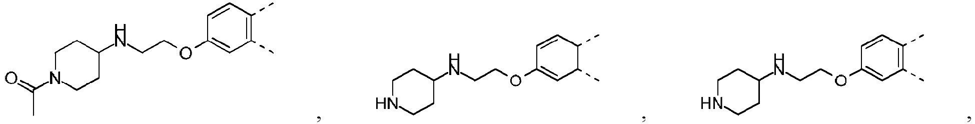 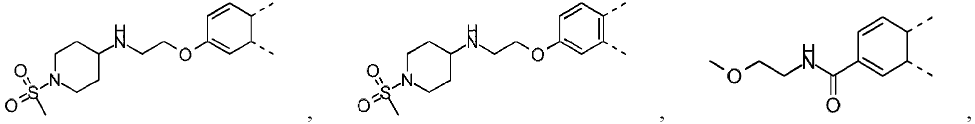 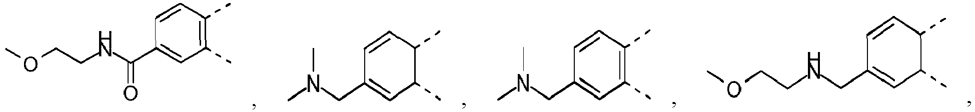 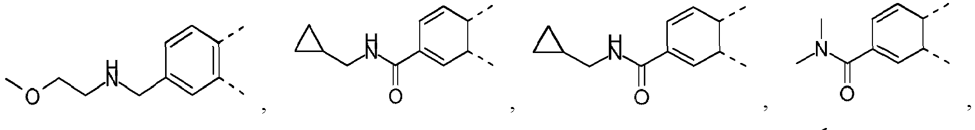 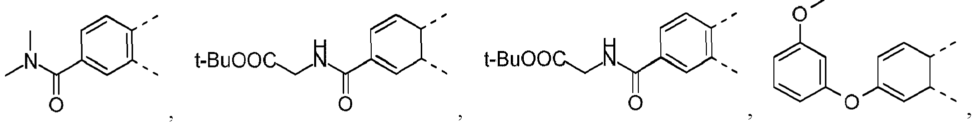 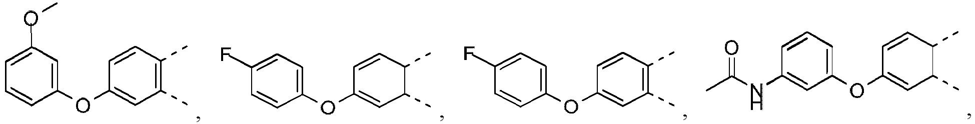 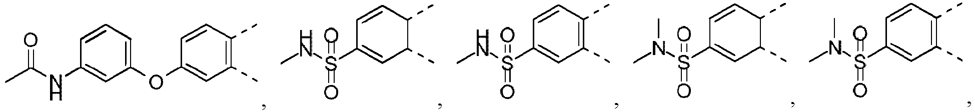 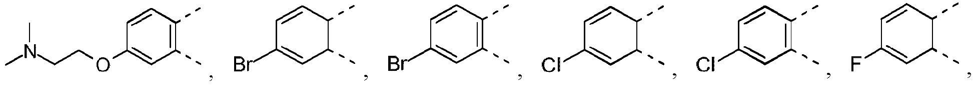 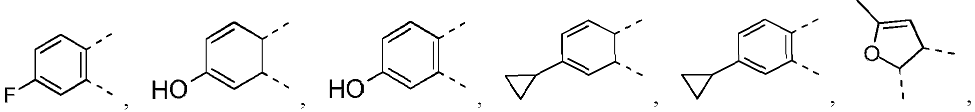 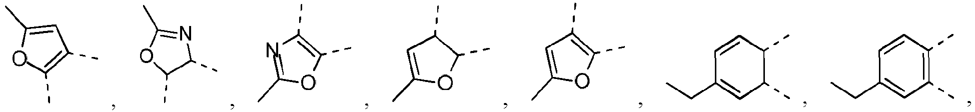 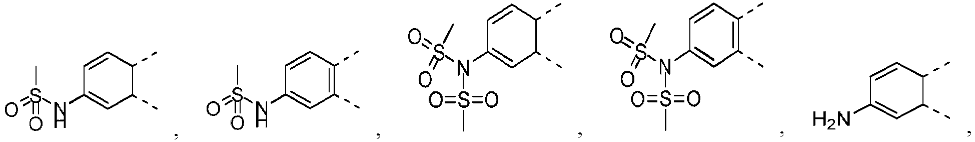 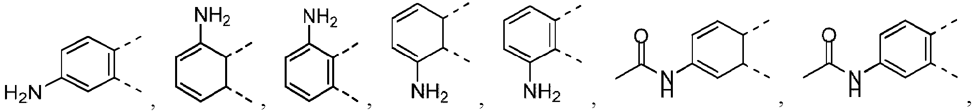 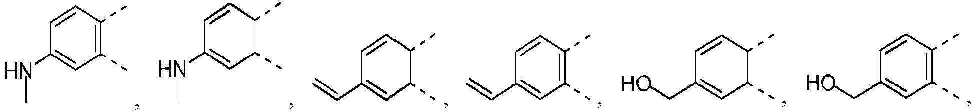 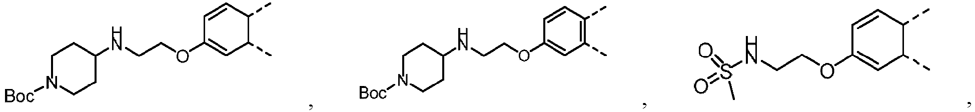 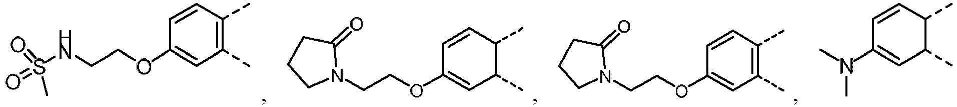 and 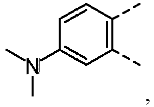 while the other variables are defined as herein.

In some embodiments of the present invention, ring B is selected from phenyl.

In some embodiments of the present invention, the structural unit 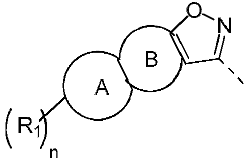 is selected from the group consisting of 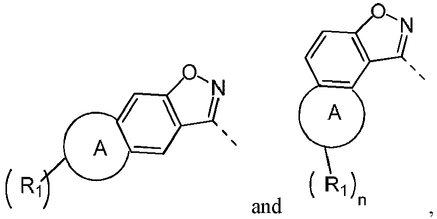 while the other variables are defined as herein.

In some embodiments of the present invention, the structural unit 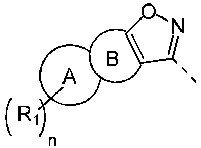 is selected from the group consisting of 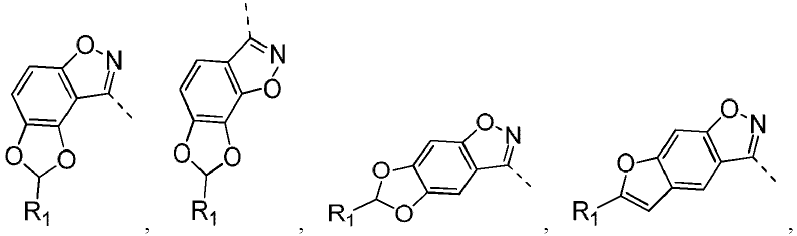 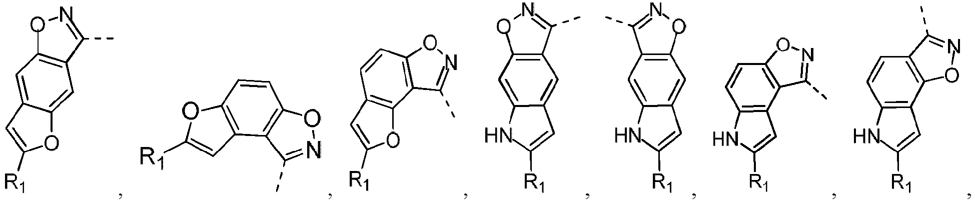 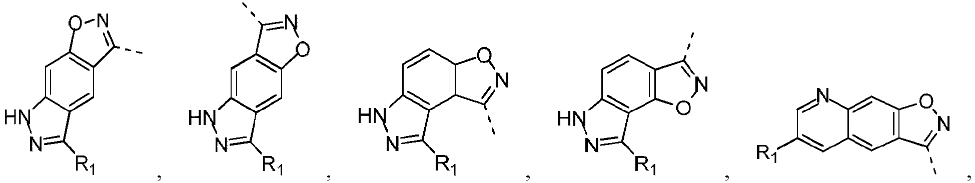 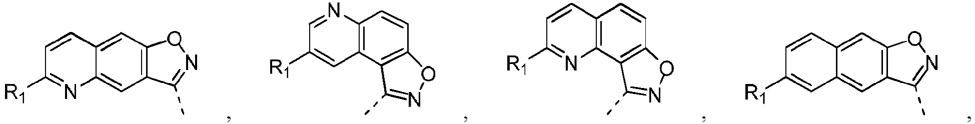 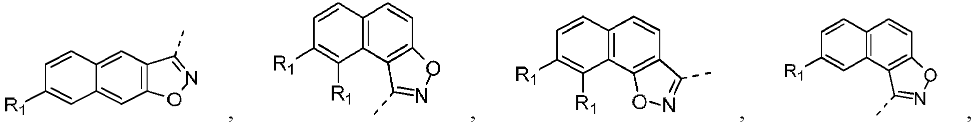 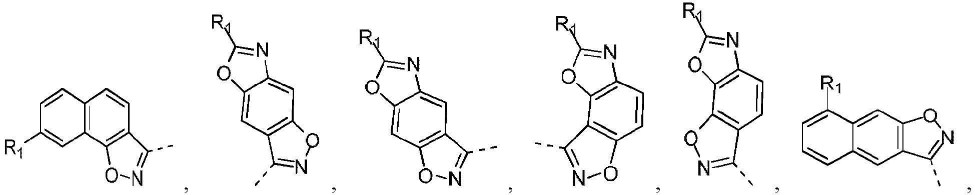 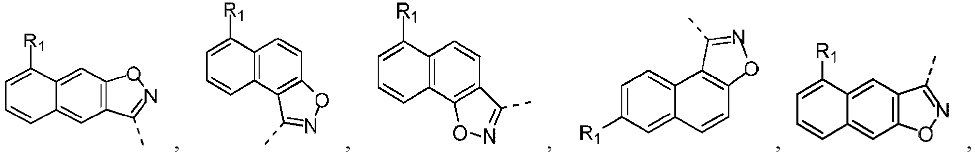 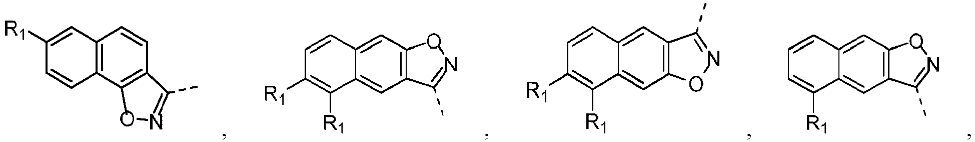 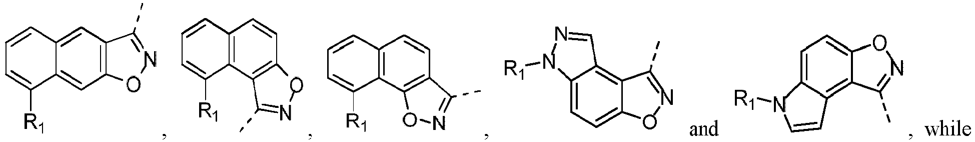 the other variables are defined as herein.

In some embodiments of the present invention, the structural unit 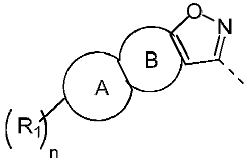 is selected from the group consisting of 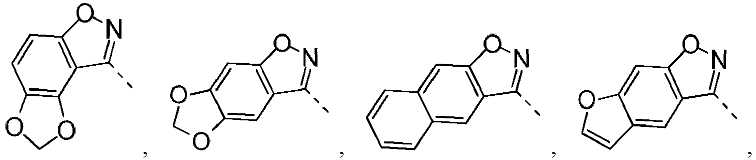 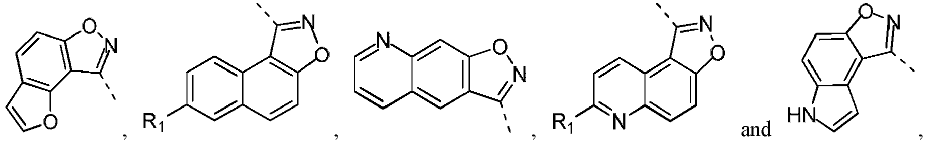 while the other variables are defined as herein.

In some embodiments of the present invention, the structural unit 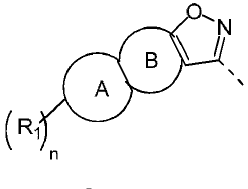 is selected from the group consisting of 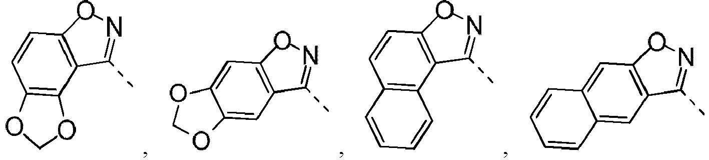 and 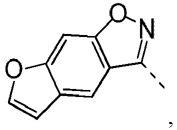 while the other variables are defined as herein.

In some embodiments of the present invention, provided is the compound, the isomer thereof or the pharmaceutically acceptable salt thereof, selected from the group consisting of: 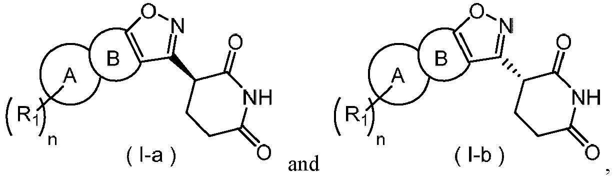 wherein, n, ring A, ring B, R₁, structural unit 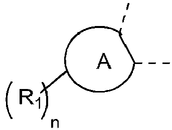 and structural unit 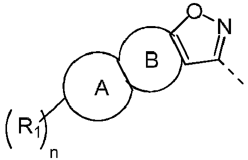 are defined as herein.

In some embodiments of the present invention, provided is the compound, the isomer thereof or the pharmaceutically acceptable salt thereof, selected from the group consisting of: 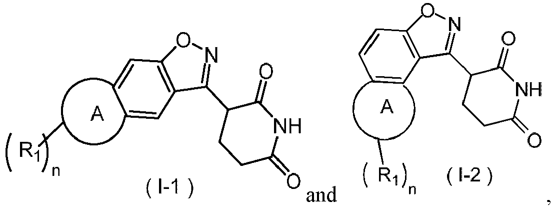 wherein, n, ring A and R₁ are defined as herein.

In some embodiments of the present invention, provided is the compound, the isomer thereof or the pharmaceutically acceptable salt thereof, selected from the group consisting of: 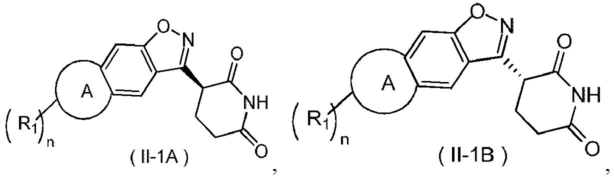 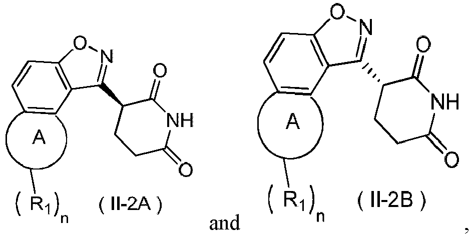 wherein n, R₁, ring A are defined as herein.

Still some other embodiments of the present invention are derived from any combination of the variables as described above.

The present invention also provides a compound of a formula below and a pharmaceutically acceptable salt thereof defined as herein, selected from the group consisting of:

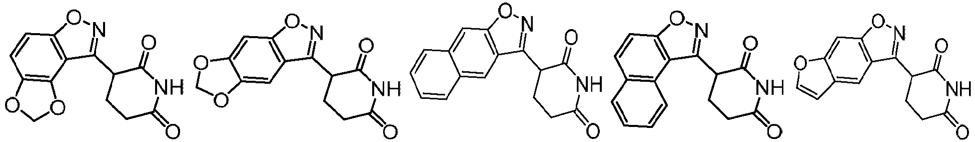

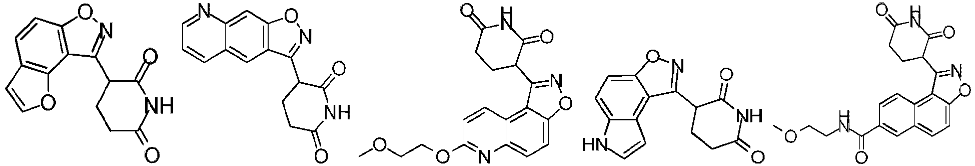

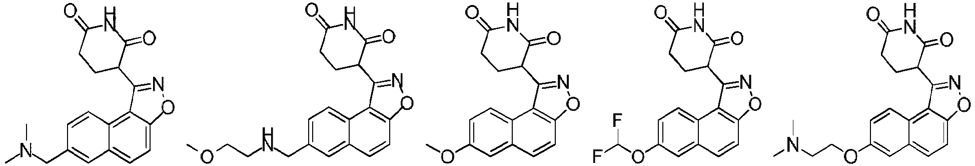

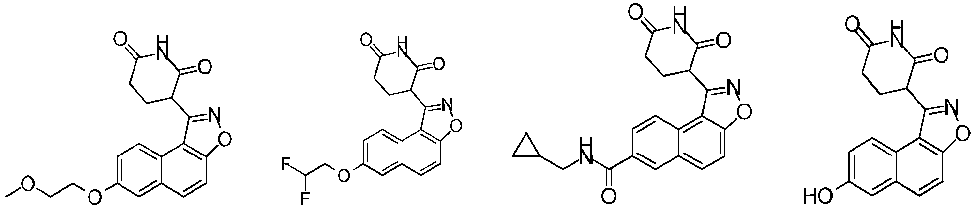

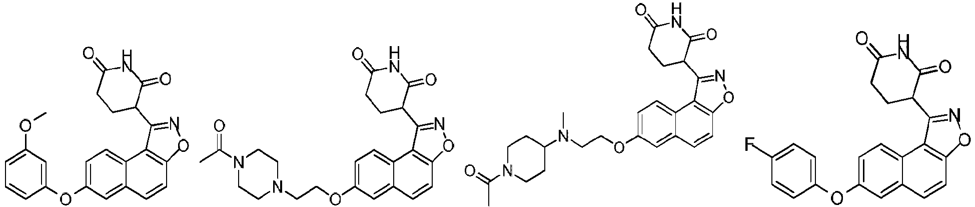

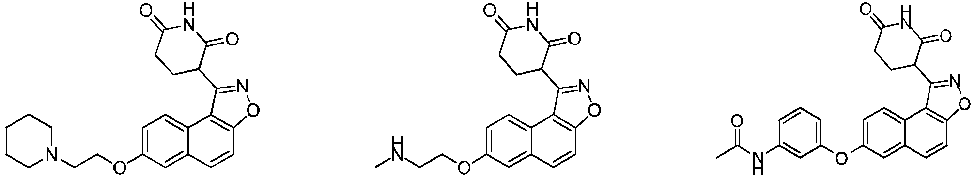

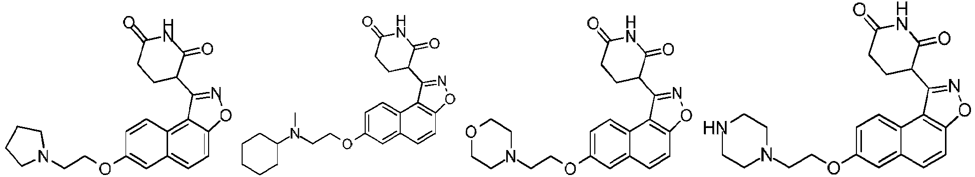

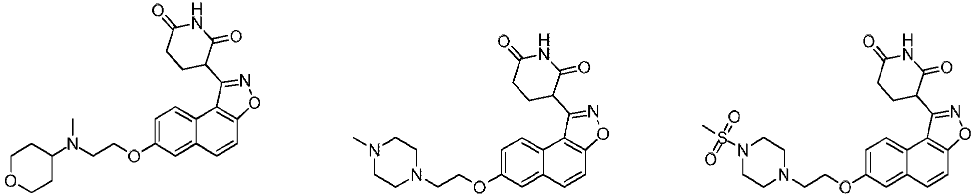

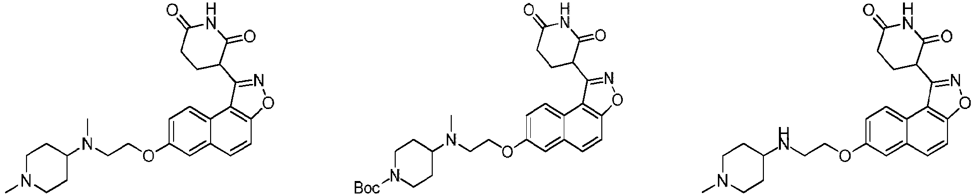

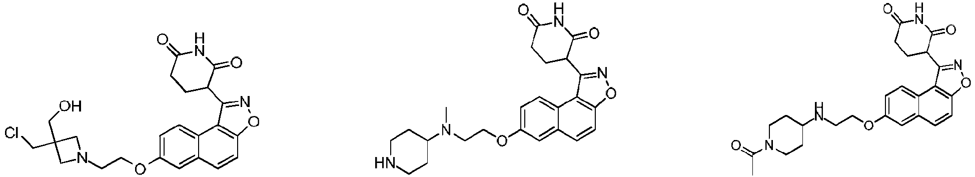

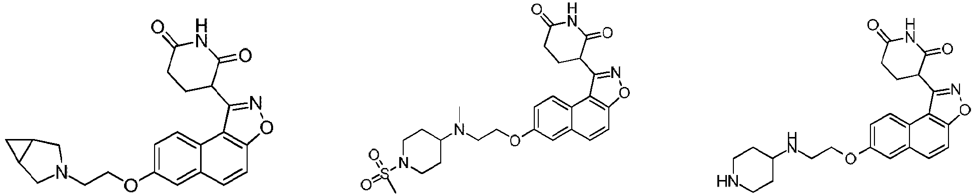

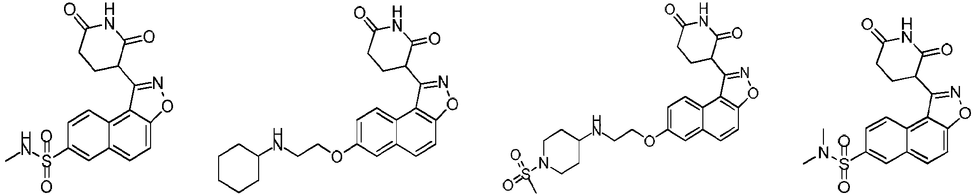

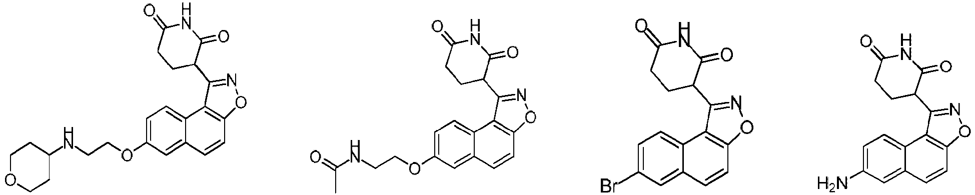

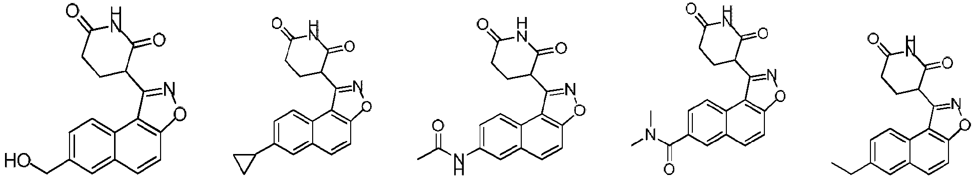

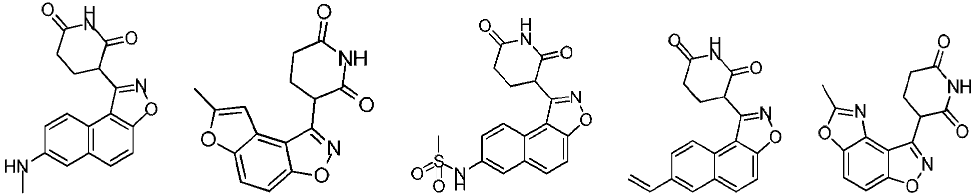

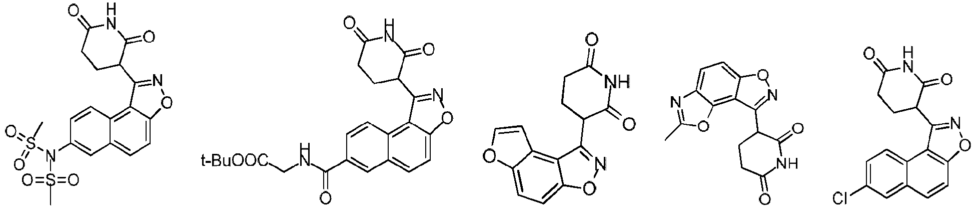

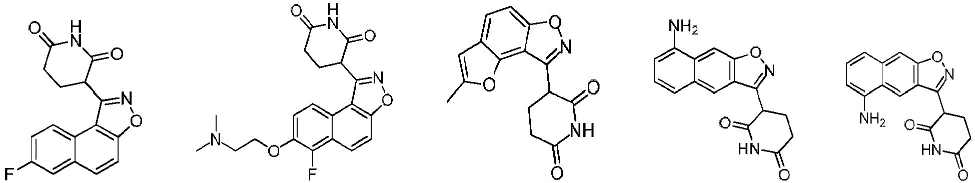

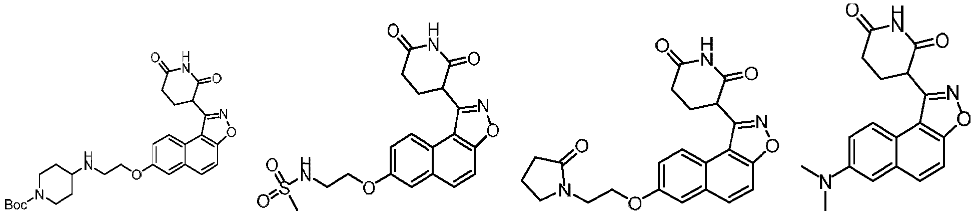

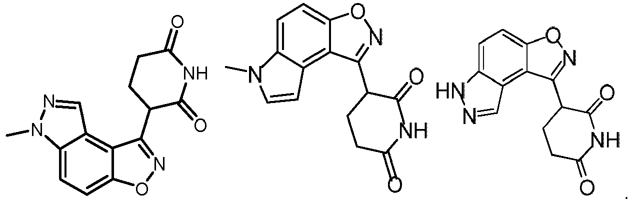

In some embodiments of the present invention, provided is the compound, the isomer thereof or the pharmaceutically acceptable salt thereof, selected from the group consisting of:

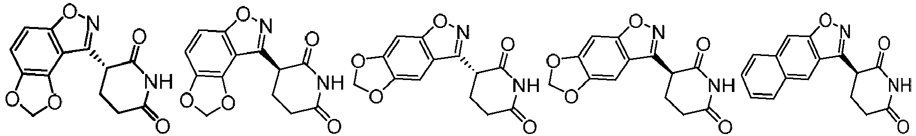

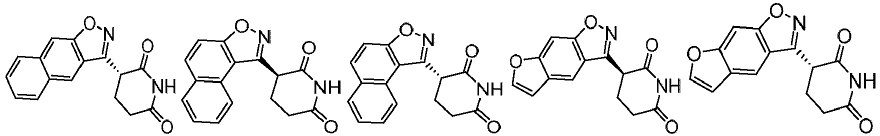

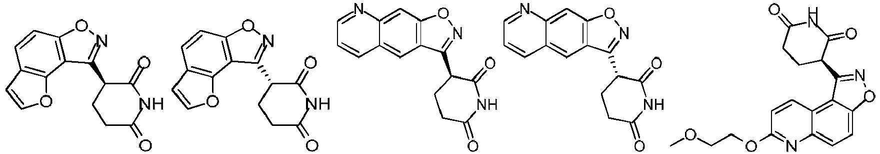

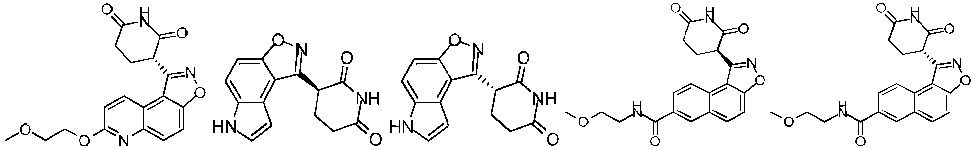

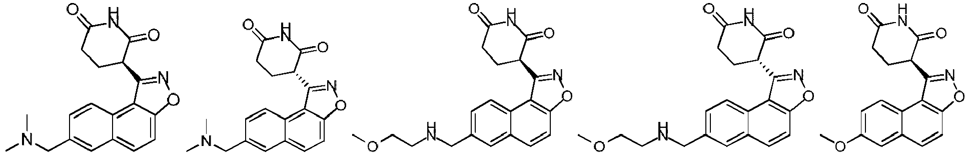

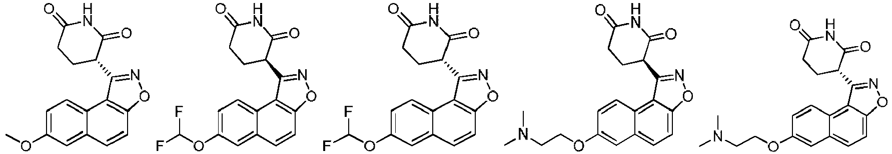

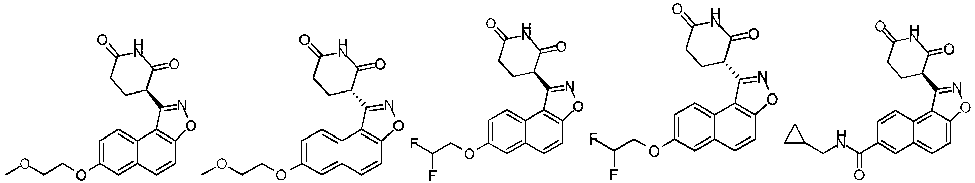

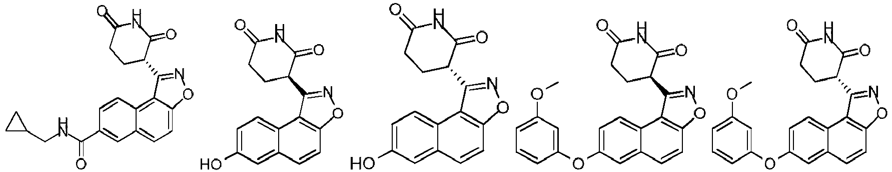

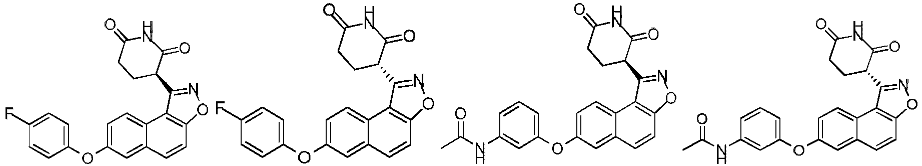

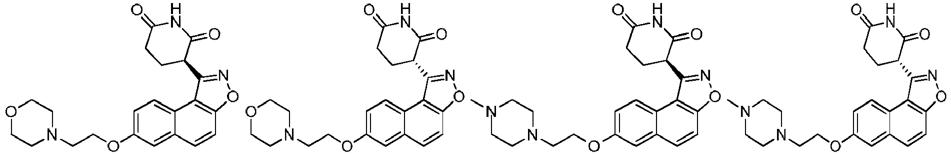

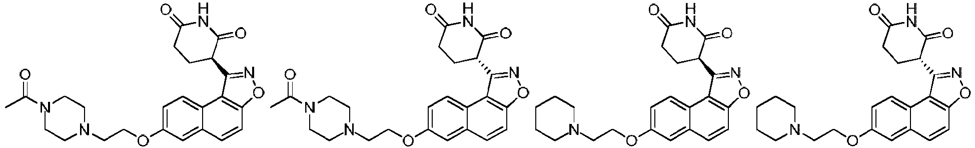

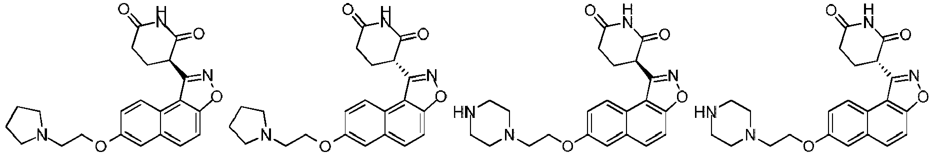

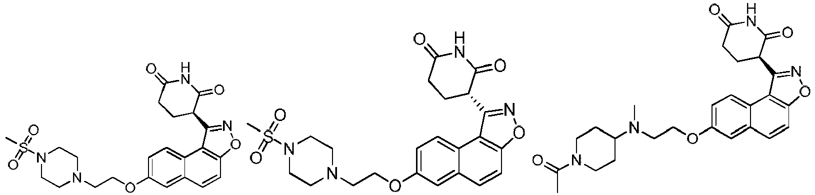

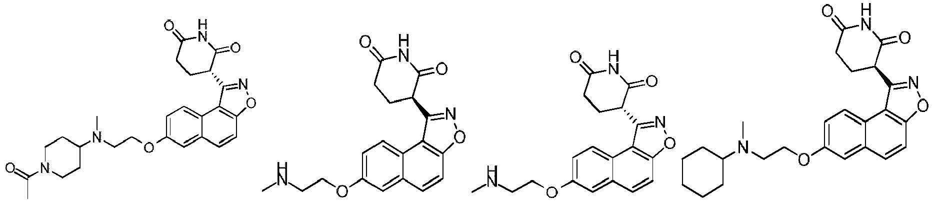

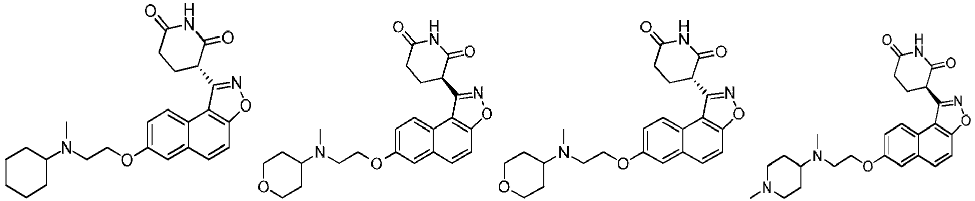

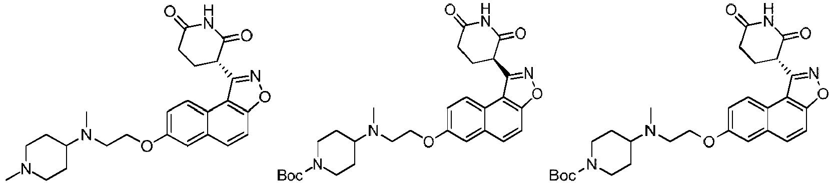

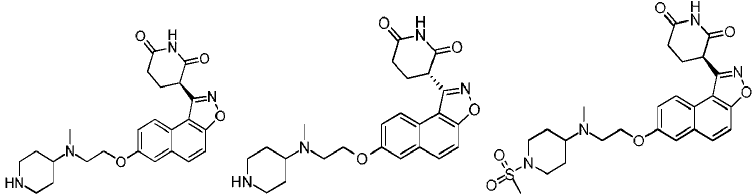

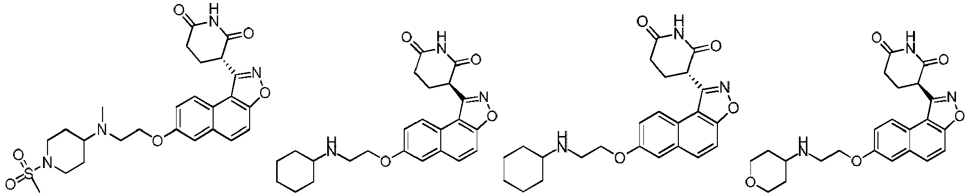

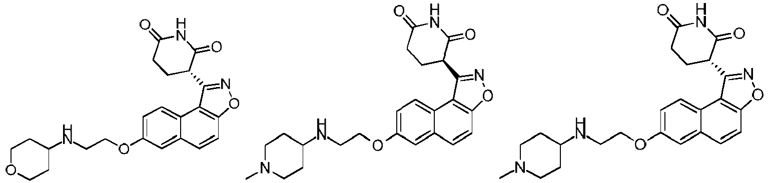

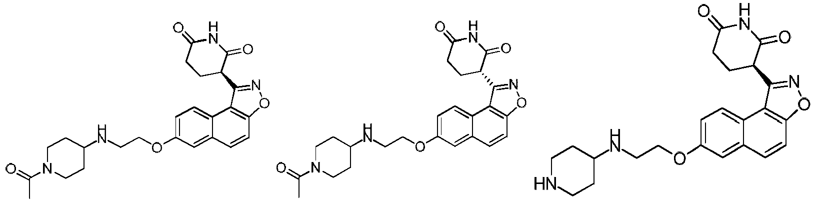

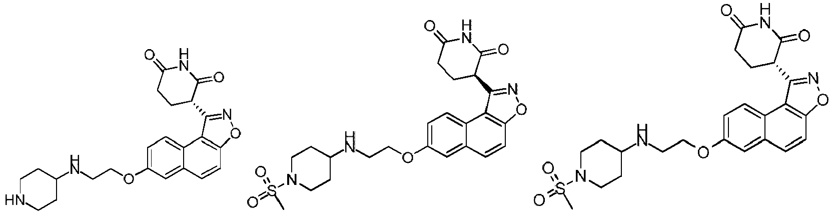

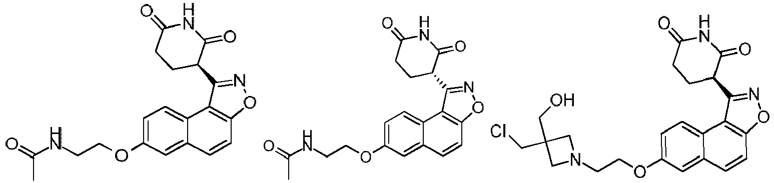

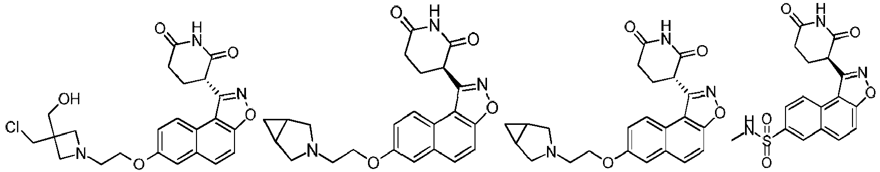

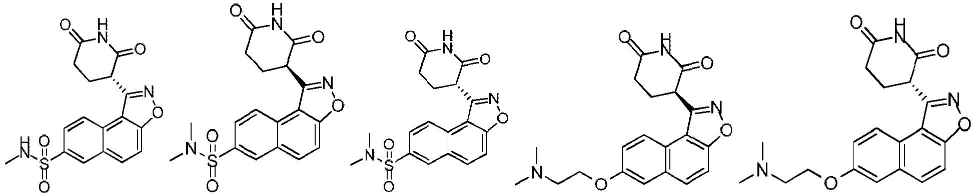

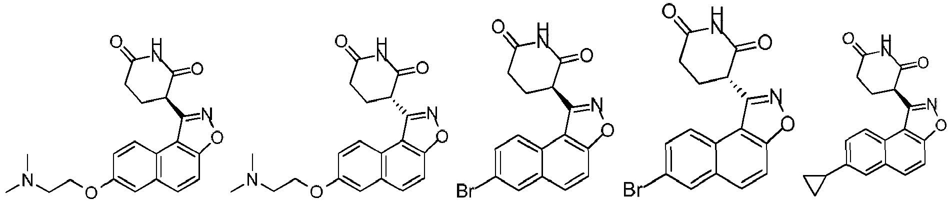

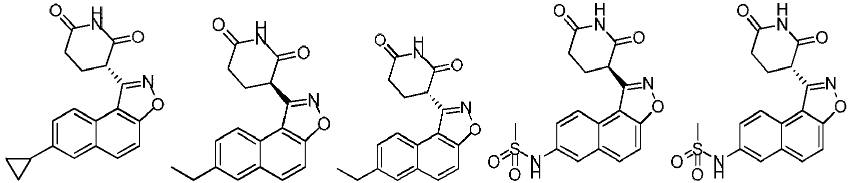

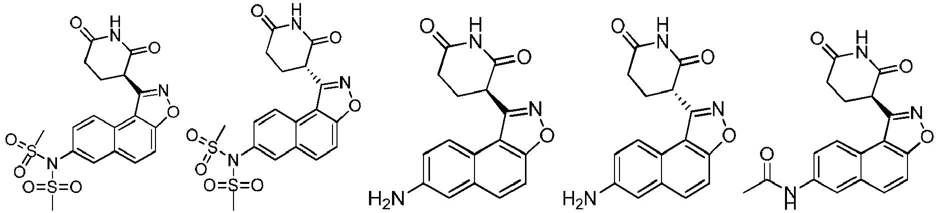

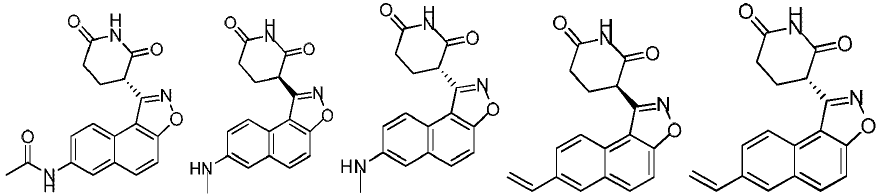

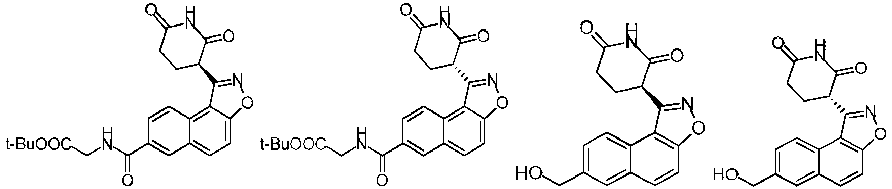

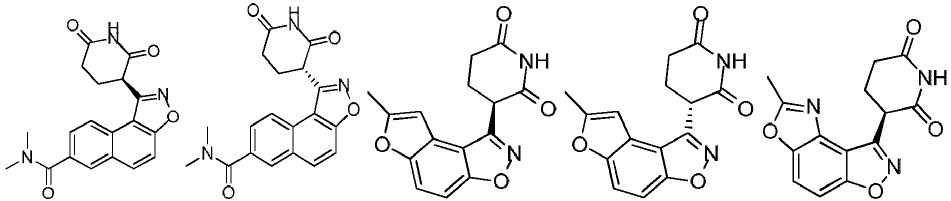

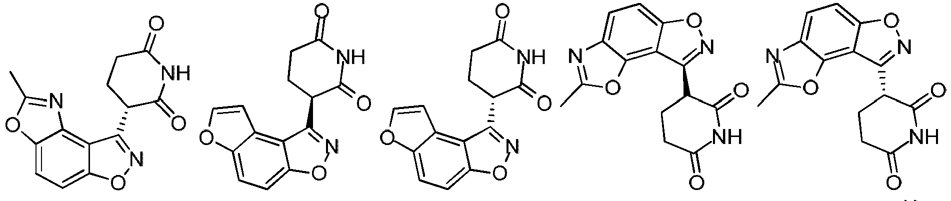

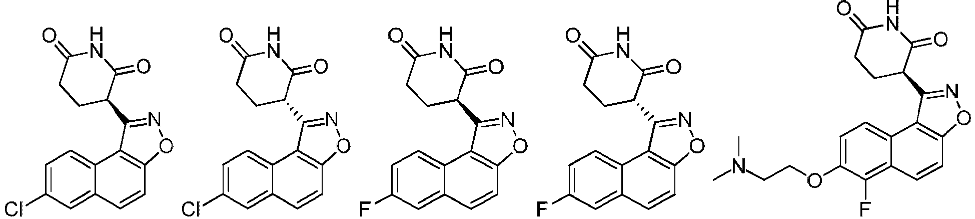

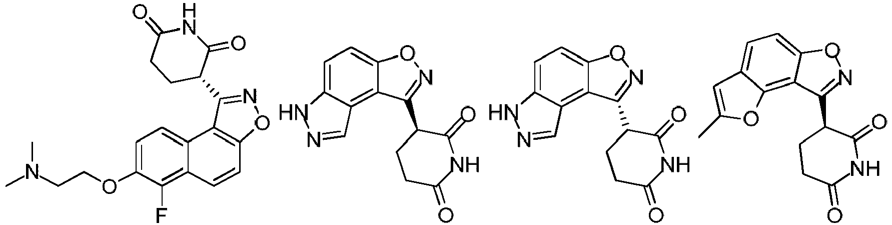

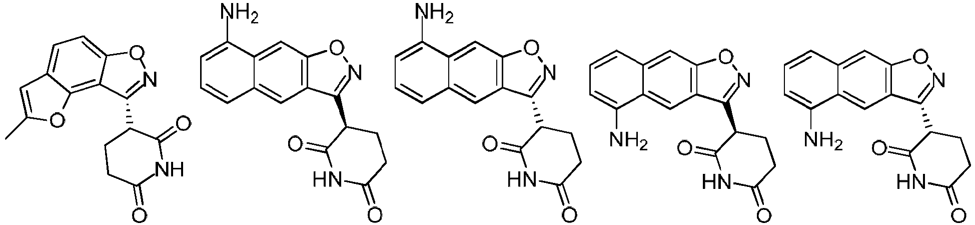

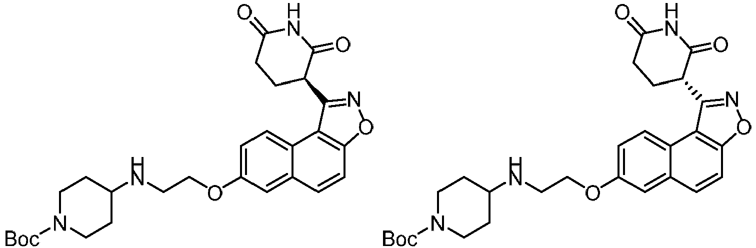

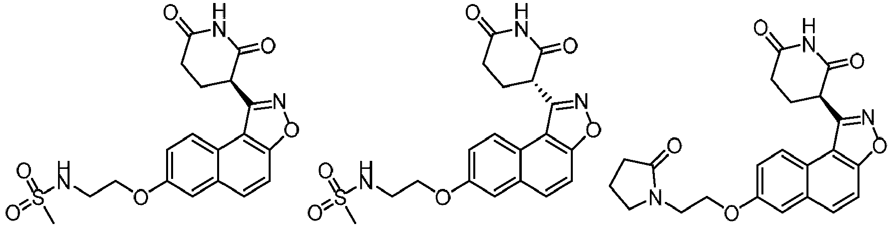

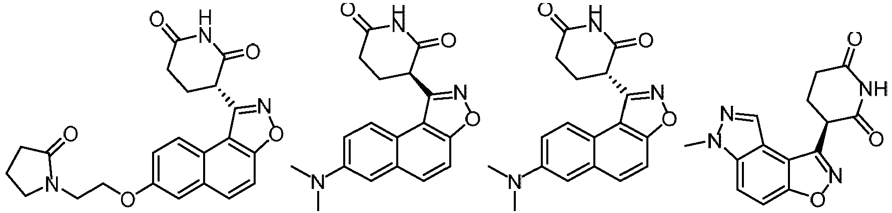

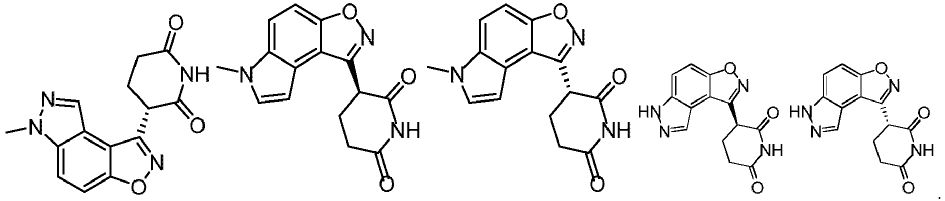

The present invention also provides a pharmaceutical composition comprising a therapeutically effective amount of the compound or the pharmaceutically acceptable salt thereof as an active ingredient, and a pharmaceutically acceptable carrier.

The present invention also provides use of the compound or the pharmaceutically acceptable salt thereof and the pharmaceutical composition in preparing a medicament for treating a disease related to CRBN protein.

The present invention also provides use of the compound or the pharmaceutically acceptable salt thereof and the pharmaceutical composition in treating a disease related to CRBN protein.

The present invention also provides a method for treating a disease related to CRBN protein, comprising administering a therapeutically effective amount of the compound or the pharmaceutically acceptable salt thereof and the pharmaceutical composition.

The present invention also provides the compound or the pharmaceutically acceptable salt thereof and the pharmaceutical composition for treating a disease related to CRBN protein.

The disease related to CRBN protein described herein is multiple myeloma.

### DEFINITIONS AND DESCRIPTION

Unless otherwise stated, the following terms and phrases used herein are intended to have the following meanings. A particular term or phrase, unless otherwise specifically defined, should not be considered as uncertain or unclear, but construed according to its common meaning. When referring to a trade name, it is intended to refer to its corresponding commercial product or its active ingredient.

The term "pharmaceutically acceptable" is used herein for those compounds, materials, compositions, and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" refers to a salt of the compound disclosed herein, which is prepared from the compound having particular substituents disclosed herein and a relatively nontoxic acid or base. When the compound of the present invention contains a relatively acidic functional group, a base addition salt can be given by contacting the neutral form of such a compound with a sufficient amount of a base in a pure solution or a suitable inert solvent. Pharmaceutically acceptable base addition salts include sodium, potassium, calcium, ammonium, organic amine, or magnesium salts, or similar salts. When the compound of the present invention contains a relatively basic functional group, an acid addition salt can be given by contacting the neutral form of such a compound with a sufficient amount of an acid in a pure solution or a suitable inert solvent.

The pharmaceutically acceptable salts of the present invention can be synthesized from a parent compound having an acidic or basic group by conventional chemical methods. In general, such salts are prepared by the following method: the free acid or base form of the compound reacting with a stoichiometric amount of the appropriate base or acid in water or an organic solvent or a mixture thereof.

The compound of the present invention can be in the form of a geometric isomer or stereoisomer. All such compounds are contemplated herein, including *cis-* and *trans*-isomers, (-)- and (+)-enantiomers, (*R*)- and (*S*)-enantiomers, diastereoisomers, (*D*)-isomers, (*L*)-isomers, and racemic mixtures and other mixtures thereof, such as an enantiomer or diastereoisomer enriched mixture, all of which are encompassed within the scope of the present invention. Substituents such as alkyl may have an additional asymmetric carbon atom. All these isomers and mixtures thereof are encompassed within the scope of the present invention.

Unless otherwise stated, the term "enantiomer" or "optical isomer" refers to stereoisomers that are mirror images of each other.

Unless otherwise stated, the term "diastereoisomer" refers to stereoisomers in which molecules each have two or more chiral centers and are not mirror images of each other.

Unless otherwise stated, "(+)" stands for dextrorotation, "(-)" stands for levorotation, and "(±)" stands for racemization.

Unless otherwise stated, the absolute configuration of a stereogenic center is represented by a wedged solid bond ( 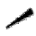 ) and a wedged dashed bond ( 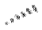 ), and the relative configuration of a stereogenic center is represented by a straight solid bond ( 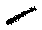 ) and a straight dashed bond ( 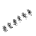 ). A wavy line ( 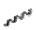 ) represents a wedged solid bond ( 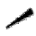 ) or a wedged dashed bond ( 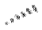 ), or a wavy line ( 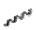 ) represents a straight solid bond ( 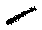 ) and a straight dashed bond ( 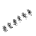 ).

The compound of the present invention may be present in a particular form. Unless otherwise stated, the term "tautomer" or "tautomeric form" means that different functional isomers are in dynamic equilibrium at room temperature and can be rapidly converted into each other. If tautomers are possible (*e.g*., in solution), the chemical equilibrium of the tautomers can be achieved. For example, a proton tautomer, also known as a prototropic tautomer, includes the interconversion by proton transfer, such as keto-enol isomerization and imine-enamine isomerization. A valence isomer includes the interconversion by recombination of some bonding electrons. A specific example of the keto-enol tautomerization is the interconversion between two tautomers pentane-2,4-dione and 4-hydroxypent-3-en-2-one.

Unless otherwise stated, the term "be rich in one isomer", "isomer enriched", "be rich in one enantiomer", or "enantiomer enriched" means that the content of one of the isomers or enantiomers is less than 100% and more than or equal to 60%, 70%, 80%, 90%, 95%, 96%, 97%, 98%, 99%, 99.5%, 99.6%, 99.7%, 99.8%, or 99.9%. Unless otherwise stated, the term "isomeric excess" or "enantiomeric excess" refers to the difference between the relative percentages of two isomers or enantiomers. For example, if the content of one isomer or enantiomer is 90% and the content of the other isomer or enantiomer is 10%, the isomeric or enantiomeric excess (ee) is 80%. Optically active (*R*)- and (*S*)-isomers and *D*- and *L*-isomers can be prepared by chiral synthesis or chiral reagents or other conventional techniques. An enantiomer of certain compound of the present invention can be prepared by asymmetric synthesis or derivatization using a chiral auxiliary, wherein the resulting diastereoisomeric mixture is separated and the auxiliary group is cleaved so as to give the desired pure enantiomer. Alternatively, when the molecule contains a basic functional group (such as amino) or an acidic functional group (such as carboxyl), the compound reacts with an appropriate optically active acid or base to form a salt of the diastereoisomer, which is then subjected to diastereoisomeric resolution through conventional methods in the art to get the pure enantiomer. Furthermore, the enantiomer and the diastereoisomer are generally isolated through chromatography using a chiral stationary phase, optionally in combination with chemical derivatization (*e.g.*, carbamate formation from amines). Unless otherwise specified, when a group has one or more connectable sites, any one or more of the sites of the group may be connected to other groups by chemical bonds. When there is no designated connecting mode for a chemical bond and H atoms are present at a connectable site, the number of the H atoms at the connectable site is correspondingly reduced based on the number of the connected chemical bonds, and a group with a corresponding valence number is thus formed. The chemical bond that connects the site to another group may be represented by a straight solid bond ( 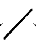 ), a straight dashed line bond ( 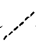 ), or a wavy line ( 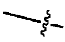 ). For example, the straight solid bond in -OCH₃ refers to being connected to another group via the oxygen atom in the group; the straight dashed bond in 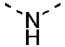 refers to being connected to another group via two ends of the nitrogen atom in the group; the wavy line in 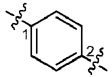 refers to being connected to another group via the carbon atoms at positions 1 and 2 in the phenyl group; 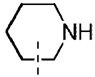 means that any connectable site on the piperidinyl can be connected to another group via 1 bond, and at least 4 connecting modes 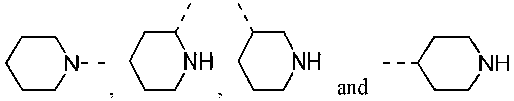 are possible; even if -N- is connected to a H atom, 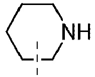 includes the connecting mode of 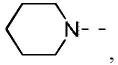 except that when 1 bond is connected to a site, the number of H at that site is correspondingly reduced by 1 and a monovalent piperidinyl is thus formed.

The compound of the present invention may contain an unnatural proportion of atomic isotope at one or more of the atoms that constitute the compound. For example, the compound may be labeled with a radioisotope, such as tritium (³H), iodine-125 (¹²⁵I), or C-14 (¹⁴C). For another example, hydrogen can be substituted by deuterium to form a deuterated drug, and the bond formed by deuterium and carbon is firmer than that formed by common hydrogen and carbon. Compared with an un-deuterated drug, the deuterated drug has the advantages of reduced toxic side effect, increased stability, enhanced efficacy, prolonged biological half-life and the like. All isotopic variations of the compound of the present invention, whether radioactive or not, are encompassed within the scope of the present invention. "Optional" or "optionally" means that the subsequently described event or circumstance may, but not necessarily, occur, and the description includes instances where the event or circumstance occurs and instances where it does not.

The term "substituted" means that one or more hydrogen atoms on a specific atom are substituted with substituents which may include deuterium and hydrogen variants, as long as the valence of the specific atom is normal and the compound after substitution is stable. When the substituent is oxygen (*i.e*., =O), it means that two hydrogen atoms are substituted. Substitution by oxygen does not occur on aromatic groups. The term "optionally substituted" means that an atom can be or cannot be substituted by a substituent. Unless otherwise specified, the type and number of the substituent may be arbitrary as long as being chemically achievable. When any variable (*e.g*., R) occurs more than once in the constitution or structure of a compound, the definition of the variable in each case is independent. Thus, for example, if a group is substituted by 0-2 R, the group can be optionally substituted by two R at most, and the definition of R in each case is independent. Furthermore, a combination of a substituent and/or a variant thereof is permissible only if the combination can result in a stable compound.

When the number of a connecting group is 0, for example, -(CRR)₀-, it means that the connecting group is a single bond.

When a substituent is absent, it means that the substituent does not exist. For example, when X is absent in A-X, the structure of A-X is actually A. When it is not specified by which atom the listed substituent is linked to the group to be substituted, the substituent can be linked via any atom of the group. For example, pyridinyl as a substituent can be linked to the group to be substituted through any carbon atom on the pyridine ring.

Unless otherwise specified, the term "C₁₋₁₀ alkyl" refers to a linear or branched saturated hydrocarbon group consisting of 1 to 10 carbon atoms. The C₁₋₁₀ alkyl includes C₁₋₁₀, C₁₋₉, C₁₋₈, C₁₋₆, C₁₋₅, C₁₋₄, C₁₋₃, C₁₋₂, C₂₋₆, C₂₋₄, C₁₀, C₈, C₇, C₆ and C₅ alkyl, and the like, and it may be monovalent (*e.g.,* methyl), divalent (*e.g.,* methylene), or polyvalent (*e.g*., methenyl). Examples of C₁₋₁₂ alkyl include, but are not limited to, methyl (Me), ethyl (Et), propyl (including *n*-propyl and isopropyl), butyl (including *n*-butyl, isobutyl, *s*-butyl, and *t*-butyl), pentyl (including *n*-pentyl, isopentyl, and neopentyl), hexyl, heptyl, octyl, and the like. Examples of C₁₋₆ alkyl include, but are not limited to, methyl (Me), ethyl (Et), propyl (including *n*-propyl and isopropyl), butyl (including *n*-butyl, isobutyl, *s*-butyl, and *t*-butyl), pentyl (including *n*-pentyl, isopentyl, and neopentyl), hexyl, and the like. Examples of C₁₋₃ alkyl include, but are not limited to, methyl (Me), ethyl (Et), propyl (including *n*-propyl and isopropyl), and the like.

Unless otherwise specified, "C₂₋₆ alkenyl" is used to denote a linear or branched hydrocarbon group consisting of 2 to 6 carbon atoms and containing at least one carbon-carbon double bond, which may be located anywhere in the group. The C₂₋₆ alkenyl includes C₂₋₄, C₂₋₃, C₄, C₃ and C₂ alkenyl, and the like, and may be monovalent, divalent or polyvalent. Examples of C₂₋₆ alkenyl include, but are not limited to, ethenyl, propenyl, butenyl, pentenyl, hexenyl, butadienyl, 1,3-pentadienyl, 1,3-hexadienyl, and the like. Examples of C₂₋₄ alkenyl include, but are not limited to, ethenyl, propenyl, butenyl, butadienyl, and the like. Examples of C₂₋₃ alkenyl include, but are not limited to, ethenyl, propenyl, and the like.

Unless otherwise specified, the term "C₁₋₁₀ alkoxy" refers to those alkyl groups that each contain 1 to 6 carbon atoms and are connected to the rest of the molecule via an oxygen atom. The C₁₋₁₀ alkoxy includes C₁₋₁₀, C₁₋₉, C₁₋₈, C₁₋₆, C₁₋₅, C₁₋₄, C₁₋₃, C₁₋₂, C₂₋₆, C₂₋₄, C₁₀, C₈, C₇, C₆ and C₅ alkoxy, and the like. Examples of C₁₋₁₀ alkoxy include, but are not limited to, methoxy, ethoxy, propoxy (including *n*-propoxy and isopropoxy), butoxy (including *n*-butoxy, isobutoxy, s-butoxy and *t*-butoxy), pentyloxy (including *n*-pentyloxy, isopentyloxy and neopentyloxy), hexyloxy, and the like. Examples of C₁₋₆ alkoxy include, but are not limited to, methoxy, ethoxy, propoxy (including *n*-propoxy and isopropoxy), butoxy (including *n*-butoxy, isobutoxy, *s*-butoxy and *t*-butoxy), pentyloxy (including *n*-pentyloxy, isopentyloxy and neopentyloxy), hexyloxy, and the like. Examples of C₁₋₃ alkoxy include, but are not limited to, methoxy, ethoxy, propoxy (including *n*-propoxy and isopropoxy), and the like.

Unless otherwise specified, the term "C₁₋₁₀ alkylamino" refers to those alkyl groups that each contain 1 to 10 carbon atoms and are connected to the rest of the molecule via an amino group. The C₁₋₁₀ alkylamino includes C₁₋₁₀, C₁₋₉, C₁₋₈, C₁₋₆, C₁₋₅, C₁₋₄, C₁₋₃, C₁₋₂, C₂₋₆, C₂₋₄, C₁₀, C₈, C₇, C₆ and C₅ alkylamino, and the like. Examples of C₁₋₁₀ alkylamino include, but are not limited to, -NHCH₃, -N(CH₃)₂, -NHCH₂CH₃, -N(CH₃)CH₂CH₃, -N(CH₂CH₃)(CH₂CH₃), -NHCH₂CH₂CH₃, -NHCH₂(CH₃)₂, -NHCH₂CH₂CH₂CH₃, and the like. Examples of C₁₋₆ alkylamino include, but are not limited to, -NHCH₃, -N(CH₃)₂, -NHCH₂CH₃, -N(CH₃)CH₂CH₃, -N(CH₂CH₃)(CH₂CH₃), -NHCH₂CH₂CH₃, -NHCH₂(CH₃)₂, -NHCH₂CH₂CH₂CH₃, and the like. Examples of C₁₋₅ alkylamino include, but are not limited to, -NHCH₃, -N(CH₃)₂, -NHCH₂CH₃, -N(CH₃)CH₂CH₃, -N(CH₂CH₃)(CH₂CH₃), -NHCH₂CH₂CH₃, -NHCH₂(CH₃)₂, -NHCH₂CH₂CH₂CH₃, and the like. Examples of C₁₋₃ alkylamino include, but are not limited to, -NHCH₃, -N(CH₃)₂, -NHCH₂CH₃, -N(CH₃)CH₂CH₃, -NHCH₂CH₂CH₃, -NHCH₂(CH₃)₂, and the like.

Unless otherwise specified, "C₃₋₁₀ cycloalkyl" refers to a saturated cyclic hydrocarbon group consisting of 3 to 10 carbon atoms, and it may be a monocyclic, a bicyclic, or a tricyclic system, wherein the bicyclic and tricyclic systems include spirocyclic, fused and bridged rings. The C₃₋₁₀ cycloalkyl includes C₃₋₈, C₃₋₆, C₃₋₅, C₄₋₁₀, C₄₋₈, C₄₋₆, C₄₋₅, C₅₋₈ and C₅₋₆ cycloalkyl, and the like, and it may be monovalent, divalent, or polyvalent. Examples of C₃₋₁₀ cycloalkyl include, but are not limited to, cyclopropyl, cyclobutyl, cyclopentyl, cyclohexyl, cycloheptyl, norbornyl, [2.2.2]bicyclooctane, [4.4.0]bicyclodecane, and the like. Examples of C₃₋₈ cycloalkyl include, but are not limited to, cyclopropyl, cyclobutyl, cyclopentyl, cyclohexyl, cycloheptyl, norbornyl, [2.2.2]bicyclooctane, and the like. Examples of C₃₋₅ cycloalkyl include, but are not limited to, cyclopropyl, cyclobutyl, cyclopentyl, and the like. Examples of C₃₋₆ cycloalkyl include, but are not limited to, cyclopropyl, cyclobutyl, cyclopentyl, cyclohexyl, and the like.

Unless otherwise specified, the term "4-10 membered heterocycloalkyl", by itself or in combination with other terms, refers to a saturated cyclic group consisting of 4 to 10 ring atoms, of which 1, 2, 3, or 4 ring atoms are heteroatoms independently selected from the group consisting of O, S, and N, with the remaining being carbon atoms. The nitrogen atom is optionally quaternized, and the nitrogen and sulfur heteroatoms can be optionally oxidized (*i.e.,* NO and S(O)ₚ, where p is 1 or 2). This includes monocyclic, bicyclic and tricyclic systems, wherein the bicyclic and tricyclic systems include spirocyclic, fused and bridged rings. Furthermore, with respect to the "4-10 membered heterocycloalkyl", a heteroatom may occupy the position where the heterocycloalkyl is connected to the rest of the molecule. The 4-10 membered heterocycloalkyl includes 4-8 membered, 4-6 membered, 4-5 membered, 5-6 membered, 4 membered, 5 membered and 6 membered heterocycloalkyl, and the like. Examples of 4-10 membered heterocycloalkyl include, but are not limited to, azetidinyl, oxetanyl, thietanyl, pyrrolidinyl, pyrazolidinyl, imidazolidinyl, tetrahydrothienyl (including tetrahydrothien-2-yl, tetrahydrothien-3-yl, *etc*.), tetrahydrofuranyl (including tetrahydrofuran-2-yl, *etc*.), tetrahydropyranyl, piperidinyl (including 1-piperidinyl, 2-piperidinyl, 3-piperidinyl, *etc*.), piperazinyl (including 1-piperazinyl, 2-piperazinyl, *etc*.), morpholinyl (including 3-morpholinyl, 4-morpholinyl, *etc*.), dioxanyl, dithianyl, isoxazolidinyl, isothiazolidinyl, 1,2-oxazinyl, 1,2-thiazinyl, hexahydropyridazinyl, homopiperazinyl, homopiperidinyl, dioxepanyl, or the like. Unless otherwise specified, the term "4-7 membered heterocycloalkyl", by itself or in combination with other terms, refers to a saturated cyclic group consisting of 4 to 7 ring atoms, of which 1, 2, 3, or 4 ring atoms are heteroatoms independently selected from the group consisting of O, S, and N, with the remaining being carbon atoms. The nitrogen atom is optionally quaternized, and the nitrogen and sulfur heteroatoms can be optionally oxidized (*i.e.,* NO and S(O)ₚ, where p is 1 or 2). This includes monocyclic and bicyclic systems, wherein the bicyclic system includes spirocyclic, fused, and bridged rings. Furthermore, with respect to the "4-7 membered heterocycloalkyl", a heteroatom may occupy the position where the heterocycloalkyl is connected to the rest of the molecule. The 4-7 membered heterocycloalkyl includes 4-6 membered, 4-5 membered, 4-7 membered, 5-6 membered, 4 membered, 5 membered and 6 membered heterocycloalkyl, and the like. Examples of 4-7 membered heterocycloalkyl include, but are not limited to, azetidinyl, oxetanyl, thietanyl, pyrrolidinyl, pyrazolidinyl, imidazolidinyl, tetrahydrothienyl (including tetrahydrothien-2-yl, tetrahydrothien-3-yl, *etc*.), tetrahydrofuranyl (including tetrahydrofuran-2-yl, *etc*.), tetrahydropyranyl, piperidinyl (including 1-piperidinyl, 2-piperidinyl, 3-piperidinyl, *etc*.), piperazinyl (including 1-piperazinyl, 2-piperazinyl, *etc*.), morpholinyl (including 3-morpholinyl, 4-morpholinyl, *etc*.), dioxanyl, dithianyl, isoxazolidinyl, isothiazolidinyl, 1,2-oxazinyl, 1,2-thiazinyl, hexahydropyridazinyl, homopiperazinyl, homopiperidinyl, dioxepanyl, or the like.

Unless otherwise specified, the terms "5-6 membered heteroaromatic ring" and "5-6 membered heteroaryl" can be used interchangeably herein. The term "5-6 membered heteroaryl" refers to a monocyclic group which consists of 5 to 6 ring atoms and has a conjugated pi-electron system, of which 1, 2, 3 or 4 ring atoms are heteroatoms independently selected from the group consisting of O, S, and N, the others being carbon atoms. The nitrogen atom is optionally quaternized and the nitrogen and sulfur heteroatoms are optionally oxidized (*i.e.,* NO and S(O)ₚ, where p is 1 or 2). The 5-6 membered heteroaryl can be connected to the rest of the molecule via a heteroatom or a carbon atom. The 5-6 membered heteroaryl includes 5-membered and 6-membered heteroaryl. Examples of the 5-6 membered heteroaryl include, but are not limited to, pyrrolyl (including *N*-pyrrolyl, 2-pyrrolyl, 3-pyrrolyl, *etc*.), pyrazolyl (including 2-pyrazolyl, 3-pyrazolyl, *etc*.), imidazolyl (including *N*-imidazolyl, 2-imidazolyl, 4-imidazolyl, 5-imidazolyl, *etc*.), oxazolyl (including 2-oxazolyl, 4-oxazolyl, 5-oxazolyl, *etc*.), triazolyl (including 1*H*-1,2,3-triazolyl, 2*H-*1,2,3-triazolyl, 1*H*-1,2,4-triazolyl, 4*H*-1,2,4-triazolyl, *etc*.), tetrazolyl, isoxazolyl (3-isoxazolyl, 4-isoxazolyl, 5-isoxazolyl, *etc*.), thiazolyl (including 2-thiazolyl, 4-thiazolyl, 5-thiazolyl, *etc*.), furanyl (including 2-furanyl, 3-furanyl, *etc.*), thienyl (including 2-thienyl, 3-thienyl, *etc*.), pyridinyl (including 2-pyridinyl, 3-pyridinyl, 4-pyridinyl, *etc*.), pyrazinyl, or pyrimidinyl (including 2-pyrimidinyl, 4-pyrimidinyl, *etc*.).

Unless otherwise specified, the term "4-7 membered heterocycloalkenyl", by itself or in combination with other terms, refers to a partially unsaturated cyclic group containing at least one carbon-carbon double bond and consisting of 4 to 7 ring atoms, of which 1, 2, 3, or 4 ring atoms are heteroatoms independently selected from the group consisting of O, S, and N, with the remaining being carbon atoms. The nitrogen atom is optionally quaternized, and the nitrogen and sulfur heteroatoms can be optionally oxidized (*i.e.,* NO and S(O)ₚ, where p is 1 or 2). This includes monocyclic, bicyclic and tricyclic systems, wherein the bicyclic and tricyclic systems include spirocyclic, fused and bridged rings, and any ring of these systems is nonaromatic. Furthermore, with respect to the "4-7 membered heterocycloalkenyl", a heteroatom may occupy the position where the heterocycloalkenyl is connected to the rest of the molecule. The 4-7 membered heterocycloalkenyl includes 5-7 membered, 5-6 membered, 4-5 membered, 4 membered, 5 membered and 6 membered heterocycloalkenyl, and the like. Examples of 4-8 membered heterocycloalkenyl include, but are not limited to 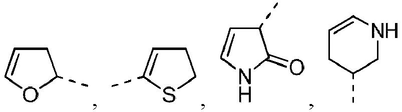 or

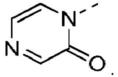

Unless otherwise specified, Cₙ₋ₙ₊ₘ or Cₙ-Cₙ₊ₘ includes any one of the specific cases of n to n+m carbons; for example, C₁₋₁₂ includes C₁, C₂, C₃, C₄, C₅, C₆, C₇, C₈, C₉, C₁₀, C₁₁ and C₁₂; Cₙ₋ₙ₊ₘ or Cₙ-Cₙ₊ₘ also includes any range in n to n+m; for example, C₁₊₁₂ includes C₁₋₃, C₁₋₆, C₁₋₉, C₃₋₆, C₃₋₉, C₃₋₁₂, C₆₋₉, C₆₋₁₂, C₉₋₁₂, *etc.* Similarly, n-n+m membered represents the number of atoms on the ring is n to n+m; for example, 3-12 membered ring includes 3 membered ring, 4 membered ring, 5 membered ring, 6 membered ring, 7 membered ring, 8 membered ring, 9 membered ring , 10 membered ring, 11 membered ring and 12 membered ring; n-n+m membered also represents any range in n to n+m; for example, 3-12 membered ring includes 3-6 membered ring, 3-9 membered ring, 5-6 membered ring, 5-7 membered ring, 6-7 membered ring, 6-8 membered ring, 6-10 membered ring, *etc.* Unless otherwise specified, the term "halo" or "halogen", by itself or as part of another substituent, refers to a fluorine, chlorine, bromine or iodine atom.

The compounds of the present invention can be prepared by a variety of synthetic methods well known to those skilled in the art, including the specific embodiments listed below, embodiments formed by combinations thereof with other chemical synthetic methods, and equivalents thereof known to those skilled in the art. Preferred embodiments include, but are not limited to, the examples disclosed herein.

The solvent used in the present invention can be commercially available. The following abbreviations are used in the present invention: aq for water; HATU for O-(7-azabenzotriazol-l-yl)-*N*,*N,N',N'*-tetramethyluronium hexafluorophosphate; EDC for *N*-(3-dimethylaminopropyl)-*N'*-ethylcarbodiimide hydrochloride; m-CPBA for 3-chloroperoxybenzoic acid; eq for equivalent; CDI for carbonyldiimidazole; DCM for dichloromethane; PE for petroleum ether; DIAD for diisopropyl azodicarboxylate; DMF for *N,N-*dimethylfortnamide; DMSO for dimethyl sulfoxide; EtOAc for ethyl acetate; EtOH for ethanol; MeOH for methanol; CBz for benzyloxycarbonyl, an amine protecting group; BOC for t-butoxycarbonyl, an amine protecting group; HOAc for acetic acid; NaCNBH₃ for sodium cyanoborohydride; r.t. for room temperature; O/N for overnight; THF for tetrahydrofuran; Boc₂O for di-*tert*-butyl dicarbonate; TFA for trifluoroacetic acid; DIPEA for diisopropylethylamine; SOCl₂ for thionyl chloride; CS₂ for carbon disulphide; TsOH for *p*-toluenesulfonic acid; NFSI for *N*-fluoro-*N*-(phenylsulfonyl) benzenesulfonamide; *n*-Bu₄NF for tetrabutylammonium fluoride; iPrOH for 2-propanol; mp for melting point; LDA for lithium diisopropylamide; M for mol/L.

Compounds are named according to conventional nomenclature rules in the art or using ChemDraw® software, and supplier's catalog names are given for commercially available compounds.

### TECHNICAL EFFECTS

The compounds of the present invention exhibit obvious down-regulation effect on the IKZF3 protein level in multiple myeloma cells MM. 1S, and excellent inhibition against cell proliferation in multiple myeloma cell lines MM.1S and NCI-H929. In addition, the compounds of the present invention have low plasma clearance and high oral bioavailability, showing excellent pharmacokinetic properties. The compounds of the present invention also exhibit obvious tumor reduction effect on a human myeloma MM.1S model.

### BRIEF DESCRIPTION OF THE DRAWINGS

FIG. 1 shows the changes in intracellular IKZF3 protein levels assayed by WB after treatment of multiple myeloma cells MM.1S with compounds WX001-WX019 of the present invention at concentrations of 50 nM and 500 nM.
FIG. 2 shows the changes in intracellular IKZF3 protein levels assayed by WB after treatment of multiple myeloma cells MM.1S with compounds WX020-WX038, WX042-WX046, WX054, WX056-WX058, WX063, WX065, WX069 and WX071-WX073 of the present invention at concentrations of 50 nM and 500 nM.
FIG. 3 shows the changes in intracellular IKZF3 protein levels assayed by WB after treatment of multiple myeloma cells MM.IS with compounds WX039-WX041, WX047-WX053, WX055, WX059-WX062, WX064, WX066-WX068, WX070 and WX074-WX079 of the present invention at concentrations of 50 nM and 500 nM.

### DETAILED DESCRIPTION

The present invention is described in detail below by way of examples. However, this is by no means disadvantageously limiting the scope of the present invention. Although the present invention has been described in detail herein and specific examples have also been disclosed, it will be apparent to those skilled in the art that various changes and modifications can be made to the specific examples without departing from the spirit and scope of the present invention.

### Example 1: WX001

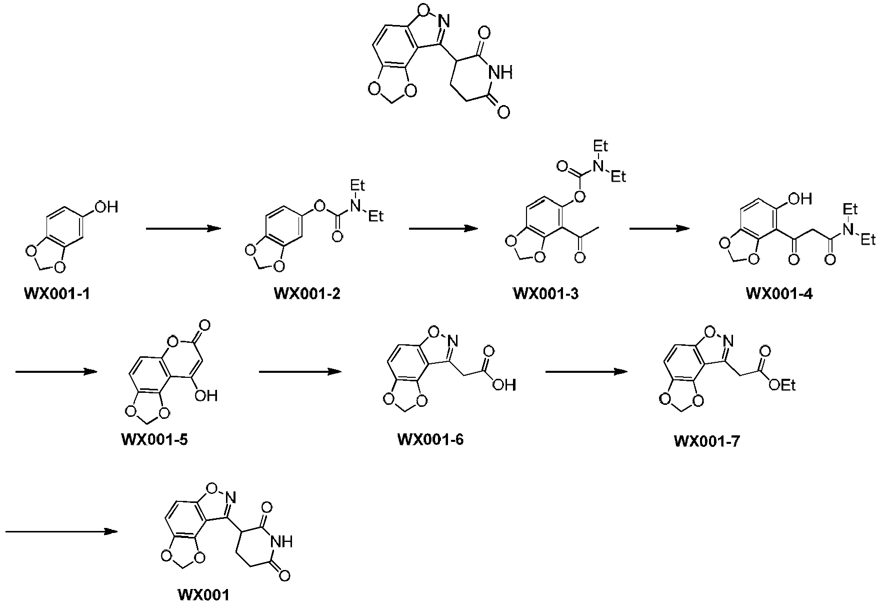

### Step 1: synthesis of intermediate WX001-2

**WX001-1** (2.01 g, 14.55 mmol) was dissolved in acetonitrile (20 mL) at room temperature, and then *N,N*-diethylchloroformamide (1.97 g, 14.55 mmol, 1.84 mL) and potassium carbonate (4.02 g, 29.11 mmol) were added. The reaction mixture was warmed to 100 °C and stirred for 14 h. After the reaction was completed, the reaction mixture was cooled to room temperature, the reaction was quenched with water (30 mL), and ethyl acetate (30 mL × 3) was added for extraction. The organic phases were combined, washed with saturated brine (60 mL × 2), dried over anhydrous sodium sulfate and filtered, and the filtrate was concentrated under reduced pressure to remove the solvent, thus giving target intermediate **WX001-2.** ¹H NMR (400 MHz, CDCl₃) δ: 6.76 (d, *J*=8.0 Hz, 1H), 6.65 (d, *J*=2.4 Hz, 1H), 6.55 (dd, *J*=2.0, 8.4 Hz, 1H), 5.97 (s, 2H), 3.42-3.37 (m, 4H), 1.28-1.17 (m, 6H).

### Step 2: synthesis of intermediate WX001-3

The intermediate **WX001-2** (2.43 g, 10.24 mmol) was dissolved in tetrahydrofuran (20 mL) in reaction flask 1 at -78 °C, and then a solution of *tert*-butyllithium in tetrahydrofuran (1.3 M, 9.45 mL) was added dropwise slowly. After the reaction mixture was stirred at -78 °C for 1 h, a solution of zinc chloride (1.40 g, 10.24 mmol) in tetrahydrofuran (20 mL) was added dropwise to the above reaction mixture, and the resulting reaction mixture was stirred at -78 °C for 2 h. Acetyl chloride (804.00 mg, 10.24 mmol, 730.91 µL) and bis(triphenylphosphine)palladium(II) dichloride (718.90 mg, 1.02 mmol) were added to tetrahydrofuran (20 mL) in reaction flask 2 at room temperature. After the reaction mixture was cooled to 0 °C, a solution of diisobutylaluminum hydride in tetrahydrofuran (1 M, 10.24 mL) was added dropwise, and then the resulting reaction mixture was stirred at 0 °C for 1 h. Lastly, the reaction mixture in reaction flask 1 was added dropwise to reaction flask 2 at 0 °C, and the reaction mixture was warmed to room temperature and then stirred for 12 h. After the reaction was completed, saturated ammonium chloride solution (50 mL) was added and then ethyl acetate (50 mL × 3) was added for extraction. The organic phases were combined, washed with saturated brine (100 mL × 2), dried over anhydrous sodium sulfate and filtered, and the filtrate was concentrated under reduced pressure to remove the solvent. The resulting residue was separated by column chromatography (eluent: petroleum ether/ethyl acetate = 20/1-10/1, volume ratio) to give intermediate **WX001-3.** MS-ESI *m*/*z:* 280.1 [M+H]⁺. ¹H NMR (400 MHz, CDCl₃) δ: 6.85 (d, *J*=8.4 Hz, 1H), 6.57 (d, *J*=8.4 Hz, 1H), 6.07 (s, 2H), 3.48-3.40 (m, 2H), 3.38-3.32 (m, 2H), 2.55 (s, 3H), 1.27 (t, *J*=7.2 Hz, 3H), 1.19 (t, *J*=7.2 Hz, 3H).

### Step 3: synthesis of intermediate WX001-4

Intermediate **WX001-3** (996 mg, 3.52 mmol, purity: 98.75%) was dissolved in tetrahydrofuran (10 mL) at room temperature, and then sodium hydride (352.13 mg, 8.80 mmol, purity: 60%) was added in batches. The reaction mixture was warmed to 100 °C and stirred for 2 h. After the reaction was completed, the reaction mixture was cooled to room temperature, the reaction was quenched with water (30 mL), and ethyl acetate (30 mL × 3) was added for extraction. The organic phases were combined, washed with saturated brine (60 mL × 2), dried over anhydrous sodium sulfate and filtered, and the filtrate was concentrated under reduced pressure to remove the solvent. The resulting residue was separated by column chromatography (eluent: petroleum ether/ethyl acetate = 5/1-3/1, volume ratio) to give target intermediate **WX001-4.** ¹H NMR (400 MHz, CDCl₃) δ: 11.54 (s, 1H), 6.95 (d, *J*=8.8 Hz, 1H), 6.43 (d, *J*=8.8 Hz, 1H), 6.01 (s, 2H), 4.06 (s, 2H), 3.43 (q, *J*=7.2 Hz, 2H), 3.30 (q, *J*=7.2 Hz, 2H), 1.24-1.21 (m, 3H), 1.19-1.16 (m, 3H).

### Step 4: synthesis of intermediate WX001-5

Intermediate **WX001-4** (762 mg, 2.73 mmol) was dissolved in toluene (8 mL) at room temperature, and then trifluoroacetic acid (933.29 mg, 8.19 mmol, 606.03 µL) was added dropwise to the above solution. The reaction mixture was warmed to 100 °C and stirred for 16 h. After the reaction was completed, the reaction mixture was cooled to room temperature and then filtered. The filter cake was collected and dried under reduced pressure to give intermediate **WX001-5.** MS-ESI *m*/*z*: 207.0 [M+H]⁺. ¹H NMR (400 MHz, DMSO_*d*₆) δ: 12.44 (s, 1H), 7.18 (d, *J*=8.4 Hz, 1H), 6.81 (d, *J*=8.4 Hz, 1H), 6.16 (s, 2H), 5.50 (s, 1H).

### Step 5: synthesis of intermediate WX001-6

Intermediate **WX001-5** (525 mg, 2.29 mmol, purity: 90.10%) was dissolved in ethanol (10.00 mL) at room temperature, and then hydroxylamine hydrochloride (478.35 mg, 6.88 mmol) and sodium acetate (564.67 mg, 6.88 mmol) were added. The reaction mixture was warmed to 80 °C and stirred for 48 h. After the reaction was completed, the reaction mixture was cooled to room temperature and concentrated under reduced pressure to remove the solvent ethanol. Water (10 mL) was added to the resulting residue, and then diluted hydrochloric acid (4 M, 8 mL) was added to adjust the pH to 1-2 and ethyl acetate (20 mL × 3) was added for extraction. The organic phases were combined, washed with saturated brine (50 mL × 2), dried over anhydrous sodium sulfate and filtered, and the filtrate was concentrated under reduced pressure to remove the solvent. The resulting residue was slurried with petroleum ether/ethyl acetate (10:1, 5 mL), stirred at room temperature for 0.5 h and filtered. After being washed with petroleum ether (2 mL), the filter cake was collected and dried under reduced pressure to give intermediate **WX001-6**.MS-ESI *m*/*z*: 221.9 [M+H]⁺.

### Step 6: synthesis of intermediate WX001-7

Intermediate **WX001-6** (427 mg, 543.68 µmol, purity: 28.16%) was dissolved in ethanol (10 mL) at room temperature, and then concentrated sulfuric acid (920.00 mg, 9.19 mmol, 0.5 mL, purity: 98%) was added. The reaction mixture was warmed to 75 °C and stirred for 14 h. After the reaction was completed, the reaction mixture was cooled to room temperature and concentrated under reduced pressure to remove the solvent ethanol, and the residue was dissolved in dichloromethane (10 mL). The organic phase was washed with water (10 mL × 2), then washed with saturated sodium bicarbonate solution (20 mL), dried over anhydrous sodium sulfate and filtered. The filtrate was concentrated under reduced pressure to remove the solvent, thus giving intermediate **WX001-7**.MS-ESI *m*/*z*: 250.0 [M+H]⁺.

### Step 7: synthesis of compound WX001

Intermediate **WX001-7** (103 mg, 282.28 µmol, purity: 68.30%) was dissolved in *N,N*-dimethylformamide (5.00 mL) at 0 °C under nitrogen atmosphere, and then potassium *tert*-butoxide (31.67 mg, 282.28 µmol) was added. After the reaction mixture was stirred at 0 °C for 0.5 h, acrylamide (20.06 mg, 282.28 µmol) was added, and the reaction mixture was warmed to room temperature and stirred for 1 h. After the reaction was completed, water (50 mL) was added and then ethyl acetate (50 mL × 3) was added for extraction. The organic phases were combined, washed with saturated brine (80 mL × 2), dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) and then separated by chromatography plate (developing solvent: petroleum ether/ethyl acetate = 1/1, volume ratio) to give compound **WX001.** MS-ESI *m*/*z:* 275.1 [M+H]⁺. ¹H NMR (400 MHz, MeOD_*d*₄) δ: 7.20 (d, *J*=8.8 Hz, 1H), 7.10 (d, *J*=8.8 Hz, 1H), 6.12 (dd, *J*=1.2, 14.8 Hz, 2H), 4.58 (s, 1H), 4.44 (dd, *J*=5.2, 12.4 Hz, 1H), 2.91-2.82 (m, 1H), 2.78-2.72 (m, 1H), 2.58-2.47 (m, 1H), 2.32-2.25 (m, 1H).

### Example 2: WX002

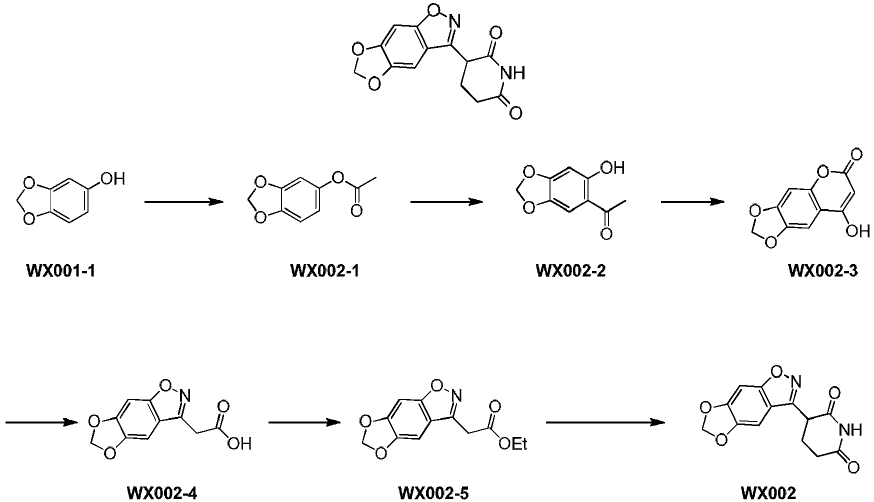

### Step 1: synthesis of intermediate WX002-1

**WX001-1** (6.05 g, 43.80 mmol) and triethylamine (6.65 g, 65.70 mmol, 9.15 mL) were dissolved in dichloromethane (80 mL) at room temperature under nitrogen atmosphere. After the reaction mixture was cooled to 0 °C, acetyl chloride (4.13 g, 52.56 mmol, 3.75 mL) was added. The resulting reaction mixture was warmed to room temperature and stirred at room temperature for 2 h. After the reaction was completed, the reaction mixture was slowly poured into ice water (300 mL) and then dichloromethane (200 mL × 3) was added for extraction. The organic phases were combined, washed with saturated brine (200 mL × 2), dried over anhydrous sodium sulfate and filtered, and the filtrate was concentrated under reduced pressure to remove the solvent. The resulting residue was separated by column chromatography (eluent: petroleum ether/ethyl acetate = 50/1-20/1, volume ratio) to give target intermediate **WX002-1.** ¹H NMR (400 MHz, CDCl₃) δ: 6.79 (d, *J*=8.4 Hz, 1H), 6.62 (d, *J*=2.4 Hz, 1H), 6.54 (dd, *J*=2.4, 8.4 Hz, 1H), 6.00 (s, 2H), 2.29 (s, 3H).

### Step 2: synthesis of intermediate WX002-2

Intermediate **WX002-1** (3.02 g, 16.76 mmol) was dissolved in a mixture of acetic acid (5 mL) and boron trifluoride diethyl etherate (5 mL) at room temperature under nitrogen atmosphere, and then the reaction mixture was heated to 100 °C and stirred at 100 °C for 0.5 h. After the reaction was completed, the reaction mixture was cooled to room temperature and adjusted to pH 9-10 with saturated aqueous sodium bicarbonate solution (80 mL), and ethyl acetate (60 mL × 3) was then added for extraction. The organic phases were combined, washed with water (60 mL × 2), dried over anhydrous sodium sulfate and filtered, and the filtrate was concentrated under reduced pressure to remove the solvent. The resulting residue was separated by column chromatography (eluent: petroleum ether/ethyl acetate = 100/1-10/1, volume ratio) to give target intermediate **WX002-2.** MS-ESI *m*/*z*: 181.0 [M+H]⁺.¹H NMR (400 MHz, CDCl₃) δ: 12.96 (s, 1H), 6.98 (s, 1H), 6.38 (s, 1H), 5.92 (s, 2H), 2.45 (s, 3H).

### Step 3: synthesis of intermediate WX002-3

Intermediate **WX002-2** (0.808 g, 3.91 mmol, purity: 87.23%) was dissolved in diethyl carbonate (15 mL) at 0 °C under nitrogen atmosphere, and then sodium hydride (625.96 mg, 15.65 mmol, purity: 60%) was added. The reaction mixture was heated to 130 °C and stirred at 130 °C for 5 h. After the reaction was completed, the reaction mixture was cooled to room temperature and poured into ice water (50 mL), and methyl *tert*-butyl ether (20 mL × 3) was added for extraction. The organic phase was discarded, and the aqueous phase was adjusted to pH = 3-4 with 2 M aqueous diluted hydrochloric acid solution (5 mL). The reaction mixture was filtered, the filtrate was discarded, and the filter cake was concentrated under reduced pressure to remove the solvent. The resulting residue was slurried with petroleum ether/ethyl acetate (10:1, 11 mL, volume ratio), filtered, and washed with petroleum ether (20 mL) to give intermediate **WX002-3.** MS-ESI *m*/*z*: 206.9 [M+H]⁺.¹H NMR (400 MHz, DMSO_*d*₆) δ: 12.38 (s, 1H), 7.18 (s, 1H), 7.06 (s, 1H), 6.16 (s, 2H), 5.47 (s, 1H).

### Step 4: synthesis of intermediate WX002-4

Intermediate **WX002-3** (1.17 g, 5.38 mmol), hydroxylamine hydrochloride (727.42 mg, 10.47 mmol) and sodium acetate (858.67 mg, 10.47 mmol) were dissolved in ethanol (20.00 mL) at room temperature under nitrogen atmosphere, and then the reaction mixture was heated to 80 °C and stirred at 80 °C for 24 h. After the reaction was completed, the reaction mixture was cooled to room temperature and concentrated under reduced pressure to remove ethanol. Water (50 mL) was added to dilute the resulting residue, and then 2 M aqueous diluted hydrochloric acid solution was added to adjust the pH to 1-2 and ethyl acetate (30 mL × 3) was added for extraction. The organic phases were combined, washed with saturated brine (30 mL × 2), dried over anhydrous sodium sulfate and filtered. The filtrate was concentrated under reduced pressure to remove the solvent, and the resulting residue was slurried with petroleum ether/ethyl acetate (10:1, 50 mL, volume ratio) and washed with petroleum ether (20 mL) to give intermediate **WX002-4.** ¹H NMR (400 MHz, DMSO_*d*₆) δ: 12.79 (s, 1H), 7.35 (s, 1H), 7.21 (s, 1H), 6.18 (s, 2H), 3.97 (s, 2H).

### Step 5: synthesis of intermediate WX002-5

Intermediate **WX002-4** (1.21 g, 3.64 mmol, purity: 66.6%) was dissolved in ethanol (20 mL) at room temperature, and then concentrated sulfuric acid (364.67 mg, 3.64 mmol, 198.19 µL, purity: 98%) was added. The reaction mixture was heated to 80 °C and stirred at 80 °C for 12 h. After the reaction was completed, the reaction mixture was cooled to room temperature. Ice water (50 mL) was added, and ethyl acetate (30 mL × 3) was added for extraction. The organic phases were combined, washed with saturated brine (20 mL × 2), dried over anhydrous sodium sulfate and filtered, and the filtrate was concentrated under reduced pressure to remove the solvent. The resulting residue was separated by column chromatography (eluent: petroleum ether/ethyl acetate = 10/1-5/1, volume ratio) to give intermediate **WX002-5.** MS-ESI *m*/*z:* 250.1 [M+H]⁺. ¹H NMR (400 MHz, DMSO_*d*₆) δ: 7.36 (s, 1H), 7.22 (s, 1H), 6.18 (s, 2H), 4.14 (q, *J*=7.0 Hz, 2H), 4.08 (s, 2H), 1.20 (t, *J*=7.0 Hz, 3H).

### Step 6: synthesis of intermediate WX002

Intermediate **WX002-5** (0.485 g, 1.92 mmol, purity: 98.71%) was dissolved in *N,N*-dimethylformamide (10 mL) at 0 °C under nitrogen atmosphere, and then potassium *tert*-butoxide (215.55 mg, 1.92 mmol) and acrylamide (273.08 mg, 3.84 mmol) were added sequentially. The reaction mixture was stirred at 0 °C for 2 h. After the reaction was completed, water (50 mL) was added for dilution and then ethyl acetate (50 mL × 3) was added for extraction. The organic phases were combined, washed with saturated brine (30 mL × 2), dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give compound **WX002.** MS-ESI *m*/*z:* 275.0 [M+H]⁺. ¹H NMR (400 MHz, DMSO_*d*₆) δ: 11.05 (s, 1H), 7.37 (s, 1H), 7.27 (s, 1H), 6.18 (s, 2H), 4.47 (dd, *J*=5.0, 11.8 Hz, 1H), 2.81-2.69 (m, 1H), 2.63-2.53 (m, 1H), 2.49-2.40 (m, 1H), 2.22-2.10 (m, 1H).

### Example 3: WX003

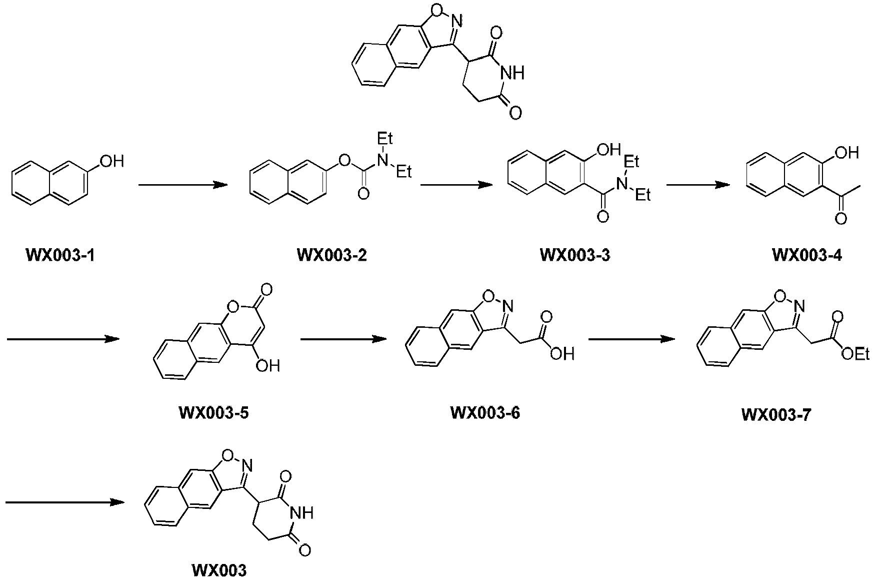

### Step 1: synthesis of intermediate WX003-2

**WX003-1** (50 g, 346.81 mmol) and diethylcarbamoyl chloride (70.54 g, 520.22 mmol) were dissolved in acetonitrile (500 mL) at room temperature, and then potassium carbonate (71.90 g, 520.22 mmol) was added. The reaction mixture was stirred at room temperature for 12 h. After the reaction was completed, the reaction mixture was filtered, and the filtrate was concentrated under reduced pressure to remove the solvent. The resulting residue was separated by column chromatography (eluent: petroleum ether/ethyl acetate = 1/0-3/1, volume ratio) to give target intermediate **WX003-2.** ¹H NMR (400 MHz, CDCl₃) δ: 7.90-7.77 (m, 3H), 7.62 (d, *J*=2.2 Hz, 1H), 7.52-7.42 (m, 2H), 7.32 (dd, *J*=2.3, 8.8 Hz, 1H), 3.59-3.33 (m, 4H), 1.41-1.19 (m, 6H).

### Step 2: synthesis of intermediate WX003-3

Intermediate **WX003-2** (10 g, 41.10 mmol) was dissolved in tetrahydrofuran (100 mL) at room temperature under nitrogen atmosphere. After the reaction mixture was cooled to -78 °C, a mixed solution of lithium diisopropylamide in tetrahydrofuran and *n*-heptane (2 M, 24.66 mL) was added slowly. The reaction mixture was warmed to room temperature and stirred for 12 h. After the reaction was completed, a solution of crude intermediate **WX003-3** in tetrahydrofuran (124 mL) was obtained and used directly in next step.

### Step 3: synthesis of intermediate WX003-4

A mixed solution of lithium diisopropylamide tetrahydrofuran and *n*-heptane (2 M, 46.03 mL) was added to a solution of crude intermediate **WX003-3** in tetrahydrofuran (32.64 mmol, 99 mL) at -78 °C. After the reaction mixture was heated to 0 °C, trimethylchlorosilane (4.29 g, 39.46 mmol, 5.01 mL) was added. The reaction mixture was stirred at 0 °C for 15 min, and then warmed to room temperature and stirred for another 15 min. After the reaction was completed, the reaction mixture was poured into ice water (100 mL). 2 M diluted hydrochloric acid was added to adjust the pH to 6-7 and ethyl acetate (50 mL × 3) was added for extraction. The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 1/0-20/1, volume ratio) to give target intermediate **WX003-4.** ¹H NMR (400 MHz, CDCl₃) δ: 11.57 (s, 1H), 8.35 (s, 1H), 7.82 (d, *J*=8.0 Hz, 1H), 7.68 (d, *J*=8.4 Hz, 1H), 7.52 (dt, *J*=1.0, 7.6 Hz, 1H), 7.39-7.31 (m, 1H), 7.28 (s, 1 H), 2.79 (s, 3H).

### Step 4: synthesis of intermediate WX003-5

Intermediate **WX003-4** (3.8 g, 20.41 mmol) was dissolved in toluene (80 mL) at room temperature under nitrogen atmosphere, and then sodium hydride (3.26 g, 81.63 mmol, purity: 60%) and diethyl carbonate (9.64 g, 81.63 mmol, 9.89 mL) were added sequentially. The reaction mixture was heated to 120 °C and stirred for 16 h. After the reaction was completed, the reaction mixture was cooled to room temperature, and then ice water (100 mL) was added and ethyl acetate (50 mL) was added for dilution. The organic phase was removed after liquid separation, and the aqueous phase was extracted with ethyl acetate (30 mL × 2) and the organic phase was removed. The aqueous phase was adjusted to pH = 5-6 with 2 M diluted hydrochloric acid and yellowish solid was generated. After filtration, the solid was collected and dried in vacuum to give target intermediate **WX003-5.** ¹H NMR (400MHz, DMSO_*d*₆) δ: 8.46 (s, 1H), 8.11 (d, *J*=8.4 Hz, 1H), 7.98 (d, *J*=8.0 Hz, 1H), 7.84 (s, 1H), 7.67-7.60 (m, 1H), 7.56-7.43 (m, 1H), 5.69 (s, 1H).

### Step 5: synthesis of intermediate WX003-6

Intermediate **WX003-5** (2.2 g, 10.37 mmol) was dissolved in ethanol (30 mL) at room temperature under nitrogen atmosphere, and then hydroxylamine hydrochloride (2.52 g, 36.29 mmol) and sodium acetate (2.98 g, 36.29 mmol) were added sequentially. The reaction mixture was heated to 80 °C and stirred for 7 h. After the reaction was completed, the reaction mixture was cooled to room temperature and concentrated under reduced pressure to remove the solvent. The resulting residue was added to saturated sodium bicarbonate solution (100 mL) and then filtered. The filtrate was adjusted to pH 3-4 with 1 M diluted hydrochloric acid, and ethyl acetate (50 mL × 3) was added for extraction. The organic phases were combined, dried over anhydrous sodium sulfate and filtered, and the filtrate was concentrated under reduced pressure to remove the solvent, thus giving intermediate **WX003-6.** ¹H NMR (400 MHz, DMSO_*d*₆) δ: 12.99 (br s, 1H), 8.53 (s, 1H), 8.20 (s, 1H), 8.16 (d, *J*=8.4 Hz, 1H), 8.09 (d, *J*=8.4 Hz, 1H), 7.63 (ddd, *J*=1.2, 6.8, 8.2 Hz, 1H), 7.53 (ddd, *J*=1.2, 6.8, 8.2 Hz, 1H), 4.23 (s, 2H).

### Step 6: synthesis of intermediate WX003-7

Intermediate **WX003-6** (350 mg, 1.54 mmol) was dissolved in ethanol (10 mL) at room temperature under nitrogen atmosphere, and then concentrated sulfuric acid (644.00 mg, 6.57 mmol, 350.00 µL) was added. The reaction mixture was heated to 70 °C and stirred for 5 h. After the reaction was completed, the reaction mixture was cooled to room temperature and concentrated under reduced pressure to remove the solvent, and water (50 mL) and ethyl acetate (30 mL) were added to the resulting residue for dilution. The organic phase was collected after liquid separation, and the aqueous phase was extracted with ethyl acetate (20 mL × 2). The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 1/0-5/1, volume ratio) to give intermediate **WX003-7.** ¹H NMR (400 MHz, CDCl₃) δ: 8.28 (s, 1H), 8.05-7.95 (m, 3H), 7.65-7.54 (m, 1H), 7.52-7.44 (m, 1H), 4.25 (q, *J*=7.2 Hz, 2H), 4.16 (s, 2H), 1.28 (t, *J*=7.1 Hz, 3H).

### Step 7: synthesis of intermediate WX003

Intermediate **WX003-7** (250 mg, 979.36 µmol) was dissolved in tetrahydrofuran (30 mL) at room temperature under nitrogen atmosphere, and then acrylamide (69.61 mg, 979.36 µmol) and potassium *tert*-butoxide (109.90 mg, 979.36 µmol) were added sequentially. The reaction mixture was stirred at room temperature for 1 h. After the reaction was completed, water (50 mL) and ethyl acetate (50 mL) were added to the resulting residue for dilution. The organic phase was collected after liquid separation, and the aqueous phase was extracted with ethyl acetate (20 mL × 5). The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give target compound **WX003.** MS-ESI *m*/*z*: 281.0 [M+H]⁺.¹H NMR (400 MHz, DMSO_*d*₆) δ: 11.18 (s, 1H), 8.59 (s, 1H), 8.23 (s, 1H), 8.11 (dd, *J*=8.6, 11.4 Hz, 2H), 7.64 (t, *J*=7.0 Hz, 1H), 7.57-7.50 (m, 1H), 4.74 (dd, *J*=5.0, 11.8 Hz, 1H), 2.92-2.77 (m, 1H), 2.76-2.60 (m, 2H), 2.36-2.22 (m, 1H).

### Example 4: WX004

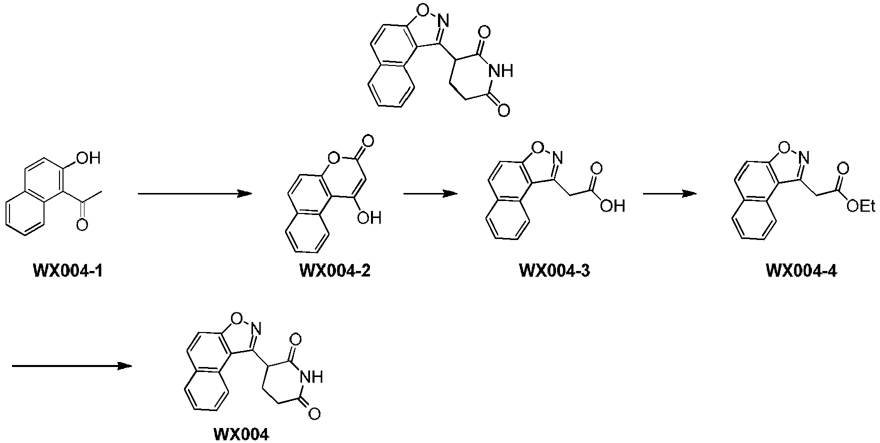

### Step 1: synthesis of intermediate WX004-2

**WX004-1** (9 g, 48.33 mmol) and diethyl carbonate (80 mL) were dissolved in toluene (80 mL) at room temperature. After the reaction mixture was cooled to 0 °C, sodium hydride (9.67 g, 241.67 mmol, purity: 60%) was added slowly in batches. The resulting reaction mixture was then heated to 120 °C and stirred at 120 °C for 12 h. After the reaction was completed, the reaction mixture was cooled to room temperature and poured into ice water (200 mL). Ethyl acetate (200 mL × 2) was added for extraction, and the organic phase was discarded. The aqueous phase was adjusted to pH = 1-2 with concentrated hydrochloric acid and extracted with ethyl acetate (200 mL × 3). The organic phases were combined, dried over anhydrous sodium sulfate and filtered, and the filtrate was concentrated under reduced pressure to remove the solvent, thus giving target intermediate **WX004-2.**

### Step 2: synthesis of intermediate WX004-3

Intermediate **WX004-2** (7 g, 32.99 mmol) was dissolved in methanol (80 mL) at room temperature, and then sodium bicarbonate (9.70 g, 115.46 mmol) and hydroxylamine hydrochloride (8.02 g, 115.46 mmol) were added sequentially. The reaction mixture was heated to 75 °C and stirred at 75 °C for 3 h. After the reaction was completed, the reaction mixture was cooled to room temperature and concentrated under reduced pressure to remove the solvent. The resulting residue was added to 1 M aqueous sodium hydroxide solution (50 mL). Ethyl acetate (50 mL) was added for extraction, and the organic phase was discarded. The aqueous phase was adjusted to pH = 2 with concentrated hydrochloric acid and extracted with ethyl acetate (30 mL × 3). The organic phases were combined, dried over anhydrous sodium sulfate and filtered, and the filtrate was concentrated under reduced pressure to remove the solvent, thus giving intermediate **WX004-3.** ¹H NMR (400MHz, DMSO_*d*₆) δ: 13.03 (br s, 1H), 8.26-8.09 (m, 3H), 7.91 (d, *J*=8.8 Hz, 1H), 7.76 (dt, *J*=1.4, 7.6 Hz, 1H), 7.67-7.58 (m, 1H), 4.39 (s, 2H).

### Step 3: synthesis of intermediate WX004-4

Intermediate **WX004-3** (3.5 g, 15.40 mmol) was dissolved in ethanol (50 mL) at room temperature, and then concentrated sulfuric acid (6.44 g, 64.35 mmol, 3.50 mL, purity: 98%) was added. The reaction mixture was heated to 90 °C and stirred at 90 °C for 2 h. After the reaction was completed, the reaction mixture was cooled to room temperature and concentrated under reduced pressure to remove the solvent. Water (30 mL) was added to dilute the resulting residue, and then aqueous sodium hydroxide solution (1N) was added to adjust the pH to 9 and ethyl acetate (30 mL × 3) was added for extraction. The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 30/1-20/1, volume ratio) to give intermediate **WX004-4.** ¹H NMR (400MHz, DMSO_*d*₆) δ: 8.24-8.08 (m, 3H), 7.92 (d, *J*=8.8 Hz, 1H), 7.75 (ddd, *J*=1.3, 7.1, 8.3 Hz, 1H), 7.63 (dt, *J*=1.2, 7.5 Hz, 1H), 4.50 (s, 2H), 4.15 (q, *J*=7.2 Hz, 2H), 1.16 (t, *J*=7.1 Hz, 3H).

### Step 4: synthesis of intermediate WX004

Intermediate **WX004-4** (3.4 g, 13.32 mmol) was dissolved in tetrahydrofuran (100 mL) at room temperature. After the reaction mixture was cooled to 0 °C, acrylamide (946.71 mg, 13.32 mmol) and potassium *tert*-butoxide (1.49 g, 13.32 mmol) were added. The resulting reaction mixture was warmed to room temperature and stirred at room temperature for 3 h. After the reaction was completed, the reaction was quenched with water (200 mL), and ethyl acetate (200 mL) was added for extraction. The organic phase was washed with saturated brine (150 mL), dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give target compound **WX004.** MS-ESI *m*/*z:* 281.1 [M+H]⁺. ¹H NMR (400MHz, DMSO_*d*₆) δ: 11.16 (s, 1H), 8.29-8.12 (m, 3H), 7.94 (d, *J*=8.8 Hz, 1H), 7.79-7.72 (m, 1H), 7.69-7.60 (m, 1H), 5.07 (dd, *J*=4.4, 11.6 Hz, 1H), 2.92-2.77 (m, 1H), 2.72-2.54 (m, 2H), 2.42-2.27 (m, 1H).

### Example 5: WX005

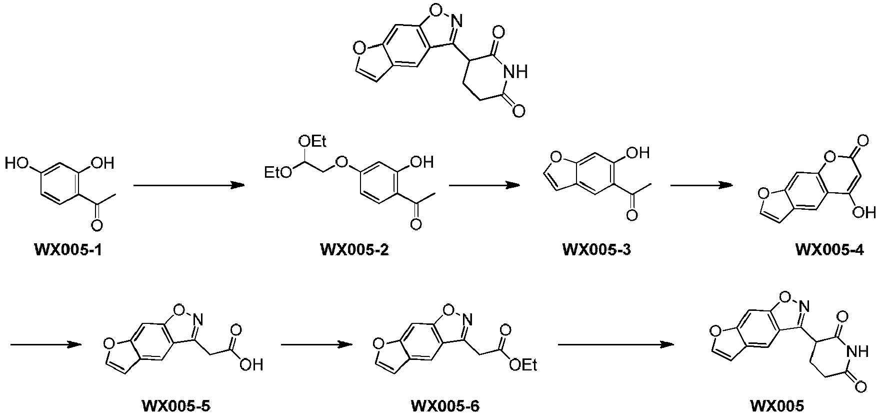

### Step 1: synthesis of intermediate WX005-2

**WX005-1** (20 g, 131.45 mmol, 16.95 mL) was dissolved in *N,N*-dimethylformamide (300 mL) at room temperature under nitrogen atmosphere, and then potassium carbonate (18.17 g, 131.45 mmol) and bromoacetaldehyde diethyl acetal (25.91 g, 131.45 mmol, 19.77 mL) were added sequentially. The reaction mixture was heated to 100 °C and stirred at 100 °C for 16 h. After the reaction was completed, the reaction mixture was cooled to room temperature. Water (400 mL) was added for dilution, and a mixed solution of petroleum ether/ethyl acetate (volume ratio: 3/1, 100 mL × 4) was added for extraction. The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 1/0-4/1, volume ratio) to give target intermediate **WX005-2.** ¹H NMR (400MHz, CDCl₃) δ: 12.70 (s, 1H), 7.64 (d, *J*=8.8 Hz, 1H), 6.48 (dd, *J*=2.4, 8.8 Hz, 1H), 6.43 (d, *J*=2.4 Hz, 1H), 4.84 (t, *J*=5.2 Hz, 1H), 4.04 (d, *J*=5.3 Hz, 2H), 3.77 (qd, *J*=7.1, 9.4 Hz, 2H), 3.64 (qd, *J*=7.0, 9.4 Hz, 2H), 2.56 (s, 3H), 1.26 (t, *J*=7.2 Hz, 6H).

### Step 2: synthesis of intermediate WX005-3

Intermediate **WX005-2** (14 g, 52.18 mmol) was dissolved in toluene (200 mL) at room temperature under nitrogen atmosphere, and then polyphosphoric acid (14 g) was added. The reaction mixture was heated to 120 °C and stirred at 120 °C for 20 min. After the reaction was completed, the reaction mixture was cooled to room temperature. The liquid supernatant was collected, and the lower suspension was poured into ice water (200 mL) and ethyl acetate (50 mL × 3) was added for extraction. The organic phases were combined, dried over anhydrous sodium sulfate and filtered. The filtrate was combined with the supernatant, and the solvent was remove under reduced pressure. The resulting residue was separated by column chromatography (eluent: petroleum ether/ethyl acetate = 1/0-95/1, volume ratio) to give target intermediate **WX005-3.** ¹H NMR (400MHz, CDCl₃) δ: 12.43 (s, 1H), 8.01 (s, 1H), 7.57 (d, *J*=2.4 Hz, 1H), 7.05 (s, 1H), 6.74 (dd, *J*=0.8, 2.0 Hz, 1H), 2.71 (s, 3H).

### Step 3: synthesis of intermediate WX005-4

Intermediate **WX005-3** (2.1 g, 11.92 mmol) was dissolved in toluene (30 mL) at room temperature under nitrogen atmosphere, and then diethyl carbonate (5.63 g, 47.68 mmol, 5.78 mL) was added, followed by further addition of sodium hydride (1.91 g, 47.68 mmol, purity: 60%) in batches. The reaction mixture was heated to 120 °C and stirred at 120 °C for 2.5 h. After the reaction was completed, the reaction mixture was cooled to room temperature and poured into ice water (100 mL). Ethyl acetate (50 mL × 3) was added for extraction, and the organic phase was discarded. The aqueous phase was adjusted to pH = 3-4 with 1 M diluted hydrochloric acid, and a large amount of solid was generated. After filtration, the filter cake was collected and dried in vacuum to give intermediate **WX005-4.** ¹H NMR (400MHz, DMSO_*d*₆) δ: 12.55 (br s, 1H), 8.11 (s, 1H), 8.09 (d, *J*=2.0 Hz, 1H), 7.67 (s, 1H), 7.09 (d, *J*=1.2 Hz, 1H), 5.58 (s, 1H).

### Step 4: synthesis of intermediate WX005-5

Intermediate **WX005-4** (2.1 g, 10.39 mmol) was dissolved in ethanol (30 mL) at room temperature under nitrogen atmosphere, and then hydroxylamine hydrochloride (2.53 g, 36.36 mmol) and sodium acetate (2.98 g, 36.36 mmol) were added sequentially. The reaction mixture was heated to 80 °C and stirred at 80 °C for 6 h. After the reaction was completed, the reaction mixture was cooled to room temperature and concentrated under reduced pressure to remove the solvent. The resulting residue was added to saturated aqueous sodium bicarbonate solution (100 mL) and then filtered. The filtrate was adjusted to pH 3-4 with 1 M aqueous diluted hydrochloric acid solution, and ethyl acetate (30 mL × 3) was added for extraction. The organic phases were combined, dried over anhydrous sodium sulfate and filtered, and the filtrate was concentrated under reduced pressure to remove the solvent, thus giving intermediate **WX005-5.** ¹H NMR (400MHz, DMSO_*d*₆) δ: 12.89 (br s, 1H), 8.11 (d, *J*=2.4 Hz, 1H), 8.06 (s, 1H), 7.98 (s, 1H), 7.13 (dd, *J*=0.8, 2.4 Hz, 1H), 4.13 (s, 2H).

### Step 5: synthesis of intermediate WX005-6

Intermediate **WX005-5** (400 mg, 1.84 mmol) was dissolved in ethanol (10 mL) at room temperature under nitrogen atmosphere, and then concentrated sulfuric acid (368.00 mg, 3.68 mmol, 0.2 mL, purity: 98%) was added. The reaction mixture was heated to 70 °C and stirred at 70 °C for 1 h. After the reaction was completed, the reaction mixture was cooled to room temperature and concentrated under reduced pressure to remove the solvent. The resulting residue was added to water (20 mL) and ethyl acetate (20 mL × 3) was added for extraction. The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 1/0-9/1, volume ratio) to give intermediate **WX005-6.** ¹H NMR (400MHz, CDCl₃) δ: 7.86 (s, 1H), 7.70 (d, *J*=2.0 Hz, 1H), 7.65 (s, 1H), 6.87 (dd, *J*=0.8, 2.4 Hz, 1H), 4.23 (q, *J*=7.2 Hz, 2H), 4.08 (s, 2H), 1.27 (t, *J*=7.0 Hz, 3H).

### Step 6: synthesis of intermediate WX005

Intermediate **WX005-6** (0.5 g, 2.04 mmol) was dissolved in tetrahydrofuran (20 mL) at room temperature under nitrogen atmosphere, and then acrylamide (144.92 mg, 2.04 mmol) and potassium *tert*-butoxide (228.79 mg, 2.04 mmol) were added. The reaction mixture was stirred at room temperature for 1.5 h. After the reaction was completed, the reaction mixture was poured into ice water (50 mL), and ethyl acetate (30 mL × 3) was added for extraction. The organic phases were combined, dried over anhydrous sodium sulfate and filtered, and the filtrate was concentrated under reduced pressure to remove the solvent. The resulting residue was added with *N*,*N*-dimethylformamide (3 mL), stirred at room temperature for 0.5 h and filtered. The filter cake was rinsed with acetonitrile (20 mL × 3), and then the filter cake was collected and dried in vacuum to give target compound **WX005.** MS-ESI *m*/*z:* 271.0 [M+H]⁺.¹H NMR (400MHz, DMSO_*d₆*) δ: 11.13 (s, 1H), 8.12 (d, *J*=2.0 Hz, 1H), 8.10 (s, 1H), 8.01 (s, 1H), 7.11 (d, *J*=2.0 Hz, 1H), 4.65 (dd, *J*=5.2, 11.6 Hz, 1H), 2.90-2.72 (m, 1H), 2.69-2.54 (m, 2H), 2.35-2.17 (m, 1H).

### Example 6: WX006

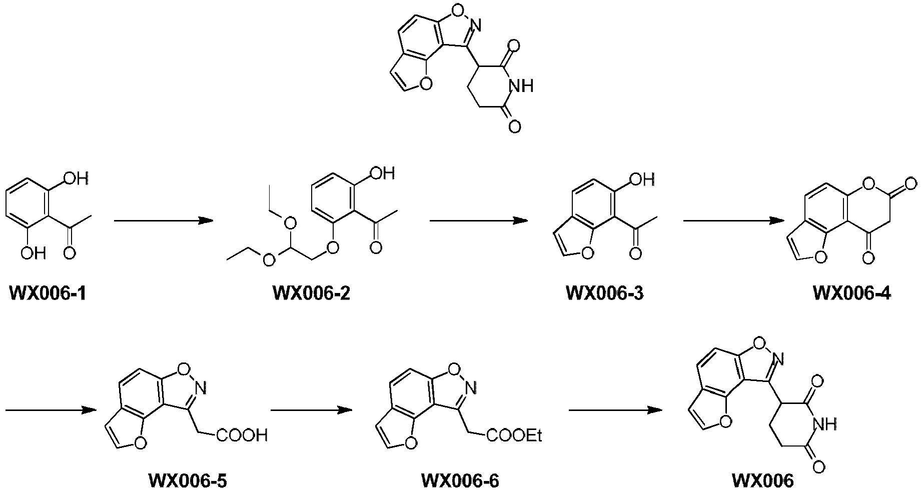

### Step 1: synthesis of intermediate WX006-2

**WX006-1** (30 g, 197.18 mmol) was dissolved in *N*,*N*-dimethylformamide (300 mL) at room temperature under nitrogen atmosphere, and then sodium hydride (7.89 g, 197.18 mmol, purity: 60%) was added in batches at 18-25 °C, and bromoacetaldehyde diethyl acetal (38.86 g, 197.18 mmol, 29.66 mL) was added lastly. The reaction mixture was heated to 100 °C and stirred at 100 °C for 36 h. After the reaction was completed, the reaction mixture was cooled to room temperature and poured into ice water (400 mL), and methyl *tert*-butyl ether (100 mL × 3) was added for extraction. The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 1/0-9/1, volume ratio) to give intermediate **WX006-2.** ¹H NMR (399 MHz, CDCl₃) δ: 13.25 (s, 1H), 7.33 (t, *J*=8.4 Hz, 1H), 6.58 (dd, *J*=1.2, 8.4 Hz, 1H), 6.36 (dd, *J*=0.8, 8.4 Hz, 1H), 4.93 (t, *J*=5.2 Hz, 1H), 4.07 (d, *J*=5.6 Hz, 2H), 3.81-3.73 (m, 2H), 3.67-3.59 (m, 2H), 2.73 (s, 3H), 1.26 (t, *J*=7.0 Hz, 6H).

### Step 2: synthesis of intermediate WX006-3

Intermediate **WX006-2** (5 g, 18.64 mmol) was dissolved in toluene (80 mL) at room temperature under nitrogen atmosphere, and then polyphosphoric acid (3 g) was added. The reaction mixture was placed directly in a preheated oil bath at 120 °C and stirred at 120 °C for 20 min. After the reaction was completed, the liquid supernatant was directly poured into a round-bottomed flask and concentrated under reduced pressure to remove the solvent. The resulting residue was separated by column chromatography (eluent: petroleum ether/ethyl acetate = 1/0-9/1, volume ratio) to give intermediate **WX006-3.** ¹H NMR (399 MHz, CDCl₃) δ: 12.86 (s, 1H), 7.67 (d, *J*=8.4 Hz, 1H), 7.63 (d, *J*=2.0 Hz, 1H), 6.91 (d, *J*=8.8 Hz, 1H), 6.77 (d, *J*=2.4 Hz, 1H), 2.92 (s, 3H).

### Step 3: synthesis of intermediate WX006-4

Intermediate **WX006-3** (1.3 g, 7.38 mmol) was dissolved in toluene (15 mL) at room temperature under nitrogen atmosphere, and then diethyl carbonate (14.63 g, 123.80 mmol, 15 mL) was added, and lastly, sodium hydride (885.43 mg, 22.14 mmol, purity: 60%) was added in batches at 0-15 °C. The reaction mixture was heated to 120 °C and stirred at 120 °C for 6 h. After the reaction was completed, the reaction mixture was cooled to room temperature and poured into ice water (100 mL), and ethyl acetate (30 mL × 3) was added for extraction. The organic phase was removed. The aqueous phase was adjusted to pH 2-3 with 1 M diluted hydrochloric acid, and yellowish solid was precipitated. 2-methyltetrahydrofuran (100 mL × 3) was added for extraction. The organic phases were combined, dried over anhydrous sodium sulfate and filtered, and the filtrate was concentrated under reduced pressure to remove the solvent, thus giving intermediate **WX006-4.** ¹H NMR (400 MHz, DMSO_*d₆*) δ: 12.63 (br s, 1H), 8.13 (d, *J*=2.0 Hz, 1H), 7.89 (d, *J*=8.4 Hz, 1H), 7.29 (d, *J*=8.4 Hz, 1H), 7.06 (d, *J*=2.4 Hz, 1H), 5.65 (s, 1H).

### Step 4: synthesis of intermediate WX006-5

Intermediate **WX006-4** (1.74 g, 8.61 mmol) was dissolved in ethanol (30 mL) at room temperature under nitrogen atmosphere, and then hydroxylamine hydrochloride (2.09 g, 30.12 mmol) and sodium acetate (2.47 g, 30.12 mmol) were added sequentially. The reaction mixture was heated to 80 °C and stirred at 80 °C for 5 h. After the reaction was completed, the reaction mixture was cooled to room temperature and concentrated under reduced pressure to remove most of the solvent. Water (100 mL) was added, the pH was adjusted to 3-4 with 1 M diluted hydrochloric acid, and solid was generated. Ethyl acetate (100 mL × 3) was added for extraction. The organic phases were combined, dried over anhydrous sodium sulfate and filtered, and the filtrate was concentrated under reduced pressure to remove the solvent, thus giving intermediate **WX006-5.**

### Step 5: synthesis of intermediate WX006-6

Intermediate **WX006-5** (1.5 g, 6.91 mmol) was dissolved in ethanol (20 mL) at room temperature under nitrogen atmosphere, and then sulfuric acid (552.00 mg, 5.52 mmol, 300.00 µL, purity: 98%) was added. The reaction mixture was heated to 70 °C and stirred at 70 °C for 2 h. After the reaction was completed, the reaction mixture was cooled to room temperature and concentrated under reduced pressure to remove most of the solvent, and water (50 mL) and ethyl acetate (50 mL) were added for dilution. The organic phase was collected after liquid separation, and the aqueous phase was extracted with ethyl acetate (30 mL × 2). The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 1/0-10/1, volume ratio) to give intermediate **WX006-6.** ¹H NMR (400 MHz, CDCl₃) δ: 7.76 (d, *J*=8.8 Hz, 1H), 7.71 (d, *J*=2.0 Hz, 1H), 7.50 (d, *J*=8.8 Hz, 1H), 6.92 (d, *J*=2.4 Hz, 1H), 4.25 (d, *J*=6.8 Hz, 2H), 4.24 (s, 2H), 1.23 (t, *J*=7.2 Hz, 3H).

### Step 6: synthesis of WX006

Intermediate **WX006-6** (300 mg, 1.22 mmol) was dissolved in tetrahydrofuran (15 mL) at room temperature, and then acrylamide (86.95 mg, 1.22 mmol, 84.42 µL) and potassium *tert*-butoxide (137.27 mg, 1.22 mmol) were added simultaneously. The reaction mixture was stirred at room temperature (10 °C) for 12 h. After the reaction was completed, water (100 mL) was added and then ethyl acetate (50 mL × 3) was added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give target compound **WX006.** MS-ESI *m*/*z:* 271.1 [M+H]⁺.¹H NMR (400 MHz, DMSO_*d₆*) δ: 11.22 (s, 1H), 8.15 (d, *J*=2.4 Hz, 1H), 7.98 (d, *J*=8.4 Hz, 1H), 7.71 (d, *J*=8.8 Hz, 1H), 7.18 (d, *J*=2.0 Hz, 1H), 4.71 (dd, *J*=4.8, 12.8 Hz, 1H), 2.96-2.87 (m, 1H), 2.70-2.66 (m, 1H), 2.60-2.55 (m, 1H), 2.33-2.22 (m, 1H).

### Example 7: WX007

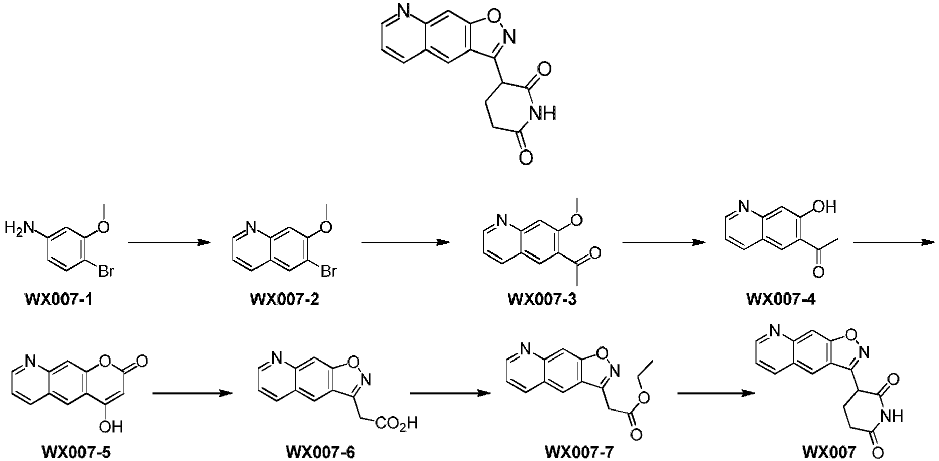

### Step 1: synthesis of intermediate WX007-2

Propane-1,2,3-triol (25.30 g, 274.69 mmol, 20.57 mL) was added to a solution of concentrated sulfuric acid (29.72 g, 296.96 mmol, 16.15 mL, purity: 98%) in water (15 mL) at room temperature under nitrogen atmosphere. The reaction mixture was heated to 110 °C, and then **WX007-1** (15 g, 74.24 mmol), concentrated sulfuric acid (15 mL, purity: 98%), water (15 mL) and propane-1,2,3-triol (15 mL) were added sequentially. The resulting reaction mixture was heated to 140 °C and stirred at 140 °C for 3 h. After the reaction was completed, the reaction mixture was cooled to room temperature and poured into ice water (300 mL). 2 N aqueous sodium hydroxide solution was added to adjust the pH to 8, and ethyl acetate (400 mL × 3) was added for extraction. The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 1/0-1/1, volume ratio) to give intermediate **WX007-2.** ¹H NMR (400 MHz, DMSO_*d₆*) δ: 8.85 (dd, *J*=1.8, 4.2 Hz, 1H), 8.31 (s, 1H), 8.25 (dd, *J*=1.6, 8.0 Hz, 1H), 7.50 (s, 1H), 7.41 (dd, *J*=4.4, 8.4 Hz, 1H), 4.00 (s, 3H).

### Step 2: synthesis of intermediate WX007-3

Intermediate **WX007-2** (8 g, 33.60 mmol) was dissolved in 1,4-dioxane (100 mL) at room temperature under nitrogen atmosphere, and then tributyl(1-ethoxyvinyl)stannane (18.20 g, 50.40 mmol, 17.01 mL) and bis(triphenylphosphine)palladium(II) dichloride (1.18 g, 1.68 mmol) were added sequentially. The reaction mixture was heated to 90 °C and stirred at 90 °C for 4 h. After the reaction mixture was cooled to room temperature (25 °C), diluted hydrochloric acid (2 M, 16.80 mL) was added, and the reaction mixture was stirred for 1 h. After the reaction was completed, an aqueous potassium fluoride solution (100 mL) and ethyl acetate (100 mL) were added, followed by filtration. The organic phase was collected after liquid separation, and the aqueous phase was extracted with ethyl acetate (100 mL × 2). The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 1/0-1/1, volume ratio) to give intermediate **WX007-3.** MS-ESI *m*/*z:* 202.2 [M+H]^{+ 1}H NMR (400 MHz, DMSO_*d₆*) δ: 8.89 (dd, *J*=1.6, 4.0 Hz, 1H), 8.39 (dd, *J*=1.2, 8.0 Hz, 1H), 8.17 (s, 1H), 7.51 (s, 1H), 7.42 (dd, *J*=4.2, 8.2 Hz, 1H), 4.01 (s, 3H), 2.61 (s, 3H).

### Step 3: synthesis of intermediate WX007-4

Intermediate **WX007-3** (2.4 g, 11.93 mmol) was dissolved in aqueous hydrobromic acid solution (50 mL) at room temperature under nitrogen atmosphere, and then the reaction mixture was heated to 120 °C and stirred at 120 °C for 12 h. After the reaction was completed, the reaction mixture was cooled to room temperature and adjusted to pH = 7 with 2 N aqueous sodium hydroxide solution, and ethyl acetate (100 mL × 3) was then added for extraction. The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 1/0-1/1, volume ratio) to give intermediate **WX007-4.** ¹H NMR (400 MHz, DMSO_*d₆*) δ: 11.43 (s, 1H), 8.88 (dd, *J*=1.6, 4.4 Hz, 1H), 8.59 (s, 1H), 8.39 (d, *J*=8.0 Hz, 1H), 7.38 (dd, *J*=4.2, 8.2 Hz, 1H), 7.36 (s, 1H), 2.76 (s, 3H).

### Step 4: synthesis of intermediate WX007-5

Intermediate **WX007-4** (0.8 g, 4.27 mmol) was dissolved in diethyl carbonate (9 mL) at room temperature under nitrogen atmosphere. After the reaction mixture was cooled to 0 °C, sodium hydride (854.73 mg, 21.37 mmol, purity: 60%) was added in batches. The reaction mixture was heated to 120 °C and stirred at 120 °C for 12 h. After the reaction was completed, the reaction mixture was cooled to room temperature and poured into ice water (200 mL). Ethyl acetate (100 mL × 2) was added for extraction, and the organic phase was discarded. The aqueous phase was concentrated under reduced pressure to remove water, thus giving intermediate **WX007-5.**

### Step 5: synthesis of intermediate WX007-6

Intermediate **WX007-5** (1.6 g, 7.51 mmol) was dissolved in methanol (30 mL) at room temperature under nitrogen atmosphere, and then sodium acetate (2.15 g, 26.27 mmol) and hydroxylamine hydrochloride (1.83 g, 26.27 mmol) were added sequentially. The reaction mixture was stirred at room temperature (25 °C) for 1.5 h, and then heated to 80 °C and stirred at 80 °C for 12 h. After the reaction was completed, the reaction mixture was cooled to room temperature and concentrated under reduced pressure to remove the solvent, thus giving intermediate **WX007-6.**

### Step 6: synthesis of intermediate WX007-7

Intermediate **WX007-6** (6 g, 26.29 mmol) was dissolved in ethanol (80 mL) at room temperature under nitrogen atmosphere, and then concentrated sulfuric acid (5 mL, purity: 98%) was added dropwise. The reaction mixture was heated to 90 °C and stirred at 90 °C for 12 h. After the reaction was completed, the reaction mixture was cooled to room temperature and concentrated under reduced pressure to remove the solvent. Water (50 mL) was added for dilution, saturated aqueous sodium bicarbonate solution was added to adjust the pH to 6-7, and ethyl acetate (100 mL × 3) was added for extraction. The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 1/0-5/1, volume ratio) to give intermediate **WX007-7.** ¹H NMR (400 MHz, DMSO_*d₆*) δ: 9.05 (dd, *J*=1.8, 4.2 Hz, 1H), 8.63 (d, *J*=8.4 Hz, 2H), 8.27 (s, 1H), 7.57 (dd, *J*=4.0, 8.4 Hz, 1H), 4.37 (s, 2H), 4.18 (q, *J*=7.2 Hz, 2H), 1.21 (t, *J*=7.0 Hz, 3H).

### Step 7: synthesis of WX007

Intermediate **WX007-7** (0.27 g, 1.05 mmol) was dissolved in tetrahydrofuran (8 mL) at 15 °C under nitrogen atmosphere, and then acrylamide (78.63 mg, 1.11 mmol) and a solution of potassium *tert*-butoxide in tetrahydrofuran (1 M, 1.11 mL) were added sequentially. The reaction mixture was stirred at 15 °C for 12 h, and then heated to 30 °C and stirred at 30 °C for 1 h. After the reaction was completed, the reaction mixture was cooled to room temperature. Water (15 mL) was added, and ethyl acetate (10 mL × 3) was added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give target compound **WX007.** MS-ESI *m*/*z:* 282.1 [M+H]⁺. ¹H NMR (400 MHz, DMSO_*d₆*) δ: 11.23 (s, 1H), 9.17 (dd, *J*=1.4, 4.2 Hz, 1H), 8.81 (s, 1H), 8.78 (d, *J*=8.4 Hz, 1H), 8.37 (s, 1H), 7.72 (dd, *J*=4.6, 8.6 Hz, 1H), 4.81 (dd, *J*=5.0, 12.2 Hz, 1H), 2.89-2.81 (m, 1H), 2.70-2.66 (m, 2H), 2.33-2.28 (m, 1H).

### Example 8: WX008

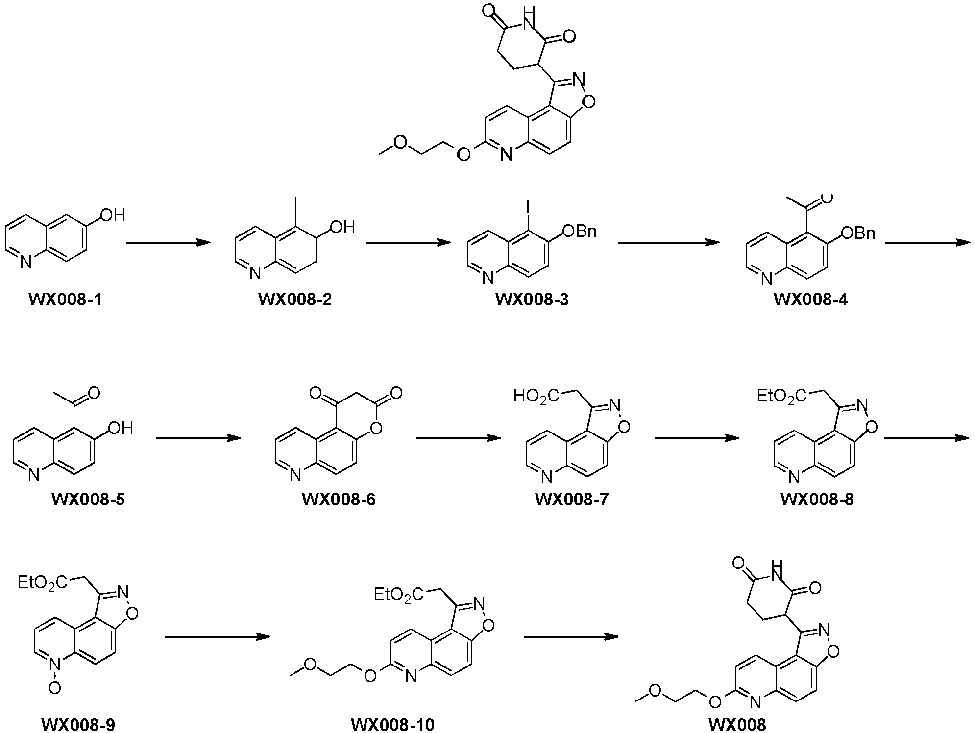

### Step 1: synthesis of intermediate WX008-2

**WX008-1** (10 g, 68.89 mmol) was dissolved in dichloromethane (100 mL) at room temperature, and then *N*-iodosuccinimide (15.50 g, 68.89 mmol) was added. The reaction mixture was stirred at 25 °C for 12 h and solid was precipitated. After the reaction was completed, the reaction mixture was filtered, and filter cake was collected and concentrated under reduced pressure to give intermediate **WX008-2.** ¹H NMR (400 MHz, DMSO_*d₆*) δ: 10.93 (s, 1H), 8.66 (dd, *J*=1.4, 4.2 Hz, 1H), 8.27 (d, *J*=8.0 Hz, 1H), 7.88 (d, *J*=8.8 Hz, 1H), 7.51 (dd, *J*=4.2, 8.4 Hz, 1H), 7.45 (d, *J*=9.2 Hz, 1H).

### Step 2: synthesis of intermediate WX008-3

Intermediate **WX008-2** (15 g, 55.34 mmol) was dissolved in *N*,*N*-dimethylformamide (150 mL) at room temperature under nitrogen atmosphere, and then potassium carbonate (22.94 g, 166.02 mmol) and benzyl bromide (11.36 g, 66.41 mmol) were added. The reaction mixture was heated to 60 °C and stirred for 12 h. After the reaction was completed, the reaction mixture was cooled to room temperature. Water (400 mL) was added, and ethyl acetate (300 mL × 2) was added for extraction. The organic phases were combined, washed with half-saturated brine (100 mL × 3), dried over anhydrous sodium sulfate and filtered, and the filtrate was concentrated under reduced pressure to remove the solvent. The resulting residue was separated by column chromatography (eluent: petroleum ether/ethyl acetate = 1/0-1/1, volume ratio) to give intermediate **WX008-3.**

### Step 3: synthesis of intermediate WX008-4

Intermediate **WX008-3** (20 g, 55.37 mmol) was dissolved in 1,4-dioxane (200 mL) at room temperature under nitrogen atmosphere, and then compounds tributyl(1-ethoxyvinyl)stannane (26.00 g, 71.99 mmol) and bis(triphenylphosphine)palladium(II) dichloride (2.73 g, 15.94 mmol) were added sequentially. The reaction mixture was heated to 90 °C and stirred for 12 h. After the reaction was completed, the reaction mixture was cooled to 25 °C and stirred for 1 h. Aqueous saturated potassium fluoride solution (200 mL) was then added and the reaction mixture was stirred for 1 h, followed by extraction with ethyl acetate (300 mL × 2). The organic phases were combined, dried over anhydrous sodium sulfate, filtered, and concentrated under reduced pressure to remove the solvent. The resulting residue was separated by column chromatography (eluent: petroleum ether/ethyl acetate = 1/0-0/1, volume ratio) to give intermediate **WX008-4.**

### Step 4: synthesis of intermediate WX008-5

Intermediate **WX008-4** (10 g, 36.06 mmol) was dissolved in trifluoroacetic acid (80 mL) at room temperature, and then the reaction mixture was heated to 80 °C and stirred for 12 h. After the reaction was completed, the reaction mixture was cooled to room temperature. Water (150 mL) was added, solid sodium carbonate was added to adjust the pH to 9, and ethyl acetate (200 mL × 3) was added for extraction. The organic phases were combined, dried over anhydrous sodium sulfate and filtered, and the filtrate was concentrated under reduced pressure to remove the solvent, thus giving intermediate **WX008-5.** ¹H NMR (400 MHz, CDCl₃) δ: 13.58 (s, 1H), 8.81 (dd, *J*=1.4, 4.2 Hz, 1H), 8.50 (d, *J*=8.8 Hz, 1H), 8.17 (d, *J*=9.2 Hz, 1H), 7.49 (dd, *J*=4.4, 8.8 Hz, 1H), 7.40 (d, *J*=9.2 Hz, 1H), 2.88 (s, 3H).

### Step 5: synthesis of intermediate WX008-6

Intermediate **WX008-5** (5.5 g, 29.38 mmol) was dissolved in diethyl carbonate (100 mL) at room temperature. After the reaction was cooled to 0 °C, sodium hydride (5.88 g, 146.91 mmol, purity: 60%) was added in batches. The reaction mixture was heated to 120 °C and stirred for 10 h. After the reaction was completed, the reaction mixture was cooled to room temperature and poured into ice water (200 mL). Ethyl acetate (150 mL × 2) was added for extraction, and the organic phase was discarded and the aqueous phase was concentrated under reduced pressure to remove water, thus giving intermediate **WX008-6.**

### Step 6: synthesis of intermediate WX008-7

Intermediate **WX008-6** (13.5 g, 63.32 mmol) was dissolved in ethanol (150 mL) at room temperature, and then sodium acetate (18.18 g, 221.63 mmol) and hydroxylamine hydrochloride (15.40 g, 221.63 mmol) were added. The reaction mixture was stirred at 15 °C for 1.5 h and then heated to 80 °C and stirred for 12 h. After the reaction was completed, the reaction mixture was cooled to room temperature, adjusted to pH = 5 with 3 N hydrochloric acid, and concentrated under reduced pressure to remove water, thus giving intermediate **WX008-7.**

### Step 7: synthesis of intermediate WX008-8

Intermediate **WX008-7** (34 g, 13.28 mmol) was dissolved in ethanol (500 mL) at room temperature, and then concentrated sulfuric acid (15 mL) was added. The reaction mixture was heated to 90 °C and stirred for 12 h. After the reaction was completed, the reaction mixture was cooled to room temperature and diluted with ethyl acetate (300 mL) and water (300 mL), and then sodium bicarbonate solid was added to adjust the pH to 7. Phases were separated and the aqueous phase was extracted with ethyl acetate (300 mL × 2). The organic phases were combined, washed with saturated brine (300 mL), dried over anhydrous sodium sulfate and filtered, and the filtrate was concentrated under reduced pressure to remove the solvent. The resulting residue was separated by column chromatography (eluent: petroleum ether/ethyl acetate = 1/0-0/1, volume ratio) to give intermediate **WX008-8.** ¹H NMR (400 MHz, DMSO_*d₆*) δ: 8.99 (dd, *J*=1.6, 4.4 Hz, 1H), 8.60-8.56 (m, 1H), 8.24 (d, *J*=9.2 Hz, 1H), 8.18 (d, *J*=9.2 Hz, 1H), 7.76 (dd, *J*=4.4, 8.4 Hz, 1H), 4.54 (s, 2H), 4.14 (q, *J*=7.2 Hz, 2H), 1.14 (t, *J*=7.2 Hz, 3H).

### Step 8: synthesis of intermediate WX008-9

Intermediate **WX008-8** (2.2 g, 8.59 mmol) was dissolved in dichloromethane (45 mL) at 0 °C, and then m-chloroperoxybenzoic acid (2.09 g, 10.30 mmol) was added. The reaction mixture was warmed to room temperature (15 °C) and stirred for 12 h. After the reaction was completed, the resulting reaction mixture was directly separated by column chromatography (eluent: dichloromethane/ethanol = 1/0-20/1, volume ratio), thus giving intermediate **WX008-9.**

¹H NMR (400 MHz, DMSO_*d₆*) δ: 8.83 (d, *J*=9.6 Hz, 1H), 8.68 (d, *J*=6.0 Hz, 1H), 8.21 (d, *J*=9.6 Hz, 1H), 8.07 (d, *J*=8.4 Hz, 1H), 7.71 (dd, *J*=6.4, 8.4 Hz, 1H), 4.55 (s, 2H), 4.15 (q, *J*=7.0 Hz, 2H), 1.16 (t, *J*=7.2 Hz, 3H).

### Step 9: synthesis of intermediate WX008-10

Intermediate **WX008-9** (0.5 g, 1.84 mmol) was dissolved in 2-methoxyethanol (4.19 g, 55.10 mmol, 4.34 mL) at room temperature, and then *p*-toluenesulfonyl chloride (451.67 mg, 2.37 mmol) was added. After the reaction mixture was cooled to 0 °C, triethylamine (464.59 mg, 4.59 mmol, 639.05 µL) was added, and the reaction mixture was stirred at room temperature (15 °C) for 16 h. After the reaction was completed, saturated sodium carbonate solution (10 mL) was added to the reaction mixture, and ethyl acetate (10 mL × 3) was added for extraction. The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 5/1-3/1, volume ratio), thus giving intermediate **WX008-10.**

### Step 10: synthesis of WX008

Intermediate **WX008-10** (0.3 g, 908.17 µmol) was dissolved in tetrahydrofuran (5 mL) at 0 °C under nitrogen atmosphere, and then acrylamide (64.55 mg, 908.17 µmοl, 62.67 µL) and potassium *tert*-butoxide (101.91 mg, 908.17 µmol) were added sequentially. The reaction mixture was stirred at room temperature (10 °C) for 14 h. After the reaction was completed, the reaction mixture was added into water (10 mL), and a mixed solution (ethyl acetate/2-methyltetrahydrofuran = 1/1) (10 mL × 3) was added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give target compound **WX008.** MS-ESI *m*/*z:* 356.1 [M+H]⁺. ¹H NMR (400 MHz, CDCl₃) δ: 8.34 (d, *J*=8.8 Hz, 1H), 8.24 (d, *J*=9.2 Hz, 1H), 8.02 (s, 1H), 7.90 (d, *J*=9.2 Hz, 1H), 7.22 (d, *J*=8.8 Hz, 1H), 4.76 (t, *J*=4.6 Hz, 2H), 4.67-4.58 (m, 1H), 3.85 (t, *J*=4.4 Hz, 2H), 3.48 (s, 3H), 3.10-2.99 (m, 1H), 2.87-2.72 (m, 2H), 2.62-2.56 (m, 1H).

### Example 9: WX009

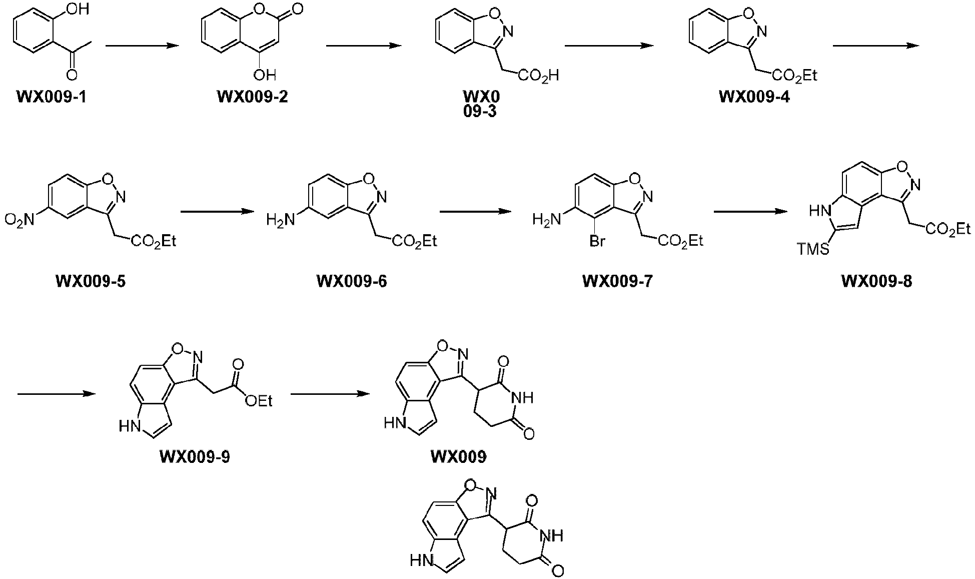

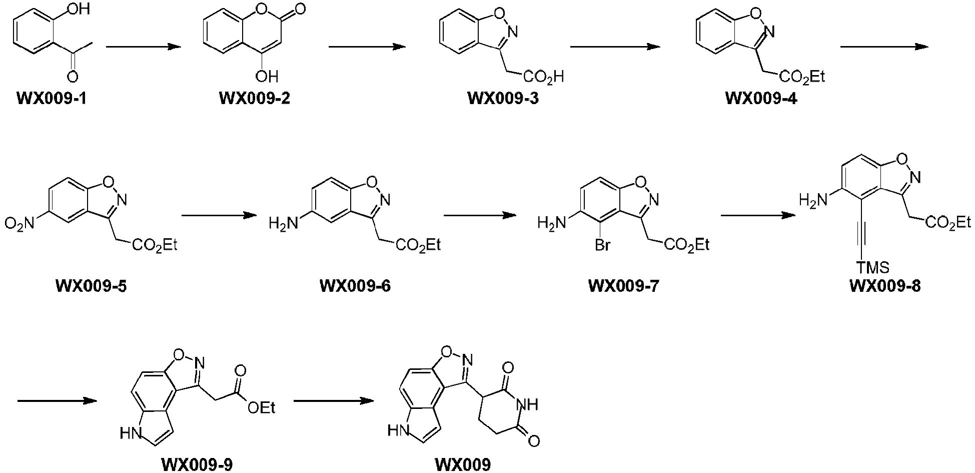

### Step 1: synthesis of intermediate WX009-2

**WX009-1** (25.00 g, 183.62 mmol) was dissolved in a mixed solution of diethyl carbonate (250 mL) and toluene (250 mL) at room temperature. After the reaction mixture was cooled to 0 °C, sodium hydride (36.72 g, 918.12 mmol, purity: 60%) was added in batches. The reaction mixture was heated to 120 °C and stirred for 12 h. After the reaction was completed, the reaction mixture was cooled to room temperature and poured slowly into ice water (500 mL), and ethyl acetate (500 mL × 2) was added for extraction. The organic phase was discarded, and the aqueous phase was adjusted to pH 3 with 3 N hydrochloric acid and extracted with ethyl acetate (1 L × 3). The organic phases were combined, dried over anhydrous sodium sulfate and filtered, and the filtrate was concentrated under reduced pressure to remove the solvent, thus giving intermediate **WX009-2.** ¹H NMR (400 MHz, DMSO_*d₆*) δ: 12.52 (s, 1H), 7.82 (dd, *J*=1.6, 7.6 Hz, 1H), 7.68-7.62 (m, 1H), 7.40-7.32 (m, 2H), 5.59 (s, 1H).

### Step 2: synthesis of intermediate WX009-3

Intermediate **WX009-2** (25.00 g, 154.19 mmol) was dissolved in methanol (300 mL) at room temperature, and then sodium acetate (44.27 g, 539.65 mmol) and hydroxylamine hydrochloride (37.50 g, 539.65 mmol) were added sequentially. The reaction mixture was stirred at 15 °C for 1.5 h and then heated to 80 °C and stirred for 12 h. After the reaction was completed, the reaction mixture was cooled to room temperature, adjusted to pH = 5 with 3 N hydrochloric acid, and then concentrated under reduced pressure to remove the solvent. Water (200 mL) was added, and the reaction flask was placed in an ice-water bath to cool while 3 N hydrochloric acid was added to adjust the pH to 3. After being stirred for 30 min, the reaction mixture was filtered, and the filter cake was collected and concentrated under reduced pressure to remove the solvent, thus giving intermediate **WX009-3.**

### Step 3: synthesis of intermediate WX009-4

Intermediate **WX009-3** (28.00 g, 158.05 mmol) was dissolved in ethanol (600 mL) at room temperature, and then concentrated sulfuric acid (28 mL, purity: 98%) was added. The reaction mixture was heated to 90 °C and stirred for 6 h. After the reaction was completed, the reaction mixture was cooled to room temperature and concentrated under reduced pressure to remove the solvent. The resulting residue was diluted with ethyl acetate (300 mL) and water (300 mL), and solid sodium bicarbonate was added to adjust the pH to 7. After liquid separation, the organic phase was collected and the aqueous phase was extracted with ethyl acetate (300 mL × 2). The organic phases were combined, washed with saturated brine (300 mL), dried over anhydrous sodium sulfate, filtered, and concentrated under reduced pressure to remove the solvent. The resulting residue was separated by column chromatography (eluent: petroleum ether/ethyl acetate = 1/0-0/1, volume ratio) to give intermediate **WX009-4.** ¹H NMR (400 MHz, DMSO_*d₆*) δ: 7.84 (d, *J*=8.0 Hz, 1H), 7.74 (d, *J*=8.4 Hz, 1H), 7.70-7.63 (m, 1H), 7.44-7.37 (m, 1H), 4.21 (s, 2H), 4.14 (q, *J*=7.2 Hz, 2H), 1.19 (t, *J*=7.2 Hz, 3H).

### Step 4: synthesis of intermediate WX009-5

Intermediate **WX009-4** (24.00 g, 116.95 mmol) was added to fuming nitric acid (180 mL) at 0 °C, and the reaction mixture was stirred at 0 °C for 2 h. After the reaction was completed, the reaction mixture was poured into ice water (500 mL), and ethyl acetate (500 mL × 3) was added for extraction. The organic phases were combined, washed with water (200 mL × 2), dried over anhydrous sodium sulfate and filtered, and the filtrate was concentrated under reduced pressure to remove the solvent. The resulting residue was separated by column chromatography (eluent: petroleum ether/ethyl acetate = 1/0-10/1, volume ratio) to give intermediate **WX009-5.** ¹H NMR (400 MHz, DMSO_*d₆*) δ: 8.93 (d, *J*=2.4 Hz, 1H), 8.51 (dd, *J*=2.0, 9.2 Hz, 1H), 7.99 (d, *J*=9.2 Hz, 1H), 4.34 (s, 2H), 4.15 (q, *J*=7.4 Hz, 2H), 1.2 (t, *J*=7.0 Hz, 3H).

### Step 5: synthesis of intermediate WX009-6

Intermediate **WX009-5** (5.50 g, 21.98 mmol) was dissolved in ethanol (120 mL) at room temperature, and then stannic chloride dihydrate (19.84 g, 87.93 mmol) was added.The reaction mixture was stirred at 15 °C for 2 h. The reaction mixture was supplemented with stannic chloride dihydrate (14.88 g, 65.95 mmol) and then stirred for 5 h. After the reaction was completed, the reaction mixture was concentrated under reduced pressure to remove the solvent. The resulting residue was diluted with dichloromethane (200 mL) and water (300 mL), and then solid sodium bicarbonate was added to adjust the pH to 7. The resulting solution was filtered through a funnel containing diatomite. The diatomite was rinsed with 2-methyltetrahydrofuran (1000 mL), and the filtrate was collected. After liquid separation, the organic phase was collected, and the aqueous phase was extracted with 2-methyltetrahydrofuran (200 mL × 3). The organic phases were combined, dried over anhydrous sodium sulfate and filtered, and the filtrate was concentrated under reduced pressure to remove the solvent. The resulting residue was separated by column chromatography (a neutral alumina column of 200-300 meshes, eluent: petroleum ether/ethyl acetate/dichloromethane = 1/0/0-6/3/1, volume ratio) to give intermediate **WX009-6.**

### Step 6: synthesis of intermediate WX009-7

Intermediate **WX009-6** (1.50 g, 6.81 mmol) was dissolved in dichloromethane (30 mL) at room temperature, and then *N*-bromosuccinimide (1.21 g, 6.81 mmol) was added at 0 °C. The reaction mixture was stirred at 0-15 °C for 2 h. After the reaction was completed, water (15 mL) was added to the reaction mixture, and dichloromethane (20 mL × 3) was then added for extraction. The organic phases were combined, washed with saturated brine (20 mL), dried over anhydrous sodium sulfate and filtered, and the filtrate was concentrated under reduced pressure to remove the solvent, thus giving intermediate **WX009-7.** ¹H NMR (400 MHz, DMSO_*d₆*) δ: 7.51 (d, *J*=8.8 Hz, 1H), 7.16 (d, *J*=8.8 Hz, 1H), 5.42 (s, 2H), 4.20-4.08 (m, 4H), 1.20 (t, *J*=7.0 Hz, 3H).

### Step 7: synthesis of intermediate WX009-8

Intermediate **WX009-7** (1.1 g, 3.68 mmol) was dissolved in *N*,*N-*dimethylformamide (10 mL) at room temperature under nitrogen atmosphere, and then trimethylsilylacetylene (722.38 mg, 7.36 mmol), triethylamine (930.30 mg, 9.20 mmol), potassium iodide (610.46 mg, 3.68 mmol), copper(I) iodide (70.04 mg, 368.00 µmol) and 1,1'-bis(diphenylphosphino)ferrocene-palladium(II)dichloride dichloromethane complex (300.31 mg, 368.00 µmol) were added. The reaction mixture was heated to 110 °C and reacted under microwave (3 bar) for 3 h. After the reaction was completed, the reaction mixture was cooled to room temperature. Water (30 mL) was added, and ethyl acetate (30 mL × 3) was added for extraction. The organic phases were combined, washed with half-saturated brine (20 mL), dried over anhydrous sodium sulfate and filtered, and the filtrate was concentrated under reduced pressure to remove the solvent. The resulting residue was separated by column chromatography (eluent: petroleum ether/ethyl acetate = 3/1, volume ratio). The resulting residue was separated by prep-HPLC (mobile phase: acetonitrile/water; acidic system: 0.05% HCl) to give intermediate **WX009-8.** ¹H NMR (400 MHz, DMSO_*d₆*) δ: 11.63 (s, 1H), 7.69 (d, *J*=9.2 Hz, 1H), 7.42 (d, *J*=9.2 Hz, 1H), 6.79 (d, *J*=1.2 Hz, 1H), 4.26 (s, 2H), 4.15 (q, *J*=7.0 Hz, 2H), 1.20 (t, *J*=7.0 Hz, 3H), 0.34 (s, 9H).

### Step 8: synthesis of intermediate WX009-9

Intermediate **WX009-8** (300.00 mg, 948.09 µmol) was dissolved in acetonitrile (20 mL) at room temperature, and then a solution of tetrabutylammonium fluoride (1 M, 948.09 µL) in tetrahydrofuran was added. The reaction mixture was heated to 70 °C and stirred for 2 h. After the reaction was completed, the reaction mixture was cooled to room temperature and concentrated under reduced pressure to remove the solvent. The resulting residue was separated by column chromatography (eluent: petroleum ether/ethyl acetate = 1/0-2/1, volume ratio) to give intermediate **WX009-9.** ¹H NMR (400 MHz, DMSO_*d₆*) δ: 11.69 (s, 1H), 7.71 (d, *J*=8.8 Hz, 1H), 7.55 (t, *J*=2.8 Hz, 1H), 7.42 (d, *J*=8.8 Hz, 1H), 6.63 (s, 1H), 4.26 (s, 2H), 4.14 (q, *J*=7.2 Hz, 2H), 1.17 (t, *J*=7.0 Hz, 3H).

### Step 9: synthesis of WX009

Intermediate **WX009-9** (125.00 mg, 511.78 µmol) was dissolved in tetrahydrofuran (7 mL) at 0 °C, and then acrylamide (36.38 mg, 511.78 µmol) and potassium *tert*-butoxide (57.43 mg, 511.78 µmol) were added sequentially. The reaction mixture was warmed to room temperature (10 °C) and stirred for 1 h. After the reaction was completed, water (10 mL) was added to the reaction mixture, and 2-methyltetrahydrofuran (20 mL × 3) was added for extraction. The organic phases were combined, washed with saturated brine (10 mL), dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give target compound **WX009.** MS-ESI *m*/*z:* 270.1 [M+H]^{+ 1}H NMR (400 MHz, DMSO_*d₆*) δ: 11.71 (s, 1H), 11.20 (s, 1H), 7.72 (d, *J*=8.8 Hz, 1H), 7.56 (t, *J*=2.6 Hz, 1H), 7.44 (d, *J*=9.2 Hz, 1H), 6.56 (s, 1H), 4.69 (dd, *J*=5.0, 11.8 Hz, 1H), 2.90-2.81 (m, 1H), 2.70-2.59 (m, 1H), 2.47-2.37 (m, 1H), 2.28-2.15 (m, 1H).

### Example 10: WX010

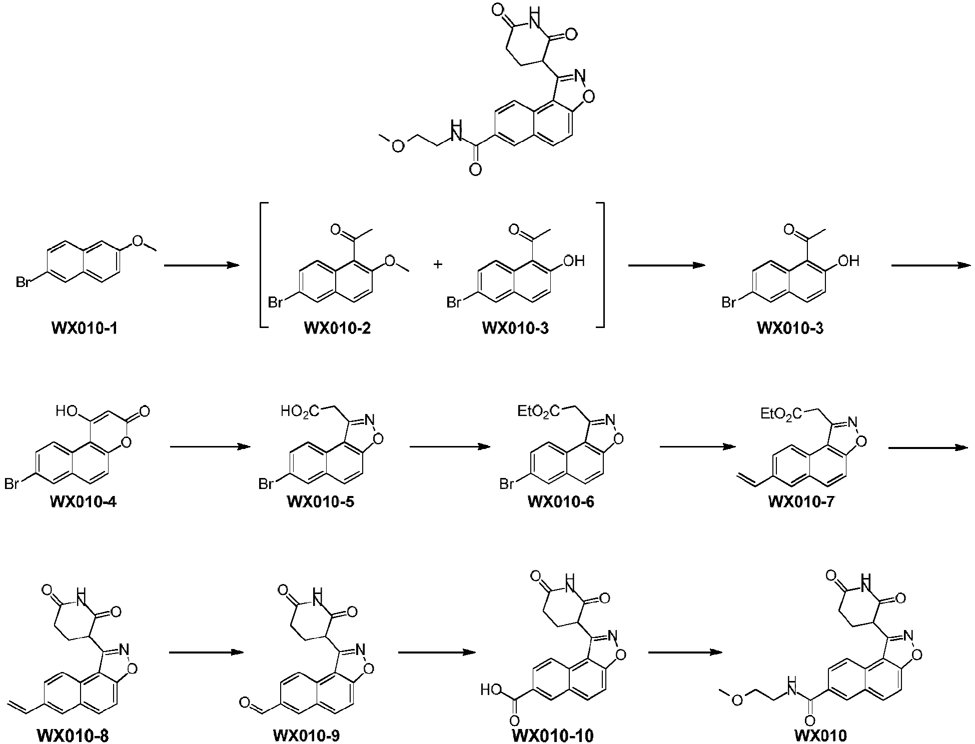

### Step 1: synthesis of intermediate WX010-2

**WX010-1** (10 g, 42.18 mmol, 25.00 mL) was dissolved in dichloromethane (200 mL) at room temperature under nitrogen atmosphere, and then acetyl chloride (3.31 g, 42.18 mmol, 3.01 mL) was added, and lastly, aluminum trichloride (8.44 g, 63.27 mmol, 3.46 mL) was added in batches at 5-15 °C. The reaction mixture was stirred at room temperature (15 °C) for 1 h. After the reaction was completed, the reaction mixture was poured into ice water (200 mL) and dichloromethane (200 mL × 3) was added for extraction. The organic phases were combined, dried over anhydrous sodium sulfate and filtered, and the filtrate was concentrated under reduced pressure to remove the solvent, thus giving a mixture of intermediate **WX010-2** and **WX010-3** in a ratio of 3/10.

### Step 2: synthesis of intermediate WX010-3

The mixture of intermediate **WX010-2** and **WX010-3** (11 g, the ratio of **WX010-2** to **WX010-3** was 3/10, molar ratio) was dissolved in dichloromethane (100 mL) at room temperature under nitrogen atmosphere. After the reaction mixture was cooled to -50 °C to -30 °C, a solution of boron trichloride in dichloromethane (1 M, 39.41 mL) was added dropwise slowly. The resulting reaction mixture was warmed to 0 °C and stirred at 0 °C for 3 h. After the reaction was completed, the reaction mixture was poured into ice water (200 mL) and then dichloromethane (200 mL × 3) was added for extraction. The organic phases were combined, dried over anhydrous sodium sulfate and filtered, and the filtrate was concentrated under reduced pressure to remove the solvent, thus giving intermediate **WX010-3.** ¹H NMR (400 MHz, CDCl₃) δ: 13.39 (s, 1H), 7.96 (d, *J*=8.8 Hz, 1H), 7.92 (d, *J*=2.4 Hz, 1H), 7.79 (d, *J*=9.2 Hz, 1H), 7.64 (dd, *J*=2.2, 9.0 Hz, 1H), 7.17 (d, *J*=8.8 Hz, 1H), 2.85 (s, 3H).

### Step 3: synthesis of intermediate WX010-4

Intermediate **WX010-3** (10 g, 37.72 mmol) was dissolved in toluene (100 mL) at room temperature under nitrogen atmosphere, and then diethyl carbonate (48.75 g, 412.68 mmol, 50 mL) was added, and lastly, sodium hydride (4.53 g, 113.16 mmol, purity: 60%) was added in batches at 5-10 °C. The reaction mixture was stirred at 10 °C for 30 min, and then heated to 120 °C and stirred for 12 h. After the reaction was completed, the reaction mixture was poured into ice water (500 mL), and ethyl acetate (100 mL × 3) was added for extraction. The organic phase was removed, and the aqueous phase was adjusted to pH = 3-4 with 2 M diluted hydrochloric acid and extracted with 2-methyltetrahydrofuran (300 mL × 5). The organic phases were combined, dried over anhydrous sodium sulfate and filtered, and the filtrate was concentrated under reduced pressure to remove the solvent, thus giving intermediate **WX010-4.** ¹H NMR (400 MHz, DMSO_*d₆*) δ: 13.01 (s, 1H), 9.19 (d, *J*=9.2 Hz, 1H), 8.32 (d, *J*=2.0 Hz, 1H), 8.17 (d, *J*=9.2 Hz, 1H), 7.80 (dd, *J*=2.2, 9.4 Hz, 1H), 7.58 (d, *J*=9.2 Hz, 1H), 5.76 (s, 1H).

### Step 4: synthesis of intermediate WX010-5

Intermediate **WX010-4** (4.3 g, 14.77 mmol) was dissolved in ethanol (200 mL) at room temperature, and then hydroxylamine hydrochloride (6.67 g, 96.02 mmol) and sodium acetate (4.24 g, 51.70 mmol) were added sequentially. The reaction mixture was heated to 85 °C and stirred for 56 h, and then heated to 90 °C and stirred for 32 h. After the reaction was completed, the reaction mixture was cooled to room temperature, and then 2 M diluted hydrochloric acid (200 mL) was added. The reaction mixture was concentrated under reduced pressure to remove ethanol, and 2-methyltetrahydrofuran (50 mL × 5) was added for extraction. The organic phases were combined, dried over anhydrous sodium sulfate and filtered, and the filtrate was concentrated under reduced pressure to remove the solvent, thus giving intermediate **WX010-5.**

### Step 5: synthesis of intermediate WX010-6

Intermediate **WX010-5** (4.5 g, 14.70 mmol) was dissolved in ethanol (20 mL) at room temperature under nitrogen atmosphere, and then sulfuric acid (1.88 g, 18.76 mmol, 1.02 mL, purity: 98%) was added. The reaction mixture was warmed to 70 °C and stirred for 12 h. After the reaction was completed, the reaction mixture was cooled to room temperature and concentrated under reduced pressure to remove the solvent. Water (100 mL) was added and ethyl acetate (50 mL × 3) was added for extraction. The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 1/0-9/1, volume ratio) to give intermediate **WX010-6.** ¹H NMR (400 MHz, CDCl₃) δ: 8.16 (d, *J*=2.0 Hz, 1H), 8.00 (d, *J*=8.8 Hz, 1H), 7.88 (d, *J*=8.8 Hz, 1H), 7.76 (dd, *J*=2.0, 8.8 Hz, 1H), 7.73 (d, *J*=9.2 Hz, 1H), 4.32 (s, 2H), 4.23 (q, *J*=7.2 Hz, 2H), 1.22 (t, *J*=7.2 Hz, 3H).

### Step 6: synthesis of intermediate WX010-7

Intermediate **WX010-6** (2.7 g, 8.08 mmol) was dissolved in *N*,*N-*dimethylformamide (40 mL) at room temperature under nitrogen atmosphere, and then 1,1'-bis(diphenylphosphino)ferrocene-palladium(II)dichloride dichloromethane complex (659.83 mg, 807.99 µmol), potassium phosphate (1.89 g, 8.89 mmol) and potassium vinylfluoroborate (1.41 g, 10.50 mmol) were added sequentially. The reaction mixture was heated to 80 °C and stirred at 80 °C for 12 h. After the reaction was completed, the reaction mixture was cooled to room temperature. Water (100 mL) was added to the reaction mixture, and ethyl acetate (50 mL × 3) was added for extraction. The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 1/0-9/1, volume ratio) to give intermediate **WX010-7.** ¹H NMR (400 MHz, CDCl₃) δ: 8.06 (d, *J*=8.4 Hz, 1H), 7.93 (d, *J*=9.2 Hz, 2H), 7.80 (dd, *J*=1.8, 8.6 Hz, 1H), 7.68 (d, *J*=8.8 Hz, 1H), 6.90 (dd, *J*=11.0, 17.4 Hz, 1H), 5.91 (d, *J*=17.6 Hz, 1H), 5.39 (d, *J*=10.8 Hz, 1H), 4.33 (s, 2H), 4.23 (q, *J*=7.0 Hz, 2H), 1.22 (t, *J*=7.2 Hz, 3H).

### Step 7: synthesis of intermediate WX010-8

Intermediate **WX010-7** (1.5 g, 5.33 mmol) was dissolved in tetrahydrofuran (40 mL) at room temperature, and then acrylamide (379.01 mg, 5.33 mmol) and potassium *tert*-butoxide (598.35 mg, 5.33 mmol) were added sequentially. The reaction mixture was stirred at room temperature (10 °C) for 3 h. After the reaction was completed, water (50 mL) was added, and ethyl acetate (100 mL × 5) was added for extraction. The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 1/0-1/1, volume ratio) to give intermediate **WX010-8.** ¹H NMR (400 MHz, DMSO_*d₆*) δ: 11.18 (s, 1H), 8.19-8.10 (m, 3H), 7.92 (t, *J*=7.4 Hz, 2H), 6.94 (dd, *J*=11.0, 17.4 Hz, 1H), 6.04 (d, *J*=17.6 Hz, 1H), 5.41 (d, *J*=10.8 Hz, 1H), 5.06 (dd, *J*=4.6, 11.4 Hz, 1H), 2.90-2.81 (m, 1H), 2.68-2.54 (m, 2H), 2.40-2.37 (m, 1H).

### Step 8: synthesis of intermediate WX010-9

Intermediate **WX010-8** (1 g, 3.26 mmol) was dissolved in dichloromethane (250 mL) at room temperature, and then ozone (15 psi) was introduced for 15 min at -70 °C. After the system turned blue, oxygen was introduced for 15 min (the blue disappeared). Lastly, dimethyl sulfide (1.69 g, 27.23 mmol, 2 mL) was added and the reaction mixture was stirred at 10 °C for 12 h. After the reaction was completed, the reaction mixture was warmed to room temperature and then concentrated under reduced pressure at 35 °C to remove the solvent. The resulting residue was separated by prep-HPLC (mobile phase: acetonitrile/water; acidic system: 0.05% HCl) to give intermediate **WX010-9** (yellowish solid, 0.4 g). ¹H NMR (400 MHz, DMSO_*d₆*) δ: 11.16 (s, 1H), 10.19 (s, 1H), 8.79 (d, *J*=0.8 Hz, 1H), 8.43 (d, *J*=9.2 Hz, 2H), 8.11 (dd, *J*=1.4, 8.6 Hz, 1H), 8.08 (d, *J*=9.2 Hz, 1H), 5.12 (dd, *J*=4.6, 11.8 Hz, 1H), 2.92-2.79 (m, 1H), 2.72-2.56 (m, 2H), 2.44-2.31 (m, 1H).

### Step 9: synthesis of intermediate WX010-10

Intermediate **WX010-9** (300 mg, 973.12 µmol) was dissolved in *N*,*N*-dimethylformamide (14 mL) at room temperature, and then potassium peroxymonosulfate (598.24 mg, 973.12 µmol) was added. The reaction mixture was stirred at room temperature (10 °C) for 12 h. After the reaction was completed, the reaction mixture was filtered to collect mother solution, thus giving a solution of intermediate **WX010-10** in *N*,*N*-dimethylformamide.

### Step 10: synthesis of WX010

2-(7-azabenzotriazol-1-yl)-*N*,*N*,*N*',*N*'-tetramethyluronium hexafluorophosphate (277.50 mg, 729.82 µmol) and triethylamine (98.47 mg, 973.09 µmol, 135.44 µL) are sequentially added to the solution of intermediate **WX010-10** (0.0695 M, 7.00 mL) in *N*,*N-*dimethylformamide at 0 °C under nitrogen atmosphere. After the reaction mixture was stirred at 0 °C for 15 min, 2-methoxyethylamine (40.20 mg, 535.20 µmol, 46.53 µL) was added. The resulting reaction mixture was warmed to room temperature (10 °C) and stirred at 10 °C for 12 h. After the reaction was completed, water (20 mL) was added to the reaction mixture and then ethyl acetate (20 mL × 3) was added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give target compound **WX010.** MS-ESI *m*/*z:* 382.1 [M+H]⁺.¹H NMR (400 MHz, DMSO_*d₆*) δ: 11.17 (s, 1H), 8.79 (s, 1H), 8.69 (s, 1H), 8.30 (d, *J*=9.2 Hz, 2H), 8.13 (d, *J*=8.4 Hz, 1H), 8.02 (d, *J*=9.2 Hz, 1H), 5.10 (dd, *J*=5.2, 12.0 Hz, 1H), 3.51 (s, 4H), 3.44 (s, 3H), 2.92-2.80 (m, 1H), 2.69-2.58 (m, 2H), 2.42-2.32 (m, 1H).

### Example 11: Hydrochloride of WX011

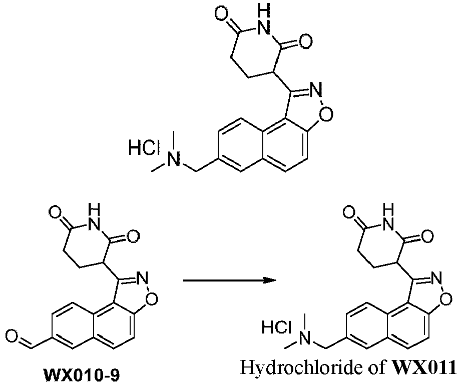

### Step 1: synthesis of WX011

Intermediate **WX010-9** (150 mg, 486.56 µmol) was dissolved in 1,2-dichloroethane (10 mL) at room temperature, and then glacial acetic acid (29.22 mg, 486.56 µmοl, 27.83 µL) and a solution of dimethylamine (2 M, 243.28 µL) in tetrahydrofuran were added. After the reaction mixture was stirred at 10 °C for 30 min, sodium triacetoxyborohydride (206.24 mg, 973.12 µmol) was added, and the resulting reaction mixture was stirred at 10 °C for 12 h. After the reaction was completed, water (30 mL) was added to the reaction mixture, and dichloromethane (20 mL × 3) was then added for extraction. The organic phases were combined, dried over anhydrous sodium sulfate and filtered. The filtrate was concentrated under reduced pressure to remove the solvent, and the aqueous phase was lyophilized directly. The residues were combined and then separated by prep-HPLC (mobile phase: acetonitrile/water; acidic system: 0.05% HCl) to give the hydrochloride of target compound **WX011.** MS-ESI *m*/*z:* 338.1 [M+H]⁺. ¹H NMR (400 MHz, DMSO_*d₆*) δ: 11.14 (s, 1H), 10.88 (s, 1H), 8.35 (d, *J*=8.4 Hz, 1H), 8.31 (s, 1H), 8.22 (d, *J*=9.2 Hz, 1H), 8.01 (d, *J*=9.2 Hz, 1H), 7.98 (d, *J*=7.6 Hz, 1H), 5.11 (dd, *J*=4.6, 11.8 Hz, 1H), 4.49 (s, 2H), 2.92-2.80 (m, 1H), 2.74 (s, 6H), 2.68-2.54 (m, 2H), 2.42-2.38 (m, 1H).

### Example 12: WX012

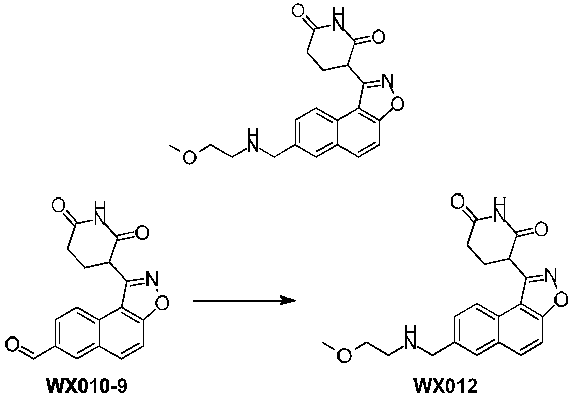

### Step 1: synthesis of WX012

Intermediate **WX010-9** (100 mg, 324.37 µmol) was dissolved in 1,2-dichloroethane (10 mL) at room temperature, and then glacial acetic acid (19.48 mg, 324.37 µmοl, 18.55 µL) and 2-methoxyethylamine (24.36 mg, 324.37 µmol, 28.20 µL) were added sequentially. After the reaction mixture was stirred at room temperature (10 °C) for 30 min, sodium triacetoxyborohydride (137.50 mg, 648.74 µmol) was added, and the resulting reaction mixture was stirred for 12 h. After the reaction was completed, water (30 mL) was added, and dichloromethane (20 mL × 3) was then added for extraction. The organic phases were combined, dried over anhydrous sodium sulfate and filtered. The filtrate was concentrated under reduced pressure to remove the solvent, and the aqueous phase was lyophilized directly. The residues were combined and then separated by prep-HPLC (mobile phase: acetonitrile/water; acidic system: 0.05% HCl) to give target compound **WX012.** MS-ESI *m*/*z:* 368.2 [M+H]⁺. ¹H NMR (400 MHz, DMSO_*d₆*) δ: 11.13 (s, 1H), 9.45 (s, 1H), 8.31 (d, *J*=11.6 Hz, 2H), 8.20 (d, *J*=9.2 Hz, 1H), 8.00 (d, *J*=9.2 Hz, 1H), 7.94 (d, *J*=7.6 Hz, 1H), 5.10 (dd, *J*=4.6, 11.4 Hz, 1H), 4.37 (s, 2H), 3.64 (t, *J*=5.0 Hz, 2H), 3.39 (s, 3H), 3.12 (s, 2H), 2.92-2.80 (m, 1H), 2.71-2.54 (m, 2H), 2.45-2.35 (m, 1H).

### Example 13: WX013

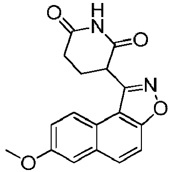

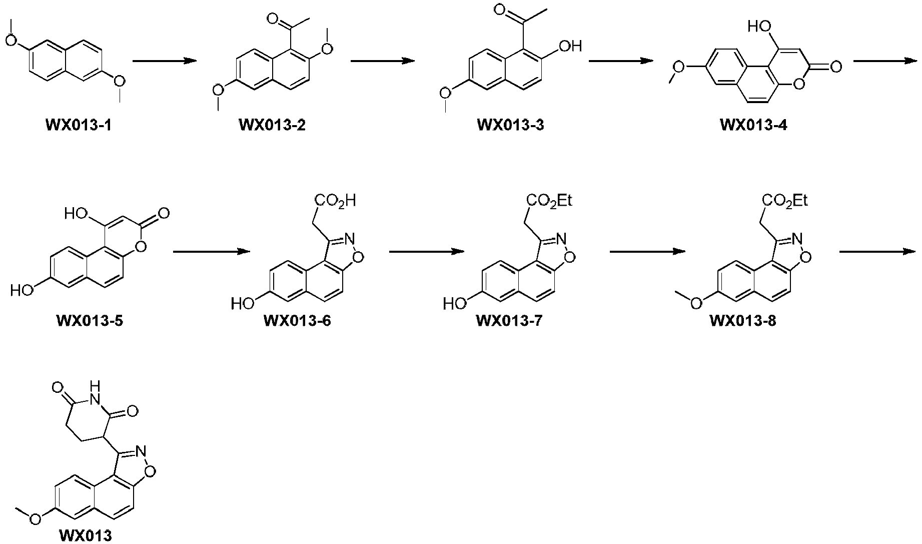

### Step 1: synthesis of intermediate WX013-2

**WX013-1** (20 g, 106.26 mmol) was dissolved in dichloromethane (300 mL) at room temperature under nitrogen atmosphere, and then acetyl chloride (8.34 g, 106.26 mmol, 7.58 mL) and aluminum trichloride (21.25 g, 159.39 mmol, 8.71 mL) were added sequentially. The reaction mixture was stirred at room temperature (18 °C) for 2 h. After the reaction was completed, the reaction mixture was slowly poured into ice water (400 mL). After liquid separation, the organic phases were collected, and the aqueous phase was extracted with dichloromethane (100 mL × 3). The organic phases were combined, washed with saturated brine (100 mL), dried over anhydrous sodium sulfate and filtered, and the filtrate was concentrated under reduced pressure to remove the solvent, thus giving intermediate **WX013-2.** ¹H NMR (400 MHz, CDCl₃) δ: 7.78 (d, *J*=9.2 Hz, 1H), 7.69 (d, *J*=9.2 Hz, 1H), 7.25 (d, *J*=9.2 Hz, 1H), 7.17 (dd, *J*=2.4, 9.2 Hz, 1H), 7.10 (d, *J*=2.4 Hz, 1H), 3.95 (s, 3H), 3.90 (s, 3H), 2.65 (s, 3H).

### Step 2: synthesis of intermediate WX013-3

Intermediate **WX013-2** (24.45 g, 106.18 mmol) was dissolved in dichloromethane (250 mL) at room temperature under nitrogen atmosphere, and then a solution of boron trichloride (1 M, 106.18 mL) in dichloromethane was added dropwise at -65 °C to -40 °C. The reaction mixture was warmed to 0 °C and stirred at 0 °C for 2 h. After the reaction was completed, the reaction mixture was slowly poured into ice water (500 mL), and dichloromethane (100 mL × 4) was added for extraction. The organic phases were combined, dried over anhydrous sodium sulfate and filtered, and the filtrate was concentrated under reduced pressure to remove the solvent, thus giving intermediate **WX013-3.** ¹H NMR (400 MHz, CDCl₃) δ: 13.17 (s, 1H), 8.01 (d, *J*=9.6 Hz, 1H), 7.80 (d, *J*=9.2 Hz, 1H), 7.24 (dd, *J*=2.8, 9.2 Hz, 1H), 7.15-7.12 (m, 2H), 3.92 (s, 3H), 2.85 (s, 3H).

### Step 3: synthesis of intermediate WX013-4

Intermediate **WX013-3** (21.5 g, 99.43 mmol) was dissolved in toluene (200 mL) at room temperature under nitrogen atmosphere, and then diethyl carbonate (195.00 g, 1.65 mol, 200 mL) was added, and lastly, sodium hydride (11.93 g, 298.29 mmol, purity: 60%) was added in batches at 10-20 °C, The reaction mixture was heated to 120 °C and stirred at 120 °C for 5 h. After the reaction was completed, the reaction mixture was cooled to room temperature and then slowly poured into ice water (800 mL), and ethyl acetate (200 mL × 3) was added for extraction. The organic phase was removed, and the aqueous phase was adjusted to pH 3-4 with 1 M diluted hydrochloric acid. Yellowish solid was precipitated and then collected by filtration. The resulting solid was stirred with methanol (200 mL) at room temperature for 30 min, and then filtered, and the solid was concentrated under reduced pressure to remove the solvent, thus giving intermediate **WX013-4.** ¹H NMR (400 MHz, DMSO_*d₆*) δ: 12.85 (s, 1H), 9.17 (d, *J*=9.6 Hz, 1H), 8.10 (d, *J*=8.8 Hz, 1H), 7.49 (d, *J*=4.4 Hz, 1H), 7.47 (d, *J*=1.6 Hz, 1H), 7.33 (dd, *J*=2.8, 9.6 Hz, 1H), 5.73 (s, 1H), 3.89 (s, 3H).

### Step 4: synthesis of intermediate WX013-5

Intermediate **WX013-4** (13 g, 53.67 mmol) was dissolved in dichloromethane (200 mL) at room temperature under nitrogen atmosphere, and then boron tribromide (49 g, 195.59 mmol, 18.85 mL) was added dropwise at - 50 °C to -30 °C. The reaction mixture was warmed to 10 °C and stirred at 10 °C for 15 h. After the reaction was completed, the reaction mixture was poured into ice water (500 mL), and 2-methyltetrahydrofuran (500 mL × 5) was added for extraction. The organic phases were combined, dried over anhydrous sodium sulfate and filtered, and the filtrate was concentrated under reduced pressure to remove the solvent, thus giving intermediate **WX013-5.**

### Step 5: synthesis of intermediate WX013-6

Intermediate **WX013-5** (9 g, 39.44 mmol) was dissolved in ethanol (150 mL) at room temperature under nitrogen atmosphere, and then hydroxylamine hydrochloride (9.59 g, 138.04 mmol) and sodium ethoxide (9.39 g, 138.04 mmol) were added sequentially. The reaction mixture was heated to 80 °C and stirred at 80 °C for 15 h. After the reaction was completed, the reaction mixture was cooled to room temperature. 1 M diluted hydrochloric acid (100 mL) was added, and the resulting reaction mixture was concentrated under reduced pressure to remove ethanol. Water (100 mL) was added, and ethyl acetate (200 mL × 3) was added for extraction. The organic phases were combined, dried over anhydrous sodium sulfate and filtered, and the filtrate was concentrated under reduced pressure to remove the solvent, thus giving intermediate **WX013-6.**

### Step 6: synthesis of intermediate WX013-7

Intermediate **WX013-6** (10 g, 41.12 mmol) was dissolved in ethanol (100 mL) at room temperature under nitrogen atmosphere, and then concentrated sulfuric acid (3.68 g, 36.77 mmol, 2 mL, purity: 98%) was added. The reaction mixture was heated to 70 °C and stirred at 70 °C for 6 h. After the reaction was completed, the reaction mixture was cooled to room temperature and concentrated under reduced pressure to remove most of the solvent. The resulting residue was diluted with water (100 mL), and ethyl acetate (70 mL × 3) was added for extraction. The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 1/0-7/3, volume ratio) to give intermediate **WX013-7.** ¹H NMR (400 MHz, CDCl₃) δ: 7.90 (d, *J*=8.8 Hz, 1H), 7.68 (d, *J*=9.2 Hz, 1H), 7.55 (d, *J*=9.2 Hz, 1H), 7.24 (d, *J*=2.4 Hz, 1H), 7.17 (dd, *J*=2.4, 7.6 Hz, 1H), 6.01 (br s, 1H), 4.24 (s, 2H), 4.15 (q, *J*=7.0 Hz, 2H), 1.14 (t, *J*=7.2 Hz, 3H).

### Step 7: synthesis of intermediate WX013-8

Intermediate **WX013-7** (500 mg, 1.84 mmol) was dissolved in *N*,*N-*dimethylformamide (8 mL) at room temperature, and then iodomethane (1.05 g, 7.37 mmol, 458.99 µL) and potassium carbonate (1.02 g, 7.37 mmol) were added sequentially. The reaction mixture was stirred at room temperature (10 °C) for 2 h. After the reaction was completed, water (30 mL) was added, and ethyl acetate (30 mL × 3) was added for extraction. The organic phases were combined, washed with half-saturated brine (30 mL × 2), dried over anhydrous sodium sulfate and filtered, and the filtrate was concentrated under reduced pressure to remove the solvent. The resulting residue was separated by column chromatography (eluent: petroleum ether/ethyl acetate = 1/0-4/1, volume ratio) to give intermediate **WX013-8.** ¹H NMR (400 MHz, CDCl₃) δ: 8.02 (d, *J*=9.6 Hz, 1H), 7.87 (d, *J*=9.2 Hz, 1H), 7.67 (d, *J*=9.2 Hz, 1H), 7.35 (s, 1H), 7.34 (dd, *J*=2.8, 8.0 Hz, 1H), 4.31 (s, 2H), 4.22 (q, *J*=7.0 Hz, 2H), 3.95 (s, 3H), 1.21 (t, *J*=7.2 Hz, 3H).

### Step 8: synthesis of WX013

Intermediate **WX013-8** (400 mg, 1.40 mmol) was dissolved in tetrahydrofuran (10 mL) at room temperature, and then acrylamide (99.66 mg, 1.40 mmol) and potassium *tert*-butoxide (157.33 mg, 1.40 mmol) were added sequentially. The reaction mixture was stirred at room temperature (10 °C) for 3 h. After the reaction was completed, water (50 mL) was added, and ethyl acetate (30 mL × 3) was added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give target compound **WX013.** MS-ESI *m*/*z:* 311.1 [M+H]⁺. ¹H NMR (400 MHz, DMSO_*d₆*) δ: 11.12 (s, 1H), 8.14 (d, *J*=8.8 Hz, 1H), 8.11 (d, *J*=9.2 Hz, 1H), 7.89 (d, *J*=9.2 Hz, 1H), 7.64 (d, *J*=2.8 Hz, 1H), 7.36 (dd, *J*=2.6, 9.0 Hz, 1H), 5.02 (dd, *J*=5.0, 11.4 Hz, 1H), 3.91 (s, 3H), 2.89-2.80 (m, 1H), 2.67-2.52 (m, 2H), 2.39-2.33 (m, 1H).

### Example 14: WX014

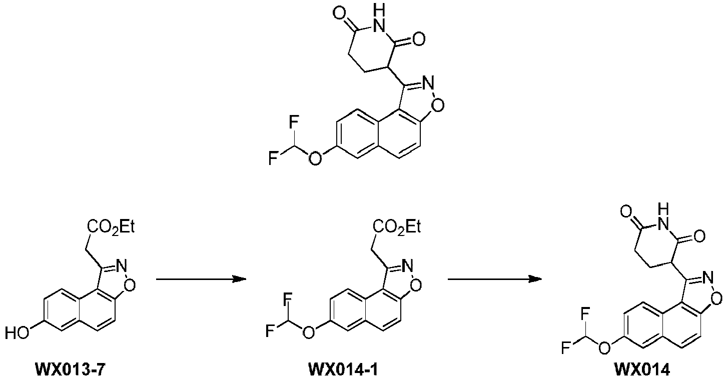

### Step 1: synthesis of intermediate WX014-1

Intermediate **WX013-7** (1 g, 3.69 mmol) was dissolved in acetonitrile (20 mL) at room temperature, and then potassium carbonate (1.02 g, 7.37 mmol) and the compound diethyl bis(bromodifluoromethyl)phosphonate (1.97 g, 7.37 mmol) were added. The reaction mixture was stirred at room temperature (10 °C) for 12 h. After the reaction was completed, water (50 mL) was added and then ethyl acetate (30 mL × 3) was added for extraction. The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 1/0-9/1, volume ratio) to give intermediate **WX014-1.** ¹H NMR (400 MHz, CDCl₃) δ: 8.13 (d, *J*=8.8 Hz, 1H), 7.92 (d, *J*=9.2 Hz, 1H), 7.74 (d, *J*=9.2 Hz, 1H), 7.71 (d, *J*=1.6 Hz, 1H), 7.49 (dd, *J*=2.0, 8.8 Hz, 1H), 6.83-6.46 (m, 1H), 4.32 (s, 2H), 4.23 (q, *J*=7.0 Hz, 2H), 1.22 (t, *J*=7.2 Hz, 3H).

### Step 2: synthesis of WX014

The recovered material **WX014-1** (100 mg, 311.26 µmol) was dissolved in tetrahydrofuran (3 mL) at room temperature, and then acrylamide (22.12 mg, 311.26 µmol) and a solution of potassium *tert*-butoxide (1 M, 311.26 µL) in tetrahydrofuran were added sequentially. The reaction mixture was stirred at room temperature (10 °C) for 2 h. After the reaction was completed, water (10 mL) was added and then ethyl acetate (20 mL × 3) was added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give target compound **WX014.** MS-ESI *m*/*z:* 347.0 [M+H]⁺.¹H NMR (400 MHz, DMSO_*d₆*) δ: 11.14 (s, 1H), 8.31 (d, *J*=8.8 Hz, 1H), 8.23 (d, *J*=9.2 Hz, 1H), 8.00 (d, *J*=9.2 Hz, 1H), 7.98 (d, *J*=2.4 Hz, 1H), 7.59-7.57 (m, 1H), 7.56-7.20 (m, 1H), 5.07 (dd, *J*=4.4, 11.6 Hz, 1H), 2.89-2.79 (m, 1H), 2.69-2.57 (m, 2H), 2.40-2.32 (m, 1H).

### Example 15: Hydrochloride of WX015

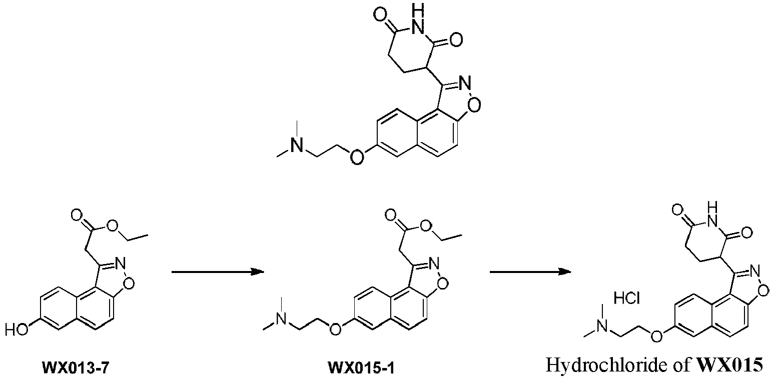

### Step 1: synthesis of intermediate WX015-1

**WX013-7** (500 mg, 1.84 mmol), 2-(dimethylamino)ethanol (180.73 mg, 2.03 mmol) and triphenylphosphine (628.49 mg, 2.40 mmol) were dissolved in tetrahydrofuran (15 mL) at room temperature under nitrogen atmosphere. After the reaction mixture was cooled to 0 °C, *N*,*N-*diisopropylethylamine (484.53 mg, 2.40 mmol, 564.89 µL) was added dropwise. The reaction mixture was warmed to room temperature (20 °C) and stirred for 12 h. After the reaction was completed, the reaction mixture was poured into water (40 mL), and 2-methyltetrahydrofuran (40 mL × 3) was added for extraction. The organic phases were combined, washed with saturated brine (50 mL × 3), dried over anhydrous sodium sulfate and filtered, and the filtrate was concentrated under reduced pressure to remove the solvent. The resulting residue was separated by column chromatography (eluent: petroleum ether/ethyl acetate = 1/0 to 0/1, dichloromethane/methanol = 1/0-19/1, volume ratio) to give intermediate **WX015-1.**

### Step 2: synthesis of WX015

Compound **WX015-1** (0.41 g, 1.2 mmol) was dissolved in tetrahydrofuran (12 mL) at room temperature under nitrogen atmosphere, and then acrylamide (85.11 mg, 1.2 mmol) and a solution of potassium *tert*-butoxide in tetrahydrofuran (1 M, 1.2 mL) were added sequentially. The reaction mixture was warmed to room temperature (20 °C) and stirred for 12 h. After the reaction was completed, the reaction mixture was adjusted to pH 3 with 2 N diluted hydrochloric acid, and concentrated under reduced pressure to remove the solvent. The resulting residue was separated by prep-HPLC (mobile phase: acetonitrile/water; acidic system: 0.05% HCl) to give the hydrochloride of target compound **WX015.** MS-ESI *m*/*z:* 368.2 [M+H]⁺. ¹H NMR (400 MHz, MeOD_d4) δ: 8.18 (d, *J*=8.8 Hz, 1H), 8.04 (d, *J*=9.2 Hz, 1H), 7.77 (d, *J*=9.2 Hz, 1H), 7.63 (d, *J*=2.8 Hz, 1H), 7.47 (dd, *J*=2.6, 9.0 Hz, 1H), 4.93 (dd, *J*=5.0, 10.2 Hz, 1H), 4.53 (t, *J*=5.0 Hz, 2H), 3.69 (t, *J*=5.0 Hz, 2H), 3.04 (s, 6H), 2.91-2.74 (m, 2H), 2.71-2.61 (m, 1H), 2.53-2.48 (m, 1H).

### Example 16: WX016

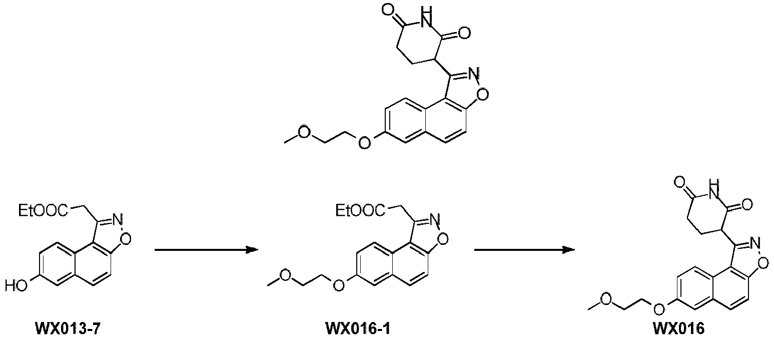

### Step 1: synthesis of intermediate WX016-1

Intermediate **WX013-7** (400 mg, 1.47 mmol) was dissolved in *N*,*N-*dimethylformamide (8 mL) at room temperature, and then potassium carbonate (815.20 mg, 5.90 mmol) and compound 1-bromo-2-methoxyethane (614.85 mg, 4.42 mmol, 415.44 µL) were added. After the reaction mixture was stirred at 10 °C for 2 h, potassium iodide (48.96 mg, 294.91 µmol) was added. The reaction mixture was heated to 50 °C and stirred at 50 °C for 12 h. After the reaction was completed, the reaction mixture was cooled to room temperature. Water (30 mL) was added, and ethyl acetate (30 mL × 3) was added for extraction. The organic phases were combined, washed with half-saturated brine (30 mL × 2), dried over anhydrous sodium sulfate and filtered, and the filtrate was concentrated under reduced pressure to remove the solvent. The resulting residue was separated by column chromatography (eluent: petroleum ether/ethyl acetate = 1/0-7/3, volume ratio) to give intermediate **WX016-1.** ¹H NMR (400 MHz, CDCl₃) δ: 8.03 (d, *J*=8.8 Hz, 1H), 7.86 (d, *J*=9.2 Hz, 1H), 7.68 (d, *J*=9.2 Hz, 1H), 7.43-7.35 (m, 2H), 4.31 (s, 2H), 4.27 (t, *J*=4.6 Hz, 2H), 4.22 (q, *J*=7.2 Hz, 2H), 3.84 (t, *J*=4.6 Hz, 2H), 3.50 (s, 3H), 1.20 (t, *J*=7.2 Hz, 3H).

### Step 2: synthesis of WX016

Intermediate **WX016-1** (350 mg, 1.06 mmol) was dissolved in tetrahydrofuran (10 mL) at room temperature, and then acrylamide (75.54 mg, 1.06 mmol, 73.33 µL) and potassium *tert*-butoxide (119.25 mg, 1.06 mmol) were added sequentially. The reaction mixture was stirred at 10 °C for 3 h. After the reaction was completed, water (50 mL) was added and then ethyl acetate (30 mL × 3) was added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give target compound **WX016.**

MS-ESI *m*/*z:* 355.1 [M+H]⁺. H NMR (400 MHz, DMSO_*d₆*) δ: 11.11 (s, 1H), 8.15 (d, *J*=9.2 Hz, 1H), 8.09 (d, *J*=9.2 Hz, 1H), 7.89 (d, *J*=9.2 Hz, 1H), 7.66 (d, *J*=2.4 Hz, 1H), 7.38 (dd, *J*=2.8, 9.2 Hz, 1H), 5.03 (dd, *J*=4.6, 11.4 Hz, 1H), 4.25 (t, *J*=4.6 Hz, 2H), 3.74 (t, *J*=4.4 Hz, 2H), 3.34 (s, 3H), 2.90-2.79 (m, 1H), 2.69-2.54 (m, 2H), 2.42-2.31 (m, 1H).

### Example 17: WX017

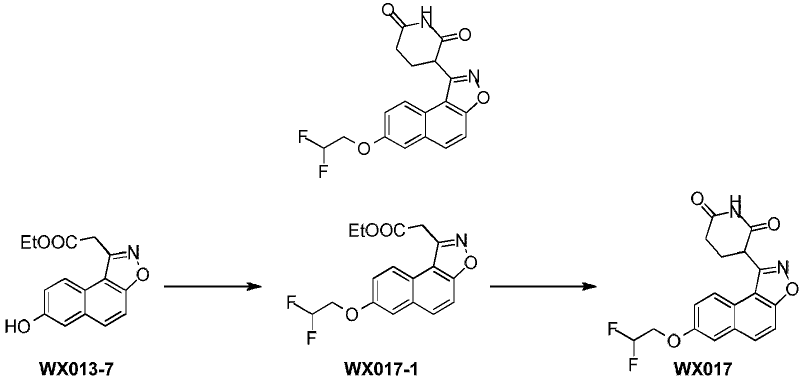

### Step 1: synthesis of intermediate WX017-1

Intermediate **WX013-7** (300 mg, 1.11 mmol) was dissolved in *N*,*N-*dimethylformamide (5 mL) at room temperature, and then potassium carbonate (611.38 mg, 4.42 mmol) and compound 2,2-difluoroethyl trifluoromethanesulfonate (710.37 mg, 3.32 mmol) were added. The reaction mixture was stirred at 10 °C for 12 h. After the reaction was completed, water (100 mL) was added and methyl *tert*-butyl ether (20 mL × 3) was then added for extraction. The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 1/0-4/1, volume ratio) to give intermediate **WX017-1.** ¹H NMR (400 MHz, CDCl₃) δ: 8.07 (d, *J*=9.2 Hz, 1H), 7.88 (d, *J*=9.2 Hz, 1H), 7.71 (d, *J*=9.2 Hz, 1H), 7.41-7.35 (m, 2H), 6.34-6.03 (m, 1H), 4.39-4.33 (m, 2H), 4.32 (s, 2H), 4.23 (q, *J*=7.0 Hz, 2H), 1.22 (t, *J*=7.2 Hz, 3H).

### Step 2: synthesis of WX017

Intermediate **WX017-1** (300 mg, 894.72 µmol) was dissolved in tetrahydrofuran (10 mL) at room temperature, and then acrylamide (63.59 mg, 894.72 µmol) and potassium *tert*-butoxide (100.40 mg, 894.72 µmol) were added simultaneously. The reaction mixture was stirred at room temperature (10 °C) for 3 h. After the reaction was completed, water (20 mL) was added and then ethyl acetate (30 mL × 3) was added for extraction. The organic phases were combined, dried over anhydrous sodium sulfate and filtered, and the filtrate was concentrated under reduced pressure to remove the solvent. The resulting residue was separated by prep-HPLC twice (mobile phase: acetonitrile/water; acidic system: 0.05% HCl) to give target compound **WX017**. MS-ESI *m*/*z:* 361.1 [M+H]⁺. ¹H NMR (400 MHz, DMSO_*d*₆) δ: 11.12 (s, 1H), 8.19 (d, *J*=9.2 Hz, 1H), 8.10 (d, *J*=9.2 Hz, 1H), 7.92 (d, *J*=9.2 Hz, 1H), 7.76 (d, *J*=2.8 Hz, 1H), 7.44 (dd, *J*=2.8, 8.8 Hz, 1H), 6.65-6.32 (m, 1H), 5.04 (dd, *J*=4.8, 11.6 Hz, 1H), 4.49 (dt, *J*=3.6, 14.6 Hz, 2H), 2.91-2.79 (m, 1H), 2.70-2.53 (m, 2H), 2.43-2.31 (m, 1H).

### Example 18: WX018

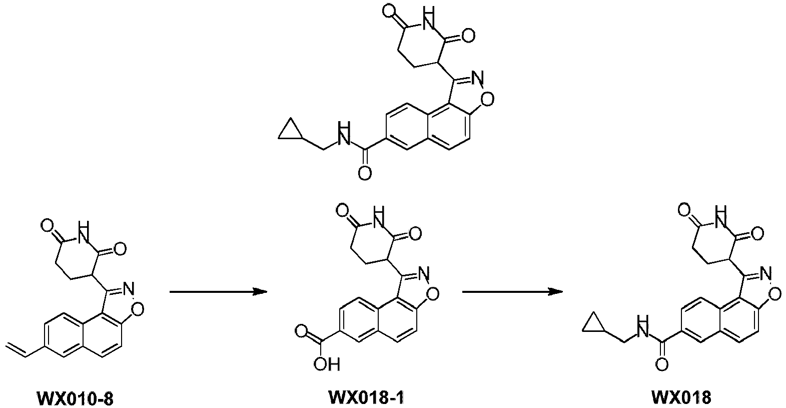

### Step 1: synthesis of intermediate WX018-1

Intermediate **WX010-8** (300 mg, 979.38 µmol) was dissolved in *N,N-*dimethylformamide (3 mL) at room temperature, and then the reaction mixture was cooled to -70 °C and ozone (15 psi) was introduced at -70 °C for 15 min. After the reaction mixture was warmed to room temperature (10 °C), dichloromethane (10 mL) was added, and then the reaction mixture was cooled to -70 °C again and ozone (15 psi) was introduced again at - 70 °C for 15 min. After oxygen was introduced for 10 min, the reaction mixture was warmed to 10 °C and concentrated under reduced pressure to remove most of the dichloromethane. Potassium hydrogen persulfate (602.09 mg, 979.38 µmol) was added and the reaction mixture was then stirred at 10 °C for 12 h. After the reaction was completed, the reaction mixture was filtered to collect mother solution, thus giving a solution of intermediate **WX018-1** in *N*,*N-*dimethylformamide.

### Step 2: synthesis of WX018

The solvent *N,N-*dimethylformamide (5 mL) was added to a solution of intermediate **WX018-1** (0.326 M, 3.00 mL) in *N*,*N*-dimethylfonnamide at 0 °C under nitrogen atmosphere, and then 2-(7-azabenzotriazol-1-yl)-*N*,*N*,*N*', *N*'-tetramethyluronium hexafluorophosphate (484.11 mg, 1.27 mmol) and triethylamine (198.21 mg, 1.96 mmol, 272.64 µL) were added sequentially. After the reaction mixture was stirred at 0 °C for 15 min, cyclopropylmethylamine (69.65 mg, 979.38 µmol) was added. The reaction mixture was allowed to return to room temperature (10 °C) and stirred for 12 h. After the reaction was completed, water (20 mL) was added and then ethyl acetate (20 mL × 3) was added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give target compound **WX018.** MS-ESI *m*/*z:* 378.1 [M+H]⁺.¹H NMR (400 MHz, DMSO_*d*₆) δ: 11.16 (s, 1H), 8.81 (t, *J=*5.6 Hz, 1H), 8.68 (s, 1H), 8.30 (d, *J*=9.2 Hz, 2H), 8.14 (d, *J*=8.4 Hz, 1H), 8.01 (d, *J*=8.8 Hz, 1H), 5.10 (dd, *J*=4.8, 11.6 Hz, 1H), 3.22 (t, *J*=6.0 Hz, 2H), 2.89-2.80 (m, 1H), 2.72-2.58 (m, 2H), 2.43-2.30 (m, 1H), 1.14-1.02 (m, 1H), 0.53-0.42 (m, 2H), 0.32-0.22 (m, 2H).

### Example 19: WX019

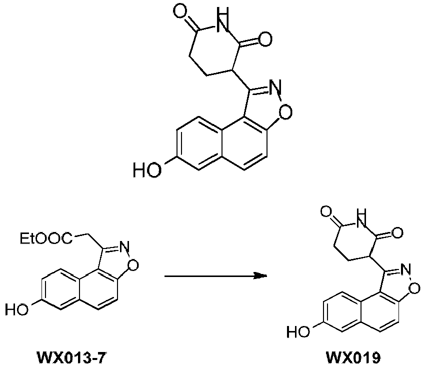

### Step 1: synthesis of WX019

Intermediate **WX013-7** (100 mg, 368.64 µmol) was dissolved in tetrahydrofuran (5 mL) at room temperature, and then acrylamide (26.20 mg, 368.64 µmol) and a solution of potassium *tert*-butoxide (1 M, 442.37 µL) in tetrahydrofuran were added. After being stirred at room temperature (10 °C) for 2 h, the reaction mixture was supplemented with the solution of potassium *tert*-butoxide (1 M, 0.2 mL) in tetrahydrofuran, and then stirred for 12 h. After the reaction was completed, the reaction mixture was slowly poured into 0.5 M diluted hydrochloric acid (20 mL) and ethyl acetate (20 mL × 3) was then added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give target compound **WX019.** MS-ESI *m*/*z:* 297.1 [M+H]^{+ 1}H NMR (400 MHz, DMSO_*d*₆) δ: 11.14 (s, 1H), 9.90 (s, 1H), 8.07 (d, *J*=9.2 Hz, 1H), 7.99 (d, *J*=9.2 Hz, 1H), 7.82 (d, *J*=9.2 Hz, 1H), 7.40 (d, *J*=2.4 Hz, 1H), 7.26 (dd, *J*=2.4, 8.8 Hz, 1H), 4.98 (dd, *J*=4.8, 11.2 Hz, 1H), 2.90-2.77 (m, 1H), 2.66-2.55 (m, 2H), 2.42-2.34 (m, 1H).

### Example 20: WX020

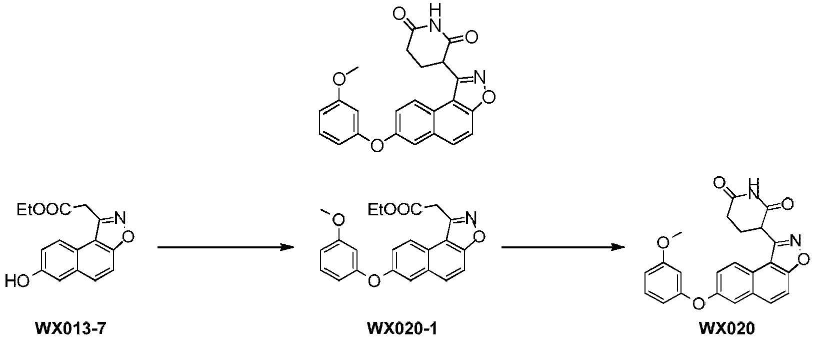

### Step 1: synthesis of WX020-1

Intermediate **WX013-7** (500 mg, 1.84 mmol) was dissolved in dichloromethane (20 mL) at 10 °C, and then copper acetate (334.78 mg, 1.84 mmol), pyridine (291.59 mg, 3.69 mmol, 297.54 µL), triethylamine (373.03 mg, 3.69 mmol, 513.10 µL) and 3-methoxyphenylboronic acid (560.17 mg, 3.69 mmol) were added sequentially. The reaction mixture was stirred open to air at 10 °C for 12 h. After the reaction was completed, water (50 mL) was added to the reaction mixture, and dichloromethane (50 mL × 3) was then added for extraction. The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 1/0-4/1, volume ratio) to give intermediate **WX020-1.** ¹H NMR (400 MHz, CDCl₃) δ: 8.11 (d, *J*=9.2 Hz, 1H), 7.85 (d, *J*=9.2 Hz, 1H), 7.70 (d, *J*=9.2 Hz, 1H), 7.54 (d, *J*=2.4 Hz, 1H), 7.46 (dd, *J*=2.2, 9.0 Hz, 1H), 7.31-7.26 (m, 1H), 6.76-6.70 (m, 1H), 6.69-6.64 (m, 1H), 6.64 (d, *J*=1.6 Hz, 1H), 4.33 (s, 2H), 4.24 (q, *J*=7.2 Hz, 2H), 3.81 (s, 3H), 1.23 (t, *J*=7.2 Hz, 3H).

### Step 2: synthesis of WX020

Intermediate **WX020-1** (310 mg, 821.43 µmol) was dissolved in tetrahydrofuran (15 mL) at 10 °C, and then acrylamide (58.39 mg, 821.43 µmol) and potassium *tert*-butoxide (92.17 mg, 821.43 µmol) were added simultaneously. The reaction mixture was stirred at 10 °C for 3 h. After the reaction was completed, water (40 mL) was added to the reaction mixture and then ethyl acetate (30 mL × 3) was added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give target compound **WX020.** MS-ESI *m*/*z:* 403.1 [M+H]⁺. ¹H NMR (399 MHz, DMSO_*d*₆) δ: 11.12 (s, 1H), 8.28 (d, *J*=9.2 Hz, 1H), 8.15 (d, *J*=9.2 Hz, 1H), 7.93 (d, *J*=9.2 Hz, 1H), 7.74 (d, *J*=2.8 Hz, 1H), 7.49 (dd, *J*=2.6, 9.0 Hz, 1H), 7.32 (t, *J*=8.2 Hz, 1H), 6.77 (dd, *J*=2.4, 8.4 Hz, 1H), 6.69 (t, *J*=2.2 Hz, 1H), 6.62 (dd, *J*=2.2, 8.2 Hz, 1H), 5.05 (dd, *J*=5.0, 11.4 Hz, 1H), 3.75 (s, 3H), 2.89-2.79 (m, 1H), 2.69-2.53 (m, 2H), 2.42-2.31 (m, 1H).

### Example 21: WX021

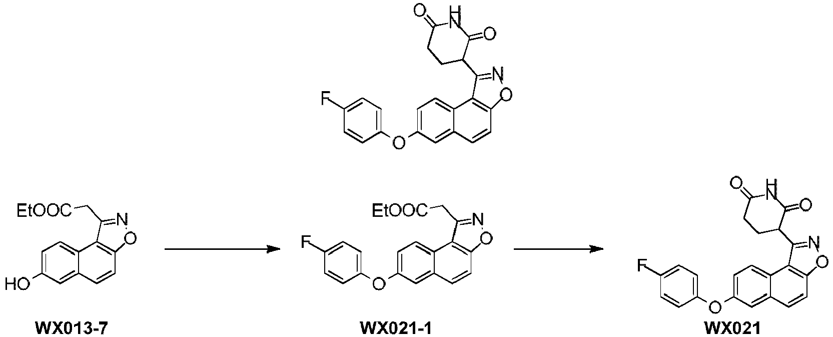

### Step 1: synthesis of intermediate WX021-1

Intermediate **WX013-7** (500 mg, 1.84 mmol) was dissolved in dichloromethane (20 mL) at room temperature, and then copper acetate (334.20 mg, 1.84 mmol), pyridine (291.09 mg, 3.68 mmol, 297.03 µL), triethylamine (372.38 mg, 3.68 mmol, 512.21 µL) and 4-fluorophenylboronic acid (514.91 mg, 3.68 mmol) were added sequentially. The reaction mixture was stirred open to air at 10 °C for 12 h. After the reaction was completed, water (50 mL) and dichloromethane (30 mL) were added to the reaction mixture. The resulting reaction mixture was filtered to collect mother solution. After liquid separation, the organic phase was collected, and the aqueous phase was extracted with dichloromethane (20 mL × 3). The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 1/0-4/1, volume ratio) to give intermediate **WX021-1.** ¹H NMR (400 MHz, CDCl₃) δ: 8.11 (d, *J*=8.4 Hz, 1H), 7.83 (d, *J*=9.2 Hz, 1H), 7.70 (d, *J*=9.2 Hz, 1H), 7.47-7.40 (m, 2H), 7.15-7.03 (m, 4H), 4.32 (s, 2H), 4.24 (q, *J*=7.0 Hz, 2H), 1.24 (t, *J*=7.2 Hz, 3H).

### Step 2: synthesis of WX021

Intermediate **WX021-1** (400 mg, 1.09 mmol) was dissolved in tetrahydrofuran (10 mL) at room temperature, and then acrylamide (77.82 mg, 1.09 mmol) and potassium *tert*-butoxide (122.85 mg, 1.09 mmol) were added simultaneously. The reaction mixture was stirred at room temperature (10 °C) for 3 h. After the reaction was completed, water (50 mL) was added to the reaction mixture and then ethyl acetate (30 mL × 3) was added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give target compound **WX021.** MS-ESI *m*/*z:* 391.1 [M+H]⁺. ¹H NMR (400 MHz, DMSO_*d*₆) δ: 11.13 (s, 1H), 8.28 (d, *J*=8.8 Hz, 1H), 8.13 (d, *J*=9.2 Hz, 1H), 7.93 (d, *J*=9.2 Hz, 1H), 7.68 (d, *J*=2.4 Hz, 1H), 7.48 (dd, *J*=2.4, 9.2 Hz, 1H), 7.33-7.23 (m, 2H), 7.21-7.14 (m, 2H), 5.04 (dd, *J*=4.4, 11.6 Hz, 1H), 2.90-2.79 (m, 1H), 2.67-2.56 (m, 2H), 2.42-2.31 (m, 1H).

### Example 22: WX022

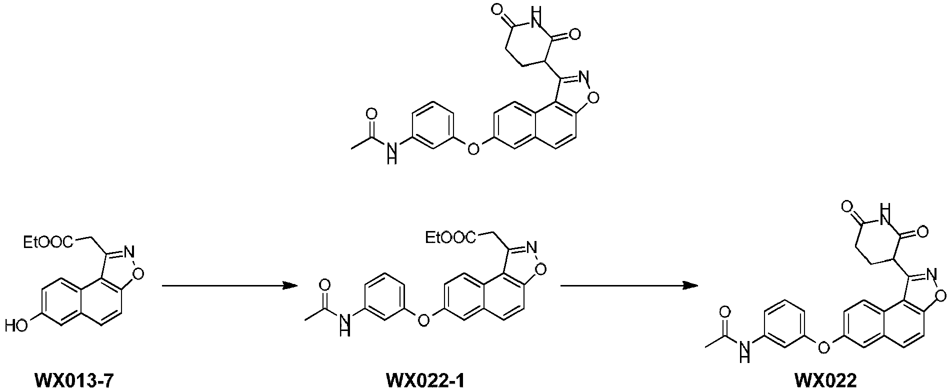

### Step 1: synthesis of intermediate WX022-1

Intermediate **WX013-7** (1 g, 3.69 mmol) was dissolved in 1,2-dichloroethane (30 mL) at room temperature, and then copper acetate (669.56 mg, 3.69 mmol), pyridine (583.19 mg, 7.37 mmol, 595.09 µL), triethylamine (746.05 mg, 7.37 mmol, 1.03 mL) and 3-acetamidophenylboronic acid (1.32 g, 7.37 mmol) were added sequentially. The reaction mixture was heated to 35 °C and stirred open to air for 12 h. After the reaction was completed, the reaction mixture was diluted with water (50 mL) and dichloromethane (30 mL). The resulting reaction mixture was filtered to collect mother solution. After liquid separation, the organic phase was collected, and the aqueous phase was extracted with dichloromethane (20 mL × 3). The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 1/0-2/1, volume ratio) to give intermediate **WX022-1.** ¹H NMR (400 MHz, CDCl₃) δ: 8.01 (d, *J*=8.8 Hz, 1H), 7.74 (d, *J*=9.2 Hz, 1H), 7.58 (d, *J*=9.2 Hz, 1H), 7.44 (d, *J*=2.4 Hz, 1H), 7.35 (dd, *J*=2.4, 8.8 Hz, 1H), 7.22-7.15 (m, 3H), 6.73 (d, *J*=7.2 Hz, 1H), 4.24 (s, 2H), 4.15 (q, *J*=7.2 Hz, 2H), 2.04 (s, 3H), 1.16 (t, *J*=7.2 Hz, 3H).

### Step 2: synthesis of WX022

Intermediate **WX022-1** (280 mg, 656.08 µmol) was dissolved in tetrahydrofuran (10 mL) at 10 °C, and then acrylamide (54.13 mg, 761.56 µmol) and a solution of potassium *tert*-butoxide (1 M, 721.69 µL) in tetrahydrofuran were added sequentially. The reaction mixture was stirred at 10 °C for 4 h. After the reaction was completed, 1 M diluted hydrochloric acid (1 mL) and water (5 mL) were added to the reaction mixture, and then ethyl acetate (20 mL × 3) was added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl), the resulting residue from which was then separated by prep-HPLC (mobile phase: acetonitrile/water; neutral system: 10 mM NH₄HCO₃) to give target compound **WX022.** MS-ESI *m*/*z:* 430.1 [M+H]⁺. ¹H NMR (400 MHz, DMSO_*d*₆) δ: 11.11 (s, 1H), 10.00 (s, 1H), 8.30 (d, *J*=8.8 Hz, 1H), 8.16 (d, *J*=9.2 Hz, 1H), 7.95 (d, *J*=9.2 Hz, 1H), 7.77 (d, *J*=2.4 Hz, 1H), 7.50 (dd, *J*=2.4, 9.2 Hz, 1H), 7.40-7.30 (m, 3H), 6.78 (d, *J*=8.0 Hz, 1H), 5.06 (dd, *J*=4.8, 11.6 Hz, 1H), 2.94-2.79 (m, 1H), 2.70-2.59 (m, 2H), 2.44-2.32 (m, 1H), 2.00 (s, 3H).

### Example 23: Hydrochloride of WX023

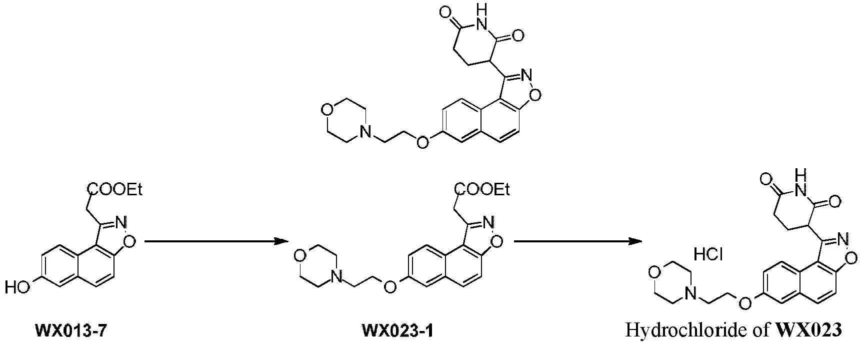

### Step 1: synthesis of intermediate WX023-1

Intermediate **WX013-7** (500 mg, 1.84 mmol) was dissolved in tetrahydrofuran (10 mL) at 15 °C under nitrogen atmosphere, and then 4-(2-hydroxyethyl)morpholine (265.96 mg, 2.03 mmol, 248.56 µL) and triphenylphosphine (628.49 mg, 2.40 mmol) were added sequentially. After the reaction mixture was cooled to 0 °C, diisopropyl azodicarboxylate (484.53 mg, 2.40 mmol, 465.89 µL) was added dropwise. The resulting reaction mixture was heated to 15 °C and stirred at 15 °C for 12 h. After the reaction was completed, the reaction mixture was directly concentrated under reduced pressure to remove the solvent. The resulting residue was separated by column chromatography (eluent: petroleum ether/ethyl acetate = 1/1-0/1, volume ratio) to give intermediate **WX023-1.** ¹H NMR (400 MHz, CDCl₃) δ: 8.03 (d, *J*=9.6 Hz, 1H), 7.86 (d, *J*=9.2 Hz, 1H), 7.68 (d, *J*=9.2 Hz, 1H), 7.43-7.32 (m, 2H), 4.31 (s, 2H), 4.26 (t, *J*=5.8 Hz, 2H), 4.22 (q, *J*=7.2 Hz, 2H), 3.77 (t, *J*=8.6 Hz, 4H), 2.90 (t, *J*=5.8 Hz, 2H), 2.64 (t, *J*=4.6 Hz, 4H), 1.21 (t, *J*=7.2 Hz, 3H).

### Step 2: synthesis of WX023

Intermediate **WX023-1** (250 mg, 650.32 µmol) was dissolved in tetrahydrofuran (10 mL) at 15 °C, and then acrylamide (46.22 mg, 650.32 µmol) and a solution of potassium *tert*-butoxide (1 M, 455.23 µL) in tetrahydrofuran were added sequentially. The reaction mixture was stirred at 15 °C for 2 h. After the reaction was completed, 4 M ethyl acetate hydrochloride was added dropwise to the reaction mixture to adjust the pH to 5-6, and the reaction mixture was then concentrated under reduced pressure to remove the solvent. The resulting residue was separated by prep-HPLC (mobile phase: acetonitrile/water; acidic system: 0.05% HCl) to give the hydrochloride of target compound **WX023.** MS-ESI *m*/*z:* 410.1 [M+H]⁺. ¹H NMR (400 MHz, D₂O) δ: 7.76 (t, *J*=9.4 Hz, 2H), 7.52 (d, *J*=9.2 Hz, 1H), 7.29 (s, 1H), 7.23 (d, *J*=8.8 Hz, 1H), 4.72 (d, *J*=5.6 Hz, 1H), 4.45 (t, *J*=4.4 Hz, 2H), 4.15 (d, *J*=11.6 Hz, 2H), 3.91 (t, *J*=12.0 Hz, 2H), 3.72 (t, *J*=4.4Hz, 2H), 3.70-3.61 (m, 2H), 3.37 (t, *J*=10.0 Hz, 2H), 2.90-2.63 (m, 2H), 2.57-2.35 (m, 2H).

### Example 24: Hydrochloride of WX024

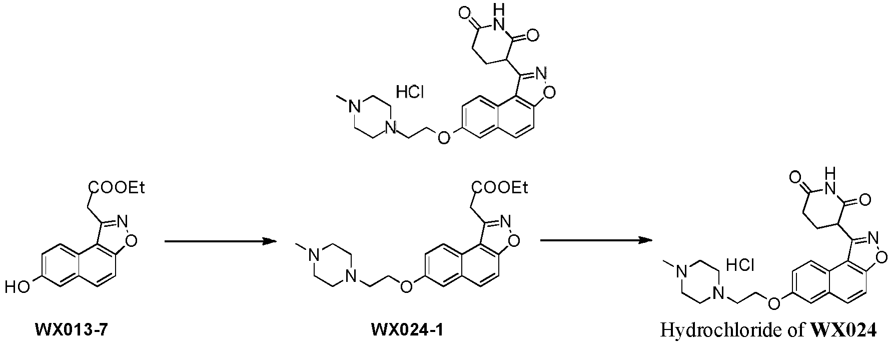

### Step 1: synthesis of intermediate WX024-1

Intermediate **WX013-7** (500 mg, 1.84 mmol) was dissolved in tetrahydrofuran (10 mL) at 15 °C under nitrogen atmosphere, and then 1-(2-hydroxyethyl)-4-methylpiperazine (291.89 mg, 2.02 mmol) and triphenylphosphine (627.40 mg, 2.39 mmol) were added sequentially. After the reaction mixture was cooled to 0-5 °C, diisopropyl azodicarboxylate (483.69 mg, 2.39 mmol, 465.08 µL) was added dropwise. The resulting reaction mixture was warmed to 15 °C and stirred for 12 h. After the reaction was completed, the reaction mixture was directly concentrated under reduced pressure to remove the solvent. The resulting residue was separated by column chromatography (eluent: petroleum ether/ethyl acetate = 1/1-0/1, and methanol/dichloromethane = 0/1-1/9, volume ratio) to give intermediate **WX024-1.**

### Step 2: synthesis of WX024

Intermediate **WX024-1** (330 mg, 830.26 µmol) was dissolved in tetrahydrofuran (10 mL) at 15 °C, and then acrylamide (59.01 mg, 830.26 µmol) and a solution of potassium *tert*-butoxide (1 M, 747.23 µL) in tetrahydrofuran were added sequentially. The reaction mixture was stirred at 15 °C for 1 h. After the reaction was completed, 4 M ethyl acetate hydrochloride was added dropwise to the reaction mixture to adjust pH to 5-6, and the reaction mixture was then concentrated under reduced pressure to remove the solvent. The resulting residue was separated by prep-HPLC (mobile phase: acetonitrile/water; acidic system: 0.05% HCl) to give the hydrochloride of target compound **WX024.** MS-ESI *m*/*z:* 423.2 [M+H]⁺.¹H NMR (400 MHz, MeOD_*d₄*) δ: 8.17 (d, *J*=9.2 Hz, 1H), 8.04 (d, *J*=9.2 Hz, 1H), 7.77 (d, *J*=8.8 Hz, 1H), 7.64 (d, *J*=2.4 Hz, 1H), 7.49 (dd, *J*=2.6, 9.0 Hz, 1H), 4.93 (dd, *J*=5.0, 10.2 Hz, 1H), 4.61 (t, *J*=4.6 Hz, 2H), 3.83 (t, *J*=4.6 Hz, 2H), 3.82-3.55 (m, 8H), 3.05 (s, 3H), 2.93-2.74 (m, 2H), 2.72-2.60 (m, 1H), 2.58-2.44 (m, 1H).

### Example 25: Hydrochloride of WX025

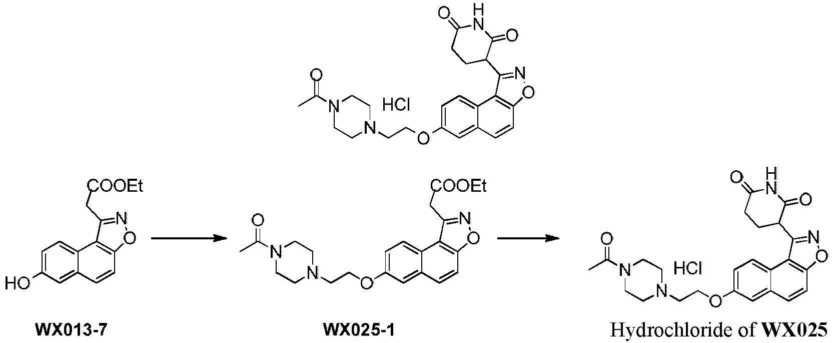

### Step 1: synthesis of intermediate WX025-1

Intermediate **WX013-7** (500 mg, 1.84 mmol) was dissolved in tetrahydrofuran (10 mL) at 15 °C under nitrogen atmosphere, and then 1-[4-(2-hydroxyethyl)-1-piperazinyl]ethanone (348.58 mg, 2.02 mmol) and triphenylphosphine (627.40 mg, 2.39 mmol) were added sequentially. After the reaction mixture was cooled to 0 °C, diisopropyl azodicarboxylate (483.69 mg, 2.39 mmol, 465.08 µL) was added dropwise. The resulting reaction mixture was warmed to 15 °C and stirred for 12 h. After the reaction was completed, the reaction mixture was directly concentrated under reduced pressure to remove the solvent. The resulting residue was separated by column chromatography (eluent: petroleum ether/ethyl acetate = 1/1-0/1, dichloromethane/methanol = 1/0-9/1, volume ratio) to give intermediate **WX025-1.**

### Step 2: synthesis of WX025

Intermediate **WX025-1** (400 mg, 940.12 µmol) was dissolved in tetrahydrofuran (15 mL) at 15 °C, and then acrylamide (66.82 mg, 940.12 µmol) and a solution of potassium *tert*-butoxide (1 M, 752.10 µL) in tetrahydrofuran were added sequentially. The reaction mixture was stirred at 15 °C for 2 h. After the reaction was completed, 4 M ethyl acetate hydrochloride was added dropwise to the reaction mixture to adjust the pH to 5-6, and the reaction mixture was then concentrated under reduced pressure to remove the solvent. The resulting residue was separated by prep-HPLC (mobile phase: acetonitrile/water; acidic system: 0.05% HCl) to give the hydrochloride of target compound **WX025.** MS-ESI *m*/*z:* 451.2 [M+H]^{+ 1}H NMR (400 MHz, DMSO_*d*₆) δ: 11.45 (s, 1H), 11.13 (s, 1H), 8.19 (d, *J*=8.8 Hz, 1H), 8.13 (d, *J*=9.2 Hz, 1H), 7.93 (d, *J*=9.2 Hz, 1H), 7.74 (d, *J*=2.4 Hz, 1H), 7.44 (dd, *J*=2.6, 9.0 Hz, 1H), 5.04 (dd, *J*=4.8, 11.2 Hz, 1H), 4.60 (t, *J*=4.4Hz, 2H), 4.44 (d, *J*=11.6 Hz, 1H), 4.02 (d, *J*=13.6 Hz, 1H), 3.70-3.55 (m, 5H), 3.28-3.00 (m, 3H), 2.92-2.78 (m, 1H), 2.73-2.54 (m, 2H), 2.43-2.30 (m, 1H), 2.05 (s, 3H).

### Example 26: WX026

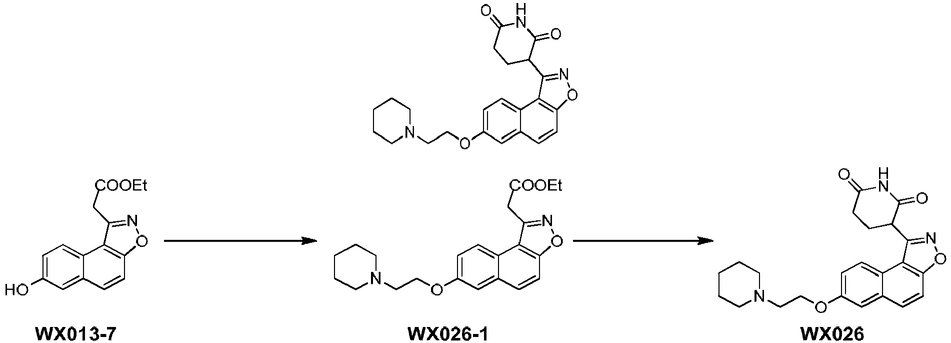

### Step 1: synthesis of intermediate WX026-1

Intermediate **WX013-7** (500 mg, 1.84 mmol) was dissolved in tetrahydrofuran (10 mL) at 15 °C under nitrogen atmosphere, and then *N*-hydroxyethyl piperidine (261.50 mg, 2.02 mmol) and triphenylphosphine (627.40 mg, 2.39 mmol) were added sequentially. After the reaction mixture was cooled to 0 °C, diisopropyl azodicarboxylate (483.69 mg, 2.39 mmol, 465.08 µL) was added dropwise. The resulting reaction mixture was warmed to 15 °C and stirred for 12 h. After the reaction was completed, the reaction mixture was directly concentrated under reduced pressure to remove the solvent. The resulting residue was separated by column chromatography (eluent: petroleum ether/ethyl acetate = 1/1-0/1, volume ratio), the resulting residue from which was then separated by prep-HPLC (mobile phase: acetonitrile/water; acidic system: 0.05% HCl) to give intermediate **WX026-1.** ¹H NMR (400 MHz, MeOD_*d₄*) δ: 8.18-8.11 (m, 1H), 8.05-7.99 (m, 1H), 7.79-7.71 (m, 1H), 7.63-7.57 (m, 1H), 7.49-7.42 (m, 1H), 4.55 (t, *J=*5.0 Hz, 2H), 4.42-4.36 (m, 2H), 4.21 (q, *J*=7.4 Hz, 2H), 3.72-3.64 (m, 4H), 3.13 (t, *J*=11.4 Hz, 2H), 2.06-1.95 (m, 2H), 1.93-1.78 (m, 3H), 1.67-1.49 (m, 1H), 1.21 (t, *J*=7.0 Hz, 3H).

### Step 2: synthesis of WX026

Intermediate **WX026-1** (150 mg, 358.07 µmol, hydrochloride) was dissolved in tetrahydrofuran (5 mL) at 15 °C, and then acrylamide (25.45 mg, 358.07 µmol) and a solution of potassium *tert*-butoxide (1 M, 429.68 µL) in tetrahydrofuran were added sequentially. After the reaction mixture was stirred at 15 °C for 1 h, a solution of potassium *tert*-butoxide (1 M, 300 µL) in tetrahydrofuran was added. The resulting reaction mixture was stirred at 15 °C for 2 h. After the reaction was completed, 4 M ethyl acetate hydrochloride was added dropwise to the reaction mixture to adjust pH to 5-6, and the reaction mixture was then concentrated under reduced pressure to remove the solvent. The resulting residue was separated by prep-HPLC (mobile phase: acetonitrile/water; acidic system: 0.05% HCl), the resulting residue from which was then separated by prep-HPLC (mobile phase: acetonitrile/water; neutral system: 10 mM NH₄HCO₃). The resulting fraction was adjusted to pH 5-6 with 6 M hydrochloric acid, and concentrated under reduced pressure to remove the solvent, thus giving target compound **WX026.** MS-ESI *m*/*z:* 408.2 [M+H]^{+ 1}H NMR (400 MHz, MeOD_*d₄*) δ: 8.18 (d, *J*=8.8 Hz, 1H), 8.05 (d, *J*=8.8 Hz, 1H), 7.82-7.70 (m, 1H), 7.64 (s, 1H), 7.46 (dd, *J*=2.4, 8.8 Hz, 1H), 4.94 (dd, *J*=5.2, 10.4 Hz, 1H), 4.56 (t, *J*=5.0 Hz, 2H), 3.75-3.60 (m, 4H), 3.14 (t, *J*=11.4 Hz, 2H), 2.95-2.73 (m, 2H), 2.72-2.60 (m, 1H), 2.57-2.44 (m, 1H), 2.09-1.76 (m, 5H), 1.69-1.47 (m, 1H).

### Example 27: Hydrochloride of WX027

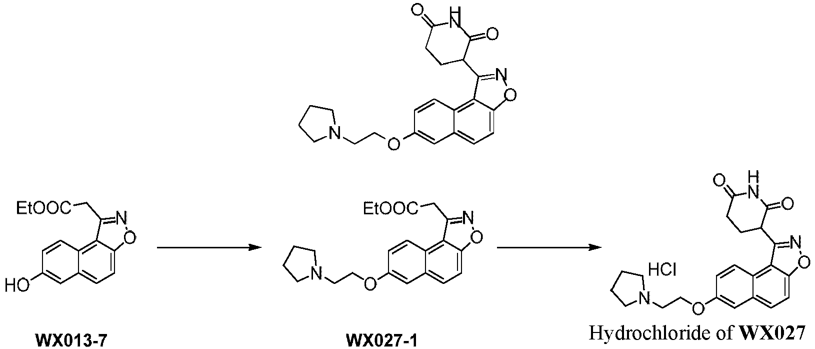

### Step 1: synthesis of intermediate WX027-1

Intermediate **WX013-7** (0.5 g, 1.84 mmol), *N*-hydroxyethylpyrrolidine (233.52 mg, 2.03 mmol) and triphenylphosphine (628.49 mg, 2.40 mmol) were dissolved in tetrahydrofuran (15 mL) at room temperature under nitrogen atmosphere. After the reaction mixture was cooled to 0 °C, diisopropyl azodicarboxylate (484.53 mg, 2.40 mmol, 465.89 µL) was added dropwise. The resulting reaction mixture was warmed to 20 °C and stirred for 12 h. After the reaction was completed, the reaction mixture was poured into water (40 mL), and ethyl acetate (40 mL × 3) was added for extraction. The organic phases were combined, washed with saturated brine (40 mL × 3), dried over anhydrous sodium sulfate, filtered, and concentrated under reduced pressure to remove the solvent. The resulting residue was separated by column chromatography (eluent: petroleum ether/ethyl acetate = 1/0-0/1, volume ratio) to give intermediate **WX027-1.**

### Step 2: synthesis of WX027

Intermediate **WX027-1** (0.2 g, 542.85 µmol) and acrylamide (38.58 mg, 542.85 µmol) were dissolved in tetrahydrofuran (6 mL) at room temperature, and then a solution of potassium *tert*-butoxide in tetrahydrofuran (1 M, 434.28 µL) was added. The reaction mixture was stirred at room temperature for 3 h. After the reaction was completed, the reaction mixture was adjusted to pH 5-6 with ethyl acetate hydrochloride (4 M), and then concentrated under reduced pressure to remove the solvent. The resulting residue was separated by prep-HPLC (mobile phase: acetonitrile/water; acidic system: 0.05% HCl) to give the hydrochloride of target compound **WX027.** MS-ESI *m*/*z:* 394.1 [M+H]⁺. ¹H NMR (400 MHz, MeOD_*d₄*) δ: 8.16 (d, *J*=9.2 Hz, 1H), 8.02 (d, *J*=8.8 Hz, 1H), 7.74 (d, *J*=9.2 Hz, 1H), 7.60 (d, *J*=2.8 Hz, 1H), 7.46 (dd, *J*=2.6, 9.0 Hz, 1H), 4.93 (dd, *J*=5.0, 10.2 Hz, 1H), 4.50 (t, *J*=4.8 Hz, 2H), 3.83-3.70 (m, 4H), 3.29-3.24 (m, 2H), 2.90-2.73 (m, 2H), 2.70-2.60 (m, 1H), 2.53-2.45 (m, 1H), 2.27-2.15 (m, 2H), 2.14-2.02 (m, 2H).

### Example 28: Hydrochloride of WX028

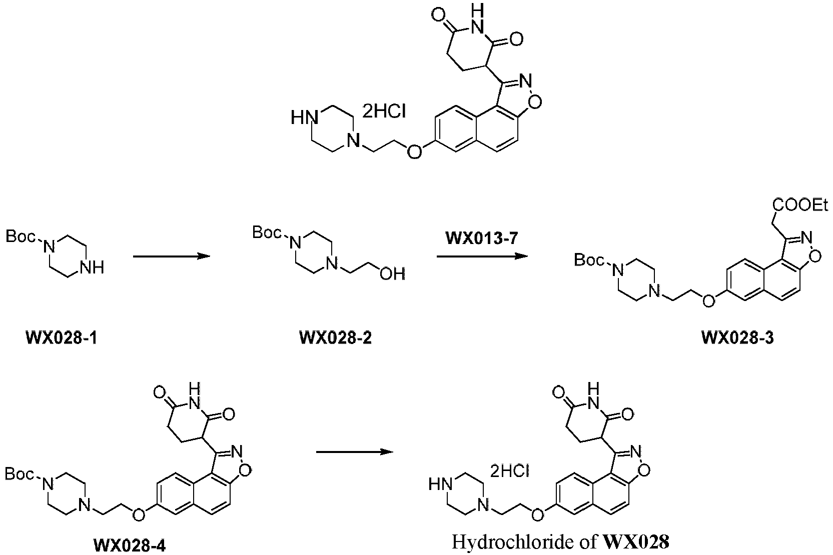

### Step 1: synthesis of intermediate WX028-2

Intermediate **WX028-1** (4 g, 21.48 mmol) was dissolved in acetonitrile (50 mL) at room temperature, and then potassium carbonate (4.45 g, 32.21 mmol) and 2-bromoethanol (3.22 g, 25.77 mmol, 1.83 mL) were added sequentially. The reaction mixture was heated to 50 °C and stirred for 12 h. After the reaction was completed, the reaction mixture was cooled to room temperature and filtered to collect mother solution, which was concentrated under reduced pressure to remove the solvent. The resulting residue was separated by column chromatography (eluent: petroleum ether/ethyl acetate = 1/1-0/1, volume ratio) to give intermediate **WX028-2.** ¹H NMR (400 MHz, CDCl₃) δ: 3.63 (t, *J*=5.2 Hz, 2H), 3.45 (t, *J*=5.0 Hz, 4H), 2.67 (s, 1H), 2.56 (t, *J*=5.4 Hz, 2H), 2.46 (t, *J*=5.0 Hz, 4H), 1.47 (s, 9H).

### Step 2: synthesis of intermediate WX028-3

Intermediate **WX013-7** (1 g, 3.69 mmol) was dissolved in tetrahydrofuran (30 mL) at 15 °C under nitrogen atmosphere, and then intermediate **WX028-2** (933.89 mg, 4.06 mmol) and triphenylphosphine (1.26 g, 4.79 mmol) were added sequentially. After the reaction mixture was cooled to 0 °C, diisopropyl azodicarboxylate (969.05 mg, 4.79 mmol, 931.78 µL) was added dropwise. The resulting reaction mixture was warmed to 15 °C and stirred for 12 h. After the reaction was completed, water (50 mL) was added to the reaction mixture, and 2-methyltetrahydrofuran (100 mL × 5) was added for extraction. The organic phases were combined, dried over anhydrous sodium sulfate, filtered, and concentrated under reduced pressure to remove the solvent. The resulting residue was separated by column chromatography (eluent: petroleum ether/ethyl acetate = 1/1-1/4, volume ratio), the resulting residue from which was then separated by prep-HPLC (mobile phase: water/acetonitrile; neutral system: 10 mM ammonium bicarbonate) to give intermediate **WX028-3.** ¹H NMR (400 MHz, CDCl₃) δ: 7.99 (d, *J*=8.8 Hz, 1H), 7.85 (d, *J*=8.8 Hz, 1H), 7.65 (d, *J*=8.8 Hz, 1H), 7.37-7.29 (m, 2H), 4.35-4.25 (m, 4H), 4.23-4.15 (m, 2H), 3.49 (s, 4H), 3.23 (s, 2H), 2.95 (s, 1H), 2.63 (s, 3H), 1.43 (s, 9H), 1.22-1.11 (m, 3H).

### Step 3: synthesis of intermediate WX028-4

Intermediate **WX028-3** (900 mg, 1.73 mmol, hydrochloride) was dissolved in tetrahydrofuran (20 mL) at room temperature, and then acrylamide (122.96 mg, 1.73 mmol) and a solution of potassium *tert*-butoxide (1 M, 1.73 mL) in tetrahydrofuran were added sequentially. The reaction mixture was stirred at 15 °C for 3 h. After the reaction was completed, the reaction mixture was adjusted to pH 6-7 with ethyl acetate hydrochloride (4 M), and then concentrated under reduced pressure to remove the solvent. The resulting residue was separated by prep-HPLC (mobile phase: acetonitrile/water; acidic system: 0.05% HCl) to give the hydrochloride of intermediate **WX028-4.** ¹H NMR (400 MHz, MeOD_*d₄*) δ: 8.16 (d, *J*=9.2 Hz, 1H), 8.02 (d, *J*=9.2 Hz, 1H), 7.75 (d, *J*=9.2 Hz, 1H), 7.61 (d, *J*=2.8 Hz, 1H), 7.46 (dd, *J*=2.6, 9.0 Hz, 1H), 4.92 (dd, *J*=5.2, 10.0 Hz, 1H), 4.57 (t, *J*=4.8 Hz, 2H), 4.24 (s, 2H), 3.75 (t, *J*=4.8 Hz, 3H), 3.72-3.60 (m, 2H), 3.30-3.18 (m, 3H), 2.95-2.73 (m, 2H), 2.71-2.59 (m, 1H), 2.57-2.45 (m, 1H), 1.49 (s, 9H).

### Step 4: synthesis of WX028

Intermediate **WX028-4** (650 mg, 1.19 mmol, hydrochloride) was dissolved in hydrochloric acid/ethyl acetate (4 M, 20 mL) at room temperature, and the reaction mixture was stirred at 15 °C for 3 h, and white solid was precipitated. After the reaction was completed, the reaction mixture was filtered to collect solid, which was concentrated under reduced pressure to give the hydrochloride of target compound **WX028.** MS-ESI *m*/*z:* 409.1 [M+H]⁺. ¹H NMR (400 MHz, DMSO_*d*₆) δ: 11.13 (s, 1H), 9.62 (s, 2H), 8.18 (d, *J*=9.2 Hz, 1H), 8.13 (d, *J*=8.8 Hz, 1H), 7.92 (d, *J*=8.8 Hz, 1H), 7.73 (d, *J*=2.4 Hz, 1H), 7.45 (dd, *J*=2.6, 9.0 Hz, 1H), 5.03 (dd, *J*=4.6, 11.4 Hz, 1H), 4.56 (s, 2H), 3.64 (s, 2H), 3.47 (s, 8H),2.90-2.79 (m, 1H), 2.70-2.55 (m, 2H), 2.41-2.34 (m, 1H).

### Example 29: WX029

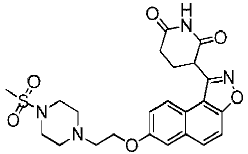

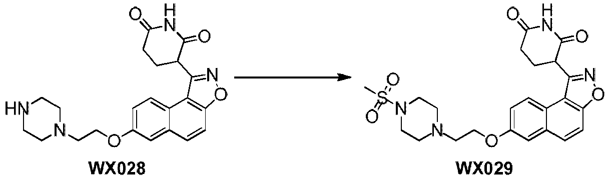

### Step 1: synthesis of WX029

Compound **WX028** (150 mg, 337.15 µmol, hydrochloride) was dissolved in 1,2-dichloroethane (10 mL) at room temperature, and then methanesulfonyl chloride (38.62 mg, 337.15 µmol, 26.09 µL) and triethylamine (170.58 mg, 1.69 mmol, 234.63 µL) were added sequentially. The reaction mixture was stirred at 15 °C for 24 h. After the reaction was completed, the reaction mixture was adjusted to pH 6-7 with ethyl acetate hydrochloride (4 M), and then concentrated under reduced pressure to remove the solvent. The resulting residue was separated by prep-HPLC (mobile phase: acetonitrile/water; neutral system: 10 mM NH₄HCO₃) to give target compound **WX029.** MS-ESI *m*/*z:* 487.1 [M+H]^{+ 1}H NMR (400 MHz, DMSO_*d*₆) δ: 11.13 (s, 1H), 8.14 (d, *J*=8.8 Hz, 1H), 8.09 (d, *J*=9.2 Hz, 1H), 7.89 (d, *J*=8.8 Hz, 1H), 7.68 (d, *J*=2.8 Hz, 1H), 7.37 (dd, *J*=2.4, 9.2 Hz, 1H), 5.02 (dd, *J*=4.8, 11.2 Hz, 1H), 4.25 (t, *J*=5.6 Hz, 2H), 3.13 (t, *J*=4.8 H, 4H), 2.87 (s, 3H), 2.83 (t, *J*=6.0 H, 3H), 2.63 (t, *J*=4.6 H, 4H), 2.60-2.52 (m, 2H), 2.42-2.31 (m, 1H).

### Example 30: WX030

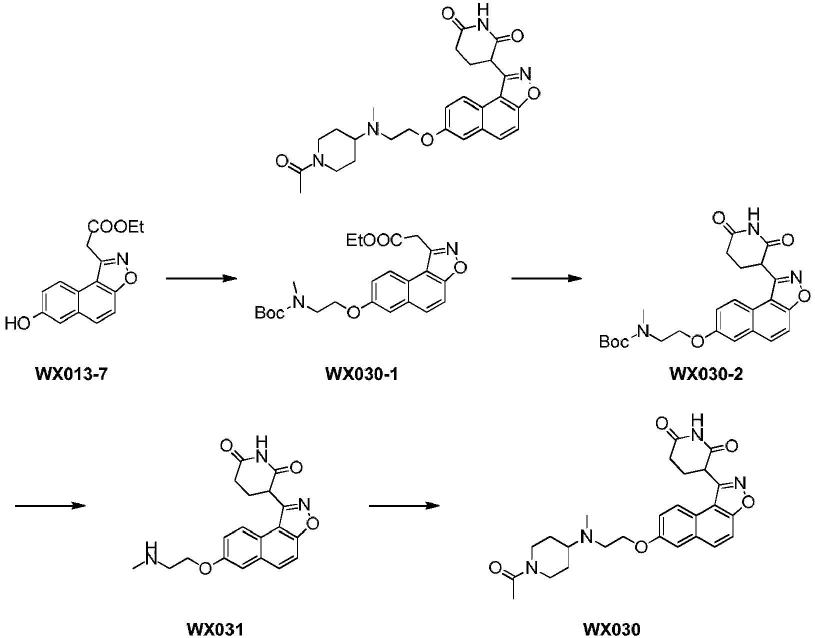

### Step 1: synthesis of intermediate WX030-1

Intermediate **WX013-7** (5 g, 18.43 mmol) was dissolved in tetrahydrofuran (80 mL) at 15 °C under nitrogen atmosphere, and then *N*-*tert*-butoxycarbonyl-*N*-methylaminoethanol (3.88 g, 22.12 mmol, 268.76 µL) and triphenylphosphine (6.28 g, 23.96 mmol) were added sequentially. After the reaction mixture was cooled to 0 °C, diisopropyl azodicarboxylate (4.85 g, 23.96 mmol, 4.66 mL) was added dropwise. The resulting reaction mixture was warmed to 15 °C and stirred for 12 h. After the reaction was completed, the reaction mixture was directly concentrated under reduced pressure to remove the solvent. The resulting residue was separated by column chromatography (eluent: petroleum ether/ethyl acetate = 1/0-4/1, volume ratio) to give intermediate **WX030-1.** ¹H NMR (400 MHz, CDCl₃) δ: 8.03 (d, *J*=8.8 Hz, 1H), 7.86 (d, *J*=9.2 Hz, 1H), 7.68 (d, *J*=8.8 Hz, 1H), 7.39-7.30 (m, 2H), 4.31 (s, 2H), 4.28-4.18 (m, 4H), 3.67 (t, *J*=5.2 Hz, 2H), 3.03 (s, 3H), 1.48 (s, 9H), 1.21 (t, *J*=7.2 Hz, 3H).

### Step 2: synthesis of intermediate WX030-2

Intermediate **WX030-1** (6.6 g, 15.40 mmol) was dissolved in tetrahydrofuran (150 mL) at 15 °C, and then acrylamide (1.09 g, 15.40 mmol) and a solution of potassium *tert*-butoxide (1 M, 13.86 mL) in tetrahydrofuran were added sequentially. The reaction mixture was stirred at 15 °C for 1 h. After the reaction was completed, water (100 mL) was added to the reaction mixture. 1 M diluted hydrochloric acid was added to adjust the pH to 6-7, and ethyl acetate (50 mL × 3) was added for extraction. The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 4/1-1/1, volume ratio) to give intermediate **WX030-2.** ¹H NMR (400 MHz, CDCl₃) δ: 8.36 (s, 1H), 7.94 (d, *J*=9.2 Hz, 1H), 7.88 (d, *J*=9.2 Hz, 1H), 7.69 (d, *J*=9.2 Hz, 1H), 7.36 (s, 1H), 7.33 (dd, *J*=2.8, 8.8 Hz, 1H), 4.74-4.67 (m, 1H), 4.24 (s, 2H), 3.69 (t, *J*=5.2 Hz, 2H), 3.03 (s, 3H), 2.95-2.85 (m, 1H), 2.81-2.64 (m, 2H), 2.61-2.50 (m, 1H), 1.48 (s, 9H).

### Step 3: synthesis of intermediate WX031

Intermediate **WX030-2** (4.1 g, 9.04 mmol) was dissolved in hydrochloric acid/ethyl acetate (4 M, 40 mL) at 15 °C, and the reaction mixture was stirred at 15 °C for 3 h. After the reaction was completed, the reaction mixture was directly filtered to collect the solid, which was concentrated under reduced pressure to remove the solvent, thus giving intermediate **WX031.**

### Step 4: synthesis of WX030

Intermediate **WX031** (100 mg, 256.52 µmol, hydrochloride) was dissolved in 1,2-dichloroethane (10 mL) at 10 °C, and then sodium acetate (21.04 mg, 256.52 µmol) and 1-acetyl-4-piperidone (36.21 mg, 256.52 µmol, 31.49 µL) were added sequentially. After the reaction mixture was stirred for 30 min at 10 °C, sodium triacetoxyborohydride (108.73 mg, 513.04 µmol) was added. After being stirred for 5 h at 10 °C, the reaction mixture was supplemented with sodium acetate (25 mg, 304.80 µmol), and then the reaction mixture was heated to 30 °C and stirred for 12 h. After the reaction was completed, the reaction mixture was directly concentrated under reduced pressure to remove the solvent. The resulting residue was separated by prep-HPLC (mobile phase: acetonitrile/water; acidic system: 0.05% HCl) to give target compound **WX030.** MS-ESI *m*/*z:* 479.2 [M+H]^{+ 1}H NMR (400 MHz, DMSO_*d*₆) δ: 11.13 (s, 1H), 8.19 (d, *J*=8.4 Hz, 1H), 8.13 (d, *J*=9.2 Hz, 1H), 7.94 (d, *J*=9.2 Hz, 1H), 7.73 (d, *J*=2.4 Hz, 1H), 7.43 (dd, *J*=2.0, 8.8 Hz, 1H), 5.03 (dd, *J*=4.4, 11.6 Hz, 1H), 4.55 (s, 3H), 4.05-3.91 (m, 1H), 3.78-3.68 (m, 1H), 3.65-3.51 (m, 3H), 3.13-3.00 (m, 1H), 2.91-2.79 (m, 4H), 2.65-2.55 (m, 2H), 2.41-2.34 (m, 1H), 2.17-2.04 (m, 2H), 2.02 (s, 3H), 1.75-1.45 (m, 2H).

### Example 31: Hydrochloride of WX031

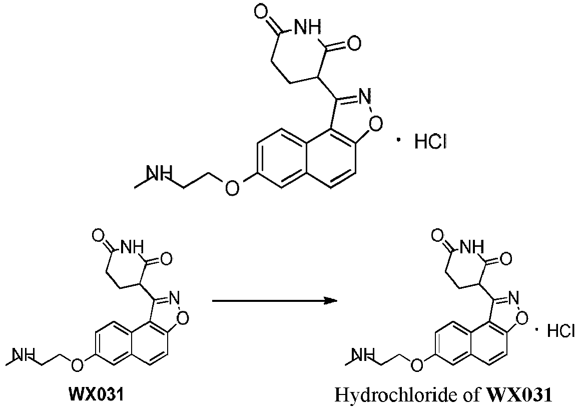

### Step 1: synthesis of WX031

Intermediate **WX031** (90 mg, 254.69 µmol, hydrochloride) was separated by prep-HPLC (mobile phase: acetonitrile/water; acidic system: 0.05% HCl) to give the hydrochloride of target compound **WX031.** MS-ESI *m*/*z:* 354.1 [M+H]⁺. ¹H NMR (400 M Hz, DMSO_*d*₆) δ: 11.13 (s, 1H), 9.14 (s, 2H), 8.20 (d, *J*=8.8 Hz, 1H), 8.14 (d, *J*=9.2 Hz, 1H), 7.92 (d, *J*=9.2 Hz, 1H), 7.72 (d, *J*=2.8 Hz, 1H), 7.42 (dd, *J*=2.8, 9.2 Hz, 1H), 5.04 (dd, *J*=4.6, 11.4 Hz, 1H), 4.43 (t, *J*=5.0 Hz, 2H), 3.45-3.38 (m, 2H), 2.91-2.77 (m, 1H), 2.70-2.55 (m, 5H), 2.43-2.28 (m, 1H).

### Example 32: Hydrochloride of WX032

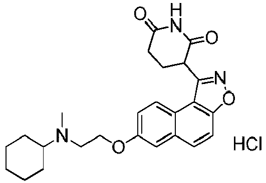

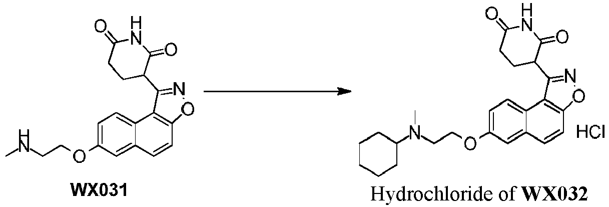

### Step 1: synthesis of WX032

Intermediate **WX031** (120 mg, 339.59 µmol, hydrochloride) was dissolved in 1,2-dichloroethane (10 mL) at 10 °C, and then acetic acid (20.39 mg, 339.59 µmol, 19.42 µL) and cyclohexanone (33.33 mg, 339.59 µmol, 35.19 µL) were added sequentially. After the reaction mixture was stirred at 10 °C for 30 min, sodium triacetoxyborohydride (143.94 mg, 679.17 µmol) was added. The reaction mixture was stirred at 10 °C for 12 h. After the reaction was completed, the reaction mixture was directly concentrated under reduced pressure to remove the solvent. The resulting residue was separated by prep-HPLC (mobile phase: acetonitrile/water; acidic system: 0.05% HCl) to give the hydrochloride of target compound **WX032.** MS-ESI *m*/*z:* 436.2 [M+H]⁺. ¹H NMR (400 MHz, DMSO_*d*₆) δ: 11.12 (s, 1H), 10.85 (s, 1H), 8.19 (d, *J*=8.4 Hz, 1H), 8.13 (d, *J*=9.2 Hz, 1H), 7.92 (d, *J*=9.2 Hz, 1H), 7.74 (d, *J*=2.4 Hz, 1H), 7.41 (dd, *J*=2.2, 9.0 Hz, 1H), 5.04 (dd, *J*=4.6, 11.4 Hz, 1H), 4.67-4.52 (m, 2H), 3.71-3.61 (m, 1H), 3.55-3.44 (m, 1H), 3.35-3.25 (m, 1H), 2.91-2.80 (m, 1H), 2.79 (d, *J*=4.8 Hz, 3H), 2.70-2.55 (m, 2H), 2.42-2.31 (m, 1H), 2.20-2.05 (m, 2H), 1.87-1.76 (m, 2H), 1.62 (d, *J*=12.4 Hz, 1H), 1.54-1.39 (m, 2H), 1.37-1.22 (m, 2H), 1.20-1.04 (m, 1H).

### Example 33: Hydrochloride of WX033

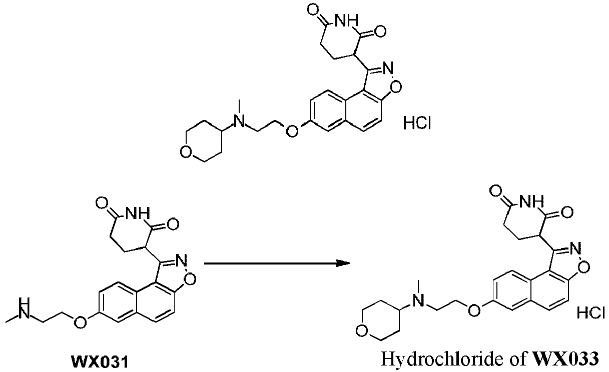

### Step 1: synthesis of WX033

Intermediate **WX031** (100 mg, 282.99 µmol, hydrochloride) was dissolved in 1,2-dichloroethane (5 mL) at 10 °C, and then acetic acid (16.99 mg, 282.99 µmol, 16.18 µL) and tetrahydropyranone (28.33 mg, 282.99 µmol. 25.99 µL) were added sequentially. After the reaction mixture was stirred at 10 °C for 30 min, sodium triacetoxyborohydride (119.95 mg, 565.98 µmol) was added. The resulting reaction mixture was stirred at 10 °C for 12 h. After the reaction was completed, the reaction mixture was directly concentrated under reduced pressure to remove the solvent. The resulting residue was separated by prep-HPLC (mobile phase: acetonitrile/water; acidic system: 0.05% HCl) to give the hydrochloride of target compound **WX033.** MS-ESI *m*/*z:* 438.1 [M+H]^{+ 1}H NMR (400 MHz, DMSO_*d*₆) δ: 11.13 (s, 1H), 11.04 (s, 1H), 8.19 (d, *J*=9.2 Hz, 1H), 8.13 (d, *J*=8.8 Hz, 1H), 7.92 (d, *J*=8.8 Hz, 1H), 7.74 (d, *J*=2.8 Hz, 1H), 7.42 (dd, *J*=2.8, 9.2 Hz, 1H), 5.04 (dd, *J*=4.8, 11.2 Hz, 1H), 4.67-4.53 (m, 2H), 4.03-3.92 (m, 2H), 3.75-3.65 (m, 1H), 3.58-3.47 (m, 2H), 3.34 (td, *J*=1.6, 12.0 Hz, 2H), 2.92-2.83 (m, 1H), 2.84 (d, *J*=5.6 Hz, 3H), 2.69-2.55 (m, 2H), 2.43-2.30 (m, 1H), 2.13-1.97 (m, 2H), 1.84-1.69 (m, 2H).

### Example 34: Hydrochloride of WX034

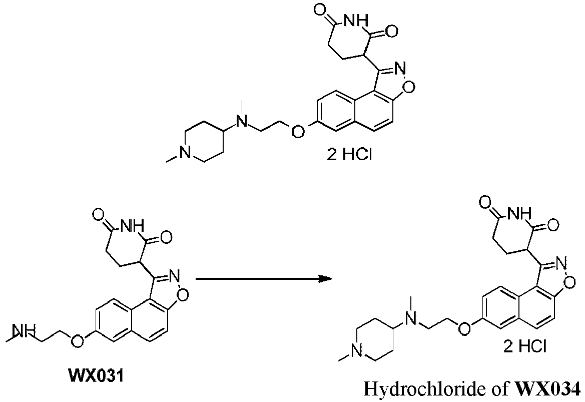

### Step 1: synthesis of WX034

Intermediate **WX031** (200 mg, 565.98 µmol, hydrochloride) was dissolved in 1,2-dichloroethane (10 mL) at 10 °C, and then acetic acid (33.99 mg, 565.98 µmol, 32.37 µL) and *N*-methyl-4-piperidone (64.04 mg, 565.98 µmol, 65.82 µL) were added sequentially. After the reaction mixture was stirred at 10 °C for 30 min, sodium triacetoxyborohydride (239.91 mg, 1.13 mmol) was added. The resulting reaction mixture was heated to 30 °C and stirred for 12 h. After the reaction was completed, the reaction mixture was directly concentrated under reduced pressure to remove the solvent. The resulting residue was separated by prep-HPLC (mobile phase: acetonitrile/water; acidic system: 0.05% HCl) to give the hydrochloride of target compound **WX034.** MS-ESI *m*/*z:* 451.2 [M+H]^{+ 1}H NMR (400 MHz, DMSO_*d*₆) δ: 11.49 (s, 1H), 11.13 (s, 1H), 11.05 (s, 1H), 8.18 (d, *J*=8.0 Hz, 1H), 8.13 (d, *J*=9.2 Hz, 1H), 7.92 (d, *J*=9.2 Hz, 1H), 7.74 (s, 1H), 7.44 (d, *J*=8.4 Hz, 1H), 5.04 (dd, *J*=2.8, 9.6 Hz, 1H), 4.61 (s, 2H), 3.76-3.52 (m, 6H), 3.15-2.97 (m, 2H), 2.92-2.80 (m, 4H), 2.77-2.61 (m, 4H), 2.44-2.30 (m, 2H), 2.26-2.12 (m, 2H).

### Example 35: WX035

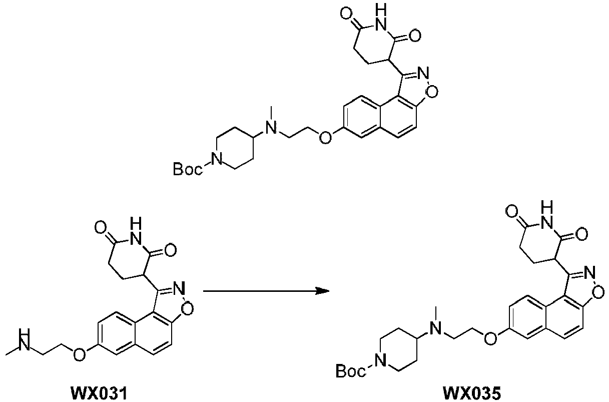

### Step 1: synthesis of WX035

Intermediate **WX031** (1 g, 2.57 mmol, hydrochloride) was dissolved in 1,2-dichloroethane (40 mL) at 10 °C, and then sodium acetate (464.29 mg, 5.66 mmol) and *N*-*tert*-butoxycarbonyl-4-piperidone (620.23 mg, 3.11 mmol, 65.82 µL) were added sequentially. After the reaction mixture was stirred at 10 °C for 30 min, sodium triacetoxyborohydride (1.2 g, 5.66 mmol) was added. The resulting reaction mixture was heated to 30 °C and stirred for 12 h. After the reaction was completed, water (50 mL) was added to the reaction mixture, and ethyl acetate (30 mL × 3) was added for extraction. The organic phases were combined, dried over anhydrous sodium sulfate, filtered and concentrated under reduced pressure to remove the solvent. The resulting residue was separated by prep-HPLC (mobile phase: acetonitrile/water; acidic system: 0.05% HCl), the resulting residue from which was then separated by prep-HPLC (mobile phase: acetonitrile/water; neutral system: 10 mM NH₄HCO₃) to give target compound **WX035.** MS-ESI *m*/*z:* 537.3 [M+H]⁺.¹H NMR (400 MHz, DMSO_*d*₆) δ: 11.13 (s, 1H), 8.14 (d, *J*=9.2 Hz, 1H), 8.10 (d, *J*=9.2 Hz, 1H), 7.88 (d, *J*=9.2 Hz, 1H), 7.66 (d, *J*=2.8 Hz, 1H), 7.35 (dd, *J*=2.6, 9.0 Hz, 1H), 5.02 (dd, *J*=4.8, 11.2 Hz, 1H), 4.18 (t, *J*=5.8 Hz, 2H), 3.97 (d, *J*=11.2 Hz, 2H), 2.92-2.79 (m, 3H), 2.76-2.54 (m, 5H), 2.42-2.33 (m, 1H), 2.30 (s, 3H), 1.71 (d, *J*=12.0 Hz, 2H), 1.38 (s, 9H), 1.34-1.20 (m, 2H).

### Example 36: Hydrochloride of WX036

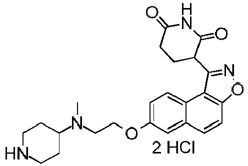

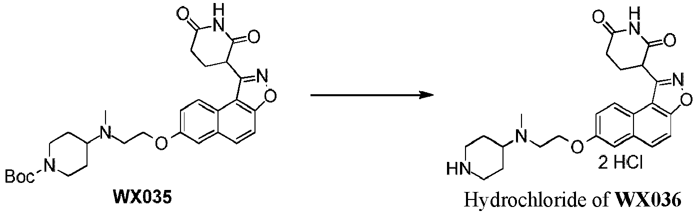

### Step 1: synthesis of WX036

Intermediate **WX035** (0.9 g, 1.68 mmol) was dissolved in hydrochloric acid/ethyl acetate (4 M, 10 mL) at room temperature, and the reaction mixture was stirred at 15 °C for 12 h. After the reaction was completed, the reaction mixture was directly filtered to collect the solid, which was concentrated under reduced pressure to remove the solvent. The resulting residue was separated by prep-HPLC (mobile phase: acetonitrile/water; acidic system: 0.05% HCl) to give the hydrochloride of target compound **WX036.** MS-ESI *m*/*z:* 437.2 [M+H]⁺.¹H NMR (400 MHz, DMSO_*d*₆) δ: 11.38 (s, 1H), 11.13 (s, 1H), 9.25 (s, 1H), 9.13 (s, 1H), 8.19 (d, *J*=8.8 Hz, 1H), 8.13 (d, *J*=9.2 Hz, 1H), 7.93 (d, *J*=8.8 Hz, 1H), 7.74 (d, *J*=2.4 Hz, 1H), 7.44 (dd, *J*=2.0, 9.2 Hz, 1H), 5.04 (dd, *J*=4.6, 11.4 Hz, 1H), 4.68-4.52 (m, 2H), 3.72-3.52 (m, 3H), 3.49-3.40 (m, 3H), 3.02-2.76 (m, 6H), 2.71-2.54 (m, 2H), 2.41-2.19 (m, 2H), 2.11-1.94 (m, 2H).

### Example 37: Hydrochloride of WX037

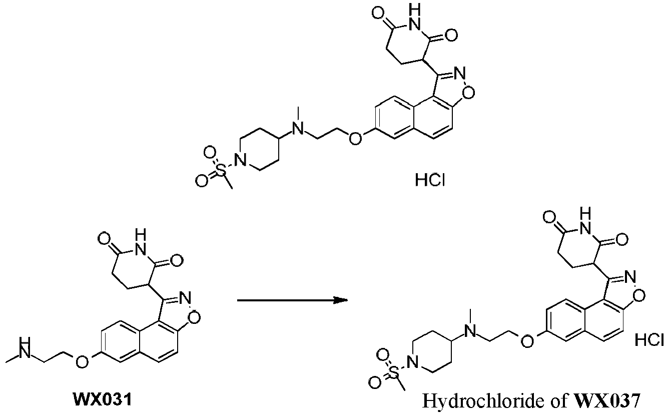

### Step 1: synthesis of WX037

Intermediate **WX031** (150 mg, 384.78 µmol, hydrochloride) was dissolved in 1,2-dichloroethane (5 mL) at room temperature, and then sodium acetate (63.13 mg, 769.56 µmol) and 1-*N*-methanesulfonyl-4-piperidone (68.19 mg, 384.78 µmol) were added sequentially. After the reaction mixture was stirred at 10 °C for 30 min, sodium triacetoxyborohydride (163.10 mg, 769.56 µmol) was added. The resulting reaction mixture was heated to 30 °C and stirred for 12 h. After the reaction was completed, the reaction mixture was directly concentrated under reduced pressure to remove the solvent. All the residue was separated by prep-HPLC (mobile phase: acetonitrile/water; acidic system: 0.05% HCl) to give the hydrochloride of target compound **WX037.** MS-ESI *m*/*z:* 515.2 [M+H]⁺. ¹H NMR (400 MHz, DMSO_*d*₆) δ: 11.14 (s, 1H), 10.97 (s, 1H), 8.19 (d, *J*=8.8 Hz, 1H), 8.13 (d, *J*=9.2 Hz, 1H), 7.93 (d, *J*=9.2 Hz, 1H), 7.74 (d, *J*=2.4 Hz, 1H), 7.44 (dd, *J*=2.6, 9.0 Hz, 1H), 5.04 (dd, *J*=4.6, 11.4 Hz, 1H), 4.64-4.52 (m, 2H), 3.77-3.67 (m, 3H), 3.61-3.52 (m, 2H), 2.92 (s, 3H), 2.88-2.74 (m, 6H), 2.71-2.55 (m, 2H), 2.43-2.31 (m, 1H), 2.30-2.14 (m, 2H), 1.87-1.71 (m, 2H).

### Example 38: Hydrochloride of WX038

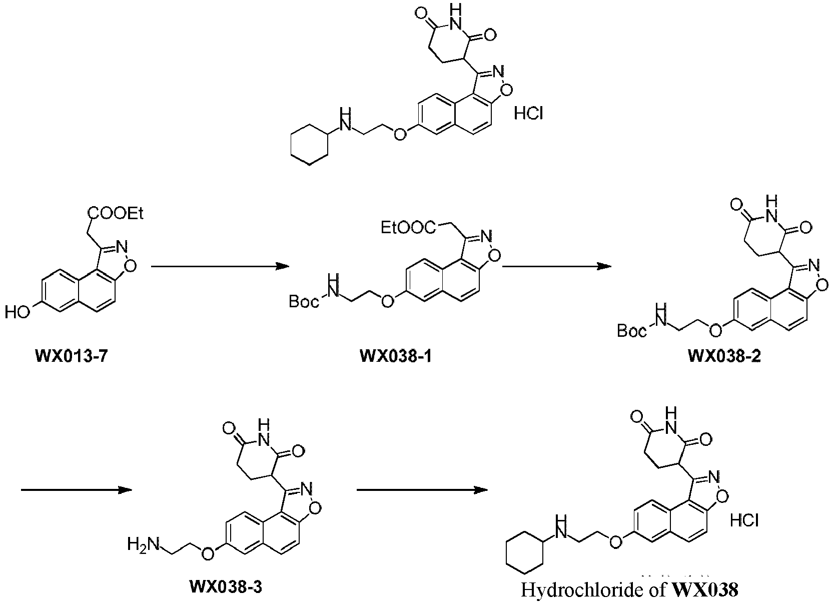

### Step 1: synthesis of intermediate WX038-1

Intermediate **WX013-7** (5 g, 18.43 mmol) was dissolved in tetrahydrofuran (80 mL) at 15 °C under nitrogen atmosphere, and then *N*-*tert*-butoxycarbonyl-ethanolamine (3.86 g, 23.96 mmol, 3.71 mL) and triphenylphosphine (6.28 g, 23.96 mmol) were added sequentially. After the reaction mixture was cooled to 0 °C, diisopropyl azodicarboxylate (4.85 g, 23.96 mmol, 4.66 mL) was added dropwise. The resulting reaction mixture was warmed to 15 °C and stirred for 12 h. After the reaction was completed, the reaction mixture was directly concentrated under reduced pressure to remove the solvent. The resulting residue was separated by column chromatography (eluent: petroleum ether/ethyl acetate = 1/0-4/1, volume ratio) to give intermediate **WX038-1.** ¹H NMR (400 MHz, CDCl₃) δ: 8.02 (d, *J*=8.8 Hz, 1H), 7.84 (d, *J*=8.8 Hz, 1H), 7.66 (d, *J*=9.2 Hz, 1H), 7.35-7.30 (m, 2H), 6.48 (s, 1H), 4.30 (s, 2H), 4.21 (q, *J*=7.4 Hz, 2H), 4.16 (t, *J*=5.0 Hz, 2H), 3.60 (q, *J*=5.0 Hz, 2H), 1.46 (s, 9H), 1.20 (t, *J*=7.0 Hz, 3H).

### Step 2: synthesis of intermediate WX038-2

Intermediate **WX038-1** (5 g, 12.06 mmol) was dissolved in tetrahydrofuran (100 mL) at 15 °C, and then acrylamide (857.49 mg, 12.06 mmol) and a solution of potassium *tert*-butoxide (1 M, 12.06 mL) in tetrahydrofuran were added sequentially. The reaction mixture was stirred at 15 °C for 1 h. After the reaction was completed, water (200 mL) was added to the reaction mixture. 1 M diluted hydrochloric acid was added to adjust the pH to 6-7, and ethyl acetate (100 mL × 3) was added for extraction. The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 4/1-1/1, volume ratio) to give intermediate **WX038-2.** ¹HNMR (400 MHz, DMSO_*d*₆) δ: 11.12 (s, 1H), 8.16 (d, *J*=8.8 Hz, 1H), 8.10 (d, *J*=9.2 Hz, 1H), 7.89 (d, *J*=9.2 Hz, 1H), 7.65 (d, *J*=2.8 Hz, 1H), 7.35 (dd, *J*=2.4, 9.2 Hz, 1H), 7.08 (t, *J*=5.4 Hz, 1H), 5.02 (dd, *J*=4.8, 11.2 Hz, 1H), 4.12 (t, *J*=5.6 Hz, 2H), 3.37 (q, *J*=5.6 Hz, 2H), 2.92-2.77 (m, 1H), 2.70-2.54 (m, 2H), 2.44-2.30 (m, 1H), 1.39 (s, 9H).

### Step 3: synthesis of intermediate WX038-3

Intermediate **WX038-2** (3.1 g, 7.05 mmol) was dissolved in hydrochloric acid/ethyl acetate (4 M, 78.73 mL) at 15 °C, and the reaction mixture was stirred at 15 °C for 12 h and a yellowish solid was generated. After the reaction was completed, the reaction mixture was directly filtered to collect the solid, which was concentrated under reduced pressure to remove the solvent, thus giving intermediate **WX038-3.** ¹H NMR (400 MHz, DMSO_*d*₆) δ: 11.12 (s, 1H), 8.29 (s, 2H), 8.20 (d, *J*=8.8 Hz, 1H), 8.14 (d, *J*=9.2 Hz, 1H), 7.92 (d, *J*=9.2 Hz, 1H), 7.71 (d, *J*=2.8 Hz, 1H), 7.41 (dd, *J*=2.4, 9.2 Hz, 1H), 5.04 (dd, *J*=4.8, 11.2 Hz, 1H), 4.36 (t, *J*=5.0 Hz, 2H), 3.30 (s, 2H), 2.91-2.79 (m, 1H), 2.70-2.54 (m, 2H), 2.43-2.34 (m, 1H).

### Step 4: synthesis of WX038

Intermediate **WX038-3** (100 mg, 266.10 µmol, hydrochloride) was dissolved in 1,2-dichloroethane (5 mL) at 10 °C, and then sodium acetate (43.66 mg, 532.19 µmol) and cyclohexanone (26.12 mg, 266.10 µmol, 27.58 µL) were added sequentially. After the reaction mixture was stirred at 10 °C for 30 min, sodium triacetoxyborohydride (112.79 mg, 532.19 µmol) was added. The resulting reaction mixture was heated to 30 °C and stirred for 12 h. After the reaction was completed, the reaction mixture was directly concentrated under reduced pressure to remove the solvent. The resulting residue was separated by prep-HPLC (mobile phase: acetonitrile/water; acidic system: 0.05% HCl) to give the hydrochloride of target compound **WX038.** MS-ESI *m*/*z:* 422.1 [M+H]⁺.¹H NMR (400 MHz, DMSO_*d*₆) δ: 11.13 (s, 1H), 8.96 (s, 2H), 8.20 (d, *J*=9.2 Hz, 1H), 8.14 (d, *J*=9.2 Hz, 1H), 7.93 (d, *J*=9.2 Hz, 1H), 7.72 (d, *J*=2.4 Hz, 1H), 7.42 (dd, *J*=2.6, 9.0 Hz, 1H), 5.03 (dd, *J*=4.8, 11.2 Hz, 1H), 4.44 (t, *J*=5.2 Hz, 2H), 3.51-3.39 (m, 2H), 3.17-3.05 (m, 1H), 2.93-2.79 (m, 1H), 2.70-2.55 (m, 2H), 2.44-2.33 (m, 1H), 2.10 (d, *J*=10.4 Hz, 2H), 1.79 (d, *J*=13.2 Hz, 2H), 1.62 (d, *J*=12.0 Hz, 1H), 1.44-1.09 (m, 5H).

### Example 39: Hydrochloride of WX039

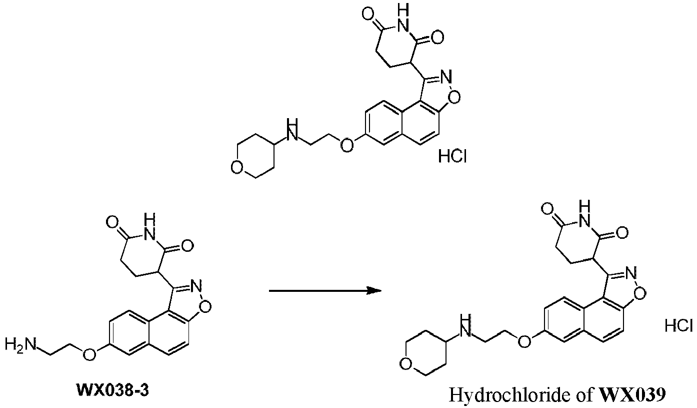

### Step 1: synthesis of WX039

Intermediate WX038-3 (150 mg, 399.14 µmol, hydrochloride) was dissolved in 1,2-dichloroethane (5 mL) at 10 °C, and then sodium acetate (65.48 mg, 798.29 µmol) and tetrahydropyranone (39.96 mg, 399.14 µmol, 36.66 µL) were added sequentially. After the reaction mixture was stirred at 10 °C for 30 min, sodium triacetoxyborohydride (169.19 mg, 798.29 µmol) was added. The resulting reaction mixture was heated to 30 °C and stirred for 12 h. After the reaction was completed, the reaction mixture was directly concentrated under reduced pressure to remove the solvent. The resulting residue was separated by prep-HPLC (mobile phase: acetonitrile/water; acidic system: 0.05% HCl) to give the hydrochloride of target compound **WX039.** MS-ESI *m*/*z:* 424.2 [M+H]^{+ 1}H NMR (400 MHz, DMSO_*d*₆) δ: 11.12 (s, 1H), 9.03 (s, 2H), 8.21 (d, *J*=9.2 Hz, 1H), 8.15 (d, *J*=9.2 Hz, 1H), 7.93 (d, *J*=9.2 Hz, 1H), 7.73 (d, *J*=2.8 Hz, 1H), 7.43 (dd, *J*=2.6, 9.0 Hz, 1H), 5.03 (dd, *J*=4.6, 11.4 Hz, 1H), 4.43 (t, *J*=4.8 Hz, 2H), 3.94 (dd, *J*=4.0, 10.8 Hz, 2H), 3.53-3.35 (m, 5H), 2.92-2.78 (m, 1H), 2.65-2.55 (m, 2H), 2.43-2.34 (m, 1H), 2.06-1.96 (m, 2H), 1.70-1.56 (m, 2H).

### Example 40: Hydrochloride of WX040

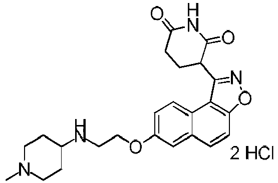

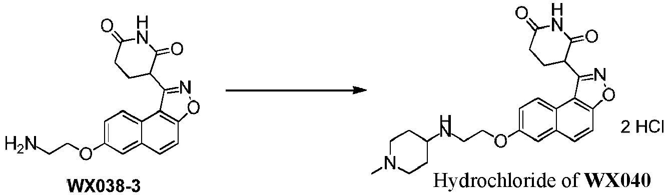

### Step 1: synthesis of WX040

Intermediate **WX038-3** (150 mg, 399.14 µmol, hydrochloride) was dissolved in 1,2-dichloroethane (5 mL) at 10 °C, and then sodium acetate (65.48 mg, 798.28 µmol) and *N*-methyl-4-piperidone (45.17 mg, 399.14 µmol, 46.42 µL) were added sequentially. After the reaction mixture was stirred at 10 °C for 30 min, sodium triacetoxyborohydride (169.19 mg, 798.28 µmol) was added. The resulting reaction mixture was heated to 30 °C and stirred for 12 h. After the reaction was completed, water (30 mL) was added to the reaction mixture, and ethyl acetate (20 mL × 3) was added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give the hydrochloride of target compound **WX040.** MS-ESI *m*/*z:* 437.2 [M+H]^{+ 1}H NMR (400 MHz, DMSO_*d₆*) δ: 11.13 (s, 1H), 10.81 (s, 1H), 9.71 (s, 2H), 8.19 (d, *J*=8.4 Hz, 1H), 8.14 (d, *J*=8.8 Hz, 1H), 7.92 (d, *J*=8.8 Hz, 1H), 7.73 (s, 1H), 7.43 (d, *J*=8.0 Hz, 1H), 5.03 (dd, *J*=4.6, 11.4 Hz, 1H), 4.58-4.48 (m, 2H), 3.62-3.42 (m, 2H), 3.10-2.93 (m, 2H), 2.92-2.54 (m, 8H), 2.42-2.16 (m, 4H), 2.14-1.97 (m, 2H).

### Example 41: Hydrochloride of WX041

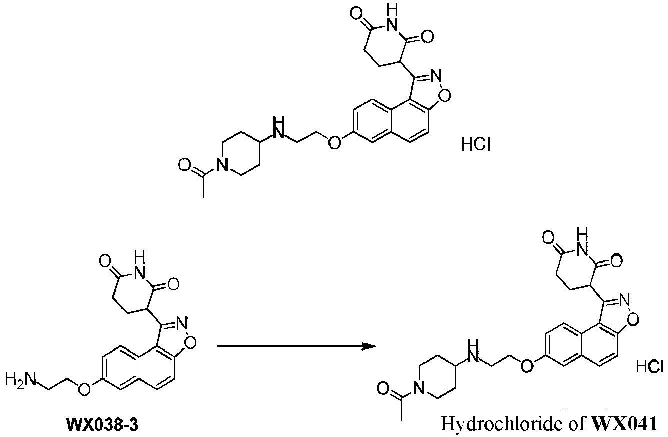

### Step 1: synthesis of WX041

Intermediate **WX038-3** (150 mg, 399.14 µmol, hydrochloride) was dissolved in 1,2-dichloroethane (5 mL) at 10 °C, and then sodium acetate (65.48 mg, 798.29 µmol) and 1-acetyl-4-piperidone (56.35 mg, 399.14 µmol, 49.00 µL) were added sequentially. After the reaction mixture was stirred at 10 °C for 30 min, sodium triacetoxyborohydride (169.19 mg, 798.29 µmol) was added. The resulting reaction mixture was heated to 30 °C and stirred for 12 h. After the reaction was completed, water (30 mL) was added to the reaction mixture, and ethyl acetate (20 mL × 3) was added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give the hydrochloride of target compound **WX041.** MS-ESI *m*/*z:* 465.2 [M+H]^{+ 1}H NMR (400 MHz, DMSO_*d₆*) δ: 11.12 (s, 1H), 9.27 (s, 2H), 8.20 (*J*=8.8 Hz, 1H), 8.14 (d, *J*=9.2 Hz, 1H), 7.93 (d, *J*=9.2 Hz, 1H), 7.73 (d, *J*=2.8 Hz, 1H), 7.43 (dd, *J*=2.6, 9.0 Hz, 1H), 5.03 (dd, *J*=4.4, 11.2 Hz, 1H), 4.46 (t, *J*=4.8 Hz, 3H), 3.92 (d, *J*=13.2 Hz, 1H), 3.52-3.43 (m, 3H), 3.06 (t, *J*=12.4 Hz, 1H), 2.91-2.78 (m, 1H), 2.69-2.55 (m, 3H), 2.42-2.34 (m, 1H), 2.19-2.05 (m, 2H), 2.02 (s, 3H), 1.66-1.36 (m, 2H).

### Example 42: Hydrochloride of WX042

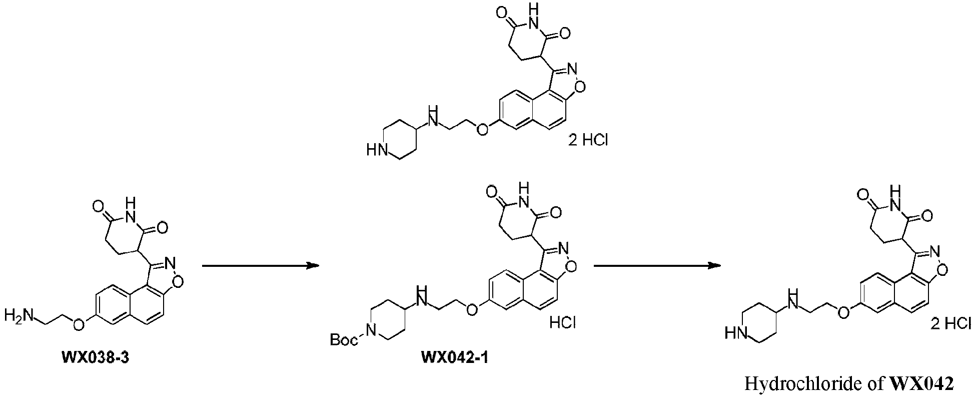

### Step 1: synthesis of intermediate WX042-1

Intermediate **WX038-3** (300 mg, 798.29 µmol, hydrochloride) was dissolved in 1,2-dichloroethane (20 mL) at 10 °C, and then sodium acetate (130.97 mg, 1.60 mmol) and *N*-*tert*-butoxycarbonyl-4-piperidone (159.06 mg, 798.29 µmol, 46.42 µL) were added sequentially. After the reaction mixture was stirred at 10 °C for 30 min, sodium triacetoxyborohydride (338.38 mg, 1.60 mmol) was added. The resulting reaction mixture was heated to 30 °C and stirred for 12 h. After the reaction was completed, water (30 mL) was added to the reaction mixture for dilution, and ethyl acetate (20 mL × 3) was added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give intermediate **WX042-1.** ¹H NMR (400 MHz, DMSO_*d*₆) δ: 11.12 (s, 1H), 8.99 (s, 2H), 8.20 (d, *J*=9.6 Hz, 1H), 8.14 (d, *J*=9.2 Hz, 1H), 7.93 (d, *J*=9.2 Hz, 1H), 7.72 (d, *J*=2.4 Hz, 1H), 7.42 (dd, *J*=2.6, 9.0 Hz, 1H), 5.03 (dd, *J*=4.6, 11.4 Hz, 1H), 4.43 (t, *J*=4.2 Hz, 2H), 4.02 (d, *J*=11.6 Hz, 2H), 3.52-3.40 (m, 2H), 2.93-2.73 (m, 3H), 2.67-2.55 (m, 3H), 2.44-2.35 (m, 1H), 2.07 (d, *J*=11.2 Hz, 2H), 1.55-1.44 (m, 2H), 1.41 (s, 9H).

### Step 2: synthesis of WX042

Intermediate **WX042-1** (200 mg, 357.75 µmol, hydrochloride) was dissolved in hydrochloric acid/ethyl acetate (4 M, 10 mL) at 15 °C, and the reaction mixture was stirred at 15 °C for 12 h and a solid was precipitated. After the reaction was completed, the reaction mixture was filtered to collect the solid, which was concentrated under reduced pressure to remove the solvent. The resulting residue was separated by prep-HPLC (mobile phase: acetonitrile/water; acidic system: 0.05% HCl) to give the hydrochloride of target compound **WX042.** MS-ESI *m*/*z:* 423.2 [M+H]^{+ 1}H NMR (400 MHz, DMSO_*d₆*) δ: 11.13 (s, 1H), 9.77 (s, 2H), 9.33 (s, 1H), 9.08 (d, *J*=8.8 Hz, 1H), 8.20 (d, *J*=8.8 Hz, 1H), 8.14 (d, *J*=9.2 Hz, 1H), 7.92 (d, *J*=9.2 Hz, 1H), 7.73 (d, *J*=2.4 Hz, 1H), 7.44 (dd, *J*=2.4, 9.2 Hz, 1H), 5.04 (dd, *J*=4.8, 11.2 Hz, 1H), 4.49 (t, *J*=4.2 Hz, 2H), 3.51-3.36 (m, 6H), 3.03-2.80 (m, 3H), 2.74-2.54 (m, 2H), 2.43-2.21 (m, 2H), 2.02-1.85 (m, 2H).

### Example 43: Hydrochloride of WX043

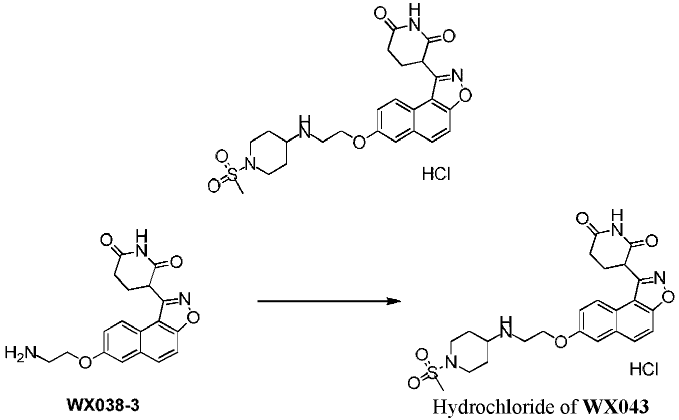

### Step 1: synthesis of WX043

Intermediate **WX038-3** (150 mg, 399.14 µmol, hydrochloride) was dissolved in 1,2-dichloroethane (5 mL) at 10 °C, and then sodium acetate (65.48 mg, 798.29 µmol) and 1-*N*-methanesulfonyl-4-piperidone (70.74 mg, 399.14 µmol) were added sequentially. After the reaction mixture was stirred at 10 °C for 30 min, sodium triacetoxyborohydride (169.19 mg, 798.28 µmol) was added. The resulting reaction mixture was heated to 30 °C and stirred for 12 h. After the reaction was completed, the reaction mixture was directly concentrated under reduced pressure to remove the solvent. The resulting residue was separated by prep-HPLC (mobile phase: acetonitrile/water; acidic system: 0.05% HCl) to give the hydrochloride of target compound **WX043.** MS-ESI *m*/*z:* 501.1 [M+H]^{+ 1}H NMR (400 MHz, DMSO_*d₆*) δ: 11.13 (s, 1H), 9.38 (s, 2H), 8.21 (d, *J*=8.8 Hz, 1H), 8.15 (d, *J*=9.2 Hz, 1H), 7.93 (d, *J*=9.2 Hz, 1H), 7.73 (d, *J*=2.4 Hz, 1H), 7.44 (dd, *J*=2.6, 9.0 Hz, 1H), 5.04 (dd, *J*=4.6, 11.4 Hz, 1H), 4.47 (t, *J*=8.4 Hz, 2H), 3.67 (d, *J*=12.0 Hz, 2H), 3.53-3.41 (m, 2H), 2.91 (s, 3H), 2.89-2.75 (m, 3H), 2.71-2.54 (m, 3H), 2.44-2.31 (m, 1H), 2.26-2.15 (m, 2H), 1.79-1.64 (m, 2H).

### Example 44: WX044

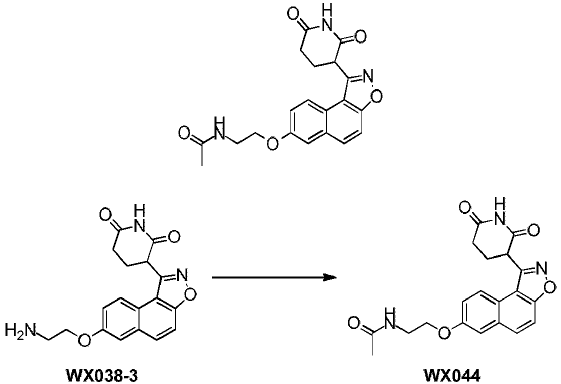

### Step 1: synthesis of WX044

Intermediate **WX038-3** (200 mg, 532.19 µmol, hydrochloride) was dissolved in 1,2-dichloroethane (10 mL) at 15 °C, and then triethylamine (269.26 mg, 2.66 mmol, 370.37 µL) and acetyl chloride (54.31 mg, 691.85 µmol, 49.37 µL) were added sequentially. The reaction mixture was stirred at 15 °C for 12 h. After the reaction was completed, 4 M ethyl acetate hydrochloride was added dropwise slowly to the reaction mixture to adjust pH to 6-7, and the reaction mixture was then concentrated under reduced pressure to remove the solvent. The resulting residue was separated by prep-HPLC (mobile phase: acetonitrile/water; acidic system: 0.05% HCl) to give target compound **WX044.** MS-ESI *m*/*z:* 382.1 [M+H]^{+ 1}H NMR (400 MHz, DMSO_*d*₆) δ: 11.12 (s, 1H), 8.17 (d, *J*=3.2 Hz, 1H), 8.15 (s, 1H), 8.10 (d, *J*=9.2 Hz, 1H), 7.89 (d, *J*=9.2 Hz, 1H), 7.67 (d, *J*=2.0 Hz, 1H), 7.37 (dd, *J*=2.2, 9.0 Hz, 1H), 5.02 (dd, *J*=4.6, 11.0 Hz, 1H), 4.14 (t, *J*=5.4 Hz, 2H), 3.49 (q, *J*=5.8 Hz, 2H), 2.91-2.78 (m, 1H), 2.69-2.54 (m, 2H), 2.42-2.30 (m, 1H), 1.85 (s, 3H).

### Example 45: WX045

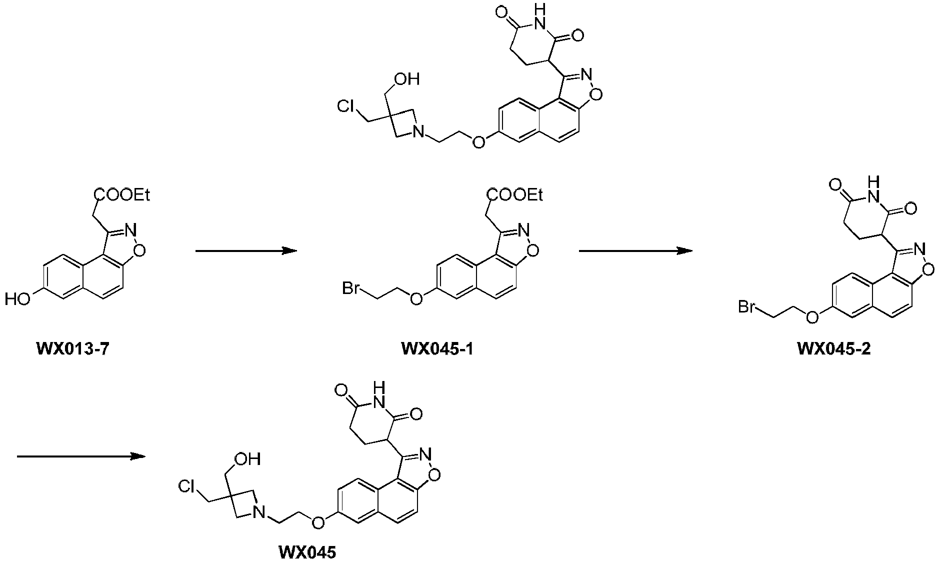

### Step 1: synthesis of intermediate WX045-1

Intermediate **WX013-7** (2 g, 7.37 mmol), bromoethanol (1.01 g, 8.11 mmol, 575.84 µL) and triphenylphosphine (2.51 g, 9.58 mmol) were dissolved in tetrahydrofuran (60 mL) at room temperature under nitrogen atmosphere. After the reaction mixture was cooled to 0 °C, diisopropyl azodicarboxylate (1.94 g, 9.58 mmol, 1.86 mL) was added dropwise. The resulting reaction mixture was warmed to room temperature and stirred for 12 h. After the reaction was completed, the reaction mixture was poured into water (40 mL), and ethyl acetate (50 mL × 3) was added for extraction. The organic phases were combined, washed with saturated brine (80 mL), dried over anhydrous sodium sulfate, filtered, and concentrated under reduced pressure to remove the solvent. The resulting residue was separated by column chromatography (eluent: petroleum ether/ethyl acetate = 1/0-10/1, volume ratio) to give intermediate **WX045-1.**

### Step 2: synthesis of intermediate WX045-2

Intermediate **WX045-1** (1 g, 2.64 mmol) and acrylamide (187.93 mg, 2.64 mmol) were dissolved in tetrahydrofuran (30 mL) at room temperature, and then a solution of potassium *tert*-butoxide in tetrahydrofuran (1 M, 2.64 mL) was added dropwise. The reaction mixture was stirred at 20 °C for 2 h. After the reaction was completed, the reaction mixture was poured into ice water (70 mL), and 2-methyltetrahydrofuran (80 mL × 3) was added for extraction. The organic phases were combined, washed with saturated brine (100 mL), dried over anhydrous sodium sulfate, filtered, and concentrated under reduced pressure to remove the solvent. The resulting residue was separated by column chromatography (eluent: petroleum ether/ethyl acetate = 1/0-3/1, volume ratio) to give intermediate **WX045-2.**

### Step 3: synthesis of WX045

Intermediate **WX045-2** (0.3 g, 744.00 µmol) and 2-oxa-6-aza-spiro[3,3]heptane (73.75 mg, 744.00 µmol) were dissolved in *N,N-*dimethylformamide (3 mL) at room temperature, and then potassium iodide (123.51 mg, 744.00 µmol) and triethylamine (75.29 mg, 744.00 µmol, 103.56 µL) were added. The reaction mixture was stirred at 20 °C for 12 h. After the reaction was completed, the reaction mixture was adjusted to pH 6-7 with ethyl acetate hydrochloride (4 M), and then concentrated under reduced pressure to remove the solvent. The resulting residue was separated by prep-HPLC (mobile phase: acetonitrile/water; acidic system: 0.05% HCl) to give target compound **WX045.** MS-ESI *m*/*z:* 458.1 [M+H]⁺. ¹H NMR (400 MHz, MeOD_*d₄*) δ: 8.18 (d, *J*=8.8 Hz, 1H), 8.04 (d, *J*=9.2 Hz, 1H), 7.78 (d, *J*=9.2 Hz, 1H), 7.61 (t, *J*=2.4 Hz, 1H), 7.49-7.40 (m, 1H), 4.94 (dd, *J*=5.2, 10.4 Hz, 1H), 4.47-4.43 (m, 2H), 4.36-4.26 (m, 4H), 3.90 (s, 1H), 3.86 (s, 1H), 3.83-3.77 (m, 3H), 3.66 (s, 1H), 2.91-2.75 (m, 2H), 2.72-2.63 (m, 1H), 2.54-2.48 (m, 1H).

### Example 46: WX046

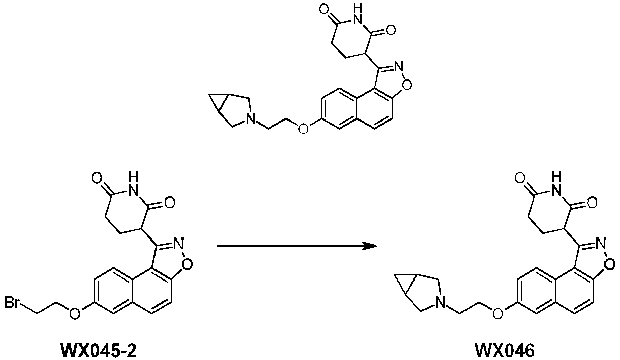

### Step 1: synthesis of WX046

Intermediate **WX045-2** (0.1 g, 248.00 µmol) and 3-azabicyclo[3.1.0]hexane (29.66 mg, 248.00 µmol, hydrochloride) were dissolved in *N,N*-dimethylformamide (1 mL) at room temperature under nitrogen atmosphere, and then potassium iodide (41.17 mg, 248.00 µmol) and *N,N*-diisopropylethylamine (32.05 mg, 248.00 µmol, 43.20 µL) were added. The reaction mixture was stirred at 20 °C for 24 h. After the reaction was completed, the reaction mixture was adjusted to pH 6-7 with ethyl acetate hydrochloride (4 M), and then concentrated under reduced pressure to remove the solvent. The resulting residue was separated by prep-HPLC (mobile phase: acetonitrile/water; acidic system: 0.05% HCl) to give target compound **WX046.** MS-ESI *m*/*z:* 406.2 [M+H]⁺. ¹H NMR (400 MHz, MeOD_*d₄*) δ: 8.19 (d, *J*=9.2 Hz, 1H), 8.05 (d, *J*=9.2 Hz, 1H), 7.78 (d, *J*=9.2 Hz, 1H), 7.62 (d, *J*=2.8 Hz, 1H), 7.46 (dd, *J*=2.8, 9.2 Hz, 1H), 4.94 (dd, *J*=5.0, 10.2 Hz, 1H), 4.50 (t, *J*=4.8 Hz, 2H), 3.83 (d, *J*=11.6 Hz, 2H), 3.75 (t, *J*=5.0 Hz, 2H), 3.58 (d, *J*=11.2 Hz, 2H), 2.92-2.75 (m, 2H), 2.73-2.63 (m, 1H), 2.55-2.48 (m, 1H), 1.92 (t, *J*=4.0 Hz, 2H), 0.92-0.86 (m, 1H), 0.77-0.73 (m, 1H).

### Example 47: WX047

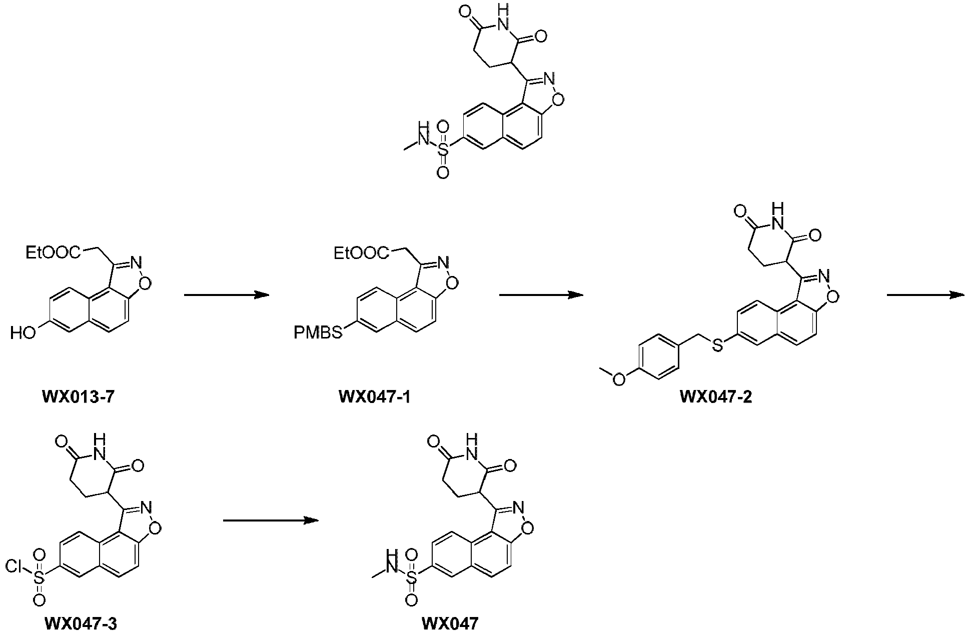

### Step 1: synthesis of intermediate WX047-1

Intermediate **WX013-7** (1 g, 2.99 mmol), *p*-methoxybenzyl mercaptan (461.54 mg, 2.99 mmol) and *N,N-*diisopropylethylamine (773.51 mg, 5.99 mmol, 1.04 mL) were dissolved in 1,4-dioxane (30 mL) at room temperature under nitrogen atmosphere, and then 4,5-bis-diphenylphosphino-9,9-dimethylxanthene (173.15 mg, 299.25 µmol) and tris(dibenzylideneacetone)dipalladium (137.02 mg, 149.63 µmol) were added. The reaction mixture was warmed to 120 °C and stirred for 12 h. After the reaction was completed, the reaction mixture was cooled to room temperature and then poured into water (80 mL), and ethyl acetate (80 mL × 3) was added for extraction. The organic phases were combined, washed with saturated brine (100 mL × 3), dried over anhydrous sodium sulfate, filtered, and concentrated under reduced pressure to remove the solvent. The resulting residue was separated by column chromatography (eluent: petroleum ether/ethyl acetate = 1/0-9/1, volume ratio) to give intermediate **WX047-1.**

### Step 2: synthesis of intermediate WX047-2

Intermediate **WX047-1** (0.74 g, 1.82 mmol) and acrylamide (129.08 mg, 1.82 mmol) were dissolved in tetrahydrofuran (22 mL) at room temperature, and then potassium *tert*-butoxide (203.78 mg, 1.82 mmol) was added. The reaction mixture was stirred at room temperature for 2 h. After the reaction was completed, the reaction mixture was poured into water (60 mL), and 2 N diluted hydrochloric acid was added to adjust the pH to 6-7, and 2-methyltetrahydrofuran (60 mL × 3) was added for extraction. The organic phases were combined, washed with saturated brine (80 mL × 3), dried over anhydrous sodium sulfate and filtered, and the filtrate was concentrated under reduced pressure to remove the solvent. The resulting residue was separated by column chromatography (eluent: petroleum ether/2-methyltetrahydrofuran = 1/0-3/1, volume ratio) to give intermediate **WX047-2.** ¹H NMR (400 MHz, DMSO) δ: 11.13 (s, 1H), 8.13-8.10 (m, 2H), 7.93 (d, *J*=9.2 Hz, 1H), 7.65 (dd, *J*=1.8, 8.6 Hz, 1H), 7.32 (d, *J*=8.4 Hz, 2H), 6.89-6.83 (m, 3H), 5.03 (dd, *J*=4.6, 11.4 Hz, 1H), 4.33 (s, 2H), 3.70 (s, 3H), 2.88-2.79 (m, 1H), 2.66-2.54 (m, 2H), 2.37-2.32 (m, 1H).

### Step 3: synthesis of intermediate WX047-3

Intermediate **WX047-2** (0.4 g, 924.88 mmol) was dissolved in acetic acid (8 mL) at room temperature, and then *N*-chlorosuccinimide (370.50 mg, 2.77 mmol) was added. The reaction mixture was stirred at room temperature for 2 h. After the reaction was completed, the reaction mixture was concentrated under reduced pressure to remove the solvent, thus giving intermediate **WX047-3.**

### Step 4: synthesis of WX047

Intermediate **WX047-3** (0.15 g, 396.00 µmol) was dissolved in tetrahydrofuran (4 mL) at room temperature, and then *N,N-*diisopropylethylamine (102.36 mg, 792.00 µmol, 137.95 µL) and a solution of methylamine (2 M, 198.00 µL) in tetrahydrofuran were added. The reaction mixture was stirred at room temperature for 12 h. After the reaction was completed, the reaction mixture was diluted with water (10 mL) and adjusted to pH 5-6 with 2 N diluted hydrochloric acid, and ethyl acetate (10 mL × 3) was added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give target compound **WX047.** MS-ESI *m*/*z:* 374.0 [M+H]⁺. ¹H NMR (400 MHz, MeOD_*d₄*) δ: 8.62 (d, *J*=1.6 Hz, 1H), 8.45 (d, *J*=8.8 Hz, 1H), 8.29 (d, *J*=9.2 Hz, 1H), 8.09 (dd, *J*=1.6, 8.8 Hz, 1H), 7.96 (d, *J*=9.2 Hz, 1H), 5.04 (dd, *J*=5.2, 10.8 Hz, 1H), 2.94-2.86 (m, 1H), 2.85-2.78 (m, 1H), 2.77-2.69 (m, 1H), 2.58 (s, 3H), 2.56-2.50 (m, 1H).

### Example 48: WX048

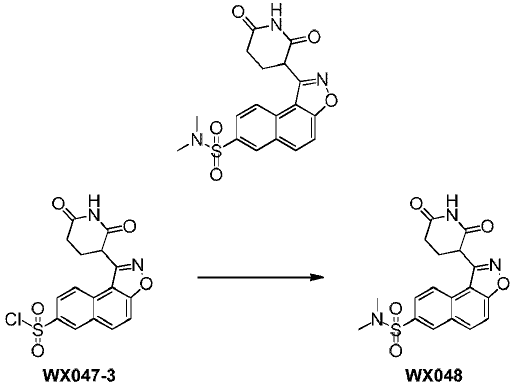

### Step 1: synthesis of WX048

Intermediate **WX047-3** (0.15 g, 396.00 µmol) was dissolved in tetrahydrofuran (4 mL) at room temperature, and then *N*,*N*-diisopropylethylamine (102.36 mg, 792.00 µmol, 137.95 µL) and a solution of dimethylamine in tetrahydrofuran (2 M, 198.00 µL) were added. The reaction mixture was stirred at room temperature for 12 h. After the reaction was completed, the reaction mixture was diluted with water (10 mL) and adjusted to pH 5-6 with 2 N diluted hydrochloric acid, and ethyl acetate (10 mL × 3) was added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give target compound **WX048.** MS-ESI *m*/*z:* 388.1 [M+H]⁺. ¹H NMR (400 MHz, MeOD_*d₄*) δ: 8.59 (d, *J*=1.6 Hz, 1H), 8.46 (d, *J*=8.8 Hz, 1H), 8.32 (d, *J*=9.2 Hz, 1H), 8.03 (dd, *J*=1.8, 8.6 Hz, 1H), 7.96 (d, *J*=9.2 Hz, 1H), 5.03 (dd, *J*=4.8, 10.8 Hz, 1H), 2.90-2.83 (m, 2H), 2.76 (s, 6H), 2.74-2.71 (m, 1H), 2.57-2.51 (m, 1H).

### Example 49 and Example 50: Hydrochloride of WX049 and Hydrochloride of WX050

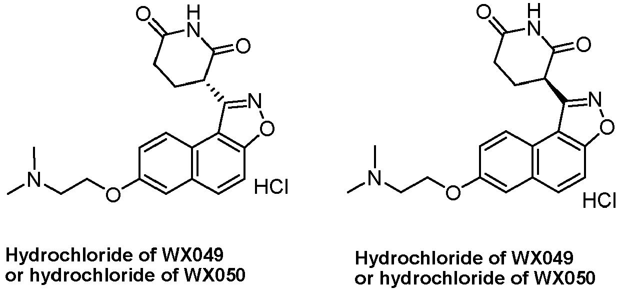

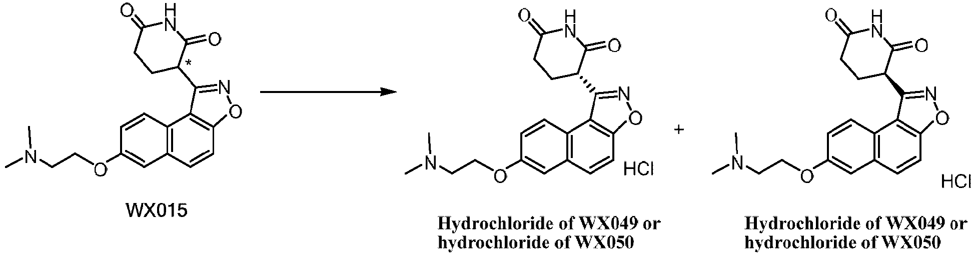

### Step 1: synthesis of WX049 and WX050

Compound **WX015** (200 mg, 495.22 µmol, hydrochloride) was separated by supercritical fluid chromatography (separation conditions: column: DAICEL CHIRALCEL OJ (250mm*30mm, 10 µm); mobile phase: [0.1% NH₃H₂O IPA]; B%: 35%-35%, 6 min), and a sample with a retention time of 3.04 min was collected to give the hydrochloride of target compound **WX049** (ee%: 96.3%), and a sample with a retention time of 2.65 min was collected to give the hydrochloride of target compound **WX050** (ee%: 99.04%). The target compound **WX049:** MS-ESI *m*/*z:* 368.1 [M+H]⁺. ¹H NMR (400 MHz, DMSO_*d₆*) δ: 11.13 (s, 1H), 10.64 (s, 1H), 8.19 (d, *J*=9.2 Hz, 1H), 8.12 (d, *J*=9.2 Hz, 1H), 7.92 (d, *J*=9.2 Hz, 1H), 7.73 (d, *J*=2.4 Hz, 1H), 7.43 (dd, *J*=2.6, 9.0 Hz, 1H), 5.04 (dd, *J*=4.8, 11.2 Hz, 1H), 4.53 (t, *J*=4.8 Hz, 2H), 3.58 (s, 2H), 2.87 (s, 6H), 2.85-2.79 (m, 1H), 2.71-2.54 (m, 2H), 2.44-2.30 (m, 1H). Target compound **WX050:** MS-ESI *m*/*z:* 368.1 [M+H]⁺.¹H NMR (400 MHz, DMSO_*d₆*) δ: 11.13 (s, 1H), 10.61 (s, 1H), 8.19 (d, *J*=8.8 Hz, 1H), 8.12 (d, *J*=9.2 Hz, 1H), 7.92 (d, *J*=9.2 Hz, 1H), 7.73 (d, *J*=2.0 Hz, 1H), 7.43 (dd, *J*=2.2, 9.0 Hz, 1H), 5.04 (dd, *J*=4.4, 11.2 Hz, 1H), 4.53 (t, *J*=4.8 Hz, 2H), 3.59 (s, 2H), 2.87 (s, 6H), 2.85-2.79 (m, 1H), 2.71-2.55 (m, 2H), 2.43-2.31 (m, 1H).

### Example 51: WX051

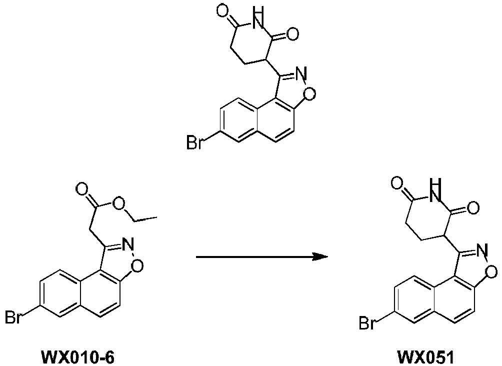

### Step 1: synthesis of WX051

Intermediate **WX010-6** (200 mg, 624.73 µmol) was dissolved in tetrahydrofuran (10 mL) at 12 °C, and then acrylamide (44.40 mg, 624.73 µmol) and potassium *tert*-butoxide (70.10 mg, 624.73 µmol) were added simultaneously. The reaction mixture was stirred at 12 °C for 3 h. After the reaction was completed, the reaction mixture was poured into 0.5 M diluted hydrochloric acid (20 mL) and ethyl acetate (20 mL × 3) was then added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give target compound **WX051.** MS-ESI *m*/*z:* 359.0 [M+H]⁺, 361.0 [M+H+2]⁺.¹H NMR (400 MHz, DMSO_*d₆*) δ: 11.13 (s, 1H), 8.47 (d, *J*=2.0Hz, 1H), 8.22 (d, *J*=8.4 Hz, 1H), 8.21 (d, *J*=8.8 Hz, 1H), 8.01 (d, *J*=9.2 Hz, 1H), 7.85 (dd, *J*=2.2, 9.0 Hz, 1H), 5.07 (dd, *J*=4.6, 11.8 Hz, 1H), 2.90-2.78 (m, 1H), 2.74-2.59 (m, 2H), 2.43-2.34 (m, 1H).

### Example 52: WX052

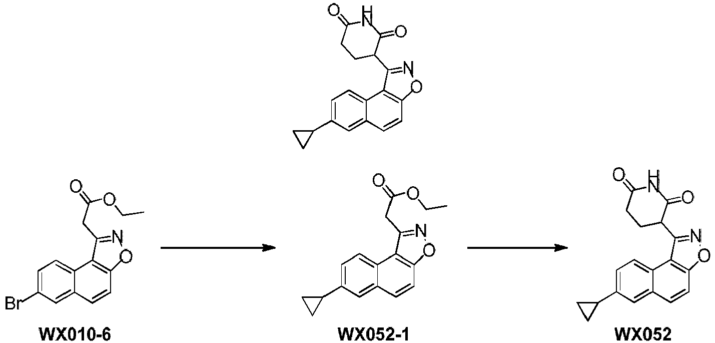

### Step 1: synthesis of intermediate WX052-1

Intermediate **WX010-6** (300 mg, 897.76 µmol) was dissolved in 1,4-dioxane (10 mL) at room temperature under nitrogen atmosphere, and then tetrakis(triphenylphosphine)palladium (51.87 mg, 44.89 µmol), sodium carbonate (333.04 mg, 3.14 mmol) and potassium cyclopropyltrifluoroborate (265.69 mg, 1.80 mmol) were added sequentially. The reaction mixture was heated to 110 °C and stirred for 12 h. After the reaction was completed, the reaction mixture was cooled to room temperature and then directly filtered. The filter cake was washed with ethyl acetate (10 mL × 2), and the filtrate was collected and concentrated under reduced pressure to remove the solvent. The resulting residue was separated by column chromatography (eluent: petroleum ether/ethyl acetate = 1/0-9/1, volume ratio) to give intermediate **WX052-1.** ¹H NMR (400 MHz, CDCl₃) δ: 8.01 (d, *J*=8.4 Hz, 1H), 7.88 (d, *J*=9.2 Hz, 1H), 7.69 (d, *J*=1.6 Hz, 1H), 7.66 (d, *J*=9.2 Hz, 1H), 7.40 (dd, *J*=2.0, 8.8 Hz, 1H), 4.31 (s, 2H), 4.22 (q, *J*=7.2 Hz, 2H), 2.16-2.06 (m, 1H), 1.22 (t, *J*=7.2 Hz, 3H), 1.12-1.03 (m, 2H), 0.88-0.77 (m, 2H).

### Step 2: synthesis of WX052

Intermediate **WX052-1** (230 mg, 778.79 µmol) was dissolved in tetrahydrofuran (20 mL) at 15 °C, and then acrylamide (55.35 mg, 778.79 µmol) and a solution of potassium *tert*-butoxide (1 M, 778.79 µL) in tetrahydrofuran were added sequentially. The reaction mixture was stirred at 15 °C for 3 h. After the reaction was completed, the reaction mixture was poured into 0.5 M diluted hydrochloric acid (20 mL) and ethyl acetate (20 mL × 3) was then added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give target compound **WX052.** MS-ESI *m*/*z:* 321.1 [M+H]⁺. ¹H NMR (400 MHz, CD₃CN) δ: 8.96 (s, 1H), 8.04 (d, *J*=8.8 Hz, 1H), 8.00 (d, *J*=9.2 Hz, 1H), 7.79 (d, *J*=1.6 Hz, 1H), 7.73 (d, *J*=8.8 Hz, 1H), 7.43 (dd, *J*=1.8, 8.6 Hz, 1H), 4.79 (dd, *J*=5.0, 10.6 Hz, 1H), 2.84-2.61 (m, 3H), 2.51-2.41 (m, 1H), 2.14-2.06 (m, 1H), 1.11-1.01 (m, 2H), 0.87-0.78 (m, 2H).

### Example 53: WX053

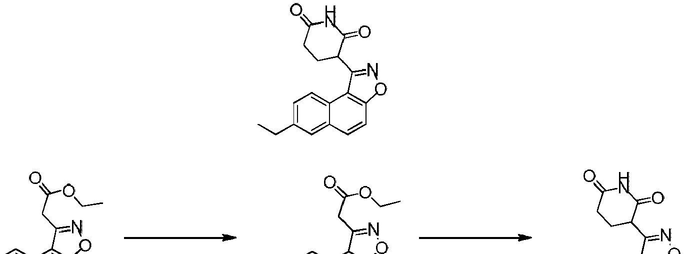

### Step 1: synthesis of intermediate WX053-1

Intermediate **WX010-6** (300 mg, 897.76 µmol) was dissolved in toluene (4 mL) and water (2 mL) at room temperature under nitrogen atmosphere, and then palladium acetate (20.16 mg, 89.78 µmol), *n*-butyl-bis(1-adamantyl)phosphine (64.38 mg, 179.55 µmol), potassium ethylfluoroborate (366.19 mg, 2.69 mmol) and cesium carbonate (877.53 mg, 2.69 mmol) were added sequentially. The reaction mixture was heated to 80 °C and stirred for 12 h. After the reaction was completed, the reaction mixture was cooled to room temperature. Water (10 mL) was added to the reaction mixture, and ethyl acetate (10 mL × 3) was added for extraction. The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 1/0-9/1, volume ratio) to give intermediate **WX053-1.**

### Step 2: synthesis of WX053

Intermediate **WX053-1** (250 mg, 882.39 µmol) was dissolved in tetrahydrofuran (20 mL) at room temperature under nitrogen atmosphere, and then acrylamide (62.72 mg, 882.39 µmol) and potassium *tert*-butoxide (99.01 mg, 882.39 µmol) were added sequentially. The reaction mixture was stirred at 15 °C for 3 h. After the reaction was completed, the reaction mixture was poured into 0.5 M diluted hydrochloric acid (20 mL) and ethyl acetate (20 mL × 3) was then added for extraction. The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 1/0-3/2, volume ratio), the resulting residue from which was separated by prep-HPLC (mobile phase: acetonitrile/water; acidic system: 0.05% HCl) to give target compound **WX053.** MS-ESI *m*/*z:* 309.1 [M+H]⁺. ¹H NMR (400 MHz, DMSO_*d*₆) δ: 11.16 (s, 1H), 8.14 (d, *J*=9.2 Hz, 2H), 7.97 (s, 1H), 7.89 (d, *J*=9.2 Hz, 1H), 7.61 (d, *J*=8.4 Hz, 1H), 5.04 (dd, *J*=4.8, 11.2 Hz, 1H), 2.93-2.76 (m, 3H), 2.70-2.55 (m, 2H), 2.42-2.30 (m, 1H), 1.29 (t, *J*=7.6 Hz, 3H).

### Example 54 and Example 55: WX054 and WX055

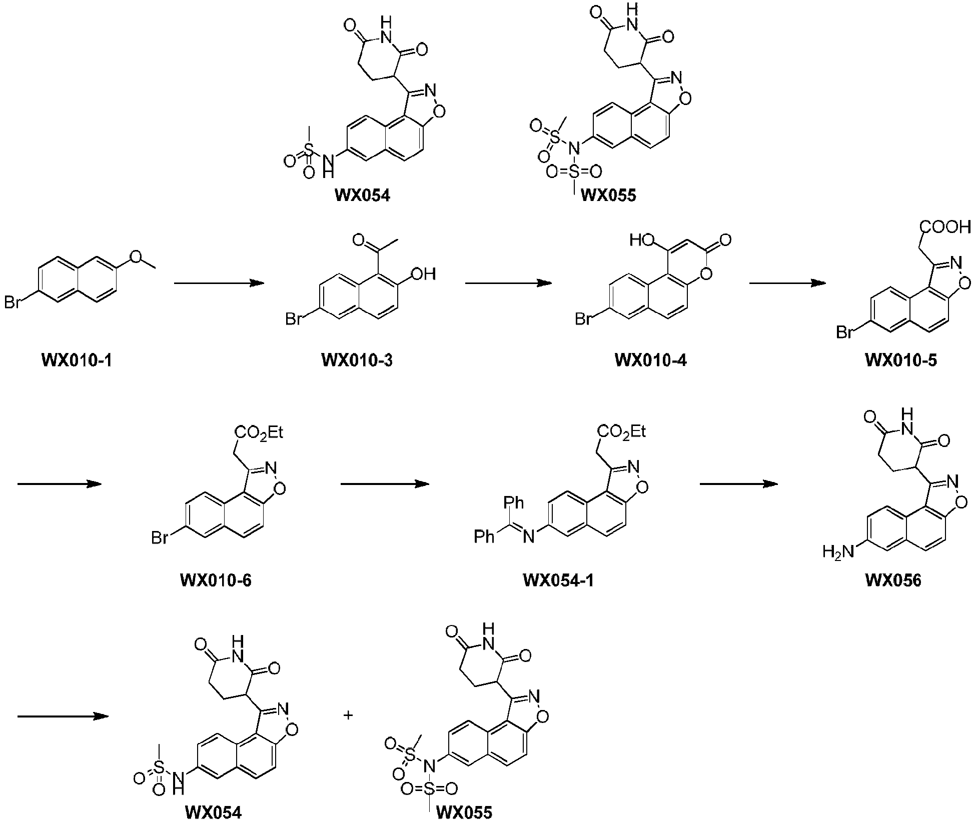

### Step 1: synthesis of intermediate WX010-3

**WX010-1** (30 g, 126.53 mmol, 75.00 mL) was dissolved in dichloromethane (400 mL) at 10 °C, and then acetyl chloride (9.93 g, 126.53 mmol, 9.03 mL) was added. After three nitrogen purges, aluminum trichloride (33.74 g, 253.07 mmol) was added in batches at 5-10 °C under nitrogen atmosphere. After being stirred at 10 °C for 2 h, the reaction mixture was supplemented with acetyl chloride (1.5 mL), and the resulting reaction was then stirred for 2 h. After the reaction was completed, the reaction mixture was poured into ice water (200 mL), and 2 M diluted hydrochloric acid (50 mL) was added for dilution, and then dichloromethane (200 mL × 3) was added for extraction. The organic phases were combined, dried over anhydrous sodium sulfate and filtered, and the filtrate was concentrated under reduced pressure to remove the solvent, thus giving intermediate **WX010-3.** ¹H NMR (400 MHz, CDCl₃) δ: 13.39 (s, 1H), 7.97 (d, *J*=9.2 Hz, 1H), 7.93 (d, *J*=2.0Hz, 1H), 7.80 (d, *J*=8.8 Hz, 1H), 7.64 (dd, *J*=2.2, 9.0 Hz, 1H), 7.17 (d, *J*=9.2 Hz, 1H), 2.85 (s, 3H).

### Step 2: synthesis of intermediate WX010-4

Intermediate **WX010-3** (31 g, 116.94 mmol) was dissolved in diethyl carbonate (302.25 g, 2.56 mol, 310.00 mL) at 10 °C under nitrogen atmosphere, and then sodium hydride (23.38 g, 584.68 mmol, purity: 60%) was added in batches at 5-10 °C. After being stirred at 10 °C for 1 h, the reaction mixture was heated to 60 °C and stirred for 1 h, and then the reaction mixture was heated to 130 °C and stirred for 16 h. After the reaction was completed, the reaction mixture was cooled to room temperature and then poured into ice water (500 mL), and ethyl acetate (500 mL) was added for extraction. The organic phase was removed, and the aqueous phase was adjusted to pH 3-4 with 6 M hydrochloric acid and extracted with 2-methyltetrahydrofuran (2000 mL × 3). The organic phases were combined, dried over anhydrous sodium sulfate and filtered, and the filtrate was concentrated under reduced pressure to remove the solvent. The resulting residue was added to methyl *tert-butyl* ether (100 mL), and the reaction mixture was stirred at room temperature for 20 min and then filtered. The filter cake was collected and concentrated under reduced pressure to remove the solvent, thus giving intermediate **WX010-4.**

### Step 3: synthesis of intermediate WX010-5

Intermediate **WX010-4** (24 g, 82.45 mmol) was dissolved in ethanol (400 mL) at room temperature, and then hydroxylamine hydrochloride (37.24 g, 535.90 mmol) and sodium acetate (23.67 g, 288.56 mmol) were added sequentially. The reaction mixture was heated to 90 °C and stirred for 60 h. After the reaction was completed, the reaction mixture was cooled to room temperature, added with 2 M diluted hydrochloric acid (200 mL), and concentrated under reduced pressure to remove most of the ethanol, and 2-methyltetrahydrofuran (500 mL × 3) was added for extraction. The organic phases were combined, dried over anhydrous sodium sulfate and filtered, and the filtrate was concentrated under reduced pressure to remove the solvent, thus giving intermediate **WX010-5.**

### Step 4: synthesis of intermediate WX010-6

Intermediate **WX010-5** (25 g, 81.67 mmol) was dissolved in ethanol (300 mL) at room temperature under nitrogen atmosphere, and then sulfuric acid (5.52 g, 55.15 mmol, 3 mL, purity: 98%) was added. The reaction mixture was heated to 80 °C and stirred for 12 h. After the reaction was completed, the reaction mixture was subjected to hot filtration at 80 °C. The mother solution was collected and cooled to room temperature, and solid was precipitated. The mixture was filtered to collect the solid, which was concentrated under reduced pressure to remove the solvent, thus giving intermediate **WX010-6.** ¹H NMR (400 MHz, CDCl₃) δ: 8.16 (d, *J*=1.6 Hz, 1H), 8.00 (d, *J*=8.8 Hz, 1H), 7.88 (d, *J*=9.2 Hz, 1H), 7.77 (dd, *J*=1.8, 9.0 Hz, 1H), 7.74 (d, *J*=9.2 Hz, 1H), 4.33 (s, 2H), 4.24 (q, *J*=7.2 Hz, 2H), 1.24 (t, *J*=7.2 Hz, 3H).

### Step 5: synthesis of intermediate WX054-1

Intermediate **WX010-6** (5 g, 14.96 mmol) was dissolved in 1,4-dioxane (70 mL) at room temperature under nitrogen atmosphere, and then benzophenone imine (3.12 g, 17.21 mmol, 2.89 mL), cesium carbonate (12.19 g, 37.41 mmol), 4,5-bis-diphenylphosphino-9,9-dimethylxanthene (1.30 g, 2.24 mmol) and palladium acetate (503.89 mg, 2.24 mmol) were added sequentially. The reaction mixture was heated to 80 °C and stirred at 80 °C for 8 h. After the reaction was completed, the reaction mixture was filtered to collect the mother solution. The filter cake was washed with ethyl acetate (100 mL × 2), and the filtrate was collected and concentrated under reduced pressure to remove the solvent. The resulting residue was separated by column chromatography (neutral alumina: 200-300 meshes, eluent: petroleum ether/ethyl acetate = 1/0-10/1, volume ratio) to give intermediate **WX054-1.** ¹H NMR (400 MHz, CDCl₃) δ: 7.87 (d, *J*=8.8 Hz, 1H), 7.83-7.79 (m, 2H), 7.76 (d, *J*=9.2 Hz, 1H), 7.60 (d, *J*=9.2 Hz, 1H), 7.54-7.49 (m, 1H), 7.48-7.42 (m, 2H), 7.34 (d, *J*=1.6 Hz, 1H), 7.26-7.19 (m, 3H), 7.18-7.13 (m, 2H), 7.09 (dd, *J*=2.4, 8.8 Hz, 1H), 4.27 (s, 2H), 4.17 (q, *J*=7.4 Hz, 2H), 1.13 (t, *J*=7.0 Hz, 3H).

### Step 6: synthesis of intermediate WX056

Intermediate **WX054-1** (2 g, 4.60 mmol) was dissolved in tetrahydrofuran (40 mL) at 15 °C, and then acrylamide (327.18 mg, 4.60 mmol) and a solution of potassium *tert*-butoxide (1 M, 4.14 mL) in tetrahydrofuran were added sequentially. The reaction mixture was stirred at 15 °C for 2 h. After the reaction was completed, water (100 mL) was added to the reaction mixture, 1 M diluted hydrochloric acid was added to adjust the pH to 4-5, and ethyl acetate (100 mL × 2) was added for extraction. The organic phases were combined and washed with 2 M diluted hydrochloric acid (20 mL × 2). The aqueous phases were combined, the organic phase was discarded, and the aqueous phase adjusted to pH 7-8 with solid sodium bicarbonate and extracted with ethyl acetate (100 mL × 3). The organic phases were combined, dried over anhydrous sodium sulfate, filtered, and concentrated under reduced pressure to remove the solvent. The resulting residue was separated by prep-HPLC (mobile phase: acetonitrile/water; neutral system: 10 mM NH₄HCO₃). The resulting fraction was adjusted to pH 6-7 with 2 M diluted hydrochloric acid and concentrated under reduced pressure to remove acetonitrile. The resulting aqueous phase was adjusted to pH 7-8 with saturated sodium bicarbonate solution and extracted with ethyl acetate (50 mL × 3). The organic phases were combined, dried over anhydrous sodium sulfate, filtered and concentrated under reduced pressure to give intermediate **WX056.** ¹H NMR (400 MHz, DMSO_*d*₆) δ: 11.11 (s, 1H), 7.90 (d, *J*=9.6 Hz, 1H), 7.81 (d, *J*=9.2 Hz, 1H), 7.69 (d, *J*=9.2 Hz, 1H), 7.10 (dd, *J*=2.6, 7.4 Hz, 2H), 5.44 (s, 2H), 4.92 (dd, *J=*5.2, 11.2 Hz, 1H), 2.87-2.76 (m, 1H), 2.70-2.53 (m, 2H), 2.42-2.28 (m, 1H).

### Step 7: synthesis of WX054 and WX055

Intermediate **WX056** (100 mg, 338.65 µmol) was dissolved in 1,2-dichloroethane (10 mL) at 15 °C, and then methanesulfonyl chloride (38.79 mg, 338.65 µmol, 26.21 µL) and triethylamine (34.27 mg, 338.65 µmol, 47.14 µL) were added. After being stirred at 15 °C for 12 h, the reaction mixture was supplemented with triethylamine (50 µL) and methanesulfonyl chloride (40 µL), stirred for 5 h, and then supplemented with triethylamine (60 µL) and methanesulfonyl chloride (20 µL). The resulting reaction mixture was then stirred at 15 °C for 12 h. After the reaction was completed, water (20 mL) was added to the reaction mixture and then ethyl acetate (50 mL × 3) was added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give target compounds **WX054** and **WX055.** Target compound **WX054:** MS-ESI *m*/*z:* 374.0 [M+H]^{+ 1}H NMR (400 MHz, DMSO_*d₆*) δ: 11.13 (s, 1H), 10.06 (s, 1H), 8.21 (d, *J*=9.2 Hz, 1H), 8.15 (d, *J*=8.4 Hz, 1H), 7.91 (d, *J*=9.2 Hz, 2H), 7.59 (dd, *J*=2.6, 9.0 Hz, 1H), 5.01 (dd, *J*=4.6, 11.8 Hz, 1H), 3.07 (s, 3H), 2.90-2.76 (m, 1H), 2.71-2.55 (m, 2H), 2.43-2.30 (m, 1H). Target compound **WX055:** MS-ESI *m*/*z:* 452.0 [M+H]^{+ 1}H NMR (400 MHz, DMSO_*d*₆) δ: 11.17 (s, 1H), 8.44 (d, *J*=2.0Hz, 1H), 8.30 (d, *J*=9.2 Hz, 2H), 8.06 (d, *J*=9.2 Hz, 1H), 7.82 (dd, *J*=2.2, 8.6 Hz, 1H), 5.12 (dd, *J*=4.6, 11.8 Hz, 1H), 3.63 (s, 6H), 2.94-2.79 (m, 1H), 2.74-2.58 (m, 2H), 2.45-2.33 (m, 1H).

### Example 56: WX056

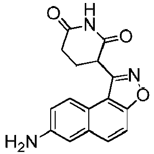

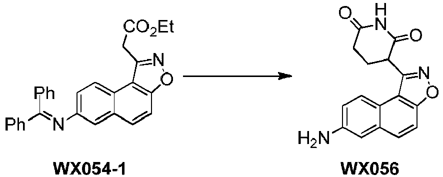

### Step 1: synthesis of WX056

Intermediate **WX054-1** (1.2 g, 2.76 mmol) was dissolved in tetrahydrofuran (30 mL) at 15 °C, and then acrylamide (196.31 mg, 2.76 mmol) and a solution of potassium *tert*-butoxide (1 M, 2.49 mL) in tetrahydrofuran were added sequentially. The reaction mixture was stirred at 15 °C for 2 h. After the reaction was completed, water (100 mL) was added to the reaction mixture and then ethyl acetate (100 mL × 3) was added for extraction. The organic phases were combined, dried over anhydrous sodium sulfate, filtered, and concentrated under reduced pressure to remove the solvent. The resulting residue was separated by prep-HPLC (mobile phase: acetonitrile/water; neutral system: 10 mM NH₄HCO₃), and the resulting fraction was adjusted to pH 3-4 with 4 M ethyl acetate hydrochloride, stirred at 15 °C for 15 min and extracted with ethyl acetate (50 mL). The organic phase was removed by washing with 2 M diluted hydrochloric acid (50 mL × 2). The aqueous phases were combined, adjusted to pH 7-8 with saturated sodium carbonate solution and extracted with ethyl acetate (50 mL × 3). The organic phases were combined, dried over anhydrous sodium sulfate, filtered and concentrated under reduced pressure to remove the solvent. The resulting residue was separated by prep-HPLC (mobile phase: acetonitrile/water; acidic system: 0.05% HCl) to give target compound **WX056.** MS-ESI *m*/*z:* 296.1 [M+H]^{+ 1}H NMR (400 MHz, DMSO_*d₆*) δ: 11.14 (s, 1H), 8.25 (s, 1H), 8.21-8.14 (m, 1H), 8.00-7.92 (m, 1H), 7.91-7.81 (m, 1H), 7.61-7.50 (m, 1H), 5.04 (dd, *J*=3.4, 11.4 Hz, 1H), 2.90-2.77 (m, 1H), 2.70-2.56 (m, 2H), 2.42-2.30 (m, 1H).

### Example 57: WX057

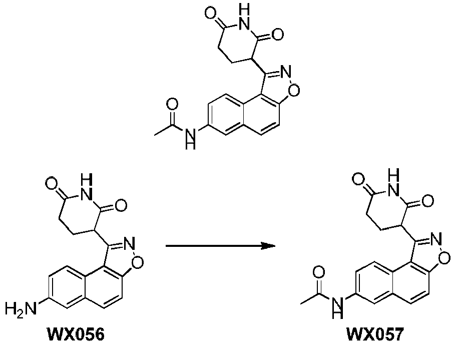

### Step 1: synthesis of WX057

Intermediate **WX056** (100 mg, 338.65 µmol) was dissolved in 1,2-dichloroethane (10 mL) at 15 °C, and then acetyl chloride (26.58 mg, 338.65 µmol, 24.17 µL) and triethylamine (34.27 mg, 338.65 µmol, 47.14 µL) were added sequentially. The reaction mixture was stirred at 15 °C for 3 h. After the reaction was completed, water (20 mL) was added to the reaction mixture, and ethyl acetate (50 mL × 3) was added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give target compound **WX057.** MS-ESI *m*/*z:* 338.1 [M+H]^{+ 1}H NMR (400 MHz, DMSO_*d₆*) δ: 11.13 (s, 1H), 10.25 (s, 1H), 8.48 (d, *J*=2.0Hz, 1H), 8.18 (d, *J*=9.2 Hz, 1H), 8.10 (d, *J*=9.2 Hz, 1H), 7.88 (d, *J*=9.2 Hz, 1H), 7.79 (dd, *J*=2.0, 8.8 Hz, 1H), 5.02 (dd, *J*=4.8, 11.6 Hz, 1H), 2.89-2.77 (m, 1H), 2.69-2.55 (m, 2H), 2.42-2.31 (m, 1H), 2.12 (s, 3H).

### Example 58: WX058

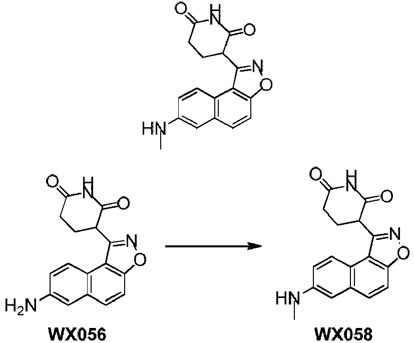

### Step 1: synthesis of WX058

Intermediate **WX056** (50 mg, 169.32 µmol) was dissolved in 1,4-dioxane (3 mL) at room temperature under nitrogen atmosphere, and then copper acetate (76.89 mg, 423.30 µmol), pyridine (46.88 mg, 592.62 µmol, 47.83 µL) and methylboronic acid (25.34 mg, 423.30 µmol) were added sequentially. The reaction mixture was heated to 110 °C and stirred for 12 h. After the reaction was completed, the reaction mixture was cooled to room temperature and filtered. The filter cake was washed with ethyl acetate (10 mL × 2), and the filtrate was collected and concentrated under reduced pressure to give the residue. The resulting residue was separated by prep-HPLC (mobile phase: acetonitrile/water; acidic system: 0.05% HCl) to give target compound **WX058.** MS-ESI *m*/*z:* 310.1 [M+H]⁺.¹H NMR (400 MHz, DMSO_*d*₆) δ: 11.13 (s, 1H), 8.15 (d, *J*=8.4 Hz, 1H), 8.09 (d, *J*=8.8 Hz, 1H), 7.88 (d, *J*=9.2 Hz, 1H), 7.62 (s, 1H), 7.48 (d, *J*=8.0 Hz, 1H), 5.01 (dd, *J*=4.6, 11.0 Hz, 1H), 2.91 (s, 3H), 2.88-2.77 (m, 1H), 2.71-2.57 (m, 2H), 2.42-2.31 (m, 1H).

### Example 59: WX059

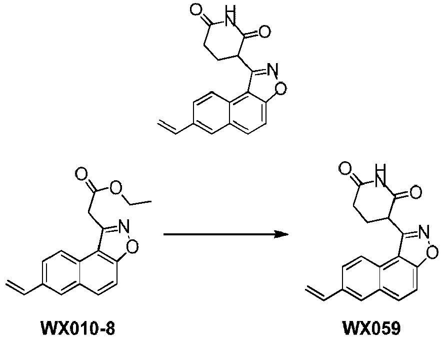

### Step 1: synthesis of WX059

Intermediate **WX010-8** (1.5 g, 5.33 mmol) was dissolved in tetrahydrofuran (40 mL) at 10 °C, and then acrylamide (379.01 mg, 5.33 mmol) and potassium *tert*-butoxide (598.35 mg, 5.33 mmol) were added sequentially. The reaction mixture was stirred at 10 °C for 3 h. After the reaction was completed, water (50 mL) was added and then ethyl acetate (100 mL × 5) was added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give target compound **WX059.** MS-ESI *m*/*z:* 307.1 [M+H]⁺. ¹H NMR (400 MHz, DMSO_*d₆*) δ: 11.17 (s, 1H), 8.22-8.16 (m, 3H), 7.96-7.89 (m, 2H), 6.94 (dd, *J*=10.8, 17.6 Hz, 1H), 6.04 (d, *J*=17.6 Hz, 1H), 5.41 (d, *J*=11.2 Hz, 1H), 5.06 (dd, *J*=4.6, 11.8 Hz, 1H), 2.92-2.79 (m, 1H), 2.70-2.56 (m, 2H), 2.42-2.31 (m, 1H).

### Example 60: WX060

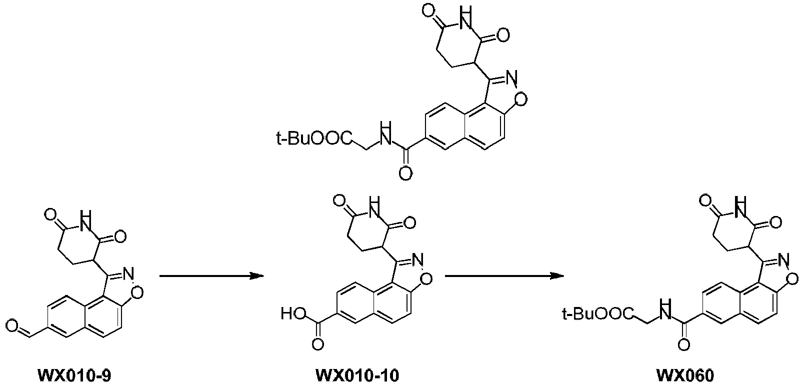

### Step 1: synthesis of intermediate WX010-10

Intermediate **WX010-9** (300 mg, 973.12 µmol) was dissolved in *N,N-*dimethylformamide (14 mL) at 10 °C, and then potassium peroxymonosulfate (598.24 mg, 973.12 µmol) was added. The reaction mixture was stirred at 10 °C for 12 h. After the reaction was completed, the reaction mixture was filtered to collect mother solution, thus giving a solution of intermediate **WX010-10** in *N,N-*dimethylformamide (0.0695 M, 14 mL).

### Step 2: synthesis of WX060

2-(7-benzotriazole oxide)-*N*,*N*,*N'*,*N'*-tetramethyluronium hexafluorophosphate (277.50 mg, 729.82 µmol) and triethylamine (98.47 mg, 973.09 µmol, 135.44 µL) were sequentially added to a solution of intermediate **WX010-10** in *N,N*-dimethylfonnamide (0.0695 M, 7.00 mL) at 0 °C under nitrogen atmosphere. After the reaction mixture was stirred at 0 °C for 15 min, *tert-butyl* glycinate (76.59 mg, 583.85 µmol) was added. The resulting reaction mixture was warmed to 10 °C and stirred for 12 h. After the reaction was completed, water (20 mL) was added to the reaction mixture and then ethyl acetate (20 mL × 3) was added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; neutral system: 10 mM NH₄HCO₃), the resulting residue from which was then separated by prep-HPLC (mobile phase: acetonitrile/water; acidic system: 0.05% HCl) to give target compound **WX060.** MS-ESI *m*/*z:* 438.1 [M+H]⁺. ¹H NMR (400 MHz, DMSO_*d₆*) δ: 11.17 (s, 1H), 9.12 (t, *J*=5.8 Hz, 1H), 8.71 (s, 1H), 8.35 (d, *J*=8.4 Hz, 1H), 8.31 (d, *J*=9.2 Hz, 1H), 8.14 (dd, *J*=1.6, 8.8 Hz, 1H), 8.03 (d, *J*=8.8 Hz, 1H), 5.12 (dd, *J*=4.4, 11.2 Hz, 1H), 3.98 (d, *J*=6.0 Hz, 2H), 2.91-2.79 (m, 1H), 2.71-2.56 (m, 2H), 2.43-2.30 (m, 1H), 1.44 (s, 9H).

### Example 61: WX061

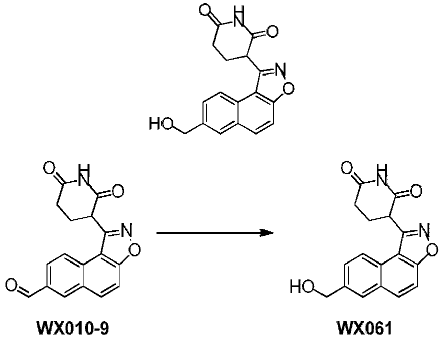

### Step 1: synthesis of WX061

Intermediate **WX010-9** (100 mg, 324.37 µmol) was dissolved in tetrahydrofuran (5 mL) at room temperature under nitrogen atmosphere, and then sodium borohydride (12.27 mg, 324.37 µmol) was added at 0 °C. The reaction mixture was warmed to 10 °C and stirred for 2 h. After the reaction was completed, the reaction mixture was poured into 0.5 M diluted hydrochloric acid (20 mL) and ethyl acetate (20 mL × 3) was then added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give target compound **WX061.** MS-ESI *m*/*z:* 311.1 [M+H]⁺. ¹H NMR (400 MHz, DMSO_*d*₆) δ: 11.13 (s, 1H), 8.20 (d, *J*=6.4 Hz, 1H), 8.18 (d, *J*=9.2 Hz, 1H), 8.08 (s, 1H), 7.91 (d, *J*=9.2 Hz, 1H), 7.68 (d, *J*=8.4 Hz, 1H), 5.39 (t, *J*=5.6 Hz, 1H), 5.05 (dd, *J*=4.6, 11.4 Hz, 1H), 4.71 (d, *J*=5.2 Hz, 2H), 2.92-2.78 (m, 1H), 2.69-2.54 (m, 2H), 2.43-2.34 (m, 1H).

### Example 62: WX062

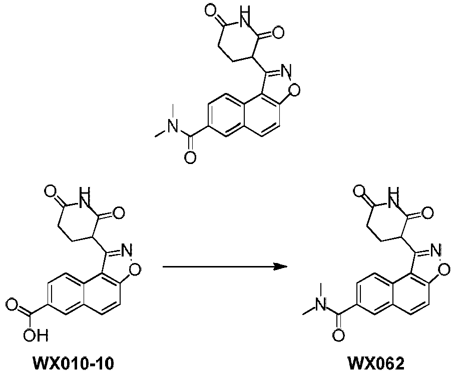

### Step 1: synthesis of WX062

Intermediate **WX010-10** (130 mg, 400.88 µmol) was dissolved in *N,N*-dimethylfonnamide (5 mL) at 0 °C under nitrogen atmosphere, and then 2-(7-benzotriazole oxide)-*N*,*N*,*N'*,*N'*-tetramethyluronium hexafluorophosphate (228.64 mg, 601.32 µmol) and triethylamine (121.69 mg, 1.20 mmol, 167.39 µL) were added sequentially. After the reaction mixture was stirred at 0 °C for 15 min, dimethylamine (35.96 mg, 440.97 µmol, 40.40 µL, HCl) was added. The resulting reaction mixture was warmed to 10 °C and stirred for 12 h. After the reaction was completed, water (40 mL) was added and then ethyl acetate (30 mL × 3) was added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give target compound **WX062.** MS-ESI *m*/*z:* 352.1 [M+H]⁺. ¹H NMR (400 MHz, DMSO_*d*₆) δ: 11.14 (s, 1H), 8.29 (d, *J*=8.4 Hz, 1H), 8.27 (d, *J*=8.8 Hz, 1H), 8.25 (s, 1H), 8.00 (d, *J*=9.2 Hz, 1H), 7.74 (d, *J*=8.4 Hz, 1H), 5.09 (dd, *J*=4.6, 11.8 Hz, 1H), 3.06 (s, 3H), 2.98 (s, 3H), 2.92-2.78 (m, 1H), 2.73-2.56 (m, 2H), 2.44-2.35 (m, 1H).

### Example 63: WX063

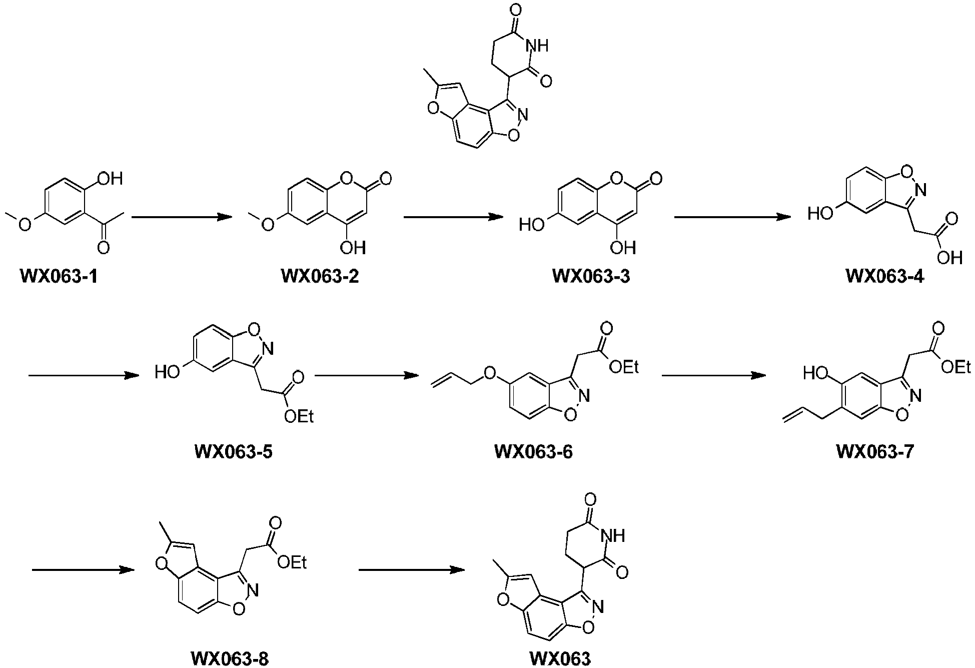

### Step 1: synthesis of intermediate WX063-2

Intermediate **WX063-1** (40 g, 240.71 mmol) was added to diethyl carbonate (300 mL) and toluene (600 mL) at room temperature. After the reaction mixture was cooled to 0 °C, sodium hydride (38.51 g, 962.85 mmol, purity: 60%) was added in batches while stirring, the reaction mixture was heated to 120 °C and stirred for 12 h. After the reaction was completed, the parallel reactions for the two batches were performed together. The reaction mixture was cooled to room temperature and then poured into water (4000 mL). Ethyl acetate (2000 mL × 2) was added for extraction and the organic phase was discarded. The aqueous phase was adjusted to pH 3 with concentrated hydrochloric acid (12 N) and extracted with 2-methyltetrahydrofuran (4000 mL × 2). The organic phases were combined, dried over anhydrous sodium sulfate, filtered and concentrated under reduced pressure to remove the solvent, thus giving intermediate **WX063-2.**

### Step 2: synthesis of intermediate WX063-3

Intermediate **WX063-2** (45 g, 234.17 mmol) was dissolved in dichloromethane (600 mL) at room temperature. After the reaction mixture was cooled to -78 °C, boron tribromide (234.66 g, 936.68 mmol, 90.25 mL) was added dropwise. The resulting reaction mixture was warmed to 20 °C and stirred for 12 h. After the reaction was completed, the parallel reactions for the two batches were performed together. The reaction mixture was slowly poured into ice water (3000 mL), and 2-methyltetrahydrofuran (3000 mL × 3) was added for extraction. The organic phases were combined, dried over anhydrous sodium sulfate and filtered, and the filtrate was concentrated under reduced pressure to remove the solvent, thus giving intermediate **WX063-3.**

### Step 3: synthesis of intermediate WX063-4

Intermediate **WX063-3** (36 g, 202.09 mmol) was dissolved in ethanol (550 mL) at room temperature, and then hydroxylamine hydrochloride (42.13 g, 606.26 mmol) and sodium ethoxide (41.26 g, 606.26 mmol) were added sequentially. The reaction mixture was heated to 90 °C and stirred for 12 h. After the reaction was completed, the parallel reactions for the two batches were performed together. The reaction mixture was cooled to room temperature, then adjusted to pH 5 with 2 N hydrochloric acid, and concentrated under reduced pressure to remove the solvent. The resulting residue was diluted with water (500 mL), and ethyl acetate (400 mL × 3) was added for extraction. The organic phases were combined, dried over anhydrous sodium sulfate, filtered and concentrated under reduced pressure to give intermediate **WX063-4.**

### Step 4: synthesis of intermediate WX063-5

Intermediate **WX063-4** (34 g, 176.02 mmol) was dissolved in ethanol (300 mL) at room temperature under nitrogen atmosphere, and then concentrated sulfuric acid (3.68 g, 36.77 mmol, 2 mL, purity: 98%) was added. The reaction mixture was heated to 90 °C and stirred for 16 h. After the reaction was completed, the parallel reactions for the two batches were performed together. The reaction mixture was cooled to room temperature and concentrated under reduced pressure to remove the solvent. The resulting residue was diluted with ethyl acetate (400 mL) and water (200 mL), and the aqueous phase was extracted with ethyl acetate (150 mL × 3) after liquid separation. The organic phases were combined, washed with water (100 mL × 6) to adjust the pH to about 6, then washed with brine (100 mL × 2), dried over anhydrous sodium sulfate, filtered, and concentrated under reduced pressure to remove the solvent. The resulting residue was separated by column chromatography (eluent: petroleum ether/ethyl acetate = 1/0-2.3/1, volume ratio) to give intermediate **WX063-5.** ¹H NMR (400 MHz, DMSO_*d*₆) δ: 9.64 (s, 1H), 7.54 (d, *J*=8.8, 1H), 7.11 (dd, *J*=2.4 Hz, 8.8 Hz, 1H), 7.02 (d, *J*=2.0 Hz, 1H), 4.13 (q, *J*=7.0 Hz, 2H), 4.131 (s, 2H), 1.20 (t, *J*=7.2, 3H).

### Step 5: synthesis of intermediate WX063-6

Intermediate **WX063-5** (3 g, 13.56 mmol) was dissolved in *N,N*-dimethylfonnamide (60 mL) at room temperature, and then potassium carbonate (5.62 g, 40.69 mmol) and allyl bromide (1.64 g, 13.56 mmol) were added. The reaction mixture was stirred at 15 °C for 12 h. After the reaction was completed, the reaction mixture was poured into ice water (100 mL), and ethyl acetate (100 mL × 3) was added for extraction. The organic phases were combined, washed with half-saturated brine (200 mL × 2), dried over anhydrous sodium sulfate, filtered, and concentrated under reduced pressure to remove the solvent. The resulting residue was separated by column chromatography (eluent: petroleum ether/ethyl acetate = 1/0-19/1, volume ratio) to give intermediate **WX063-6.**

### Step 6: synthesis of intermediate WX063-7

Intermediate **WX063-6** (2.5 g, 9.57 mmol) was dissolved in *N,N-*dimethylformamide (40 mL) at room temperature, and the reaction mixture was heated to 240 °C and stirred for 4 h. After the reaction was completed, the reaction mixture was cooled to room temperature. Half-saturated brine (100 mL) was added, and ethyl acetate (150 mL) was added for extraction. The organic phase was washed with half-saturated brine (80 mL × 3), dried over anhydrous sodium sulfate, filtered, and concentrated under reduced pressure to remove the solvent. The resulting residue was separated by column chromatography (eluent: petroleum ether/ethyl acetate = 19/1-3/1, volume ratio) to give intermediate **WX063-7.** ¹H NMR (400 MHz, CDCl₃) δ: 7.32 (d, *J*=8.8 Hz, 1H), 7.08 (d, *J*=8.8 Hz, 1H), 6.07-5.98 (m, 1H), 5.42 (s, 1H), 5.14 (dd, *J*=1.6 Hz, 10.0 Hz, 1H), 4.97 (dd, *J*=1.6 Hz, 17.2 Hz, 1H), 4.22 (q, *J*=7.0 Hz, 2H), 4.08 (s, 2H), 3.64-3.61 (m, 2 H), 1.27 (t, *J*=7.0 Hz, 3H).

### Step 7: synthesis of intermediate WX063-8

Intermediate **WX063-7** (0.8 g, 3.06 mmol) and copper acetate (1.67 g, 9.19 mmol) were added to water (2.5 mL) and *N,N*-dimethylfonnamide (12.5 mL) at room temperature, and then lithium chloride (398.42 mg, 9.19 mmol) and palladium chloride (27.15 mg, 153.10 µmol) were added. The reaction mixture was stirred at room temperature under air atmosphere for 12 h. After the reaction was completed, the reaction mixture was filtered and diluted with water (15 mL) and ethyl acetate (20 mL), followed by liquid separation. The organic phase was washed with half-saturated brine (10 mL × 3), dried over anhydrous sodium sulfate, filtered, and concentrated under reduced pressure to remove the solvent. The resulting residue was separated by column chromatography (eluent: petroleum ether/ethyl acetate = 1/0-5.25/1, volume ratio) to give intermediate **WX063-8.** ¹H NMR (400 MHz, CDCl₃) δ: 7.62 (d, *J*=9.2 Hz, 1H), 7.40 (d, *J*=8.8 Hz, 1H), 6.64 (s, 1H), 4.22 (q, *J*=7.2 Hz, 2H), 4.13 (s, 2H), 2.55 (s, 3H), 1.24 (t, *J*=7.2, 3H).

### Step 8: synthesis of WX063

Intermediate **WX063-8** (0.1 g, 385.72 µmol) was dissolved in tetrahydrofuran (3 mL) at room temperature, and then acrylamide (27.42 mg, 385.72 µmol) and a solution of potassium *tert*-butoxide in tetrahydrofuran (1 M, 385.72 µmol) were added sequentially. The reaction mixture was stirred for 12 h. After the reaction was completed, the reaction mixture was adjusted to pH 6 with 1 N diluted hydrochloric acid and diluted with water (5 mL) and ethyl acetate (10 mL), followed by liquid separation. The organic phase was dried over anhydrous sodium sulfate, filtered, and concentrated under reduced pressure. The resulting residue was purified by prep-HPLC (mobile phase: acetonitrile/water; neutral system: 10 mM NH₄HCO₃, neutral system), and the resulting fraction was adjusted to about pH 6 with 1 N hydrochloric acid and concentrated under reduced pressure to remove acetonitrile, and ethyl acetate (50 mL × 3) was added for extraction. The organic phases were combined, dried over anhydrous sodium sulfate, filtered and concentrated under reduced pressure to give target compound **WX063.** MS-ESI *m*/*z:* 285.0 [M+H]⁺.¹H NMR (400 MHz, DMSO_*d*₆) δ: 11.15 (s, 1H), 7.85 (d, *J*=9.2 Hz, 1H), 7.58 (d, *J*=8.8 Hz, 1H), 6.82 (s, 1H), 4.71 (dd, *J*=5.0 Hz, 12.2 Hz, 1H), 2.88-2.79 (m, 1H), 2.69-2.63 (m, 1H), 2.52 (s, 3H), 2.46-2.39 (m, 1H), 2.28-2.21 (m, 1H).

### Example 64: WX064

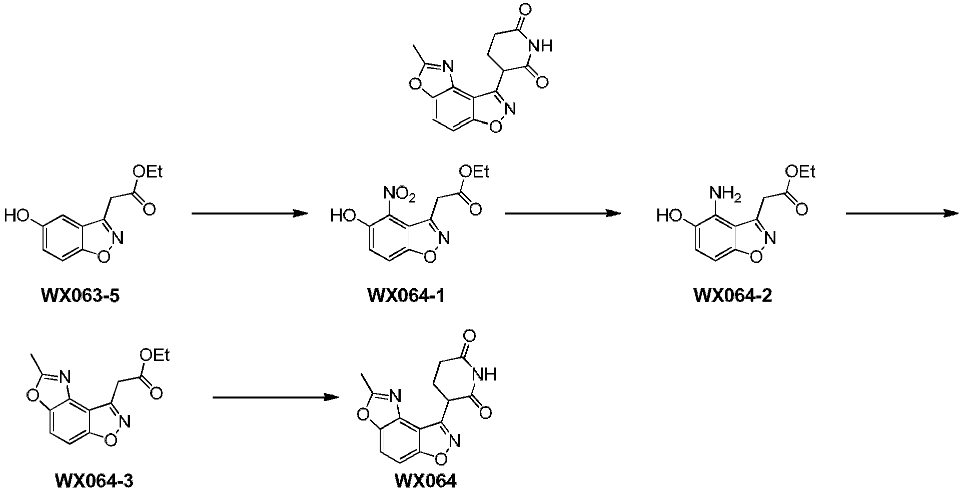

### Step 1: synthesis of intermediate WX064-1

Intermediate **WX063-5** (0.5 g, 2.26 mmol) was dissolved in concentrated sulfuric acid (5 mL, purity: 98%) at room temperature under nitrogen atmosphere. After the reaction mixture was cooled to 0 °C, potassium nitrate (239.94 mg, 2.37 mmol) was added. The resulting reaction mixture was warmed to 20 °C and stirred for 12 h. After the reaction was completed, the reaction mixture was poured into ice water (60 mL) and then dichloromethane (50 mL × 3) was added for extraction. The organic phases were combined, washed with water (60 mL × 3) and saturated brine (60 mL × 3) sequentially, dried over anhydrous sodium sulfate and filtered, and the filtrate was concentrated under reduced pressure to remove the solvent, thus giving intermediate **WX064-1.**

### Step 2: synthesis of intermediate WX064-2

Intermediate **WX064-1** (0.46 g, 1.73 mmol) was dissolved in ethanol (10 mL) at room temperature under nitrogen atmosphere. After the reaction mixture was cooled to 0 °C, stannous chloride dihydrate (2.73 g, 12.10 mmol) was added. The resulting reaction mixture was warmed to 20 °C and stirred for 12 h. After the reaction was completed, the reaction mixture was concentrated under reduced pressure to remove the solvent, diluted with water (30 mL), adjusted to pH 6-7 with saturated aqueous sodium bicarbonate solution, and filtered, and the filtrate was collected and extracted with 2-methyltetrahydrofuran (60 mL × 3). The organic phases were combined, washed with saturated brine (60 mL × 3), dried over anhydrous sodium sulfate and filtered, and the filtrate was concentrated under reduced pressure to remove the solvent, thus giving intermediate **WX064-2.**

### Step 3: synthesis of intermediate WX064-3

Intermediate **WX064-2** (0.3 g, 1.27 mmol) and triethyl orthoacetate (309.04 mg, 1.90 mmol) were dissolved in N,N-dimethylformamide (6 mL) at room temperature, and then zirconium tetrachloride (295.96, 1.27 mmol) was added. The reaction mixture was stirred at 20 °C for 12 h. After the reaction was completed, the reaction mixture was poured into ice water (50 mL), and ethyl acetate (50 mL × 3) was added for extraction. The organic phases were combined, washed with half-saturated brine (60 mL × 3) and saturated brine (60 mL × 3) sequentially, dried over anhydrous sodium sulfate and filtered, and the filtrate was concentrated under reduced pressure to remove the solvent. The resulting residue was separated by column chromatography (eluent: petroleum ether/ethyl acetate = 1/0-6/1, volume ratio) to give intermediate **WX064-3.** ¹H NMR (400 MHz, CDCl₃) δ: 7.69 (d, *J*=8.8 Hz, 1H), 7.51 (d, *J*=9.2 Hz, 1H), 4.27 (s, 2H), 4.26 (q, *J*=7.2 Hz, 2H), 2.71 (s, 3H), 1.28 (t, *J*=7.0 Hz, 3H).

### Step 4: synthesis of WX064

Intermediate **WX064-3** (0.27 g, 1.04 mmol) was dissolved in tetrahydrofuran (10 mL) at room temperature, and then acrylamide (73.74 mg, 1.04 mmol) and a solution of potassium *tert*-butoxide (1 M, 1.04 mL) in tetrahydrofuran were added sequentially. The reaction mixture was stirred at 20 °C for 3 h. After the reaction was completed, the reaction mixture was adjusted to pH 6 with 1 N diluted hydrochloric acid, added with brine (20 mL), and extracted with ethyl acetate (20 mL × 3). The organic phases were combined, dried over anhydrous sodium sulfate and filtered, and the filtrate was concentrated under reduced pressure to remove the solvent. The resulting residue was separated by prep-HPLC twice (mobile phase: acetonitrile/water; acidic system: 0.05% HCl) to give target compound **WX064.** MS-ESI *m*/*z:* 286.0 [M+H]⁺.¹H NMR (400 MHz, DMSO_*d*₆) δ: 11.16 (s, 1H), 8.04 (d, *J*=9.2 Hz, 1H), 7.77 (d, *J*=9.2 Hz, 1H), 4.66 (dd, *J*=4.8, 12.0 Hz, 1H), 2.95-2.85 (m, 1H), 2.84-2.75 (m, 1H), 2.69 (s, 3H), 2.67-2.62 (m, 1H), 2.21-2.17 (m, 1H).

### Example 65: WX065

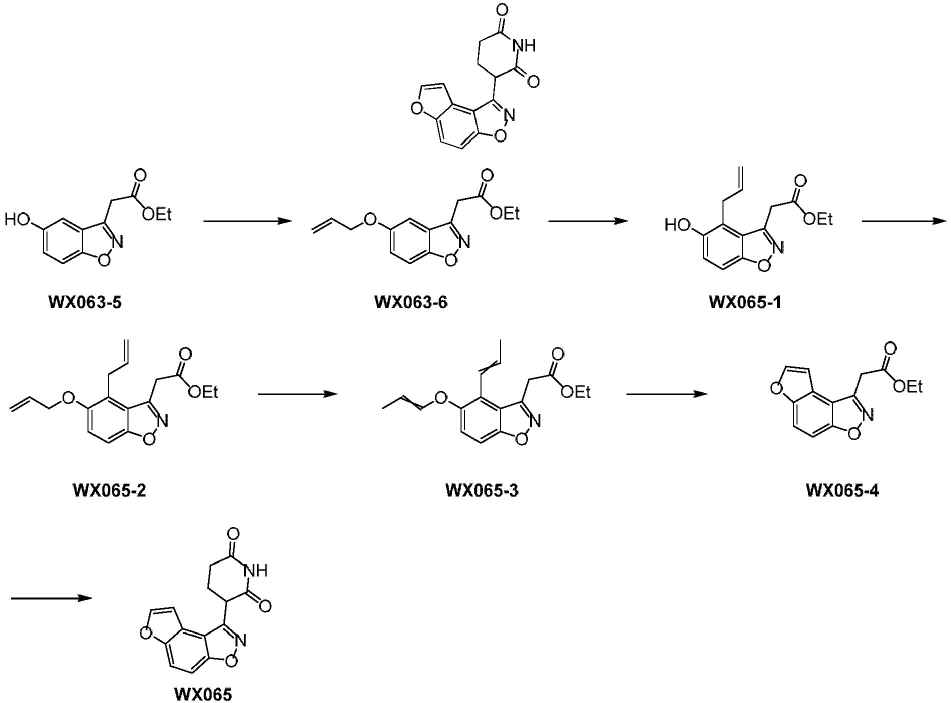

### Step 1: synthesis of intermediate WX063-6

Intermediate **WX063-5** (5 g, 22.60 mmol) was dissolved in *N,N*-dimethylfonnamide (75 mL) at 20 °C, and then potassium carbonate (10.93 g, 79.11 mmol) was added and 3-bromo-1-propene (2.87 g, 23.73 mmol) was added dropwise. The resulting reaction mixture was stirred at 20 °C for 12 h. After the reaction was completed, the reaction mixture was filtered, and water (80 mL) and ethyl acetate (80 mL) were added to the filtrate. The organic phase was washed with half-saturated brine (50 mL × 3), dried over anhydrous sodium sulfate, filtered, and concentrated under reduced pressure to remove the solvent. The resulting residue was separated by column chromatography (eluent: petroleum ether/ethyl acetate = 1/0-9/1, volume ratio) to give intermediate **WX063-6.**

### Step 2: synthesis of intermediate WX065-1

Intermediate **WX063-6** (4.5 g, 17.22 mmol) was dissolved in *N,N-*dimethylformamide (60 mL) at 20 °C under nitrogen atmosphere, and the reaction mixture was heated to 240 °C and stirred for 4 h. After the reaction was acetate (150 mL) were added to the reaction mixture. The organic phase was washed with half-saturated brine (80 mL × 3). The organic phase was dried over anhydrous sodium sulfate, filtered and concentrated under reduced pressure to remove the solvent. The resulting residue was separated by column chromatography (eluent: petroleum ether/ethyl acetate = 19/1-3/1, volume ratio) to give intermediate **WX065-1.** ¹H NMR (400 MHz, MeOD_*d*₄) δ: 7.33 (d, *J*=8.4 Hz, 1H), 7.15 (d, *J*=8.8 Hz, 1H), 6.06-5.96 (m, 1H), 5.02-4.98 (m, 1H), 4.80 (q, *J*=2.2 Hz, 1H), 4.20 (q, *J*=7.0 Hz, 2H), 4.08 (s, 2H), 3.62 (t, *J*=1.8 Hz, 1H), 3.61 (t, *J*=2.0 Hz, 1H), 1.26 (t, *J*=7.4 Hz, 3H).

### Step 3: synthesis of intermediate WX065-2

Intermediate **WX065-1** (1 g, 3.83 mmol) and 3-bromoprop-1-ene (463.03 mg, 3.83 mmol) were dissolved in *N,N*-dimethylfonnamide (20 mL) at 20 °C, and then potassium carbonate (1.59 g, 11.48 mmol) was added. The reaction mixture was stirred at 20 °C for 12 h. After the reaction was completed, the reaction mixture was filtered, and the filtrate was poured into 0.5 M diluted hydrochloric acid (60 mL) and ethyl acetate (40 mL × 3) was added for extraction. The organic phases were combined, washed with half-saturated brine (60 mL × 3), dried over anhydrous sodium sulfate, filtered, and concentrated under reduced pressure to remove the solvent. The resulting residue was separated by column chromatography (eluent: petroleum ether/ethyl acetate = 1/0-49/1, volume ratio) to give intermediate **WX065-2.** ¹H NMR (400 MHz, CDCl₃) δ: 7.41 (d, *J*=9.2 Hz, 1H), 7.22 (d, *J*=9.2 Hz, 1H), 6.10-5.97 (m, 2H), 5.41 (dd, *J*=1.4, 17.4 Hz, 1H), 5.28 (dd, *J*=1.4, 10.6 Hz, 1H), 5.04 (dd, *J*=1.6, 10.0 Hz, 1H), 4.84 (dd, *J*=1.6, 17.2 Hz, 1H), 4.59-4.57 (m, 2H), 4.22 (q, *J*=7.2 Hz, 2H), 4.09 (s, 2H), 3.72-3.62 (m, 2H), 1.27 (t, *J*=7.2 Hz, 3H).

### Step 4: synthesis of intermediate WX065-3

Intermediate **WX065-2** (850.00 mg, 2.82 mmol) was dissolved in toluene (20 mL) at room temperature under nitrogen atmosphere, and then tris(triphenylphosphine)carbonyl ruthenium hydrochloride (268.65 mg, 282.08 µmol) was added. The reaction mixture was heated to 65 °C and stirred for 12 h. After the reaction was completed, the reaction mixture was concentrated under reduced pressure to remove the solvent. The resulting residue was separated by column chromatography (eluent: petroleum ether/ethyl acetate = 1/0-49/1, volume ratio) to give intermediate **WX065-3.** ¹H NMR (400 MHz, CDCl₃) δ: 7.36-7.27 (m, 1H), 7.19-7.12 (m, 1H), 6.47-6.39 (m, 1H), 6.33-5.92 (m, 2H), 5.17-5.06 (m, 1H), 4.80-4.69 (m, 1H), 4.14-3.92 (m, 4H), 1.88-1.85 (m, 2H), 1.66-1.46 (m, 3H), 1.19-1.10 (m, 3H).

### Step 5: synthesis of intermediate WX065-4

Intermediate **WX065-3** (0.6 g, 1.99 mmol) was dissolved in toluene (18 mL) at room temperature under nitrogen atmosphere, and then GRUBBS catalyst (second generation) (84.52 mg, 99.56 µmol) was added. The reaction mixture was heated to 60 °C and stirred for 3 h. After the reaction was completed, the reaction mixture was concentrated under reduced pressure to remove the solvent. The resulting residue was separated by column chromatography (eluent: petroleum ether/ethyl acetate = 1/0-32/1, volume ratio) to give intermediate **WX065-4.**

### Step 6: synthesis of WX065

Intermediate **WX065-4** (0.365 g, 1.49 mmol) and acrylamide (105.79 mg, 1.49 mmol) were dissolved in tetrahydrofuran (10 mL) at room temperature under nitrogen atmosphere, and then a solution of potassium *tert*-butoxide in tetrahydrofuran (1 M, 1.49 mL) was added. The reaction mixture was stirred at 20 °C for 12 h. After the reaction was completed, the reaction mixture was adjusted to pH 5-6 with 2 N diluted hydrochloric acid, and concentrated under reduced pressure to remove the solvent. The resulting residue was separated by prep-HPLC twice (mobile phase: acetonitrile/water; acidic system: 0.05% HCl) to give target compound **WX065.** MS-ESI *m*/*z:* 271.0 [M+H]⁺. ¹H NMR (400 MHz, DMSO_*d*₆) δ: 11.17 (s, 1H), 8.26 (d, *J*=2.0 Hz, 1H), 7.97 (dd, *J*=0.6, 9.0 Hz, 1H), 7.71 (d, *J*=9.2 Hz, 1H), 7.18 (dd, *J*=0.8, 2.0 Hz,, 1H), 4.77 (dd, *J*=5.0, 12.2 Hz, 1H), 2.87-2.78 (m, 1H), 2.69-2.63 (m, 1H), 2.47-2.41(m, 1H), 2.33-2.25 (m, 1H).

### Example 66: WX066

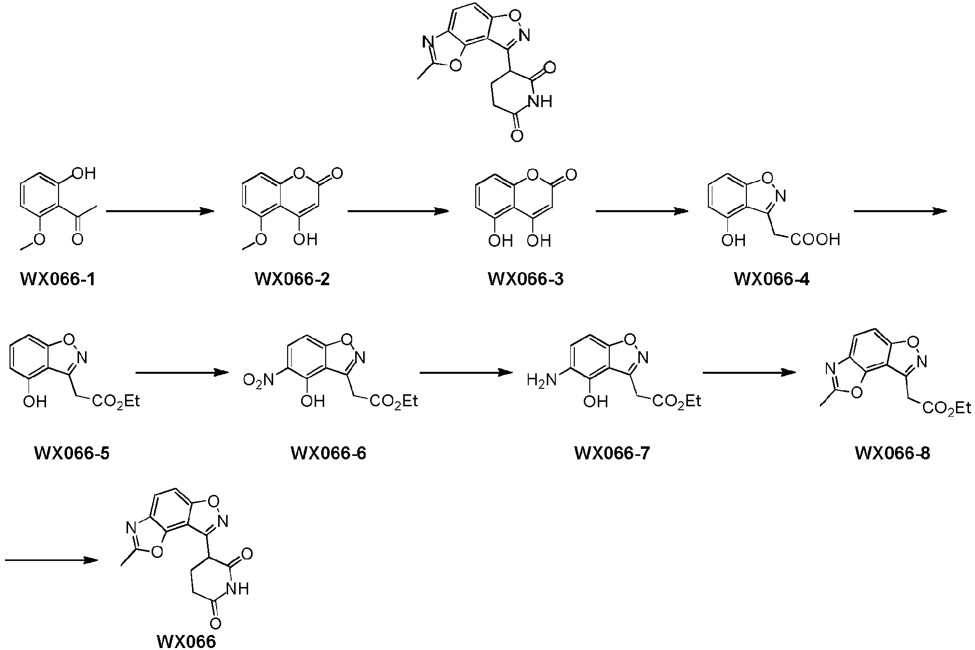

### Step 1: synthesis of intermediate WX066-2

Intermediate **WX066-1** (20 g, 120.36 mmol) was dissolved in toluene (200 mL) at room temperature under nitrogen atmosphere, and then diethyl carbonate (146.25 g, 1.24 mol, 150 mL) was added, and lastly, sodium hydride (19.26 g, 481.42 mmol, purity: 60%) was added in batches at 5-10 °C. The reaction mixture was stirred at 15 °C for 30 min, and then heated to 120 °C and stirred at 120 °C for 12 h. After the reaction was completed, the reaction mixture was cooled to room temperature and then slowly poured into ice water (500 mL), and ethyl acetate (100 mL × 2) was added for extraction. The organic phase was removed, and the aqueous phase was adjusted to pH 5-6 with 6 M diluted hydrochloric acid and extracted with 2-methyltetrahydrofuran (100 mL × 3). The organic phases were combined, dried over anhydrous sodium sulfate and filtered, and the filtrate was concentrated under reduced pressure to remove the solvent, thus giving intermediate **WX066-2.** ¹H NMR (400 MHz, DMSO_*d*₆) δ: 11.27 (s, 1H), 7.55 (t, *J*=8.2 Hz, 1H), 6.94 (d, *J*=8.4 Hz, 2H), 5.51 (s, 1H), 3.90 (s, 3H).

### Step 2: synthesis of intermediate WX066-3

Intermediate **WX066-2** (20 g, 104.08 mmol) was dissolved in dichloromethane (300 mL) at room temperature under nitrogen atmosphere, and then boron tribromide (78.22 g, 312.23 mmol, 30.08 mL) was added dropwise at -50 °C to -30 °C. The reaction mixture was warmed to 10 °C and stirred for 12 h. After the reaction was completed, the reaction mixture was slowly poured into ice water (500 mL), and 2-methyltetrahydrofuran (500 mL × 4) was added for extraction. The organic phases were combined, dried over anhydrous sodium sulfate and filtered, and the filtrate was concentrated under reduced pressure to remove the solvent, thus giving intermediate **WX066-3.**

### Step 3: synthesis of intermediate WX066-4

Intermediate **WX066-3** (5 g, 28.07 mmol) was dissolved in methanol (100 mL) at room temperature under nitrogen atmosphere, and then hydroxylamine hydrochloride (6.83 g, 98.24 mmol) and sodium acetate (8.06 g, 98.24 mmol) were added sequentially. After being stirred at 10 °C for 15 min, the reaction mixture was heated to 80 °C and stirred for 12 h, and then heated to 90 °C and stirred at 90 °C for 6 h. After the reaction was completed, the reaction mixture was cooled to room temperature, added with 2 M diluted hydrochloric acid (200 mL), and concentrated under reduced pressure to remove most of the ethanol, and ethyl acetate (100 mL × 4) was added for extraction. The organic phases were combined, dried over anhydrous sodium sulfate and filtered, and the filtrate was concentrated under reduced pressure to remove the solvent, thus giving intermediate **WX066-4.**

### Step 4: synthesis of intermediate WX066-5

Intermediate **WX066-4** (4 g, 20.71 mmol) was dissolved in ethanol (40 mL) at room temperature under nitrogen atmosphere, and then sulfuric acid (920.00 mg, 9.19 mmol, 0.5 mL, purity: 98%) was added. The reaction mixture was heated to 80 °C and stirred for 4 h. After the reaction was completed, the reaction mixture was cooled to room temperature and concentrated under reduced pressure to remove the solvent. The resulting residue was separated by column chromatography (eluent: petroleum ether/ethyl acetate = 1/0-4/1, volume ratio) to give intermediate **WX066-5.** ¹H NMR (400 MHz, CDCl₃) δ: 8.84 (s, 1H), 7.43 (t, *J*=*8.2* Hz, 1H), 7.12 (d, *J*=8.4 Hz, 1H), 6.78 (d, *J*=7.6 Hz, 1H), 4.30 (q, *J*=7.2 Hz, 2H), 4.12 (s, 2H), 1.33 (t, *J*=7.0 Hz, 3H).

### Step 5: synthesis of intermediate WX066-6

Intermediate **WX066-5** (2.6 g, 11.75 mmol) was dissolved in concentrated sulfuric acid (15 mL, purity: 98%) at room temperature under nitrogen atmosphere, and then potassium nitrate (1.19 g, 11.75 mmol) was added in batches at 0-10 °C. The resulting reaction mixture was warmed to 12 °C and stirred for 2 h. After the reaction was completed, the reaction mixture was slowly poured into ice water (100 mL) dropwise, and dichloromethane (50 mL × 3) was added for extraction. The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 1/0-7/3, volume ratio) to give intermediate **WX066-6.** ¹H NMR (400 MHz, CDCl₃) δ: 11.67 (s, 1H), 8.30 (d, *J*=9.6 Hz, 1H), 7.17 (d, *J*=9.6 Hz, 1H), 4.25 (q, *J*=7.4 Hz, 2H), 4.16 (s, 2H), 1.29 (t, *J*=7.4 Hz, 3H).

### Step 6: synthesis of intermediate WX066-7

Intermediate **WX066-6** (2.1 g, 7.89 mmol) was dissolved in ethanol (30 mL) at room temperature, and then stannous chloride dihydrate (12.46 g, 55.22 mmol) was added. The reaction mixture was stirred at 35 °C for 12 h. After the reaction was completed, the reaction mixture was cooled to room temperature and concentrated under reduced pressure to remove the solvent. Water (50 mL) was added and the pH was adjusted to 6-7 with saturated aqueous sodium bicarbonate solution. The resulting mixture was filtered, and the filtrate was extracted with dichloromethane (100 mL × 3). Dichloromethane (100 mL) was added to the filter cake, and the mixture was stirred at room temperature for 30 min and then filtered to collect the filtrate. The organic phases were combined, dried over anhydrous sodium sulfate and filtered, and the filtrate was concentrated under reduced pressure to remove the solvent, thus giving intermediate **WX066-7.**

### Step 7: synthesis of intermediate WX066-8

Intermediate **WX066-7** (1.5 g, 6.35 mmol) was dissolved in *N,N*-dimethylformamide (30 mL) at 15 °C, and then zirconium tetrachloride (739.88 mg, 3.17 mmol, 264.24 µL) and triethyl orthoacetate (1.55 g, 9.52 mmol, 1.75 mL) were added sequentially. The reaction mixture was stirred at 15 °C for 24 h. After the reaction was completed, water (100 mL) was added to the reaction mixture and then ethyl acetate (50 mL × 3) was added for extraction. The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 1/0-4/1, volume ratio) to give intermediate **WX066-8.** ¹H NMR (400 MHz, CDCl₃) δ: 7.84 (d, *J*=9.2 Hz, 1H), 7.56 (d, *J*=8.8 Hz, 1H), 4.26 (q, *J*=7.2 Hz, 2H), 4.20 (s, 2H), 2.71 (s, 3H), 1.28 (t, *J*=7.0 Hz, 3H).

### Step 8: synthesis of WX066

Intermediate **WX066-8** (55 mg, 211.34 µmol) was dissolved in tetrahydrofuran (5 mL) at room temperature, and then acrylamide (15.02 mg, 211.34 µmol) and a solution of potassium *tert*-butoxide (1 M, 211.34 µL) in tetrahydrofuran were added sequentially. The reaction mixture was stirred at 15 °C for 3 h. After the reaction was completed, the reaction mixture was poured into 0.5 M diluted hydrochloric acid (20 mL) and ethyl acetate (20 mL × 3) was then added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl), the resulting residue from which was then separated by prep-HPLC (mobile phase: acetonitrile/water; neutral system: 10 mM NH₄HCO₃) to give target compound **WX066.** MS-ESI *m*/*z*: 286.0 [M+H]⁺. ¹H NMR (400 MHz, DMSO_*d*₆) δ: 7.99 (d, *J*=8.4 Hz, 1H), 7.79 (d, *J*=8.8 Hz, 1H), 4.72 (dd, *J*=4.0, 12.4 Hz, 1H), 2.97-2.83 (m, 1H), 2.74-2.68 (m, 1H), 2.67 (s, 3H), 2.38-2.22 (m, 2H).

### Example 67: WX067

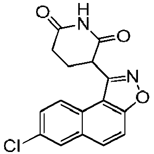

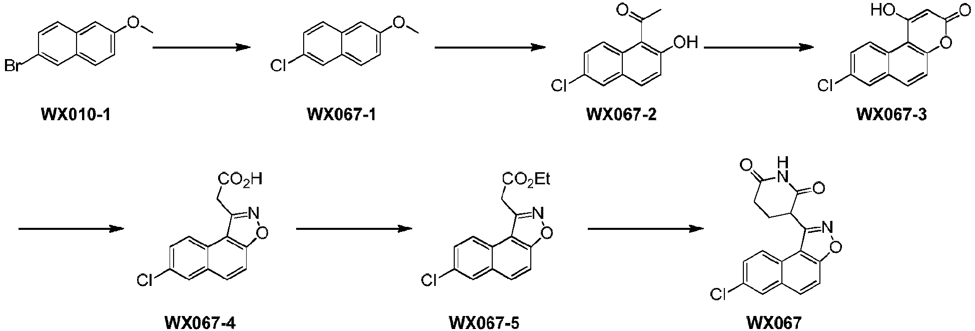

### Step 1: synthesis of intermediate WX067-1

Intermediate **WX010-1** (20 g, 84.36 mmol) was dissolved in *N,N*-dimethylfonnamide (600 mL) at room temperature under nitrogen atmosphere, and then copper(I) iodide (16.07 g, 84.36 mmol) and cuprous chloride (83.51 g, 843.55 mmol) were added sequentially. The reaction mixture was heated to 140 °C and stirred for 12 h. After the reaction was completed, the reaction mixture was cooled to room temperature, slowly poured into ice water (1000 mL) and filtered, and the filter cake was collected and concentrated under reduced pressure. Then, the resulting residue was dissolved in ethyl acetate (500 mL), insoluble materials were removed by filtration, and the filtrate was concentrated under reduced pressure to give intermediate **WX067-1.** ¹H NMR (400 MHz, CDCl₃) δ: 7.76 (s, 1H), 7.68 (d, *J*=6.4 Hz, 1H), 7.66 (d, *J*=6.0 Hz, 1H), 7.39 (d, *J*=7.6 Hz, 1H), 7.18 (d, *J*=9.2 Hz, 1H), 7.12 (s, 1H), 3.93 (s, 3H).

### Step 2: synthesis of intermediate WX067-2

Intermediate **WX067-1** (16.25 g, 84.35 mmol) was dissolved in dichloromethane (180 mL) at room temperature under nitrogen atmosphere, and then acetyl chloride (7.28 g, 92.79 mmol, 6.62 mL) was added dropwise at 0 °C, and aluminum trichloride (22.50 g, 168.71 mmol) was added in batches at 0 °C under nitrogen atmosphere. The reaction mixture was then heated to 15 °C and stirred for 4 h. After the reaction was completed, the reaction mixture was slowly poured into ice water (500 mL), and dichloromethane (100 mL × 2) was added for extraction. The organic phases were combined, washed with saturated brine (200 mL), dried over anhydrous sodium sulfate and filtered, and the filtrate was concentrated under reduced pressure to remove the solvent, thus giving intermediate **WX067-2.** ¹H NMR (400 MHz, CDCl₃) δ: 13.38 (s, 1H), 8.04 (d, *J*=9.2 Hz, 1H), 7.81 (d, *J*=8.8 Hz, 1H), 7.78 (d, *J*=2.4 Hz, 1H), 7.53 (dd, *J*=2.2, 9.0 Hz, 1H), 7.19 (d, *J*=9.2 Hz, 1 H), 2.86 (s, 3H).

### Step 3: synthesis of intermediate WX067-3

Intermediate **WX067-2** (18 g, 81.58 mmol) was dissolved in diethyl carbonate (100 mL) and toluene (100 mL) at room temperature under nitrogen atmosphere, and then sodium hydride (16.31 g, 407.88 mmol, purity: 60%) was added in batches at 0 °C. The reaction mixture was heated to 120 °C and stirred for 12 h. After the reaction was completed, the reaction mixture was cooled to room temperature and then slowly poured into ice water (500 mL), and ethyl acetate (200 mL × 2) was added for extraction. The organic phase was removed, and the aqueous phase was adjusted to pH 4-5 with 1 M diluted hydrochloric acid and extracted with ethyl acetate (300 mL × 3). The organic phases were combined, washed with saturated brine (500 mL), dried over anhydrous sodium sulfate and filtered, and the filtrate was concentrated under reduced pressure to remove the solvent, thus giving intermediate **WX067-3.** ¹H NMR (400 MHz, DMSO_*d₆*) δ: 13.07 (s, 1H), 9.28 (d, *J*=9.6 Hz, 1H), 8.19 (d, *J*=6.4 Hz, 1H), 8.18 (s, 1H), 7.71 (dd, *J*=2.4, 9.2 Hz, 1H), 7.60 (d, *J*=9.2 Hz, 1H), 5.79 (s, 1H).

### Step 4: synthesis of intermediate WX067-4

Intermediate **WX067-3** (2 g, 8.11 mmol) was dissolved in ethanol (40 mL) at room temperature under nitrogen atmosphere, and then sodium acetate (2.33 g, 28.38 mmol) and hydroxylamine hydrochloride (3.38 g, 48.65 mmol) were added sequentially. The reaction mixture was heated to 80 °C and stirred for 36 h. After the reaction was completed, the reaction mixture was cooled to room temperature and concentrated under reduced pressure to remove ethanol, and then water (20 mL) and 1 M diluted hydrochloric acid (10 mL) were added, and ethyl acetate (50 mL × 3) was added for extraction. The organic phases were combined, dried over anhydrous sodium sulfate and filtered, and the filtrate was concentrated under reduced pressure to remove the solvent, thus giving intermediate **WX067-4.**

### Step 5: synthesis of intermediate WX067-5

Intermediate **WX067-4** (2 g, 7.64 mmol) was dissolved in ethanol (40 mL) at room temperature under nitrogen atmosphere, and then sulfuric acid (1.84 g, 18.39 mmol, 1 mL, purity: 98%) was added. The reaction mixture was heated to 80 °C and stirred for 4 h. After the reaction was completed, the reaction mixture was cooled to room temperature and concentrated under reduced pressure to remove most of the solvent. The resulting residue was diluted with water (100 mL) and extracted with 2-methyltetrahydrofuran (200 mL × 3). The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 1/0-20/1, volume ratio) to give intermediate **WX067-5.** ¹H NMR (400 MHz, CDCl₃) δ: 8.07 (d, *J*=8.8 Hz, 1H), 8.00 (d, *J*=2.0 Hz, 1H), 7.90 (d, *J*=9.2 Hz, 1H), 7.76 (d, *J*=8.8 Hz, 1H), 7.64 (dd, *J*=2.4, 8.8 Hz, 1H), 4.33 (s, 2H), 4.23 (q, *J*=7.0 Hz, 2H), 1.23 (t, *J*=7.0 Hz, 3H).

### Step 6: synthesis of WX067

Intermediate **WX067-5** (560 mg, 1.93 mmol) was dissolved in tetrahydrofuran (20 mL) at 15 °C, and then acrylamide (137.39 mg, 1.93 mmol) and a solution of potassium *tert*-butoxide (1 M, 1.93 mL) in tetrahydrofuran were added sequentially at 0 °C. The reaction mixture was stirred at 15 °C for 2 h. After the reaction was completed, 1 M diluted hydrochloric acid was added to adjust pH to 4-5, water (50 mL) was added, and ethyl acetate (50 mL × 3) was added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give target compound **WX067.** MS-ESI m/z: 315.0 [M+H]⁺, 317.1 [M+H+2]⁺.¹H NMR (400 MHz, MeOD_*d*₄) δ: 8.23 (d, *J*=9.2 Hz, 1H), 8.13 (d, *J*=2.0 Hz, 1H), 8.08 (d, *J*=9.2 Hz, 1H), 7.85 (d, *J*=9.2 Hz, 1H), 7.70 (dd, *J*=2.2, 9.0 Hz, 1H), 4.97 (dd, *J*=5.0, 10.6 Hz, 1H), 2.90-2.77 (m, 2H), 2.74-2.64 (m, 1H), 2.57-2.47 (m, 1H).

### Example 68: WX068

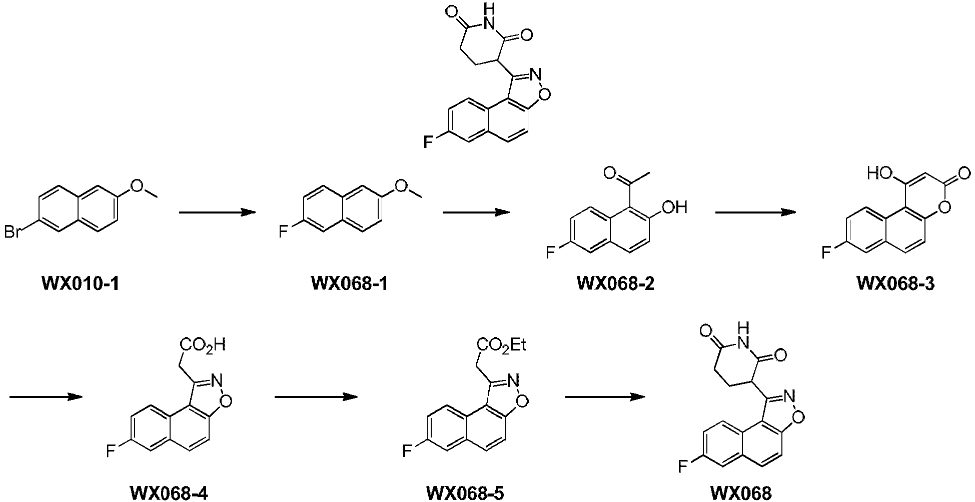

### Step 1: synthesis of intermediate WX068-1

Intermediate **WX010-1** (10 g, 42.18 mmol) was dissolved in tetrahydrofuran (300 mL) at room temperature under nitrogen atmosphere, and then a solution of *n*-butyllithium in *n*-hexane (2.5 M, 18.56 mL) was added dropwise slowly at -78 °C. The reaction was stirred at -78 °C for 0.5 h. *N*-fluorobisbenzenesulfonamide (83.51 g, 843.55 mmol, 20.17 mL) was then added in batches, and the reaction mixture was stirred at -78 °C for 1 h. The reaction mixture was warmed to 15 °C and stirred for 12 h. After the reaction was completed, the reaction mixture was slowly poured into ice water (100 mL), and ethyl acetate (100 mL × 3) was added for extraction. The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 1/0-20/1, volume ratio) to give intermediate **WX068-1.**

### Step 2: synthesis of intermediate WX068-2

Intermediate **WX068-1** (3.5 g, 19.87 mmol) was dissolved in dichloromethane (60 mL) at room temperature under nitrogen atmosphere, and then acetyl chloride (1.72 g, 21.85 mmol, 1.56 mL) was added, and aluminum trichloride (5.30 g, 39.73 mmol, 2.17 mL) was added in batches at 0 °C. The reaction mixture was then warmed to 15 °C and stirred for 4 h. After the reaction was completed, the reaction mixture was slowly poured into ice water (50 mL), and dichloromethane (50 mL × 2) was added for extraction. The organic phases were combined, washed with saturated brine (100 mL), dried over anhydrous sodium sulfate and filtered, and the filtrate was concentrated under reduced pressure to remove the solvent. The resulting residue was separated by column chromatography (eluent: petroleum ether/ethyl acetate = 1/0-5/1, volume ratio) to give intermediate **WX068-2.**

### Step 3: synthesis of intermediate WX068-3

Intermediate **WX068-2** (2.3 g, 11.26 mmol) was dissolved in diethyl carbonate (20 mL) and toluene (20 mL) at room temperature under nitrogen atmosphere, and then sodium hydride (2.25 g, 56.32 mmol, purity: 60%) was added in batches at 0 °C. The reaction mixture was heated to 120 °C and stirred for 12 h. After the reaction was completed, the reaction mixture was cooled to room temperature and then slowly poured into ice water (100 mL), and ethyl acetate (200 mL × 2) was added for extraction. The organic phase was removed, and the aqueous phase was adjusted to pH 4-5 with 1 M diluted hydrochloric acid and extracted with ethyl acetate (300 mL × 3). The organic phases were combined, washed with saturated brine (500 mL), dried over anhydrous sodium sulfate and filtered, and the filtrate was concentrated under reduced pressure to remove the solvent, thus giving intermediate **WX068-3.** ¹H NMR (400 MHz, DMSO_*d*₆) δ: 13.01 (s, 1H), 9.34 (dd, *J*=5.6, 9.6 Hz, 1H), 8.19 (d, *J*=8.8 Hz, 1H), 7.88 (dd, *J*=3.0, 9.8 Hz, 1H), 7.61 (dd, *J*=2.8, 12.8 Hz, 1H), 7.59 (d, *J*=9.2 Hz, 1H), 5.77 (s, 1H).

### Step 4: synthesis of intermediate WX068-4

Intermediate **WX068-3** (2.2 g, 9.56 mmol) was dissolved in ethanol (80 mL) at room temperature under nitrogen atmosphere, and then hydroxylamine hydrochloride (3.98 g, 57.34 mmol) and sodium ethoxide (2.28 g, 33.45 mmol) were added sequentially. The reaction mixture was heated to 80 °C and stirred for 36 h. After the reaction was completed, the reaction mixture was cooled to room temperature and concentrated under reduced pressure to remove ethanol, and then water (100 mL) and 1 M diluted hydrochloric acid (20 mL) were added, and 2-methyltetrahydrofuran (200 mL × 2) was added for extraction. The organic phases were combined, dried over anhydrous sodium sulfate and filtered, and the filtrate was concentrated under reduced pressure to remove the solvent, thus giving intermediate **WX068-4.**

### Step 5: synthesis of intermediate WX068-5

Intermediate **WX068-4** (0.9 g, 3.67 mmol) was dissolved in ethanol (20 mL) at room temperature under nitrogen atmosphere, and then sulfuric acid (920.00 mg, 9.19 mmol, 0.5 mL, purity: 98%) was added. The reaction mixture was heated to 80 °C and stirred for 1 h. After the reaction was completed, the reaction mixture was cooled to room temperature and concentrated under reduced pressure to remove most of the solvent. The resulting residue was diluted with water (50 mL) and extracted with 2-methyltetrahydrofuran (50 mL × 3). The organic phases were combined, dried over anhydrous sodium sulfate and filtered, and the filtrate was concentrated under reduced pressure to remove the solvent. The resulting residue was separated by column chromatography (eluent: petroleum ether/ethyl acetate = 1/0-20/1, volume ratio) to give intermediate **WX068-5.** ¹H NMR (400 MHz, CDCl₃) δ: 8.13 (dd, *J*=5.2, 9.2 Hz, 1H), 7.92 (d, *J*=9.2 Hz, 1H), 7.76 (d, *J*=9.2 Hz, 1H), 7.66 (dd, *J*=2.6, 9.4 Hz, 1H), 7.47 (td, *J*=2.8, 8.6 Hz, 1H), 4.33 (s, 2H), 4.23 (q, *J*=7.0 Hz, 2H), 1.22 (t, *J*=7.2 Hz, 3H).

### Step 6: synthesis of WX068

Intermediate **WX068-5** (200 mg, 731.91 µmol) was dissolved in tetrahydrofuran (10 mL) at room temperature, and then acrylamide (52.02 mg, 731.91 µmol) and a solution of potassium *tert*-butoxide (1 M, 585.53 µL) in tetrahydrofuran were added sequentially at 0 °C. The reaction mixture was stirred at 15 °C for 2 h. After the reaction was completed, 1 M diluted hydrochloric acid was added to adjust pH to 4-5, water (50 mL) was added, and ethyl acetate (50 mL × 3) was added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give target compound **WX068.** MS-ESI *m*/*z:* 299.1 [M+H]⁺. ¹H NMR (400 MHz, MeOD_*d*₄) δ: 8.28 (dd, *J*=5.8, 9.0 Hz, 1H), 8.08 (d, *J*=9.2 Hz, 1H), 7.84 (d, *J*=9.2 Hz, 1H), 7.80 (dd, *J*=2.6, 9.8 Hz, 1H), 7.53 (td, *J*=2.6, 8.6 Hz, 1H), 4.96 (dd, *J*=5.4, 10.6 Hz, 1H), 2.89-2.78 (m, 2H), 2.73-2.64 (m, 1H), 2.55-2.49 (m, 1H).

### Example 69: WX069

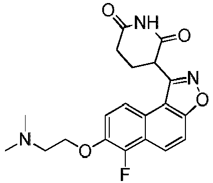

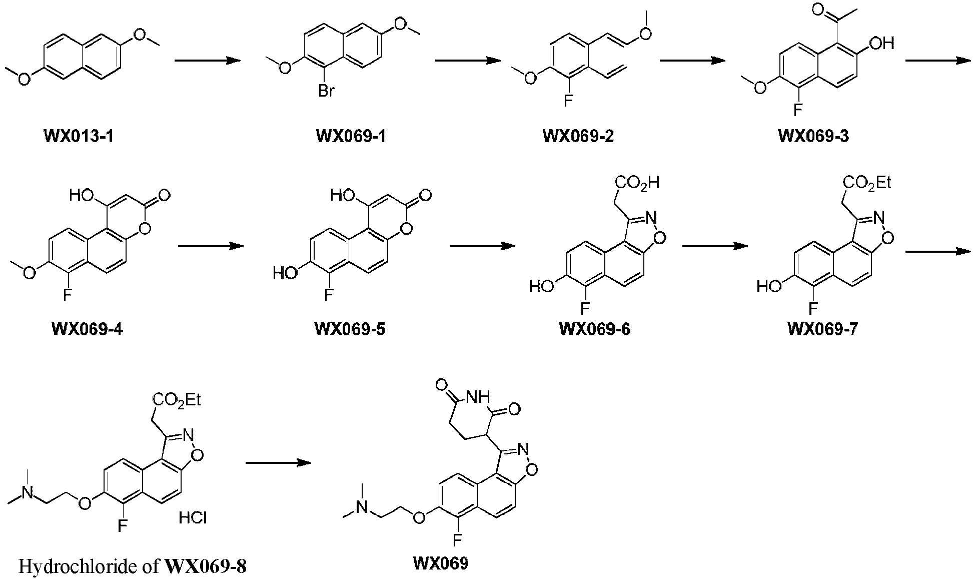

### Step 1: synthesis of intermediate WX069-1

Intermediate **WX013-1** (10 g, 53.13 mmol) was dissolved in dichloromethane (100 mL) at room temperature under nitrogen atmosphere. After the reaction mixture was cooled to 0 °C, *N*-bromosuccinimide (14.18 g, 79.69 mmol) was added in batches. The reaction mixture was stirred at 20 °C for 2 h. After the reaction was completed, the reaction mixture was filtered, and the filter cake was washed with dichloromethane (100 mL × 2). The filtrate was collected and concentrated under reduced pressure to remove the solvent. The resulting residue was separated by column chromatography (eluent: petroleum ether/2-methyltetrahydrofuran = 1/0-49/1, volume ratio) to give intermediate **WX069-1.**

### Step 2: synthesis of intermediate WX069-2

Intermediate **WX069-1** (8 g, 29.95 mmol) was dissolved in tetrahydrofuran (240 mL) at room temperature under nitrogen atmosphere. After the reaction mixture was cooled to -78 °C, n-butyllithium (2.5 M, 13.18 mL) was added dropwise. After being stirred at -78 °C for 0.5 h, the reaction mixture was further added with a solution of *N*-fluorobisbenzenesulfonamide (14.17 g, 44.92 mmol) in tetrahydrofuran (20 mL). After being stirred at -78 °C for 1 h, the resulting reaction mixture was warmed to room temperature an stirred for 12 h. After the reaction was completed, the reaction was quenched with ice water (200 mL), and 2-methyltetrahydrofuran (200 mL × 3) was added for extraction. The organic phases were combined, washed with saturated brine (100 mL × 3), dried over anhydrous sodium sulfate and filtered, and the filtrate was concentrated under reduced pressure to remove the solvent. The resulting residue was separated by column chromatography (eluent: petroleum ether/ethyl acetate = 1/0-49/1, volume ratio) to give intermediate **WX069-2.**

### Step 3: synthesis of intermediate WX069-3

Intermediate **WX069-2** (4 g, 19.40 mmol) and acetyl chloride (1.67 g, 21.34 mmol, 1.52 mL) were dissolved in dichloromethane (80 mL) at room temperature under nitrogen atmosphere. After the reaction mixture was cooled to 0 °C, aluminum trichloride (5.17 g, 38.79 mmol) was added in batches. The resulting reaction mixture was warmed to room temperature and stirred for 2 h. After the reaction was completed, the reaction was quenched with ice water (20 mL), and dichloromethane (40 mL × 3) was added for extraction. The organic phases were combined, washed with saturated brine (40 mL × 3), dried over anhydrous sodium sulfate, filtered, and concentrated under reduced pressure to remove the solvent. The resulting residue was separated by column chromatography (eluent: petroleum ether/ethyl acetate = 1/0-49/1, volume ratio) to give intermediate **WX069-3.** ¹H NMR (400 MHz, CDCl₃) δ: 13.36 (s, 1H), 8.17 (d, *J*=9.6 Hz, 1H),7.81 (d, *J*=9.2 Hz, 1H), 7.36 (t, *J*=9.2 Hz, 1H), 7.19 (d, *J*=9.2 Hz, 1H), 4.03 (s, 3H), 2.85 (s, 3H).

### Step 4: synthesis of intermediate WX069-4

Intermediate **WX069-3** (0.94 g, 4.01 mmol) was dissolved in toluene (10 mL) and diethyl carbonate (10 mL) at room temperature under nitrogen atmosphere. After the reaction mixture was cooled to 0 °C, sodium hydride (642.12 mg, 16.05 mmol, purity: 60%) was added in batches. The reaction mixture was heated to 120 °C and stirred for 12 h. After the reaction was completed, the reaction mixture was cooled to room temperature, the reaction was quenched with ice water (50 mL), and 2-methyltetrahydrofuran (50 mL × 3) was added for extraction. The organic phase was discarded, and the aqueous phase was adjusted to pH 2-3 with 2 N diluted hydrochloric acid and then extracted with 2-methyltetrahydrofuran (60 mL × 3). The organic phases were combined, washed with saturated brine (60 mL × 3), dried over anhydrous sodium sulfate, filtered, and concentrated under reduced pressure to remove the solvent, thus giving intermediate **WX069-4.**

### Step 5: synthesis of intermediate WX069-5

Intermediate **WX069-4** (1 g, 3.84 mmol) was dissolved in dichloromethane (10 mL) at room temperature under nitrogen atmosphere. After the reaction mixture was cooled to -78 °C, boron tribromide (1.93 g, 7.69 mmol, 740.57 µL) was added dropwise. The reaction mixture was warmed to room temperature and stirred for 12 h. The reaction mixture was cooled to -78 °C again and boron tribromide (1.93 g, 7.69 mmol, 740.57 µL) was added dropwise. The reaction mixture was heated to 50 °C and stirred for 3 h. After the reaction was completed, the reaction mixture was cooled to room temperature and poured into ice water (50 mL), followed by liquid separation. The aqueous phase was extracted with 2-methyltetrahydrofuran (50 mL × 3). The organic phases were combined, dried over anhydrous sodium sulfate, filtered, and concentrated under reduced pressure to remove the solvent, thus giving intermediate **WX069-5.**

### Step 6: synthesis of intermediate WX069-6

Intermediate **WX069-5** (0.7 g, 2.84 mmol) and hydroxylamine hydrochloride (691.54 mg, 9.95 mmol) were dissolved in ethanol (10 mL) at room temperature under nitrogen atmosphere, and then sodium ethoxide (677.21 mg, 9.95 mmol) was added. The reaction mixture was heated to 90 °C and stirred for 12 h. After the reaction was completed, the reaction mixture was cooled to room temperature, concentrated under reduced pressure to remove the solvent, diluted with water (40 mL) and adjusted to pH 2-3 with 2 N diluted hydrochloric acid, and ethyl acetate (40 mL × 3) was added for extraction. The organic phases were combined, washed with saturated brine (50 mL), dried over anhydrous sodium sulfate, filtered, and concentrated under reduced pressure to remove the solvent, thus giving intermediate **WX069-6.**

### Step 7: synthesis of intermediate WX069-7

Intermediate **WX069-6** (0.8 g, 3.06 mmol) was dissolved in ethanol (8 mL) at room temperature under nitrogen atmosphere, and then concentrated sulfuric acid (0.5 mL, purity: 98%) was added dropwise. The reaction mixture was heated to 90 °C and stirred for 12 h. After the reaction was completed, the reaction mixture was cooled to room temperature and concentrated under reduced pressure to remove the solvent. Water (50 mL) was added for dilution and ethyl acetate (40 mL × 3) was added for extraction. The organic phases were combined, washed with saturated brine (40 mL), dried over anhydrous sodium sulfate and filtered, and the filtrate was concentrated under reduced pressure to remove the solvent. The resulting residue was separated by column chromatography (eluent: petroleum ether/ethyl acetate = 1/0-7/1, volume ratio) to give intermediate **WX069-7.**

### Step 8: synthesis of intermediate WX069-8

Intermediate **WX069-7** (0.5 g, 1.73 mmol), 2-(dimethylamino)-ethanol (169.48 mg, 1.90 mmol, 190.86 µL) and triphenylphosphine (589.40 mg, 2.25 mmol) were dissolved in tetrahydrofuran (15 mL) at room temperature under nitrogen atmosphere. After the reaction mixture was cooled to 0 °C, diisopropyl azodicarboxylate (454.39 mg, 2.25 mmol, 436.92 µL) was added dropwise. The resulting reaction mixture was stirred at 20 °C for 12 h. After the reaction was completed, the reaction mixture was poured into water (40 mL), and 2-methyltetrahydrofuran (40 mL × 3) was added for extraction. The organic phases were combined, washed with saturated brine (50 mL × 3), dried over anhydrous sodium sulfate, filtered, and concentrated under reduced pressure to remove the solvent. The resulting residue was separated by prep-HPLC (mobile phase: acetonitrile/water; acidic system: 0.05% HCl) to give the hydrochloride of intermediate **WX069-8.** ¹H NMR (400 MHz, DMSO_*d₆*) δ: 11.11 (s, 1H), 8.24 (d, *J*=9.2 Hz, 1H), 8.01 (d, *J*=9.2 Hz, 1H), 7.94 (d, *J*=9.2 Hz, 1H), 7.84 (t, *J*=8.6 Hz, 1H), 4.66 (t, *J*=4.8 Hz, 2H), 4.51 (s, 2H), 4.15 (q, *J*=7.0 Hz, 2H),3.58 (q, *J*=4.4 Hz, 2H), 2.89 (s, 3H), 2.88 (s, 3H), 1.17 (t, *J*=7.0 Hz, 3H).

### Step 9: synthesis of WX069

Intermediate **WX069-8** (0.22 g, 554.38 µmol, hydrochloride) and acrylamide (39.40 mg, 554.38 µmol) were dissolved in tetrahydrofuran (5 mL) at room temperature under nitrogen atmosphere, and then a solution of potassium *tert*-butoxide in tetrahydrofuran (1 M, 554.38 µL) was added. The reaction mixture was stirred at room temperature for 12 h. After the reaction was completed, the reaction mixture was adjusted to pH 6-7 with ethyl acetate hydrochloride (4 M), and then concentrated under reduced pressure to remove the solvent. The resulting residue was separated by prep-HPLC (mobile phase: acetonitrile/water; neutral system: 10 mM NH₄HCO₃), and then separated by prep-HPLC (mobile phase: acetonitrile/water; acidic system: 0.05% HCl) to give target compound WX069. MS-ESI *m*/*z:* 386.2 [M+H]⁺. ¹H NMR (400 MHz, MeOD_*d*₄) δ: 8.30 (d, *J*=9.6 Hz, 1H), 8.04 (d, *J*=9.2 Hz, 1H), 7.90 (d, *J*=9.2 Hz, 1H), 7.69 (t, *J*=8.8 Hz, 1H), 4.95 (dd, *J*=4.8, 10.4 Hz, 1H), 4.60 (t, *J*=5.0 Hz, 2H), 3.69 (t, *J*=4.8 Hz, 2H), 3.07 (s, 6H), 2.90-2.78 (m, 2H), 2.75-2.66 (m, 1H), 2.55-2.49 (m, 1H).

### Example 70: WX070

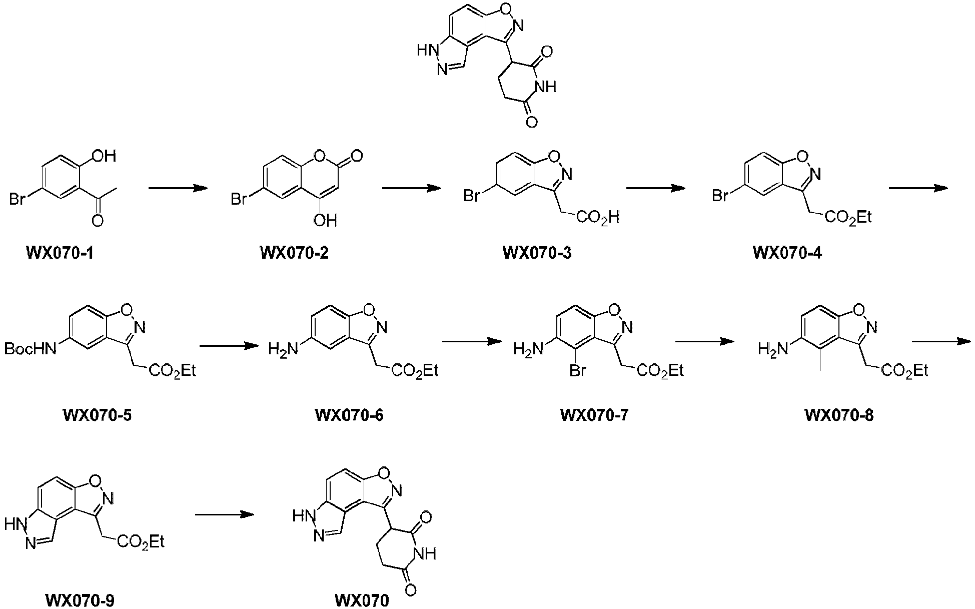

### Step 1: synthesis of intermediate WX070-2

Intermediate **WX070-1** (45 g, 209.26 mmol) was dissolved in diethyl carbonate (250 mL) and toluene (250 mL) at room temperature under nitrogen atmosphere. After the reaction mixture was cooled to 0 °C, sodium hydride (33.48 g, 837.04 mmol, purity: 60%) was added in batches. The reaction mixture was heated to 120 °C and stirred for 12 h. After the reaction was completed, the parallel reactions for the four batches were performed together. The reaction mixture was cooled to room temperature, ice water (6000 mL) was added to quench the reaction, and ethyl acetate (4000 mL × 3) was added for extraction. The organic phase was discarded, the aqueous phase was adjusted to pH 2-3 with 2 N diluted hydrochloric acid, and white solid was precipitated. The mixture was filtered and the filter cake was collected and concentrated under reduced pressure to remove the solvent, thus giving intermediate **WX070-2.** MS-ESI *m*/*z:* 240.9 [M+H]⁺, 242.9 [M+H+2]⁺

### Step 2: synthesis of intermediate WX070-3

Intermediate **WX070-2** (50 g, 207.44 mmol) was dissolved in ethanol (500 mL) at room temperature under nitrogen atmosphere, and then hydroxylamine hydrochloride (50.45 g, 726.03 mmol) and sodium acetate (59.56 g, 726.03 mmol) were added sequentially. The reaction mixture was heated to 90 °C and stirred for 12 h. After the reaction was completed, the parallel reactions for the four batches were performed together. The reaction mixture was cooled to room temperature, then adjusted to pH 6-7 with 2 N diluted hydrochloric acid, and concentrated under reduced pressure to remove the solvent. The resulting residue was diluted with water (800 mL), and extracted with ethyl acetate (1500 mL × 3). The organic phases were combined, dried over anhydrous sodium sulfate, filtered, and concentrated under reduced pressure to remove the solvent, thus giving intermediate **WX070-3.** MS-ESI *m*/*z:* 255.9 [M+H]⁺, 257.9 [M+H+2]⁺.

### Step 3: synthesis of intermediate WX070-4

Intermediate **WX070-3** (50 g, 195.27 mmol) was dissolved in ethanol (300 mL) at room temperature under nitrogen atmosphere, and then concentrated sulfuric acid (3 mL, purity: 98%) was added dropwise. The reaction mixture was heated to 90 °C and stirred for 12 h. After the reaction was completed, the parallel reactions for the four batches were performed together. The reaction mixture was cooled to room temperature and precipitates were filtered. The filter cake was collected and the mother solution was concentrated under reduced pressure to remove the solvent. The resulting residue from the concentrating of the mother solution under reduced pressure was separated by column chromatography (eluent: petroleum ether/ethyl acetate = 1/0-49/1, volume ratio) to give intermediate **WX070-4.** MS-ESI *m*/*z:* 284.0 [M+H]⁺, 286.0 [M+H+2]⁺.

### Step 4: synthesis of intermediate WX070-5

Intermediate **WX070-4** (20 g, 70.40 mmol) and *tert*-butyl carbamate (24.74 g, 211.19 mmol) were dissolved in toluene (425 mL) and water (85 mL) at room temperature under nitrogen atmosphere, and then tris(dibenzylideneacetone)dipalladium (4.51 g, 4.93 mmol), potassium phosphate (59.77 g, 281.59 mmol) and 2-di-*tert*-butylphosphine-2',4',6'-triisopropylbiphenyl (4.19 g, 9.86 mmol) were added. The reaction mixture was heated to 105 °C and stirred for 12 h. After the reaction was completed, the parallel reactions for the four batches were performed together. The reaction mixture was cooled to room temperature, poured into water (800 mL) and then filtered, and ethyl acetate (600 mL) was added for extraction. The mother solution was collected, and liquid separation was performed, and the aqueous phase was extracted with ethyl acetate (2000 mL × 2). The organic phases were combined, dried over anhydrous sodium sulfate, filtered, and concentrated under reduced pressure to remove the solvent. The resulting residue was separated by column chromatography (eluent: petroleum ether/ethyl acetate = 1/0-20/1) to give intermediate **WX070-5.** MS-ESI *m*/*z:* 319.1 [M-H]⁻.

### Step 5: synthesis of intermediate WX070-6

Intermediate **WX070-5** (45 g, 140.48 mmol) was dissolved in ethyl acetate hydrochloride (4 M, 750 mL) at room temperature, and the reaction mixture was stirred at room temperature for 12 h. After the reaction was completed, the parallel reactions for the two batches were performed together. The reaction mixture was concentrated under reduced pressure, poured into water (1000 mL), and adjusted to pH 7-8 with saturated aqueous sodium bicarbonate solution, and ethyl acetate (800 mL × 3) was added for extraction. The organic phases were combined, dried over anhydrous sodium sulfate, filtered, and concentrated under reduced pressure to remove the solvent. The resulting residue was separated by column chromatography (eluent: petroleum ether/ethyl acetate = 1/0-6/1) to give intermediate **WX070-6.** MS-ESI *m*/*z:* 221.1 [M+H]⁺.

### Step 6: synthesis of intermediate WX070-7

Intermediate **WX070-6** (45 g, 204.34 mmol) was dissolved in dichloromethane (600 mL) at room temperature, and then *N*-bromosuccinimide (40.01 g, 224.77 mmol) was added in batches. The reaction mixture was stirred at room temperature for 12 h. After the reaction was completed, the reaction mixture was filtered, and the mother solution was concentrated under reduced pressure to remove the solvent. The resulting residue was separated by column chromatography (eluent: petroleum ether/ethyl acetate = 1/0-4/1) to give intermediate **WX070-7.** ¹H NMR (400 MHz, CDCl₃) δ: 7.35 (d, *J*=8.8 Hz, 1H), 7.02 (d, *J*=8.8 Hz, 1H), 4.23 (q, *J*=7.0 Hz, 2H), 4.16 (s, 2H), 3.94 (s, 2H), 1.27 (t, *J*=7.2 Hz, 3H).

### Step 7: synthesis of intermediate WX070-8

Intermediate **WX070-7** (1.3 g, 4.35 mmol) was dissolved in 1,4-dioxane (13 mL) at room temperature under nitrogen atmosphere, and then methylboronic acid (780.47 mg, 13.04 mmol), cesium fluoride (2.24 g, 14.78 mmol) and bis(diphenylphosphino)ferrocene palladium(II) dichloride dichloromethane (354.92 mg, 434.61 µmol) were added. The reaction mixture was heated to 90 °C and stirred for 12 h. After the reaction was completed, the reaction mixture was cooled to room temperature and then filtered, and the filtrate was concentrated under reduced pressure. The resulting residue was separated by column chromatography (eluent: petroleum ether/ethyl acetate = 5/1-3/1, volume ratio) to give intermediate **WX070-8.** ¹H NMR (400 M Hz, DMSO_*d*₆) δ: 7.26 (d, *J*=7.6 Hz, 1H), 6.97 (d, *J*=8.8 Hz, 1H), 4.23 (q, *J*=7.2 Hz, 2H), 4.11 (s, 2H) 3.76 (s, 2H), 2.34 (s, 3H), 1.27 (t, *J*=*7.4* Hz, 3H).

### Step 8: synthesis of intermediate WX070-9

Intermediate **WX070-8** (0.9 g, 3.84 mmol) and potassium acetate (1.21 g, 12.29 mmol) were added to chloroform (20 mL) at room temperature. After the reaction mixture was cooled to 0 °C, acetic anhydride (1.18 g, 11.53 mmol, 1.08 mL) was added dropwise. After being stirred at room temperature for 0.5 h, the reaction mixture was heated to 60 °C and then added with isoamyl nitrite (900.16 mg, 7.68 mmol,1.03 mL). The resulting reaction mixture was stirred at 60 °C for 5.5 h. The reaction mixture was cooled to room temperature, and water (30 mL) and dichloromethane (50 mL) were added. The organic phase was dried over anhydrous sodium sulfate and concentrated under reduced pressure to remove the solvent. The residue was added with ethanol (20 mL) and hydrochloric acid (4 N, 10 mL) at 30 °C, and the mixture was stirred for 2 h. After the reaction was completed, the reaction mixture was concentrated under reduced pressure to 15 mL, and then extracted with saturated brine (30 mL) and ethyl acetate (30 mL). The organic phase was dried over anhydrous sodium sulfate, filtered and concentrated under reduced pressure to remove the solvent. The resulting residue was separated by column chromatography (eluent: petroleum ether/ethyl acetate = 1/0-4.5/1, volume ratio) to give intermediate **WX070-9.** ¹H NMR (400 MHz, CDCl₃) δ: 10.87 (s, 1H), 8.26 (s, 1H), 7.72 (d, *J*=9.2 Hz, 1H), 7.65 (d, *J*=9.2 Hz, 1H), 4.24 (q, *J*=6.8 Hz, 2H), 4.22 (s, 2H), 1.25 (t, *J*=7.2 Hz, 3H).

### Step 9: synthesis of WX070

Intermediate **WX070-9** (0.11 g, 448.55 µmol) was added to tetrahydrofuran (4 mL) at room temperature, and then acrylamide (31.88 mg, 448.55 µmol) and a solution of potassium *tert*-butoxide (1 M, 448.55 µL) in tetrahydrofuran were added. The reaction mixture was stirred at room temperature for 1 h. After the reaction was completed, 1 N hydrochloric acid was added to the reaction mixture to adjust the pH to 6, and water (20 mL) and ethyl acetate (20 mL) were added for extraction. The organic phase was washed with saturated brine (20 mL), dried over anhydrous sodium sulfate, filtered and concentrated under reduced pressure to remove the solvent. The residue was separated by prep-HPLC (mobile phase: acetonitrile/water; system: 0.05% HCl, hydrochloric acid system) to give target compound **WX070.** MS-ESI m/z: 271.0 [M+H]^{+ 1}H NMR (400 MHz, DMSO_*d*₆) δ: 11.18 (s, 1H), 8.20 (s, 1H), 7.88 (d, *J*=8.4 Hz, 1H), 7.77 (d, *J*=9.2 Hz, 1H), 4.76 (dd, *J*=4.6, 11.8 Hz, 1H), 2.91-2.78 (m, 1H), 2.72-2.65 (m, 1H), 2.45-2.37 (m, 1H), 2.30-2.15 (m, 1 H).

### Example 71: WX071

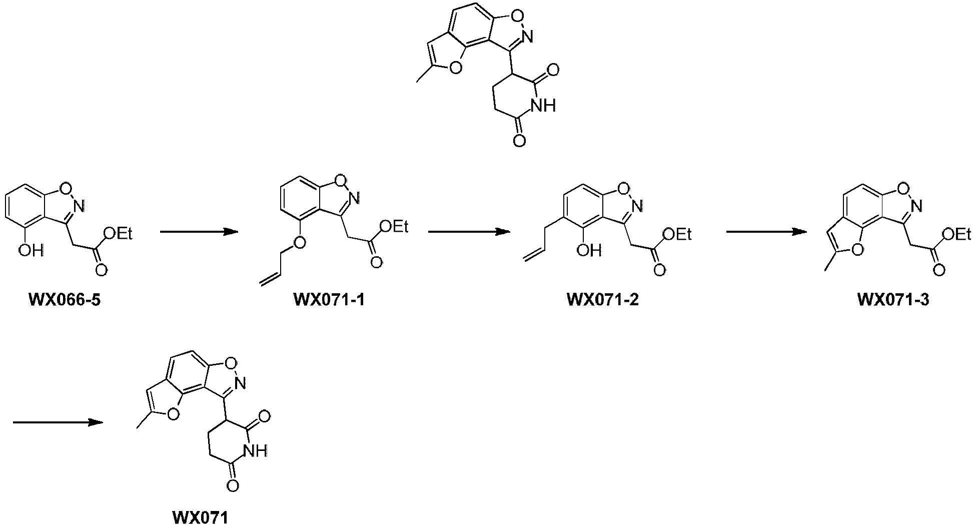

### Step 1: synthesis of intermediate WX071-1

Intermediate **WX066-5** (1 g, 4.52 mmol) was dissolved in *N,N*-dimethylfonnamide (15 mL) at 15 °C, and then potassium carbonate (1.87 g, 13.56 mmol) and allyl bromide (656.17 mg, 5.42 mmol) were added sequentially. The reaction mixture was stirred at 15 °C for 5 h. After the reaction was completed, water (100 mL) was added to the reaction mixture for dilution, and ethyl acetate (50 mL × 3) was added for extraction. The organic phases were combined, washed with half-saturated brine (50 mL × 2), dried over anhydrous sodium sulfate and filtered, and the filtrate was concentrated under reduced pressure to remove the solvent. The resulting residue was separated by column chromatography (eluent: petroleum ether/ethyl acetate = 1/0-4/1, volume ratio) to give intermediate **WX071-1.** ¹H NMR (400 MHz, CDCl₃) δ: 7.43 (t, *J*=8.0 Hz, 1H), 7.14 (d, *J*=8.4 Hz, 1H), 6.61 (d, *J*=8.0 Hz, 1H), 6.12-5.98 (m, 1H), 5.47-5.38 (m, 1H), 5.36-5.30 (m, 1H), 4.69-4.61 (m, 2H), 4.19 (q, *J*=7.2 Hz, 2H), 4.10 (s, 2H), 1.24 (t, *J*=7.2 Hz, 3H).

### Step 2: synthesis of intermediate WX071-2

Intermediate **WX071-1** (1 g, 3.83 mmol) was dissolved in *N,N-*dimethylformamide (20 mL) at room temperature under nitrogen atmosphere, and the reaction mixture was heated to 240 °C and stirred for 7 h. After the reaction was completed, the reaction mixture was cooled to room temperature, diluted with water (100 mL), and extracted with ethyl acetate (30 mL × 3). The organic phases were combined, washed with half-saturated brine (20 mL × 2), dried over anhydrous sodium sulfate and filtered, and the filtrate was concentrated under reduced pressure to give the residue. The resulting residue was separated by column chromatography (eluent: petroleum ether/ethyl acetate = 1/0-7/3, volume ratio), and then separated by prep-HPLC again (mobile phase: acetonitrile/water; acidic system: 0.05% HCl) to give intermediate **WX071-2.**

### Step 3: synthesis of intermediate WX071-3

Intermediate **WX071-2** (260.00 mg, 995.13 µmol) was dissolved in dioxane (5 mL) at room temperature under nitrogen atmosphere, and then *p*-benzoquinone (107.57 mg, 995.13 µmol) and bis(acetonitrile)palladium(II) chloride (12.91 mg, 49.76 µmol) were added sequentially. The reaction mixture was heated to 80 °C and stirred for 16 h. After the reaction was completed, the reaction mixture was cooled to room temperature and diluted with water (20 mL) and ethyl acetate (15 mL). After liquid separation, the organic phase was collected, and the aqueous phase was extracted with ethyl acetate (20 mL × 3). The organic phases were combined, washed with saturated brine (50 mL × 2), dried over anhydrous sodium sulfate and filtered, and the filtrate was concentrated under reduced pressure to remove the solvent. The resulting residue was separated by column chromatography (eluent: petroleum ether/ethyl acetate = 1/0-19/1, volume ratio) to give intermediate **WX071-3.**

### Step 4: synthesis of WX071

Intermediate **WX071-3** (129.00 mg, 497.56 µmol) was dissolved in tetrahydrofuran (3 mL) at 15 °C under nitrogen atmosphere, and then acrylamide (35.37 mg, 497.56 µmol) and potassium *tert*-butoxide (55.83 mg, 497.56 µmol) were added sequentially. The reaction mixture was stirred at 15 °C for 4 h. After the reaction was completed, water (20 mL) was added to the reaction mixture. 2 M diluted hydrochloric acid was added to adjust the pH to 6-7, and 2-methyltetrahydrofuran (15 mL) was added for dilution. After liquid separation, the organic phase was collected, and the aqueous phase was extracted with 2-methyltetrahydrofuran (20 mL × 3). The organic phases were combined, washed with saturated brine (10 mL × 2), dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give target compound **WX071.** MS-ESI *m*/*z:* 285.1 [M+H]⁺. ¹H NMR (400 MHz, DMSO_*d*₆) δ: 11.23 (s, 1H), 7.85 (d, *J*=8.8 Hz, 1H), 7.62 (d, *J*=8.8 Hz, 1H), 6.78 (d, *J*=0.8 Hz, 1H), 4.67 (dd, *J*=5.0, 12.6 Hz, 1H), 2.97-2.84 (m, 1H), 2.72-2.61 (m, 1H), 2.59-2.53 (m, 1H), 2.48 (s, 3H), 2.34-2.19 (m, 1H).

### Example 72 and Example 73: WX072 and WX073

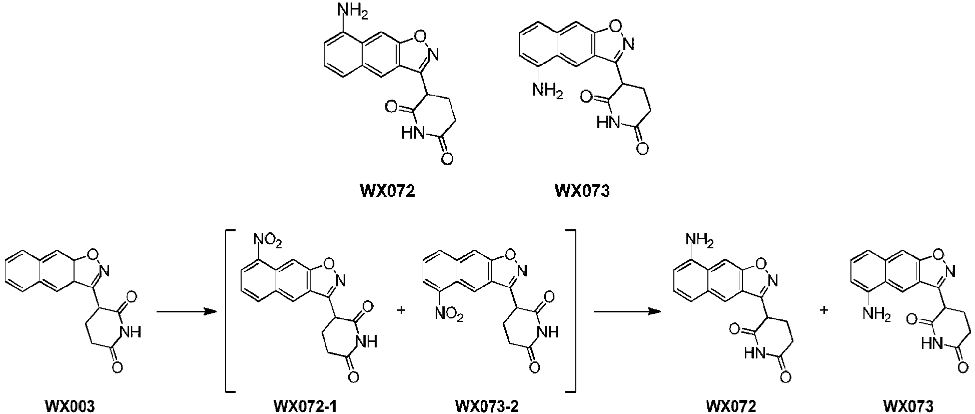

### Step 1: synthesis of intermediates WX072-1 and WX073-2

Compound **WX003** (570 mg, 2.03 mmol) was dissolved in concentrated sulfuric acid (5 mL, purity: 98%) at 0 °C under nitrogen atmosphere, and then potassium nitrate (205.61 mg, 2.03 mmol) was added. The reaction mixture was stirred at 0 °C for 1 h. After the reaction was completed, the reaction mixture was poured into ice water (200 mL), and ethyl acetate (50 mL × 3) was added for extraction. The organic phases were combined, dried over anhydrous sodium sulfate and filtered, and the filtrate was concentrated under reduced pressure to remove the solvent, thus giving 600 mg of crude product, 500 mg of which was separated by prep-HPLC (mobile phase: acetonitrile/water; acidic system: 0.05% HCl) to give a mixture of intermediates **WX072-1** and **WX073-2.**

### Step 2: synthesis of WX072 and WX073

A mixture of intermediates **WX072-1** and **WX073-2** (300 mg, 922.30 µmol) was dissolved in ethanol (5 mL) at room temperature under nitrogen atmosphere, and then stannous dichloride dihydrate (1.46 g, 6.46 mmol) was added. The reaction mixture was heated to 50 °C and stirred for 24 h. After the reaction was completed, the reaction mixture was cooled to room temperature and concentrated under reduced pressure to remove the solvent. Water (50 mL) was added for dilution, and the pH was adjusted to 6-7 with saturated aqueous sodium bicarbonate solution. A solid was precipitated and the mixture was filtered. The filter cake was washed with 2-methyltetrahydrofuran (50 mL × 2). The organic phase was collected after separating the filtrate, and the aqueous phase was extracted with 2-methyltetrahydrofuran (50 mL × 3). The organic phases were combined, dried over anhydrous sodium sulfate and filtered, and the filtrate was concentrated under reduced pressure to remove the solvent. The resulting residue was separated by prep-HPLC (mobile phase: acetonitrile/water; neutral system: 10 mM NH₄HCO₃) to give target compounds **WX072** and **WX073.** The target compound **WX072:** MS-ESI *m*/*z*: 296.1 [M+H]⁺.¹H NMR (400 MHz, DMSO_*d*₆) δ: 8.39 (s, 1H), 8.36 (s, 1H), 7.31 (d, *J*=8.4 Hz, 1H), 7.23 (t, *J*=*7.8* Hz, 1H), 6.76 (d, J=7.2 Hz, 1H), 5.87 (s, 2H), 4.70 (dd, *J*=4.6, 11.8 Hz, 1H), 2.85-2.75 (m, 1H), 2.65-2.55 (m, 2H), 2.28-2.19 (m, 1H). The target compound WX073: MS-ESI *m*/*z*: 296.1 [M+H]⁺. ¹H NMR (400 MHz, DMSO_*d*₆) δ: 8.64 (s, 1H), 7.98 (s, 1H), 7.31 (t, *J*=8.0 Hz, 1H), 7.21 (d, *J*=8.4 Hz, 1H), 6.61 (d, *J*=7.6 Hz, 1H), 6.11 (s, 2H), 4.62 (dd, *J*=5.0, 12.2 Hz, 1H), 2.98-2.75 (m, 2H), 2.65-2.57 (m, 1H), 2.24-2.12 (m, 1H).

### Example 74: Hydrochloride of WX074

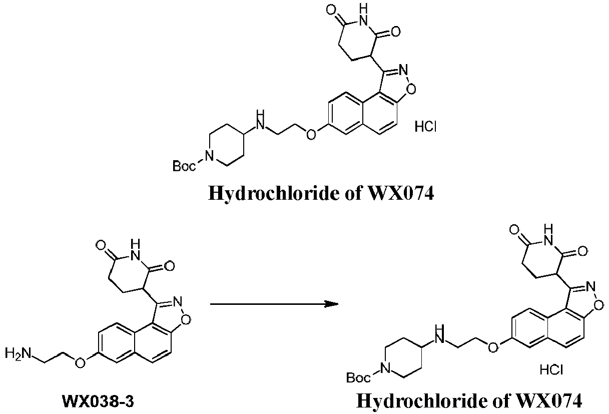

### Step 1: synthesis of WX074

Intermediate **WX038-3** (300 mg, 798.29 µmol, hydrochloride) was dissolved in 1,2-dichloroethane (20 mL) at 10 °C, and then sodium acetate (130.97 mg, 1.60 mmol) and *N*-*tert*-butoxycarbonyl-4-piperidone (159.06 mg, 798.29 µmol, 46.42 µL) were added sequentially. After the reaction mixture was stirred at 10 °C for 30 min, sodium triacetoxyborohydride (338.38 mg, 1.60 mmol) was added. The resulting reaction mixture was heated to 30 °C and stirred for 12 h. After the reaction was completed, water (30 mL) was added to the reaction mixture for dilution, and ethyl acetate (20 mL × 3) was added for extraction. The organic phases were combined, dried over anhydrous sodium sulfate and filtered, and the filtrate was concentrated under reduced pressure to remove the solvent. The resulting residue was separated by prep-HPLC twice (mobile phase: acetonitrile/water; acidic system: 0.05% HCl) to give the hydrochloride of target compound **WX074.** MS-ESI *m*/*z*: 467.2 [M-55]^{+ 1}H NMR (400 MHz, DMSO_*d*₆) δ: 11.13 (s, 1H), 9.17 (s, 2H), 8.20 (d, *J*=8.8 Hz, 1H), 8.15 (d, *J*=9.2 Hz, 1H), 7.93 (d, *J*=9.2 Hz, 1H), 7.73 (d, *J*=2.4 Hz, 1H), 7.43 (dd, *J*=2.6, 9.0 Hz, 1H), 5.04 (dd, *J*=4.6, 11.4 Hz, 1H), 4.44 (s, 2H), 4.02 (d, *J*=11.6 Hz, 2H), 3.46 (s, 2H), 2.92-2.72 (m, 3H), 2.70-2.53 (m, 3H), 2.43-2.34 (m, 1H), 2.08 (d, .7=10.0 Hz, 2H), 1.56-1.45 (m, 2H), 1.41 (s, 9H)

### Example 75: WX075

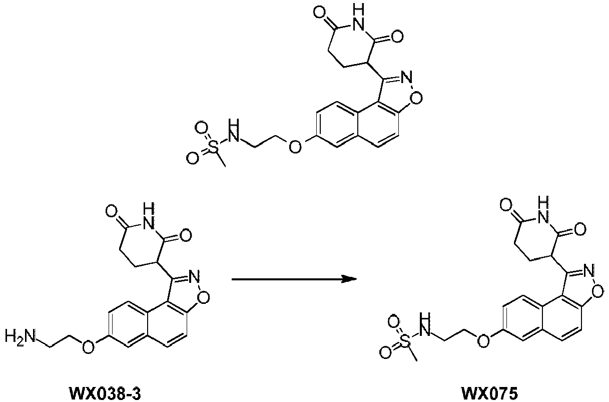

### Step 1: synthesis of WX075

Intermediate **WX038-3** (150 mg, 399.14 µmol, hydrochloride) was dissolved in 1,2-dichloroethane (3 mL) at 15 °C, and then triethylamine (201.95 mg, 2.00 mmol, 277.78 µL) and methanesulfonyl chloride (45.72 mg, 399.14 µmol. 30.89 µL) were added sequentially. After being stirred at 15 °C for 12 h, the reaction mixture was supplemented with triethylamine (100 µL) and methanesulfonyl chloride (50 µL). After being heated to 30 °C and stirred for 12 h, the reaction mixture was cooled to 15 °C, and further supplemented with triethylamine (100 µL) and methanesulfonyl chloride (50 µL). After being heated to 30 °C and stirred for 12 h again, the reaction mixture was cooled to 15 °C and supplemented with triethylamine (100 µL) and methanesulfonyl chloride (50 µL) again. The reaction mixture was then heated to 30 °C and stirred for 12 h. After the reaction was completed, the reaction mixture was cooled to room temperature, and 4 M ethyl acetate hydrochloride was added dropwise slowly to adjust pH to 6-7, and the reaction mixture was then concentrated under reduced pressure to remove the solvent. The resulting residue was separated by prep-HPLC (mobile phase: acetonitrile/water; acidic system: 0.05% HCl) to give target compound **WX075.** MS-ESI *m*/*z:* 418.0 [M+H]⁺.¹H NMR (400 MHz, DMSO_*d*₆) δ: 11.12 (s, 1H), 8.17 (d, *J*=8.8 Hz, 1H), 8.11 (d, *J*=9.2 Hz, 1H), 7.90 (d, *J*=9.2 Hz, 1H), 7.67 (d, *J*=2.8 Hz, 1H), 7.38 (dd, *J*=2.8, 9.2 Hz, 1H), 7.34 (d, *J*=6.0 Hz, 1H), 5.03 (dd, *J*=4.8, 11.6 Hz, 1H), 4.20 (t, *J*=5.8 Hz, 2H), 3.42 (q, *J*=5.8 Hz, 2H), 2.98 (s, 3H), 2.90-2.77 (m, 1H), 2.68-2.54 (m, 2H), 2.43-2.31 (m, 1H).

### Example 76: WX076

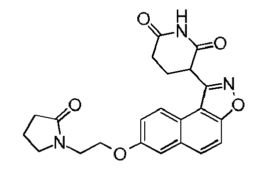

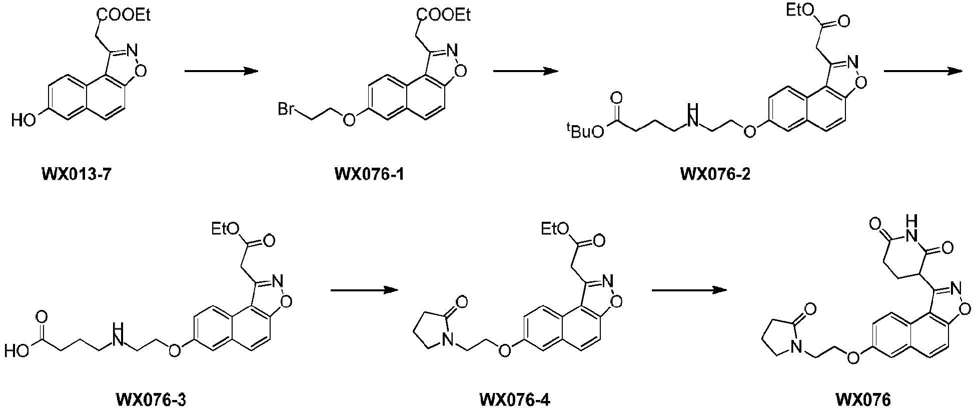

### Step 1: synthesis of intermediate WX076-1

Intermediate **WX013-7** (2 g, 7.37 mmol), bromoethanol (1.01 g, 8.11 mmol, 575.84 µL) and triphenylphosphine (2.51 g, 9.58 mmol) were dissolved in tetrahydrofuran (60 mL) at room temperature under nitrogen atmosphere. After the reaction mixture was cooled to 0 °C, diisopropyl azodicarboxylate (1.94 g, 9.58 mmol, 1.86 mL) was added dropwise. The resulting reaction mixture was warmed to room temperature and stirred for 12 h. After the reaction was completed, the reaction mixture was poured into water (60 mL), and ethyl acetate (60 mL × 3) was added for extraction. The organic phases were combined, washed with saturated brine (80 mL), dried over anhydrous sodium sulfate, filtered, and concentrated under reduced pressure to remove the solvent. The resulting residue was separated by column chromatography (eluent: petroleum ether/ethyl acetate = 1/0-11/1, volume ratio) to give intermediate **WX076-1.**

### Step 2: synthesis of intermediate WX076-2

*Tert*-butyl-4-aminobutanoate hydrochloride (277.85 mg, 1.75 mmol) and potassium iodide (241.40 mg, 1.45 mmol) were dissolved in *N,N*-dimethylfonnamide (6 mL) at room temperature, and then triethylamine (147.15 mg, 1.45 mmol, 202.41 µL) was added dropwise and intermediate **WX076-1** (0.55 g, 1.45 mmol) was added. The reaction mixture was stirred at room temperature for 12 h. After the reaction was completed, the reaction mixture was poured into water (40 mL), and ethyl acetate (50 mL × 3) was added for extraction. The organic phases were combined, washed with saturated brine (80 mL), dried over anhydrous sodium sulfate and filtered, and the filtrate was concentrated under reduced pressure to remove the solvent. The resulting residue was separated by prep-HPLC (mobile phase: acetonitrile/water; acidic system: TFA) to give intermediate **WX076-2.**

### Step 3: synthesis of intermediate WX076-3

Intermediate **WX076-2** (0.4 g, 876.17 µmol) was dissolved in dichloromethane (8 mL) at room temperature, and then trifluoroacetic acid (1.4 mL) was added. The reaction mixture was stirred at room temperature for 12 h. After the reaction was completed, the reaction mixture was concentrated under reduced pressure to remove the solvent. The resulting residue was separated by prep-HPLC (mobile phase: acetonitrile/water; acidic system: 0.05% HCl) to give intermediate **WX076-3.**

### Step 4: synthesis of intermediate WX076-4

Intermediate **WX076-3** (0.09 g, 206.00 µmol, hydrochloride) was dissolved in *N,N*-dimethylfonnamide (2 mL) at room temperature, and then O-(7-azabenzotriazol-1-yl)-*N,N,N,N-*tetramethyluronium hexafluorophosphate (117.49 mg, 309.01 µmol) and triethylamine (62.54 mg, 618.01 µmol, 86.02 µL) were added. The reaction mixture was stirred at room temperature for 12 h. After the reaction was completed, the reaction mixture was concentrated under reduced pressure to remove the solvent. The resulting residue was separated by prep-HPLC (mobile phase: acetonitrile/water; acidic system: 0.05% HCl) to give intermediate **WX076-4.**

### Step 5: synthesis of WX076

Intermediate **WX076-4** (0.06 g, 156.90 µmol) was dissolved in tetrahydrofuran (2 mL) at room temperature, and then acrylamide (11.15 mg, 156.90 µmol) and a solution of potassium *tert*-butoxide in tetrahydrofuran (1 M, 156.90 µL) were added sequentially. The reaction mixture was stirred at room temperature for 12 h. After the reaction was completed, the reaction mixture was adjusted to pH 5-6 with 4 M ethyl acetate hydrochloride, and then concentrated under reduced pressure to remove the solvent. The resulting residue was separated by prep-HPLC (mobile phase: acetonitrile/water; acidic system: 0.05% HCl) to give target compound **WX076.** MS-ESI *m*/*z:* 408.2 [M+H]⁺.¹H NMR (400 MHz, DMSO_*d*₆) δ: 11.12 (s, 1H), 8.15 (d, *J*=8.8 Hz, 1H), 8.09 (d, *J*=8.8 Hz, 1H), 7.90 (d, *J*=9.2 Hz, 1H), 7.68 (d, *J*=2.4 Hz, 1H), 7.36 (dd, *J*=2.6, 9.0 Hz, 1H), 5.02 (dd, *J*=4.6, 11.4 Hz, 1H), 4.25 (t, *J*=5.4 Hz, 2H), 3.63 (t, *J*=5.4 Hz, 2H), 3.50 (t, *J*=7.0 Hz, 2H), 2.87-2.80 (m, 1H), 2.67-2.65 (m, 1H), 2.58-2.54 (m, 1H), 2.42-2.35 (m, 1H), 2.23 (t, *J*=8.0 Hz, 2H), 1.99-1.87 (m, 2H)

### Example 77: WX077

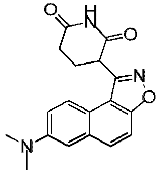

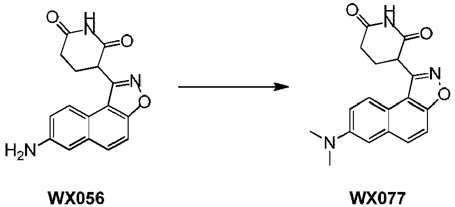

### Step 1: synthesis of WX077

Intermediate **WX056** (100 mg, 338.65 µmol) was dissolved in 1,2-dichloroethane (5 mL) at 15 °C, and then acetic acid (20.34 mg, 338.65 µmol, 19.37 µL) and an aqueous formaldehyde solution (60.46 mg, 745.02 µmol, 55.47 µL, purity: 37%) were added sequentially. After the reaction mixture was stirred at 15 °C for 30 min, sodium triacetoxyborohydride (143.55 mg, 677.30 µmol) was added. After being stirred at 15 °C for 12 h, the reaction mixture was supplemented with aqueous formaldehyde solution (30 µL, purity: 37%). The resulting reaction mixture was then stirred at 15 °C for 8 h. After the reaction was completed, the reaction mixture was directly concentrated under reduced pressure to remove the solvent. The resulting residue was separated by prep-HPLC (mobile phase: acetonitrile/water; acidic system: 0.05% HCl) to give target compound **WX077.** MS-ESI *m*/*z:* 324.0 [M+H]⁺.¹H NMR (400 MHz, DMSO_*d*₆) δ: 11.13 (s, 1H), 8.14 (d, *J*=9.2 Hz, 1H), 8.08 (d, *J*=9.2 Hz, 1H), 7.87 (d, *J*=9.2 Hz, 1H), 7.71 (s, 1H), 7.61 (s, 1H), 5.01 (dd, *J*=4.8, 11.6 Hz, 1H), 3.10 (s, 6H), 2.92-2.78 (m, 1H), 2.70-2.54 (m, 2H), 2.41-2.30 (m, 1H).

### Example 78: WX078

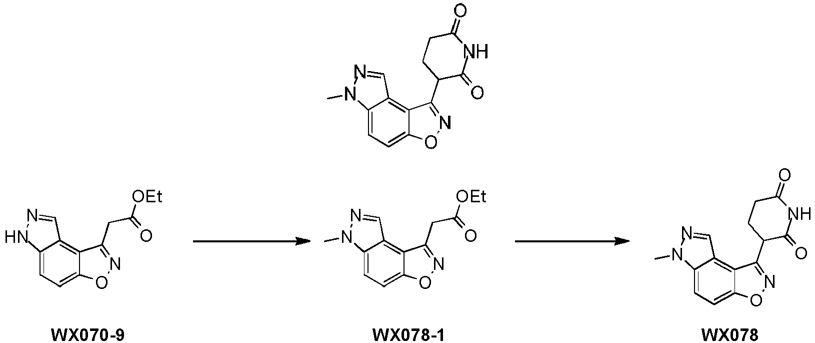

### Step 1: synthesis of intermediate WX078-1

Intermediate **WX070-9** (300 mg, 1.22 mmol) was dissolved in acetone (5 mL) at room temperature. After the reaction mixture was cooled to 0 °C, potassium carbonate (338.14 mg, 2.45 mmol) and iodomethane (156.27 mg, 1.10 mmol, 68.54 µL) were added sequentially. After being warmed to room temperature and stirred for 12 h, the reaction mixture was supplemented with iodomethane (100 µL). The reaction mixture was then stirred at room temperature for 12 h. After the reaction was completed, the reaction mixture was directly filtered. The filter cake was washed with ethyl acetate (10 mL × 2), and the mother solution was collected and concentrated under reduced pressure to remove the solvent. The resulting residue was separated by silica gel plate (developing solvent: petroleum ether/ethyl acetate = 1/1) to give intermediate **WX078-1.** ¹H NMR (400 MHz, CDCl₃) δ: 8.11 (s, 1H), 7.64 (d, *J*=9.2 Hz, 1H), 7.60 (d, *J*=9.2 Hz, 1H), 4.22 (q, *J*=7.2 Hz, 2H), 4.19 (s, 5H), 1.24 (t, *J*=7.0 Hz, 3H).

### Step 2: synthesis of WX078

Intermediate **WX078-1** (50 mg, 192.86 µmol) was dissolved in tetrahydrofuran (5 mL) at room temperature, and then acrylamide (13.71 mg, 192.86 µmol) and a solution of potassium *tert*-butoxide in tetrahydrofuran (1 M, 192.86 µL) were added sequentially. The reaction mixture was stirred at room temperature for 1 h. After the reaction was completed, water (20 mL) was added to the reaction mixture, and ethyl acetate (20 mL × 3) was added for extraction. The organic phases were combined, dried over anhydrous sodium sulfate and filtered, and the filtrate was concentrated under reduced pressure to remove the solvent. The resulting residue was separated by prep-HPLC (mobile phase: acetonitrile/water; acidic system: 0.05% HCl) to give target compound **WX078.** MS-ESI *m*/*z:* 285.1 [M+H]⁺.¹H NMR (400 MHz, DMSO_*d*₆) δ: 11.17 (s, 1H), 8.15 (s, 1H), 8.02 (d, *J*=9.2 Hz, 1H), 7.84 (d, *J*=9.6 Hz, 1H), 4.77 (dd, *J*=5.0, 12.2 Hz, 1H), 4.17 (s, 3H), 2.92-2.78 (m, 1H), 2.70-2.66 (m, 1H), 2.57-2.52 (m, 1H), 2.31-2.21 (m, 1H).

### Example 79: WX079

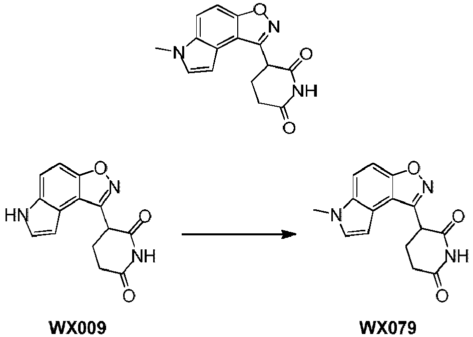

Intermediate **WX009** (50 mg, 182.41 µmol, purity: 98.23%) was dissolved in *N,N*-dimethylformamide (5 mL) at 0 °C under nitrogen atmosphere, and then potassium *tert*-butoxide (40.94 mg, 364.82 µmol) and dimethyl sulfate (98.23 mg, 778.79 µmol, 73.86 µL) were added. The reaction mixture was stirred at 0 °C for 2 h. After the reaction was completed, water (20 mL) was added, and ethyl acetate (30 mL × 3) was added for extraction. The organic phases were combined, washed with saturated brine (50 mL × 2), dried over anhydrous sodium sulfate, filtered, and concentrated under reduced pressure to remove the solvent. The resulting residue was separated by SFC (column: Chiralpak AD-3 50×4.6 mm I.D., 3 µm; mobile phase: A: CO₂, B: methanol (0.05% diethanolamine); gradient: from 5% to 40% of B in 2 min and hold 40% for 1.2 min, then 5% of B for 0.8 min; flow rate: 4 mL/min; column temperature: 35 °C; pressure: 1500 psi) to give target compound **WX079.** MS-ESI m/z: 284.1 [M+H]⁺.¹H NMR (400 MHz, DMSO_*d*₆) δ: 11.71 (s, 1H), 7.73 (d, *J*=8.8 Hz, 1H),7.54 (d, *J*=4.8 Hz, 1H), 7.44 (d, *J*=8.8 Hz, 1H), 6.50 (s, 1H), 4.78 (dd, *J*=5.0, 11.8 Hz, 1H), 3.11 (s, 3H), 3.02-2.88 (m, 1H), 2.85-2.65 (m, 1H), 2.49-2.41 (m, 1H), 2.33-2.12 (m, 1H).

### Experimental Example 1: In Vitro Assay of IKZF3 Protein Level in Multiple Myeloma Cells

### Experimental objective:

By using WB method, compounds were investigated for their modulation of IKZF3 protein level in multiple myeloma cells MM.1S at different concentrations.

### Experimental procedure:

1) MM.1S cells were thawed and passaged twice;
2) MM.1S cells were inoculated into a 6-well plate at 1 × 10⁶ cells per well and then treated with a test compound at certain concentration;
3) After 16 h of treatment, the cultured cell sample was dissolved in RIPA buffer (Sigma-Aldrich) or NETN buffer (150 mM NaCl, 1% NP-40, 50 mM Tris-HCl, pH 8.0) containing a complete histone inhibitor (Roche) placed on ice, and left to stand for 20 min;
4) After centrifugation (rotation speed: 17950 rpm) for 15 min, the supernatant was collected and quantitative determination of protein (Pierce BCA protein assay kit, Thermo) was performed;
5) An equal amount of 20 µg of protein from each sample was separated by SDS-PAGE and transferred to PVDF or nylon membrane (Invitrogen);
6) 5% skim milk powder was added, followed by overnight incubation in primary antibody (anti-IKZF3 (NBP2-24495, Novus Biologicals) and anti-Actin (1844-1, Epitomics)) 5% BSA at 4 °C;
7) After a final reaction with HRP-linked secondary antibody (Goat-anti-rabbit IgG (sc-2004, Santa Cruz)) for 1 h, the bands on the membrane were detected with a chemiluminescent substrate (Thermo Scientific).

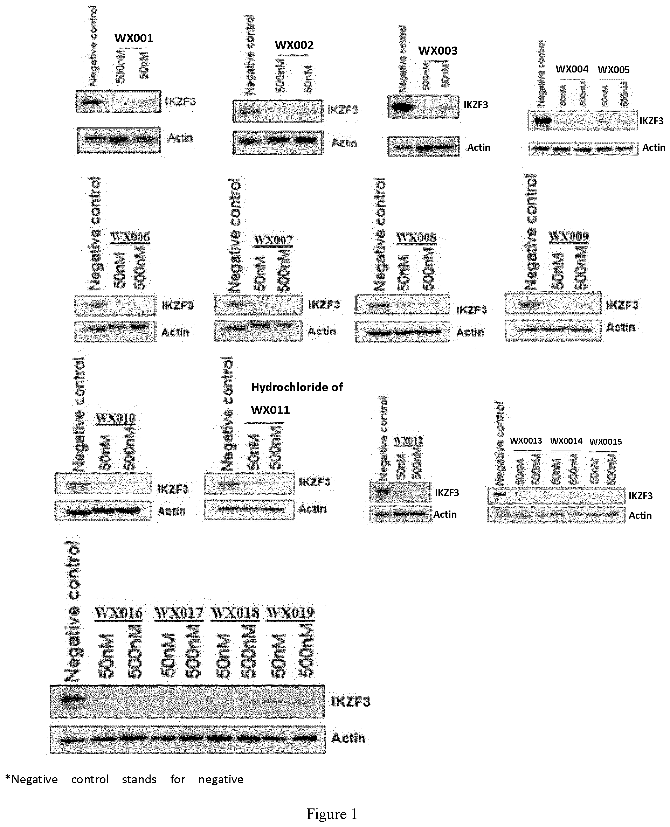

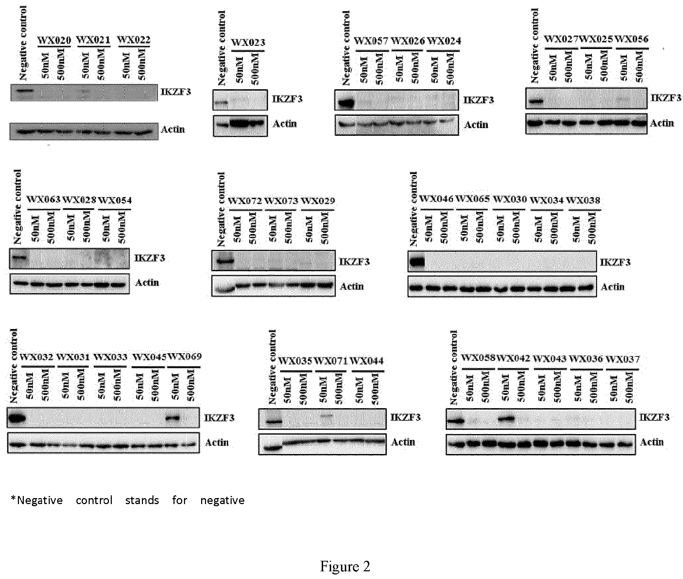

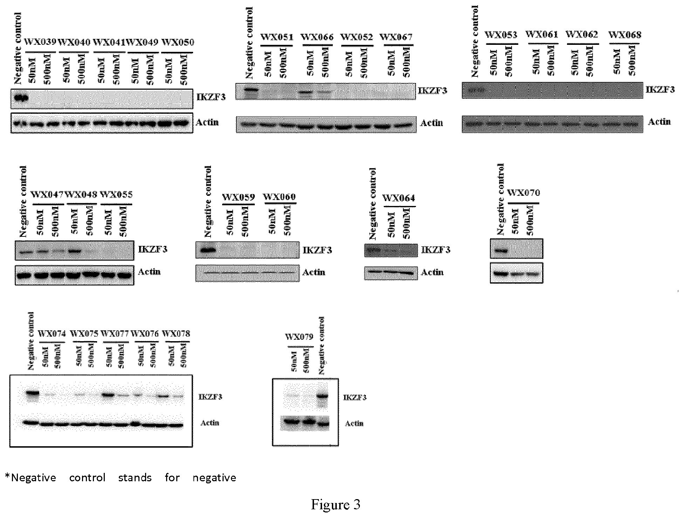

The experimental results are shown in FIGs. 1, 2 and 3.

### Experimental Example 2: Evaluation of Antiproliferative Effect in Multiple Myeloma Cell Lines MM.1S and NCI-H929

**Experimental objective:** in this experiment, the inhibition of cell proliferation in multiple myeloma cell lines MM. 1S and NCI-H929 by the test compounds was detected.

### Experimental materials:

1. Cell line and culture method

| Cell line | Tumor type | Characteristics of growth | Culture method |
|---|---|---|---|
| MM.1S | Multiple myeloma | Semi -adhesion | RPMI-1640+10%FBS |
| NCI-H929 | Myeloma | Suspension | RPMI-1640+0.05mM 2-mercaptoethanol+10%FBS |

2. Culture medium and reagent

| Culture medium and reagent | Manufacturer | Catalog number |
|---|---|---|
| RPMI 1640 | GIBCO | 22400-089 |
| DµLbecco's PBS | Hyclone | SH30256.01 |
| FBS | Hyclone | SY30087.03 |
| Anti biotic-antimycotic | GIBCO | 15240-062 |
| 0.25% Trypsin | GIBCO | 25200072 |
| DMSO | SIGMA | D2650 |
| 2-mercaptoethanol | SIGMA | 60-24-2 |

3. Multi-well plate
Greiner CELLSTAR® 96-well plate, flat-bottomed blackboard (with cover and transparent bottom), # 655090.
4. Reagent and instrument for cell viability experiment
(1) Promega CellTiter-Glo luminescence method cell viability assay kit (Promega-G7573).
(2) 2104 En Vision® plate reader, PerkinElmer.

### Experimental procedure:

### 1. Cell culturing

The tumor cell lines were cultured in an incubator at 37 °C/5% CO₂ according to the above-mentioned culturing conditions. Periodical passaging was performed, and cells in logarithmic growth phase were taken for plating.

### 2. Cell plating

(1) Cells were stained with trypan blue and viable cells were counted.
(2) The cell concentration was adjusted to an appropriate level.

| **Cell line** | **Density (per 96-well)** |
|---|---|
| MM.1S | 4000 |
| NCI-H929 | 6000 |

(3) 90 µL of cell suspension was added to each well in the culture plate and cell-free medium was added to the blank control wells according to the table above.
(4) The culture plate was incubated overnight in an incubator at 37 °C/5% CO₂ and 100% relative humidity.

### 3. Preparation of compound storage plate

Preparation of storage plate of mother solution at a concentration 400-fold higher than the initial concentration of compound: compound was diluted from highest concentration gradient to lowest concentration with DMSO. It was prepared freshly prior to use.

### 4. Preparation of working solution at a concentration 10-fold higher than the initial concentration of compound and treatment of cells with the compound

(1) Preparation of working solution at a concentration 10-fold higher than the initial concentration of compound: 76 µL of cell culture solution was added to a V-bottomed 96-well plate, and 4 µL of compound was pipetted from a storage plate of mother solution at a concentration 200-fold higher than the initial concentration of the compound and added to the cell culture in the 96-well plate. 4 µL of DMSO was added to vehicle control and blank control wells. After the compound or DMSO was added, the mixture was pipetted and mixed well, and 78 µL of the cell culture was added to the V-bottomed 96-well plate, and 2 µL of compound was pipetted from a storage plate of mother solution at a concentration 400-fold higher than the initial concentration of the compound and added to the cell culture in the 96-well plate. 2 µL of DMSO was added to vehicle control and blank control wells. After the compound or DMSO was added, the mixture was pipetted and mixed well.
(2) Dosing: 10 µL of working solution at a concentration 10-fold higher than the initial concentration of the compound was added to the cell culture plate. 10 µL of DMSO-cell culture mixture was added to the vehicle control and blank control wells.
(3) The 96-well cell plate was put back into the incubator for culturing MM.1S (3-fold dilution, 5 days of compound co-incubation) and NCI-H929 (3-fold dilution, 5 days of compound co-incubation).

### 5. Cell viability assay with CellTiter-Glo luminescence method

The following procedure was performed according to the instructions of Promega CellTiter-Glo luminescence cell viability assay kit (Promega-G7573).
(1) The CellTiter-Glo buffer was thawed and left to stand until reaching the room temperature.
(2) The CellTiter-Glo substrate was left to stand until reaching the room temperature.
(3) 10 mL of CellTiter-Glo buffer was added to a bottle of CellTiter-Glo substrate to dissolve the substrate, thus preparing the CellTiter-Glo working solution.
(4) Slow vortex shaking was performed to completely dissolve the substrate.
(5) The cell culture plate was taken out and left to stand for 30 min so as to be equilibrated to room temperature.
(6) 50 µL (equal to half the volume of cell culture in each well) of CellTiter-Glo working solution was added to each well. The cell plate was wrapped with aluminum foil to keep out of light.
(7) The plate was shaken on an orbital shaker for 2 min to induce cell lysis.
(8) The culture plate was placed at room temperature for 10 min to stabilize the luminescence signals.
(9) The luminescence signals were detected on 2104 EnVision plate reader.

### 6. Data analysis

The inhibition rates (IRs) of the detected compounds were calculated according to the following formula: IR (%) = (RLU vehicle control - RLU compound) / (RLU vehicle control - RLU blank control) × 100%. Inhibition rates of compounds at different concentrations were calculated in Excel, followed by plotting the inhibition curves and calculating relevant parameters including minimum inhibition rate (%), maximum inhibition rate (%) and IC₅₀ using GraphPad Prism software.

**Experimental results:** the test results are shown in Table 1.

**Table 1. Inhibition of cell proliferation in the MM.1S and NCI-H929 cell lines by the compounds disclosed herein**

| Compound | MM.1S IC₅₀ (nM) | NCI-H929 IC₅₀ (nM) |
|---|---|---|
| **WX004** | 6.6 | 21.2 |
| **WX005** | / | 19.1 |
| **WX006** | 2 | / |
| **WX009** | 0.4 | 0.5 |
| **WX013** | 4.5 | / |
| **WX015** | 3.8 | / |
| **WX023** | 6.4 | 10.3 |
| **WX027** | 3.4 | / |
| **Hydrochloride of WX039** | 2.6 | / |
| **Hydrochloride of WX050** | 2.9 | / |
| **WX061** | 3.4 | / |
| **WX065** | 4 | / |
| **WX070** | 8.9 | 13 |

### Conclusion:

The compounds disclosed herein exhibit excellent inhibition of cell proliferation in multiple myeloma cell lines MM.1S and NCI-H929.

### Experimental Example 3: Pharmacokinetic Evaluation of Compounds in Mice

### Experimental objective:

The test animals in the study were C57BL male mice, and the LC/MS/MS method was used to quantitatively determine the drug concentration in the plasma of the mice at different time points after intravenous injection or oral administration of the test compounds and the reference compound, thus evaluating the pharmacokinetic characteristics of the test drugs in the mice.

### Experimental materials:

C57Balb/C (C57) mice (male, 20-30 g, 7-10 weeks old, Beijing Vital River or Shanghai SLAC).

### Experimental procedure:

Clear or suspended solution of the test compound was injected into C57 mice (overnight fasting) via the tail vein or administered intragastrically to C57 mice (overnight fasting). For intravenous injection, 200 µL of blood was each collected by jugular vein puncture at 0 h (before injection) and 0.0833 h, 0.25 h, 0.5 h, 1 h, 2 h, 4 h, 6 h, 8 h and 24 h (after injection), and then placed in an anticoagulation tube added with EDTA-K2 (Jiangsu KANGJIAN Medical Apparatus Co., Ltd.), and the mixture was thoroughly vortex-mixed at 4 °C and centrifuged at 13000 rpm for 10 min; for intragastric administration, blood was collected by jugular vein puncture at 0 h (before administration) and 0.5 h, 1 h, 2 h, 4 h, 6 h, 8 h and 24 h (after administration), and then placed in an anticoagulation tube added with EDTA-K2 (Jiangsu KANGJIAN Medical Apparatus Co., Ltd.), and the mixture was thoroughly vortex-mixed and centrifuged at 13000 rpm for 10 min. The plasma concentration was measured by LC-MS/MS method, and the relevant pharmacokinetic parameters were calculated by WinNonlin™ Version 6.3 (Pharsight, Mountain View, CA) pharmacokinetic software using non-compartmental model linear logarithmic trapezoid method.

**Experimental results:** the test results are shown in Table **2.**

**Table 2. Pharmacokinetic parameters of the compounds disclosed herein in mice**

| Pharmacokinetic parameters in mice | Intravenous injection (2 mg/kg) | | | Oral administration (10 mg/kg) | | | |
|---|---|---|---|---|---|---|---|
| | Plasma clearance (mL/min/k g) | Half life (h) | Area under plasma concentration -time curve (0-inf, µM.h) | Peak concentration (µM) | Time to peak (h) | Area under plasma concentration -time curve (0-inf, µM.h) | Bioavailability F (%) |
| **WX009** | 23.2 | 0.89 | 5.34 | 10.52 | 0.25 | 12.29 | 46.1 |
| **WX023** | 11.8 | 3.37 | 6.87 | 17.50 | 0.25 | 34.40 | 100.3 |

### Experimental Example 4: Pharmacokinetic Evaluation of Compounds in Rats

### Experimental objective:

The test animals in the study were SD male rats, and the LC/MS/MS method was used to quantitatively determine the drug concentration in the plasma of the rats at different time points after intravenous injection or oral administration of the test compounds and the reference compound, thus evaluating the pharmacokinetic characteristics of the test drugs in the rats.

### Experimental materials:

Sprague Dawley (SD) rats (male, 200-300g, 7-10 weeks old, Beijing Vital River or Shanghai SLAC).

### Experimental procedure:

Clear solution of the test compound was injected into SD rats (overnight fasting) via the tail vein or administered intragastrically to SD rats (overnight fasting). For intravenous injection, 200 µL of blood was each collected by jugular vein puncture at 0 h (before injection) and 0.0833 h, 0.25 h, 0.5 h, 1 h, 2 h, 4 h, 6 h, 8 h and 24 h (after injection), and then placed in an anticoagulation tube added with EDTA-K2 (Jiangsu KANGJIAN Medical Apparatus Co., Ltd.), and the mixture was thoroughly vortex-mixed at 4 °C and centrifuged at 13000 rpm for 10 min; for intragastric administration, blood was collected by jugular vein puncture at 0 h (before administration) and 0.5 h, 1 h, 2 h, 4 h, 6 h, 8 h and 24 h (after administration), and then placed in an anticoagulation tube added with EDTA-K2 (Jiangsu KANGJIAN Medical Apparatus Co., Ltd.), and the mixture was thoroughly vortex-mixed and centrifuged at 13000 rpm for 10 min. The plasma concentration was measured by LC-MS/MS method, and the relevant pharmacokinetic parameters were calculated by WinNonlin™ Version 6.3 (Pharsight, Mountain View, CA) pharmacokinetic software using non-compartmental model linear logarithmic trapezoid method.

**Experimental results:** the test results are shown in Table **3**.

**Table 3. Pharmacokinetic parameters of the compounds disclosed herein in rats**

| Pharmacokinetic parameters in rats | Intravenous injection (2 mg/kg) | | | Intragastric administration (10 mg/kg) | | | |
|---|---|---|---|---|---|---|---|
| | Plasma clearance (mL/min/kg) | Half life (h) | Area under plasma concentration-time curve (0-inf, µM.h) | Peak concentration (µM) | Time to peak (h) | Area under plasma concentration-time curve (0-inf, µM.h) | Bioavailability F (%) |
| **WX009** | 15.9 | 0.90 | 7.92 | 12.75 | 0.50 | 26.34 | 66.5 |
| **WX023** | 21.7 | 1.24 | 3.76 | 3.08 | 0.75 | 19.69 | 73.7 |

### Experimental Example 5: In Vivo Pharmacodynamic Study of Compounds in Subcutaneous Xenograft Tumor CB-17SCID Model of Human Myeloma MM.1S Cells

Cell culturing: human multiple myeloma cells MM. 1S (ATCC® CRL-2974™) were cultured *in vitro,* in a semi-suspension manner, with an ATCC-formulated RPMI-1640 medium containing 10% fetal calf serum, 100 U/mL penicillin and 100 µg/mL streptomycin in an incubator at 37 °C/5% CO₂. Passages were performed twice a week. At a required number, the cells were taken, counted and inoculated.
Animals: CB-17 SCID mice, female, 6-8 weeks old, weight of 18-20 g.

### Experimental procedure:

0.2 mL (5 × 10⁶ cells) of MM.1S cells (along with matrigel in a volume ratio of 1:1) was subcutaneously inoculated on the right back of each mouse, and the mice were divided into groups for administration after the mean tumor volume was approximately 130 mm³. Seven days constitute an administration cycle, twice daily with a 12 h interval, and the test compound was orally administered for a total of four cycles. Test compounds WX009 and WX023 were administered at a dose of 5 mg/kg, tumor volume was measured twice weekly with a two-dimensional caliper, and the volume was measured in cubic millimeters and calculated according to the following formula: V = 0.5 a × b², where a and b are the long and short diameters, respectively, of the tumor. Anti-tumor efficacy was determined by dividing the mean tumor increase volume of compound-treated animals by the mean tumor increase volume of untreated animals.

### Experimental results:

The test results are shown in Table **4**.

**Table 4: Test results of compounds in subcutaneous xenograft tumor CB-17 SCID model of human myeloma MM.1S cells**

| Group | Administration dose | Tumor volume (mm³) (Day 0) | Tumor volume (mm³) (Day 24) | TGI (%) (Day 24) |
|---|---|---|---|---|
| Vehicle control | 0 mg/kg | 130±9 | 2,637±332 | / |
| **WX009** | 5 mg/kg | 131±12 | 14±5 | 105 |
| **WX023** | 5 mg/kg | 130±8 | 15±4 | 105 |

TGI: tumor growth inhibition. TGI(%) = [1 - (mean tumor volume of a treatment group at the end of administration - mean tumor volume of the treatment group during administration) / (mean tumor volume of a solvent control group at the end of treatment - mean tumor volume of the solvent control group at the beginning of treatment)] × 100%.

## Claims

1. A compound of formula (I) or a pharmaceutically acceptable salt thereof, 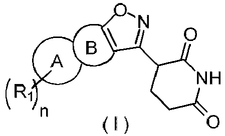 wherein,
n is selected from the group consisting of 0, 1, 2 and 3;
each R₁ is independently selected from the group consisting of H, F, Cl, Br, I, OH, NH₂, CN, C₁₋₆ alkyl, C₃₋₁₀
cycloalkyl, C₁₋₆ alkoxy, C₁₋₆ alkylamino, C₂₋₆ alkenyl, 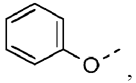 -S(=O)₂NH₂, -NHS(=O)₂-C₁₋₆ alkyl, -N[S(=O)_{z}-C₁₋₆ alkyl]₂, -N[C(=O)-C₁₋₆ alkyl]₂, -NHC(=O)-C₁₋₆ alkyl and -C(=O)NH₂, wherein the OH, NH₂, C₁₋₆ alkyl, C₃₋₁₀ cycloalkyl, C₁₋₆ alkoxy, C₁₋₆ alkylamino, C₂₋₆ alkenyl, 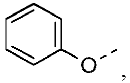 -S(=O)₂NH₂, -NHS(=O)₂-C₁₋₆ alkyl, -N[S(=O)₂-C₁₋₆ alkyl]₂, -N[C(=O)-C₁₋₆ alkyl]₂, -NHC(=O)-C₁₋₆ alkyl and -C(=O)NH₂ are optionally substituted with 1, 2 or 3 Rₐ;
ring A is selected from the group consisting of 5-6 membered heteroaryl, phenyl, C₄₋₆ cycloalkyl, 4-7 membered heterocycloalkyl and 4-7 membered heterocycloalkenyl;
ring B is selected from the group consisting of 5-6 membered heteroaryl and phenyl;
each Rₐ is independently selected from the group consisting of F, Cl, Br, I, OH, NH₂, C₁₋₁₀ alkyl, C₁₋₁₀ alkoxy, C₁₋₁₀ alkylamino, -C(=O)NH-C₁₋₁₀ alkyl, -NHC(=O)-C₁₋₁₀ alkyl, C₃₋₁₀ cycloalkyl, C₃₋₁₀ cycloalkylamino, 4-10 membered heterocycloalkyl, 4-10 membered heterocycloalkylamino and 4-10 membered heterocycloalkyl substituted with one carbonyl, wherein the OH, NH₂, C₁₋₁₀ alkyl, C₁₋₁₀ alkoxy, C₁₋₁₀ alkylamino, -C(=O)NH-C₁₋₁₀ alkyl, -NHC(=O)-C₁₋₁₀ alkyl, -COOC₁₋₁₀ alkyl, C₃₋₁₀ cycloalkyl, C₃₋₁₀ cycloalkylamino, 4-10 membered heterocycloalkyl and 4-10 membered heterocycloalkylamino are optionally substituted with 1, 2 or 3 R;
each R is independently selected from the group consisting of F, Cl, Br, I, OH, NH₂, C₁₋₃ alkyl, C₁₋₃ alkoxy, C₁₋₃ alkylamino, C₃₋₅ cycloalkyl, -C(=O)-C₁₋₃ alkyl, -C(=O)O-C₁₋₆ alkyl, -S(=O)₂-C₁₋₃ alkyl, 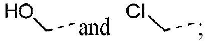
the 5-6 membered heteroaryl, 4-7 membered heterocycloalkyl, 4-10 membered heterocycloalkyl, 4-10 membered heterocycloalkylamino, 4-7 membered heterocycloalkenyl and 4-10 membered heterocycloalkyl substituted with one carbonyl each contain 1, 2, 3 or 4 heteroatoms or heteroatom groups independently selected from the group consisting of -NH-, -O-, -S- and N.

2. The compound according to claim 1, wherein ring A is selected from the group consisting of 5-6 membered heteroaryl, phenyl and 4-7 membered heterocycloalkenyl.

3. The compound according to claim 1 or 2, wherein each Rₐ is independently selected from F, Cl, Br, I, OH, NH₂, C₁₋₆ alkyl, C₁₋₆ alkoxy, C₁₋₆ alkylamino, -C(=O)NH-C₁₋₆ alkyl, -NHC(=O)-C₁₋₆ alkyl, C₃₋₈ cycloalkyl, C₃₋₈ cycloalkylamino, 4-6 membered heterocycloalkyl, 4-6 membered heterocycloalkylamino and 4-10 membered heterocycloalkyl substituted with one carbonyl, wherein the C₁₋₆ alkyl, C₁₋₆ alkoxy, C₁₋₆ alkylamino, -C(=O)NH-C₁₋₆ alkyl, -NHC(=O)-C₁₋₆ alkyl, -COOC₁₋₆ alkyl, C₃₋₈ cycloalkyl, C₃₋₈ cycloalkylamino, 4-6 membered heterocycloalkyl and 4-6 membered heterocycloalkylamino are optionally substituted with 1, 2 or 3 R; preferably, each Rₐ is independently selected from the group consisting of F, Cl, Br, I, OH, NH₂, C₁₋₃ alkyl, C₁₋₃ alkoxy, C₁₋₃ alkylamino, -C(=O)NH-C₁₋₃ alkyl, -NHC(=O)-C₁₋₃ alkyl, C₃₋₆ cycloalkyl, cyclohexylamino, azetidinyl, pyrrolidin-2-one, tetrahydropyrrolyl, piperidinyl, piperazinyl, morpholinyl, tetrahydropyranyl, 3-azabicyclo[3,1,0]hexyl, azetidinylamino, tetrahydropyrrolylamino, tetrahydropyrrolyl, piperidinylamino, piperazinylamino, morpholinylamino, tetrahydropyranylamino and 3-azabicyclo[3,1,0]hexylamino, wherein the C₁₋₃ alkyl, C₁₋₃ alkoxy, C₁₋₃ alkylamino, -C(=O)NH-C₁₋₃ alkyl, -NHC(=O)-C₁₋₃ alkyl, -COOC₁₋₄ alkyl, C₃₋₆ cycloalkyl, cyclohexylamino, azetidinyl, tetrahydropyrrolyl, piperidinyl, piperazinyl, morpholinyl, tetrahydropyranyl, 3-azabicyclo[3,1,0]hexyl, azetidinylamino, tetrahydropyrrolylamino, piperidinylamino, piperazinylamino, morpholinylamino, tetrahydropyranylamino, and 3-azabicyclo[3,1,0]hexylamino are optionally substituted with 1, 2 or 3 R; more preferably, each Rₐ is independently selected from the group consisting of F, Cl, Br, I, OH, NH₂, -CH₃, -CH₂CH₃, 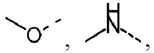 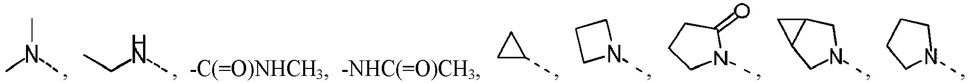 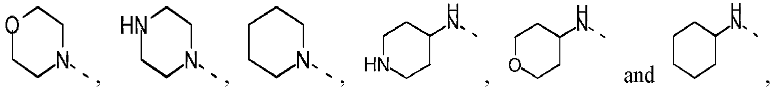 wherein the -CH₃, -CH₂CH₃, 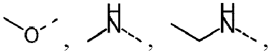 -C(=O)NHCH₃, -NHC(=O)CH₃, -COOt-Bu, 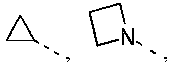 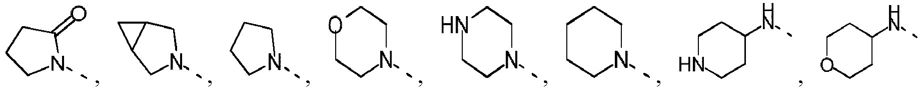 and 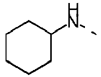 are optionally substituted with 1, 2 or 3 R.

4. The compound according to claim 1 or 2, wherein each Rₐ is selected from the group consisting of F, Cl, Br, I, OH, NH₂, -CH₂-, -CH₂CH₂-, 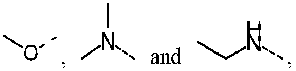 wherein the -CH₂-, -CH₂CH₂-, 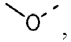 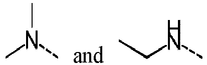 are optionally substituted with 1, 2 or 3 R.

5. The compound according to claim 1 or 2, wherein each Rₐ is independently selected from the group consisting of F, Cl, Br, I, Me, OH, NH₂, 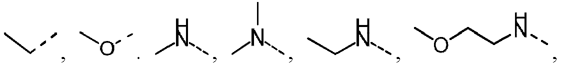 -C(=O)NHCH₃, -NHC(=O)CH₃, -CH₂COOt-Bu,

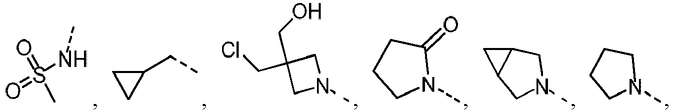

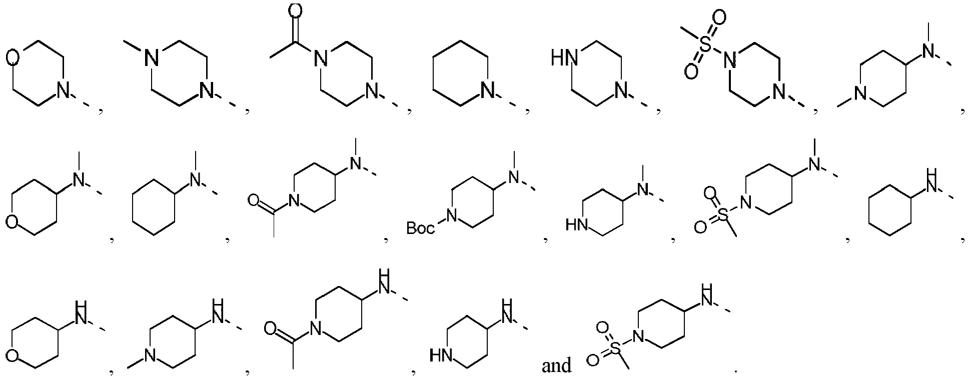

6. The compound according to claim 1 or 2, wherein each R is independently selected from the group consisting of C₁₋₃ alkyl, C₁₋₃ alkoxy, -C(=O)-C₁₋₃ alkyl, C₃₋₅ cycloalkyl, -C(=O)O-C₁₋₄ alkyl, -S(=O)₂-C₁₋₃ alkyl, 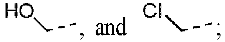 preferably, each R is independently selected from the group consisting of -CH₃,

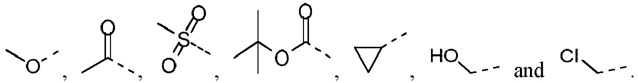

7. The compound according to claim 1 or 2, wherein each R is independently selected from the group consisting of F, Cl, Br, I, OH, NH₂, -CH₂-, -CH₂CH₂-,

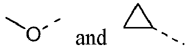

8. The compound according to claim 1 or 2, wherein each R₁ is independently selected from the group consisting of H, F, Cl, Br, I, OH, NH₂, CN, C₁₋₃ alkyl, C₃₋₅ cycloalkyl, C₁₋₃ alkoxy, C₁₋₃ alkylamino, C₂₋₄ alkenyl, 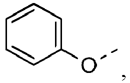 -S(=O)₂NH₂, -S(=O)₂NH-C₁₋₃ alkyl, -NHS(=O)₂-C₁₋₃ alkyl, -N[S(=O)₂-C₁₋₃ alkyl]₂, -N[C(=O)-C₁₋₃ alkyl]₂, -NHC(=O)-C₁₋₃ alkyl, -C(=O)NH₂ and -C(=O)NH-C₁₋₃ alkyl, wherein the C₁₋₃ alkyl, C₃₋₅ cycloalkyl, C₁₋₃ alkoxy, C₁₋₃ alkylamino, C₂₋₄ alkenyl, 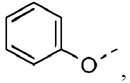 -S(=O)₂NH₂, -S(=O)₂NH-C₁₋₃ alkyl, -NHS(=O)₂-C₁₋₃ alkyl, -N[S(=O)₂-C₁₋₃ alkyl]₂, -N[C(=O)-C₁₋₃ alkyl]₂, -NHC(=O)-C₁₋₃ alkyl, -C(=O)NH₂ and -C(=O)NH-C₁₋₃ alkyl are optionally substituted with 1, 2 or 3 Rₐ; preferably, each R₁ is independently selected from the group consisting of H, F, Cl, Br, I, OH, NH₂, CN, C₁₋₃ alkyl, C₃₋₅ cycloalkyl, C₁₋₃ alkoxy, C₁₋₃ alkylamino, C₂₋₄ alkenyl, 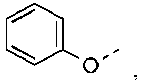 -S(=O)₂NH₂, -S(=O)₂NH-C₁₋₃ alkyl, -NHS(=O)₂-C₁₋₃ alkyl, -N[S(=O)₂-C₁₋₃ alkyl]₂, -N[C(=O)-C₁₋₃ alkyl]₂, -NHC(=O)-C₁₋₃ alkyl, -C(=O)NH₂ and -C(=O)NH-C₁₋₃ alkyl, wherein the C₁₋₃ alkyl, C₃₋₅ cycloalkyl, C₁₋₃ alkoxy, C₁₋₃ alkylamino, C₂₋₄ alkenyl, 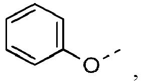 -S(=O)₂NH₂, -S(=O)₂NH-C₁₋₃ alkyl, -NHS(=O)₂-C₁₋₃ alkyl, -N[S(=O)₂-C₁₋₃ alkyl]₂, -N[C(=O)-C₁₋₃ alkyl]₂, -NHC(=O)-C₁₋₃ alkyl, -C(=O)NH₂ and -C(=O)NH-C₁₋₃ alkyl are optionally substituted with 1, 2 or 3 Rₐ.

9. The compound according to claim 1 or 2, wherein each R₁ is independently selected from the group consisting of H, F, Cl, Br, I, OH, NH₂, CN, C₁₋₃ alkyl, C₁₋₃ alkoxy and -C(=O)NH₂, wherein the C₁₋₃ alkyl, C₁₋₃ alkoxy and -C(=O)NH₂ are optionally substituted with 1, 2 or 3 Rₐ; preferably, each R₁ is independently selected from the group consisting of H, F, Cl, Br, I, OH, NH₂, CN, Me, 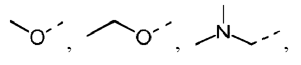 , 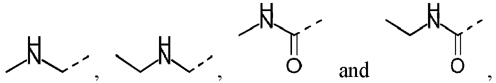 wherein the Me, 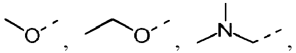 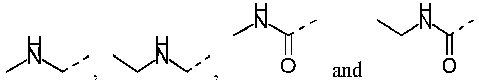 are optionally substituted with 1, 2 or 3 Rₐ; more preferably, each R₁ is independently selected from the group consisting of H, Me, OH, 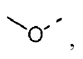 ,

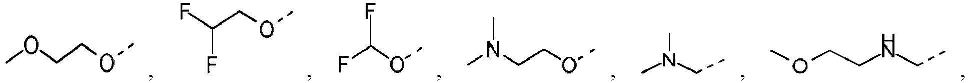

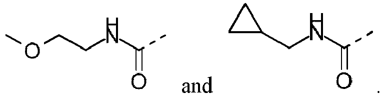

10. The compound according to claim 1 or 2, wherein each R₁ is independently selected from the group consisting of F, Cl, Br, I, OH, NH₂ and C₁₋₃ alkyl, wherein the C₁₋₃ alkyl is optionally substituted with 1, 2 or 3 Rₐ; preferably, each R₁ is independently selected from the group consisting of F, Cl, Br, I, OH, NH₂ and Me, wherein the Me is optionally substituted with 1, 2 or 3 Rₐ; more preferably, each R₁ is independently selected from Me.

11. The compound according to claim 1 or 2, wherein each R₁ is independently selected from the group consisting of H, F, Cl, Br, I, OH, NH₂, CN, Me, 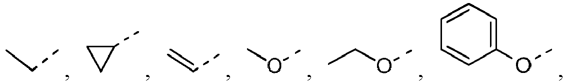 -S(=O)₂NH₂, 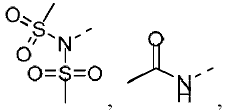 -NHCH₃ and -C(=O)NH₂, wherein the Me, 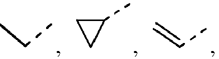 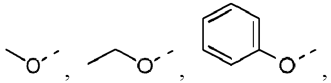 -S(=O)₂NH₂, 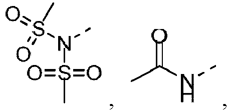 -NHCH₃ and-C(=O)NH₂ are optionally substituted with 1, 2 or 3 Rₐ; preferably, each R₁ is independently selected from the group consisting of H, F, Cl, Br, I, OH, NH₂, Me, 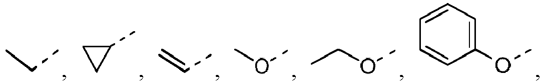 -S(=O)₂NH₂, 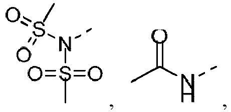 -NHCH₃ and -C(=O)NH₂, wherein the Me, 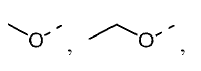 , 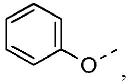 -S(=O)₂NH₂, -NHCH₃ and -C(=O)NH₂ are optionally substituted with 1, 2 or 3 Rₐ; more preferably, each R₁ is independently selected from the group consisting of H, F, Cl, Br, I, Me, OH, NH₂,

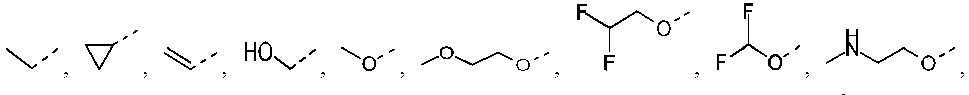

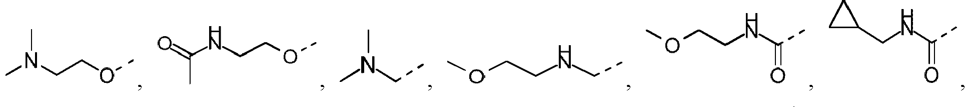

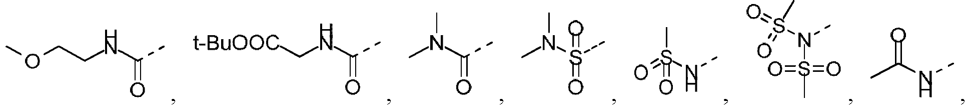

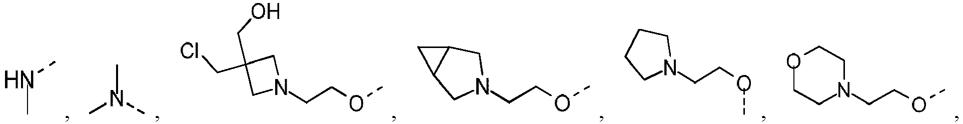

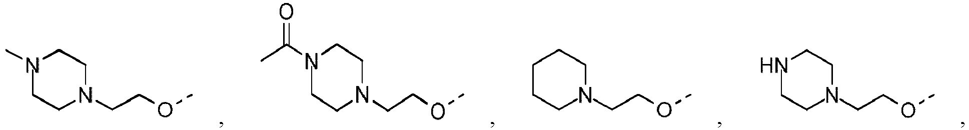

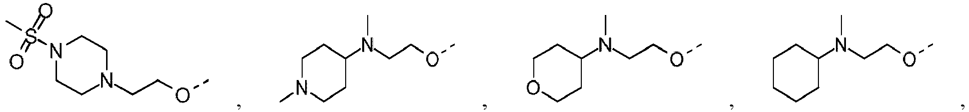

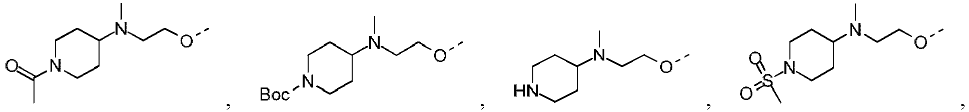

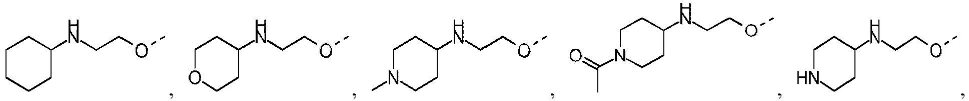

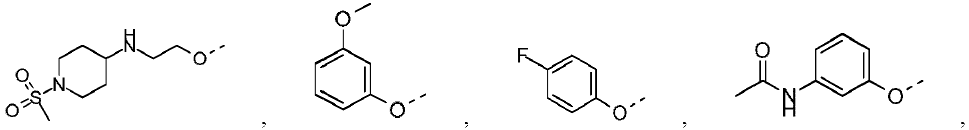

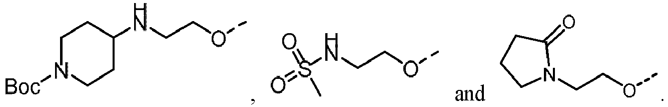

12. The compound according to claim 1 or 2, wherein ring A is selected from the group consisting of 5-6 membered heteroaryl, phenyl, 4-7 membered heterocycloalkyl and 4-7 membered heterocycloalkenyl; preferably, ring A is selected from the group consisting of 5-6 membered heteroaryl, phenyl and 4-7 membered heterocycloalkenyl; more preferably, ring A is selected from the group consisting of phenyl, 1,3-cyclohexadienyl, 1,3-dioxolanyl, 1,3-dioxolyl, morpholinyl, oxazolyl, cyclobutyl, oxapanyl, thiazolyl, tetrahydrothiazolyl, furanyl, 2,3-dihydrofuranyl, 1,4-oxazepanyl, pyridinyl, 2,3-dihydropyridinyl, pyrazolyl, 4,5-dihydro-1*H*-pyrazolyl, oxazolyl, 4,5-dihydrooxazolyl, pyrrolyl and 2,3-dihydro-1*H*-pyrrolyl; even more preferably, ring A is selected from the group consisting of phenyl, 1,3-dioxolanyl, morpholinyl, oxazolyl, cyclobutyl, oxapanyl, thiazolyl, tetrahydrothiazolyl, furanyl, tetrahydrofuranyl and 1,4-oxazepanyl; still more preferably, ring A is selected from the group consisting of phenyl, 1,3-dioxolanyl, morpholinyl, oxazolyl, cyclobutyl, oxapanyl and 1,4-oxazepanyl.

13. The compound according to claim 1 or 2, wherein ring A is selected from the group consisting of phenyl, 1,3-dioxolanyl, morpholinyl, oxazolyl, cyclobutyl, oxapanyl, thiazolyl, tetrahydrothiazolyl, furanyl, 1,4-oxazepanyl, pyridinyl and pyrrolyl; preferably, ring A is selected from the group consisting of phenyl, 1,3-dioxolanyl, furanyl, pyridinyl and pyrrolyl; more preferably, ring A is selected from the group consisting of phenyl, 1,3-dioxolanyl and furanyl.

14. The compound according to claim 1 or 2, wherein the structural unit 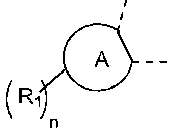 is selected from the group consisting of

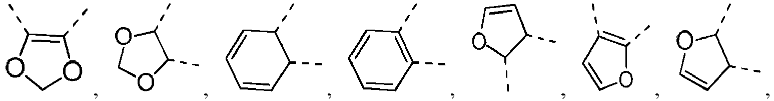

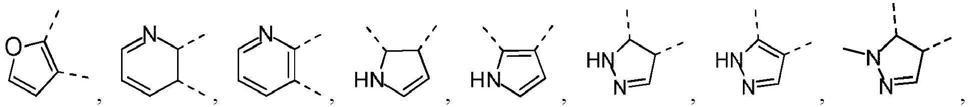

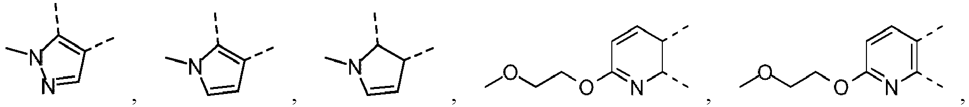

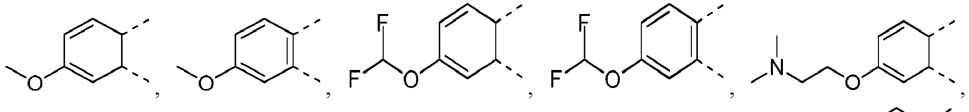

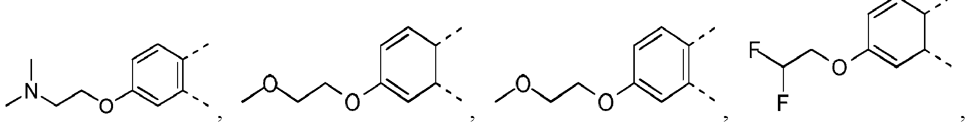

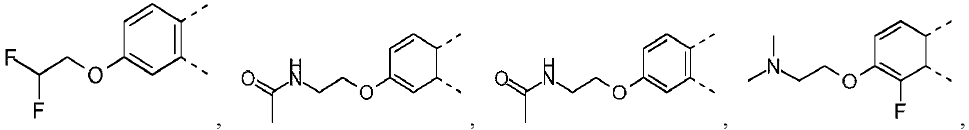

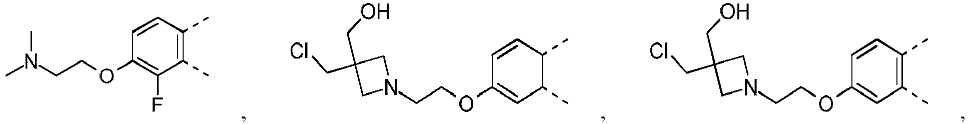

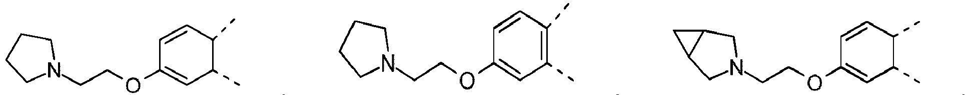

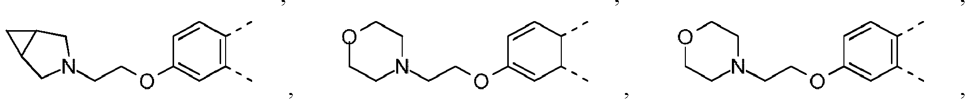

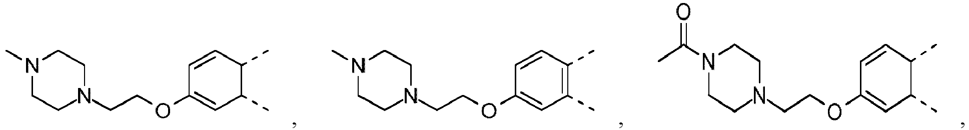

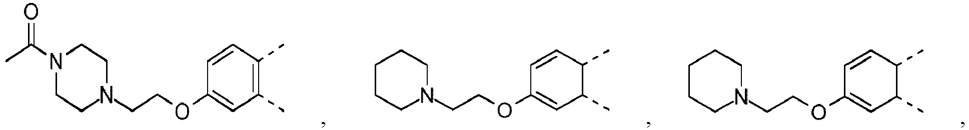

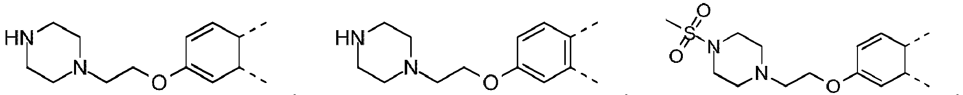

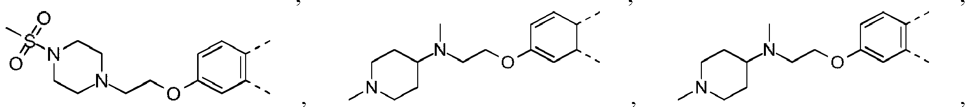

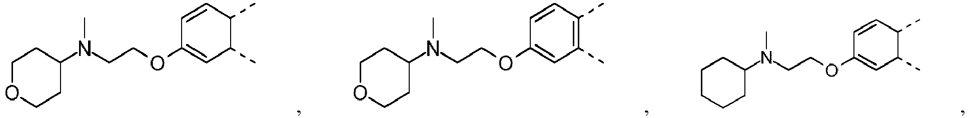

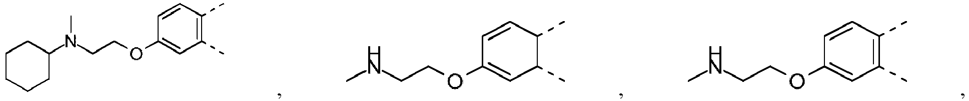

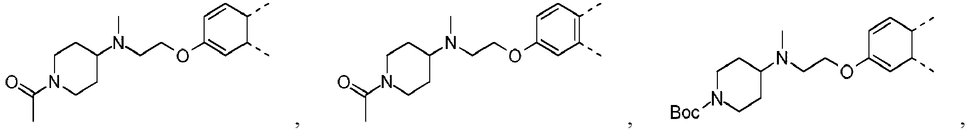

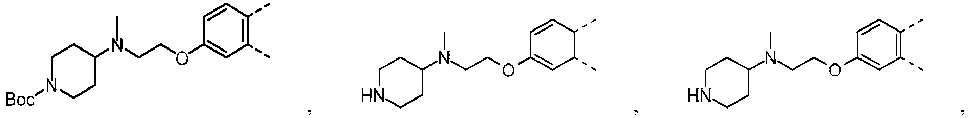

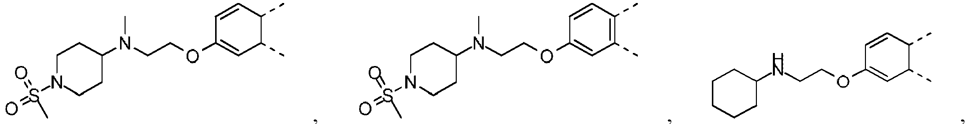

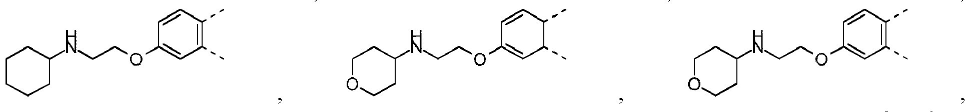

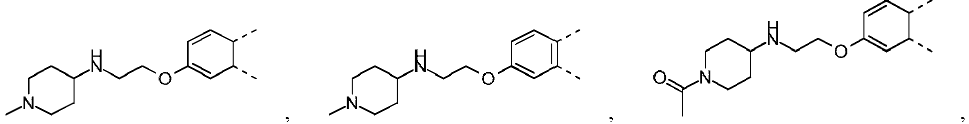

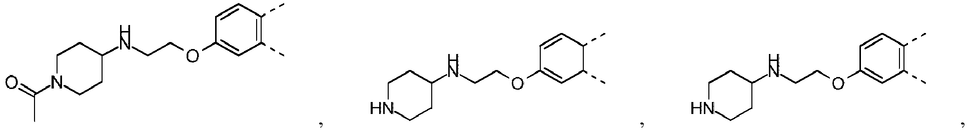

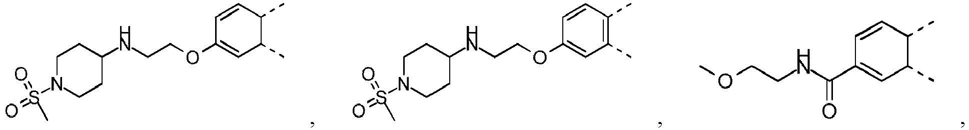

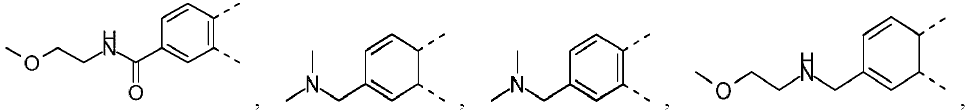

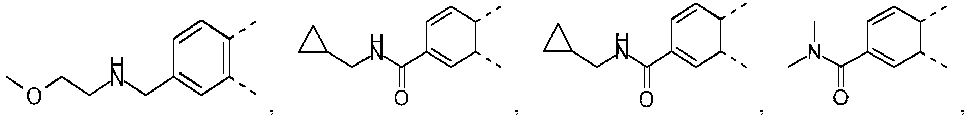

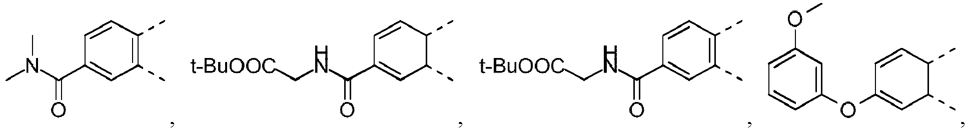

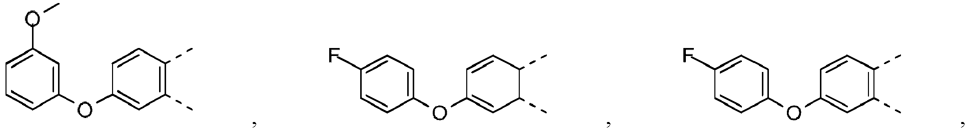

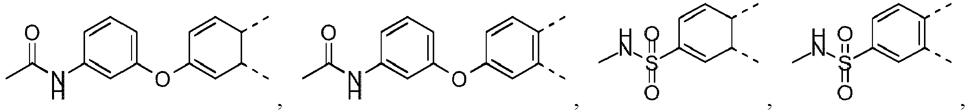

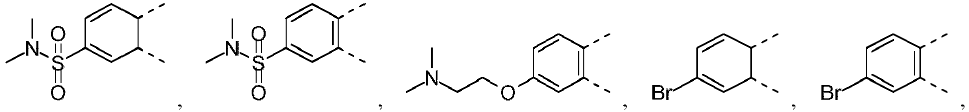

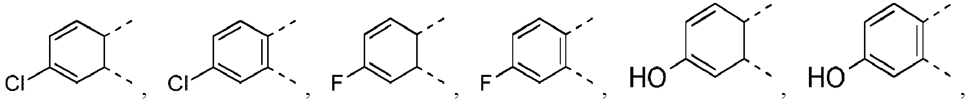

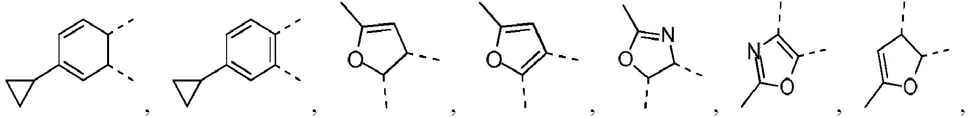

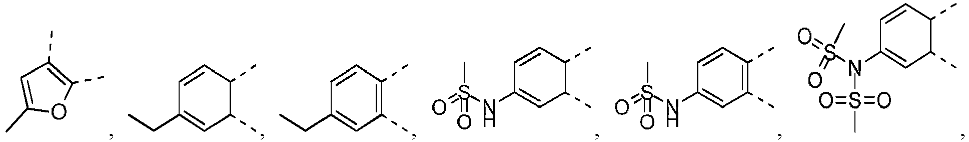

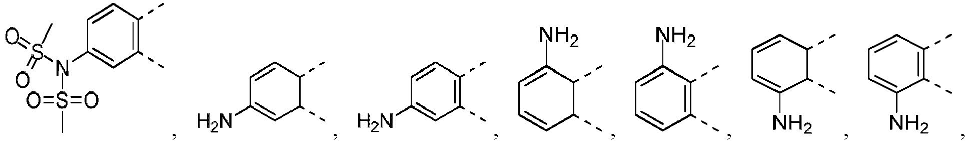

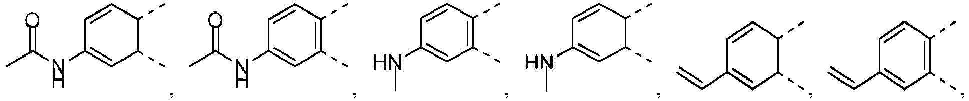

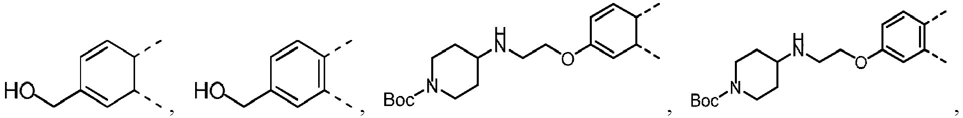

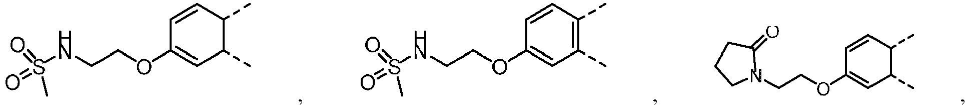

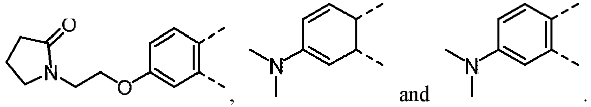

15. The compound according to claim 1 or 2, wherein the structural unit 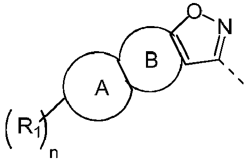 is selected from the group consisting of

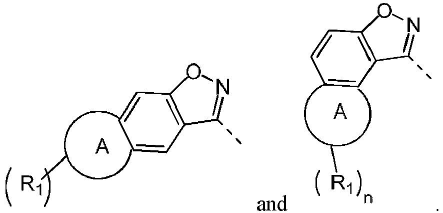

16. The compound according to claim 1 or 2, wherein the structural unit 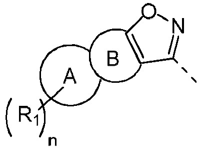 is selected from the group consisting of

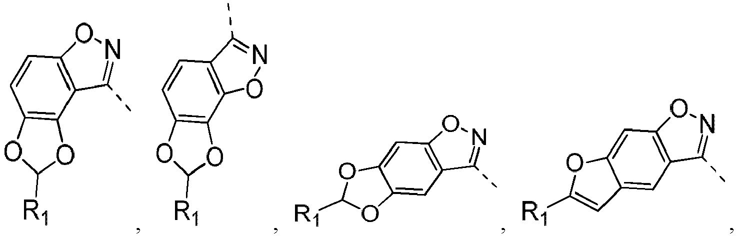

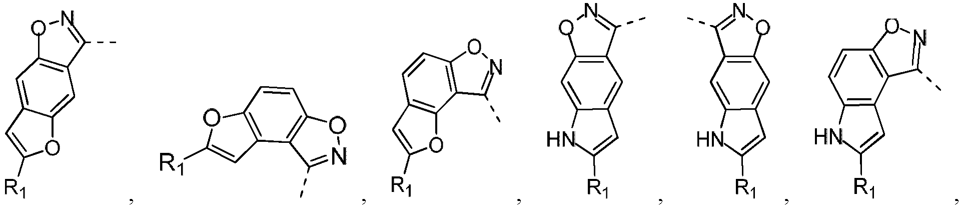

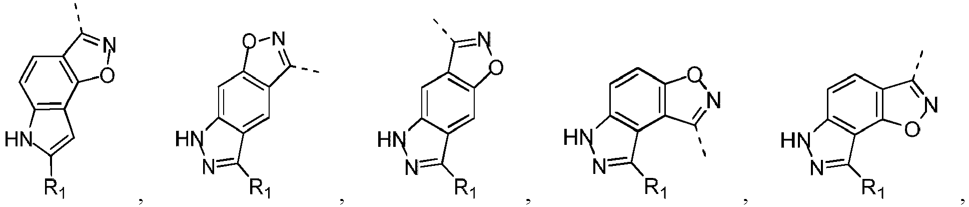

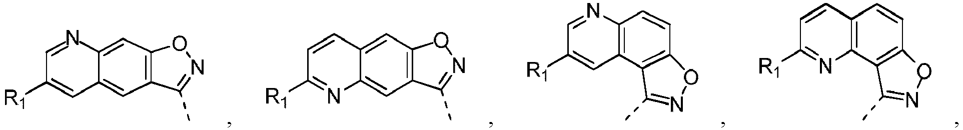

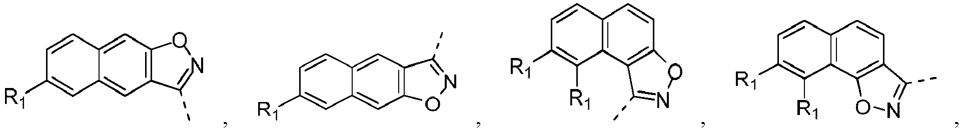

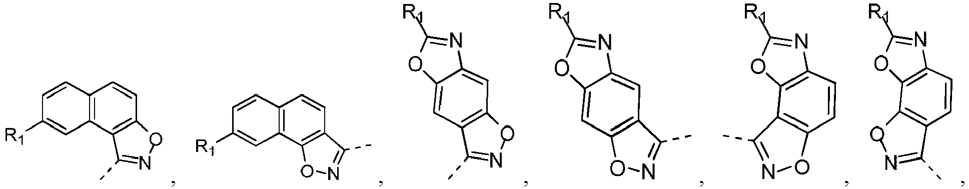

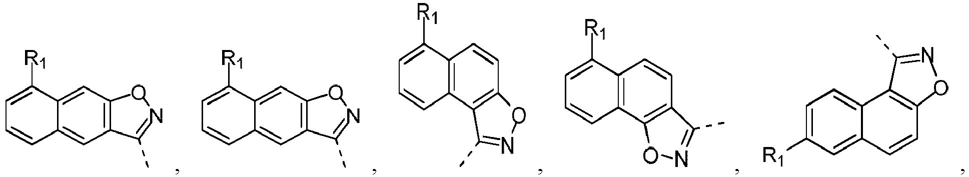

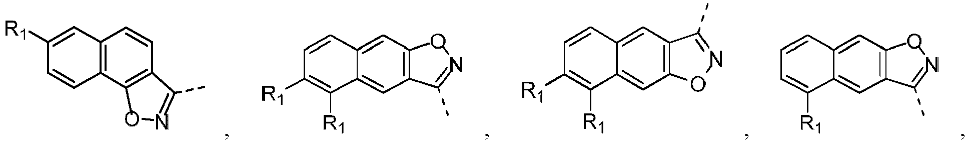

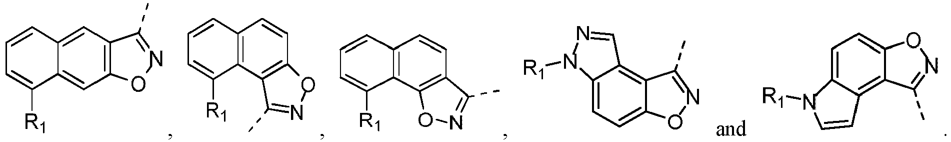

17. The compound according to claim 1 or 2, selected from the group consisting of 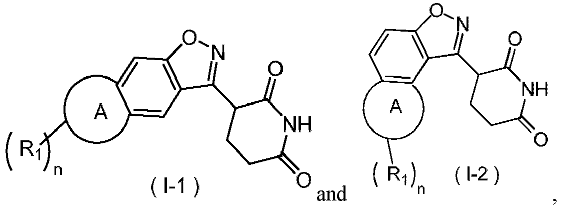 wherein n, ring A and R₁ are defined as in claim 1.

18. A compound of a formula below and a pharmaceutically acceptable salt thereof, selected from the group consisting of:

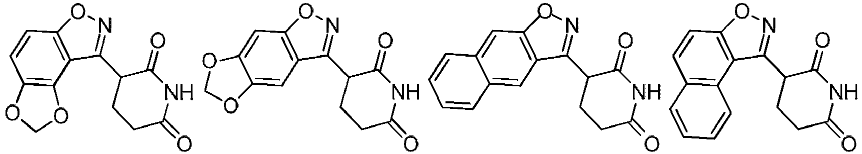

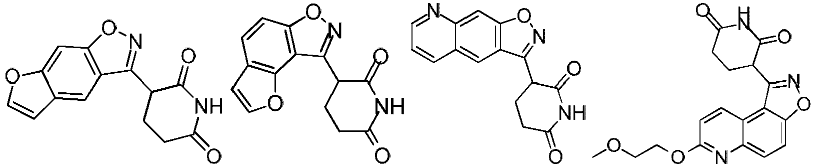

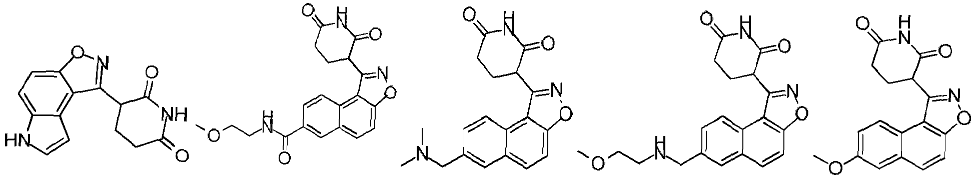

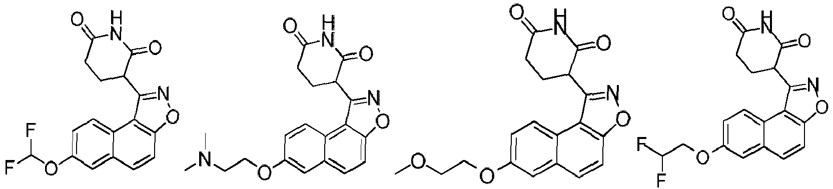

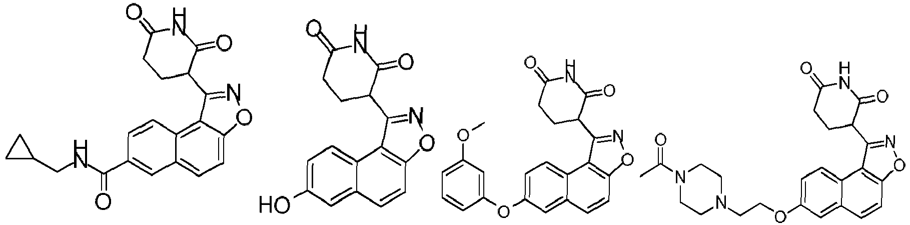

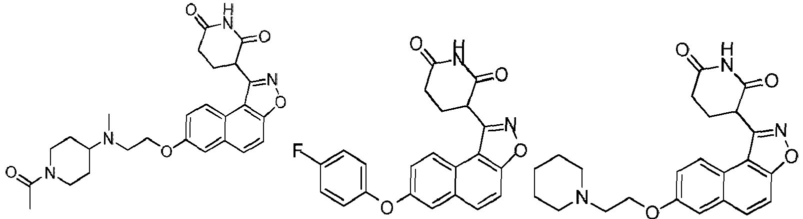

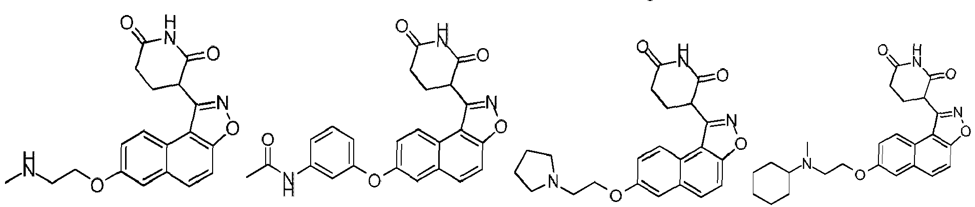

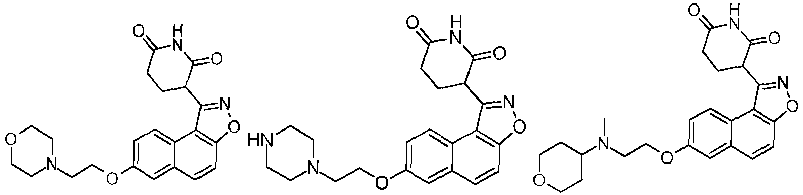

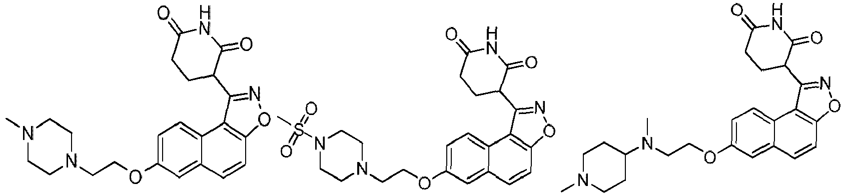

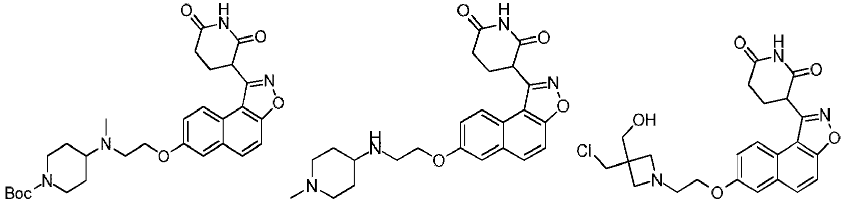

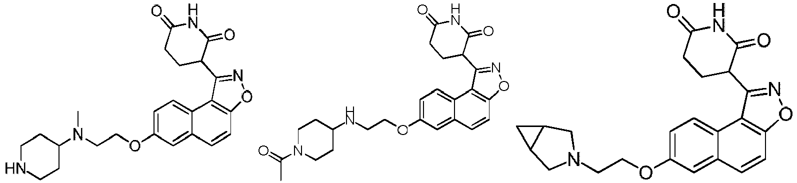

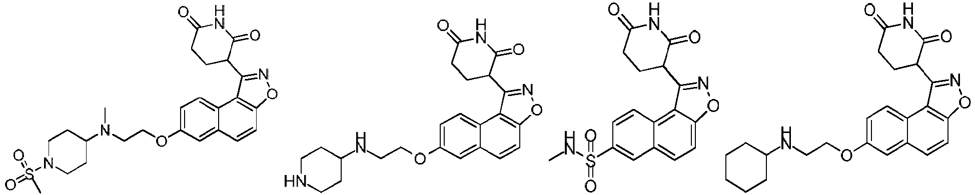

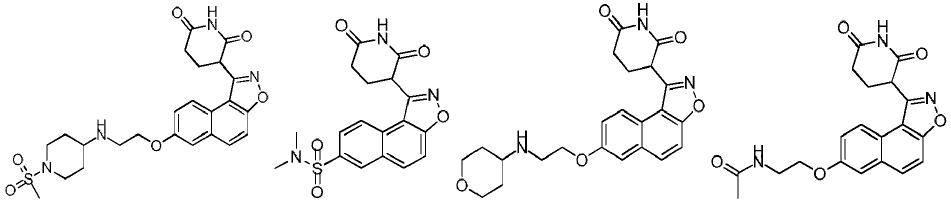

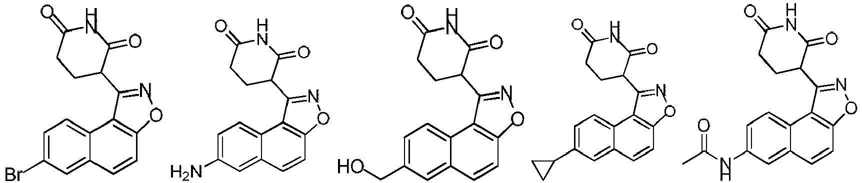

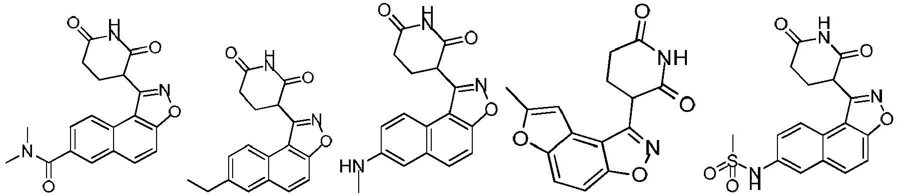

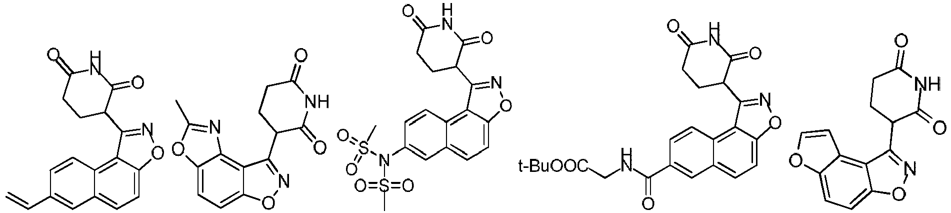

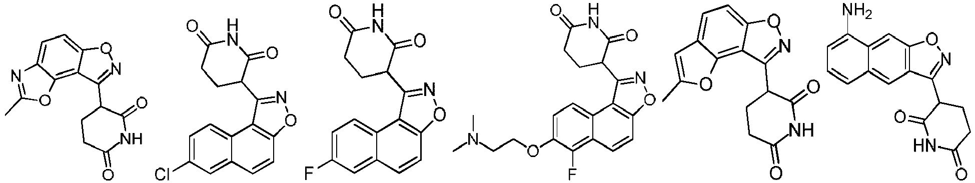

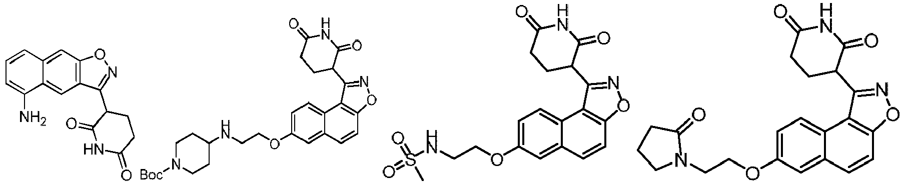

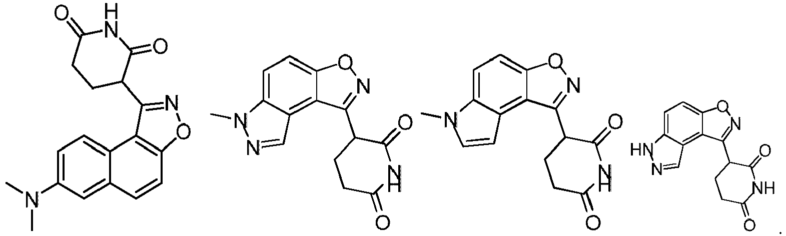

19. A pharmaceutical composition, comprising a therapeutically effective amount of the compound or the pharmaceutically acceptable salt thereof according to any one of claims 1-18 as an active ingredient, and a pharmaceutically acceptable carrier.

20. Use of the compound or the pharmaceutically acceptable salt thereof according to any one of claims 1-18 and the pharmaceutical composition according to claim 19 in preparing a medicament for treating a disease related to CRBN protein.

21. The compound or the pharmaceutically acceptable salt thereof according to any one of claims 1-18 and the pharmaceutical composition according to claim 19 for treating a disease related to CRBN protein.

22. A method for treating a disease related to CRBN protein, comprising administering a therapeutically effective amount of the compound or the pharmaceutically acceptable salt thereof according to any one of claims 1-18 and the pharmaceutical composition according to claim 19.

23. The disease related to CRBN protein according to claim 20 or 21 is multiple myeloma.
